# EUROPEAN PATENT APPLICATION

(11) **EP 2 929 883 A1**
(43) Date of publication of application: **14.10.2015**
(21) Application number: 14305516.8
(22) Date of filing: 08.04.2014
(51) Int. Cl.: A61K 31/4439, A61K 31/501, A61K 31/506, C07D 401/04, C07D 403/04, A61P 31/12

(54) **Pyrazole derivatives as dihydroorotate dehydrogenase (DHODH) inhibitors**

(71) Applicant: INSTITUT PASTEUR, 75015 Paris (FR); Centre National de la Recherche Scientifique, 75016 Paris (FR); Institut National De La Sante Et De La Recherche Medicale, 75013 Paris (FR)
(72) Inventor: Janin, Yves Louis, 75005 PARIS (FR); Guillou, Sandrine, 78700 CONFLANS SAINTE HONORINE (FR); Lucas-Hourani, Marianne, 92130 Issy-les-Moulineaux (FR); Munier-Lehmann, Hélène, 92190 MEUDON (FR); Noel, Anne, 94450 LIMEIL BREVANNES (FR); Salanouve, Elise, 31320 VIEILLE TOULOUSE (FR); Tangy, Frédéric, 93260 LES LILAS (FR); Vidalain, Pierre-Olivier, 92800 PUTEAUX (FR)
(74) Representative: Dias, Sonia

(57) **Abstract**

The present invention relates to compounds of formula (I) for their use in the treatment and/or prevention of auto-immune or auto-immune related diseases, cancer, viral infections, and central nervous system diseases and disorders, by inhibiting human dehydroorate dehydrogenase (DHODH): 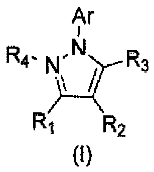 Wherein R₁, R₂, R₃, R₄ and Ar are as defined in claim 1.

## Description

The present invention relates to compounds of formula (I), pharmaceutical compositions thereof as well as to their therapeutical use as inhibitors as dihydroorotate dehydrogenase (DHODH), in the treatment of and/or prevention of auto-immune or autoimmune related diseases, cancer, viral infections, and central nervous system diseases and disorders.

The nucleosides availability is a crucial factor governing the proliferation of any living entities (viruses, bacteria, parasites or eukaryotic cells). In multicellular organisms, nucleosides are available from three sources: recycling, absorption from microenvironment and *de novo* synthesis. Inhibition of this latter mechanism is relatively well tolerated by higher organisms since it is compensated by the two other sources. Nevertheless, in human this inhibition results in immunosuppression since *de novo* nucleotide synthesis is essential for the proliferation lymphocytes and induction of an adaptive immune response. In other organisms lacking nucleosides salvage/recovery pathways (i.e. *H. pylori* or *P. falciparum)* the inhibition of the de novo pathway is currently considered to be a valid therapeutic target for anti infectious treatment. Dihydroorotate dehydrogenase (DHODH), the fourth and rate-limiting enzyme in the *de novo* pyrimidine nucleosides biosynthetic pathways is amongst the targets for inhibitors of this pathway. A very recent review (Munier et al. J. Med. Chem. 2013, 56,3148) on dihydroorotate dehydrogenases, their inhibitors and uses contains all the relevant references to the state of the art; part of which is described in the following. Many series of human DHODH inhibitors such as the compounds depicted below have been disclosed for their potential as anti-inflammatory, immunosuppressant or anticancer agents as well as against viral or parasitic infection. Inhibitors of the human DHODH such as leflunomide (**1**) or teriflunomide **(2)** are respectively used against rheumatoid arthritis or psoriatic arthritis and multiple sclerosis. More recent series of inhibitors of DHODH, such as ABR-222417 (**3**) and ABR-224050 (**4**), are currently investigated for their potential in alleviating graft rejection.

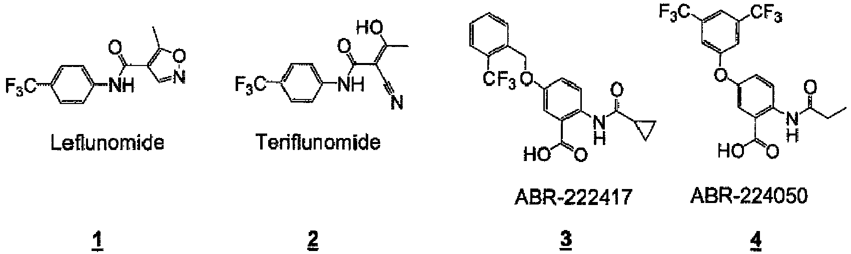

Vidofludimus (**5**), another inhibitor of human DHODH, is the subject of studies for the treatment of systemic lupus erythematosus as well as inflammatory bowel disease. Series such as the aminopyridine (**6**) are also studied for their anti-inflammatory effect. Other series of inhibitors of *P*. *falciparum* DHODH such as DSM 265 (**7**) or Genz-669178 (**8**) are undergoing preclinical studies for their potential in the treatment of malaria. Moreover, a recent series of inhibitor of fungal DHODH such as the quinazolinone (**9**) have been disclosed for their antifungal properties.

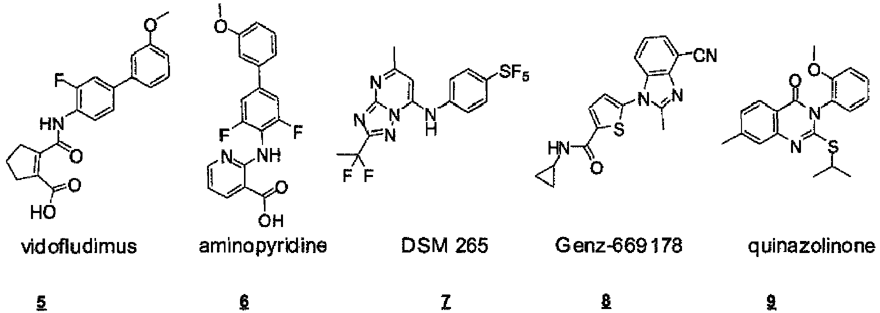

Pyrazole derivatives, more particularly pyrazole-isoxazoles (**10**) (Bonavia et al. Proc. Natl. Acad. Sci. U.S.A. 2011, 108, 6739) or carboxylic acids derivatives (**11**) (US 2011263620) have been reported or disclosed for their inhibition of human DHODH. Moreover, dicarboxypyrazoles (Copeland et al. J. Biol. Chem. 2000, 275, 33373) such as the compound (**12**) represented below have been reported as inhibitors of *H. pylori* DHODH.

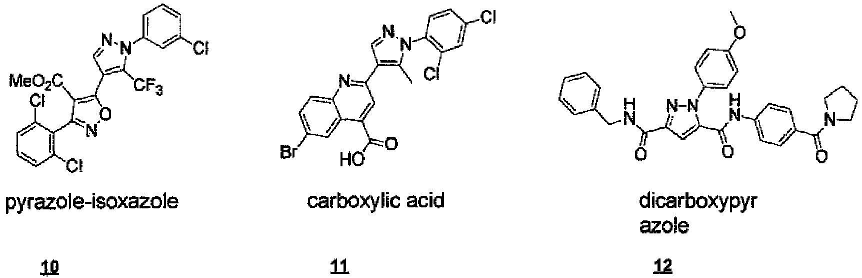

It now has been discovered a novel class of DHODH inhibitors of formula (I). Advantageously, the compounds of formula (I) are capable of strongly inhibiting DHODH. By using a cellular antiviral assay (WO 2012014181, J. Visualized Exp. 2014, in print and PLOS Pathogens 2013, 9, el003678) which is, for this type of inhibitors, reflecting their inhibition of DHODH, MIC₅₀ between 0.5 nM and 50 nM were observed. These values are, for the best examples, more than 1000 times better than the antiviral effect observed in the same assay for the approved drug Teriflunomide (2) (MIC₅₀ = 50 µM) which is a known inhibitor of human DHODH with an IC₅₀ reported to be of 1.1 µM on the isolated protein (Eur. J. Biochem. 1996, 240, 292 and Biochem. Pharmacol. 1998, 56,1259).

The present invention in one aspect is thus directed to various compounds of formula (I): 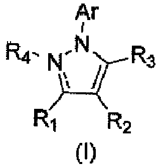 and pharmaceutically acceptable salt forms thereof, wherein the constituent members are defined *infra,* for use in the treatment and/or prevention of auto-immune or auto-immune related diseases, cancer, viral infections, and/or central nervous system diseases and disorders by inhibiting human dehydroorate dehydrogenase (DHODH).

Alternatively, the present invention is directed to compounds of formula (Ia), (Ib), (Ic), (Id), or (Ie) for use in the treatment and/or prevention of auto-immune or auto-immune related diseases, cancer, viral infections, and/or central nervous system diseases and disorders.

Another object of the present invention is to provide compounds of formula (I), notably (Ia), (Ib), (Ic), (Id), or (Ie) and pharmaceutically acceptable salts thereof.

Another object of the present invention is to provide pharmaceutical compositions comprising the compounds of the present invention wherein the compositions comprise one or more pharmaceutically acceptable excipients and a therapeutically effective amount of at least one of the compounds of the present invention, or a pharmaceutically acceptable salt form thereof.

These and other objects, features and advantages of compounds of formula (I) will be disclosed in the following detailed description of the patent disclosure.

### Compounds of formula (I) for therapeutical use

Thus, in a first object, the present invention provides a compound of formula (I): 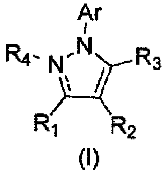 wherein:
Ar is independently selected from C₆-C₁₀ aryl or 5 to 7 membered heteroaryl, wherein said aryl or heteroaryl groups are optionally substituted by one to three R₅;
R₁ is independently selected from C₁-C₆ alkyl, C₃-C₆ cycloalkyl, C₆-C₁₀ aryl, OH, C₁-C₆ alkoxy, =O, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from H, F, Cl, Br, I, C₁-C₆ alkyl, C₆-C₁₀ aryl, C₆-C₁₀ aryloxy, (C₆-C₁₀)aryl(C₁-C₆)alkyl, 5 to 7 membered heteroaryloxy, CN, CO₂R₉, C₆-C₁₀ aroyl, C₆-C₁₀ arylthio, C₆-C₁₀ arylsulfinyl, C₆-C₁₀ arylsulfonyl, wherein said aryl groups are optionally substituted by one to three R₁₀ and wherein said alkyl groups are optionally substituted by OH;
R₃ is independently selected from H, Cl, C₁-C₆ alkyl, C₁-C₆ alkoxy, (C₁-C₆)alkoxy(C₁-C₆)alkyl, C₆-C₁₀ aryl, C₆-C₁₀ aroyl, (C₆-C₁₀)aryl(C₁-C₆)alkyl, 5 to 7 membered heterocyclyl, (5 to 7 membered heterocyclyl)-CH₂-, (CH₂)ₙCO₂R₁₁, CH₂NR₇R₈, wherein said aryl or heterocyclyl groups are optionally substituted by one to three R₁₂;
or
R₂ and R₃ together form a 5 to 7 membered heterocyclyl, optionally substituted by (C₆-C₁₀)aryl(C₁-C₆)alkyl ;
R₄ is independently selected from H, or 5 to 7 membered heteroaryl optionally substituted by R₁₃;
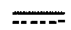 is, at each occurrence, independently selected from a double bond C=N or a single bond C-N,
provided that when 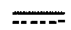 is a double bond, R₄ is absent and R₁ cannot be =O;
R₅ is at each occurrence, independently selected from C₁-C₆ alkyl, C₁-C₆ alkenyl, C₃-C₆ cycloalkyl, C₁-C₆ alkoxy, (C₆-C₁₀)aryl(C₁-C₆)alkyl, (C₆-C₁₀)aryl(C₁-C₆)alkoxy, OH, F, Cl, Br, I, NR₇R₈, CF₃, CF₂CH₃, CN, C(=O)R₆, CO₂R₆, wherein said alkyl groups are optionally substituted by one or more OH, F, Cl, or Br;
R₆, R₇, R₈, are, at each occurrence, independently selected from each independently selected from C₁-C₆ alkyl;
R₉ is, at each occurrence, independently selected from H or C₁-C₆ alkyl;
R₁₀ is at each occurrence, independently selected from C₁-C₆ alkoxy, OH, F, Cl, Br, CF₃;
R₁₁ is, at each occurrence, independently selected from H, C₁-C₆ alkyl;
R₁₂ is, at each occurrence, independently selected from F, Cl, Br, CN, C₁-C₆ alkyl or C₁-C₆ alkoxy;
R₁₃ is, at each occurrence, independently selected from F, Cl, Br, I;
n is, at each occurrence, independently selected from 0 or 1;
and pharmaceutically acceptable salts thereof,
for use in the treatment and/or prevention of auto-immune or auto-immune related diseases, cancer, viral infections, and central nervous system diseases and disorders, by inhibiting human dehydroorate dehydrogenase (DHODH).

According to the present invention, a compound inhibiting DHODH is notably a compound which exhibits a IC₅₀ value as regards DHODH inferior to 200 µM, preferably inferior to 50 µM, more preferably inferior to 5 µM, most preferably inferior to 500 nM, the IC₅₀ value being determined as indicated in the following examples, notably using the assay described in the following references : Arch. Biochem. Biophys. 1971, 146, 256-270; Methods Enzymol. 1978, 51, 63-69; and Methods Enzymol. 1978, 51, 58-63 and used in Chem. Biol. Interact. 2000, 124, 61-76. Biochem. 1996, 240, 292-301 and Biochem. Pharmacol. 1998, 56, 1259-1264.

A particular aspect of the present invention includes compounds of formula (I) for use in the treatment of rheumatoid arthritis, multiple sclerosis, melanoma, spinal cord injury, psoriasis, inflammatory bowel disease or prevention of graft rejection.

Another aspect of the present invention includes compounds of formula (I) wherein 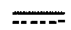 is a double bond C=N.

A further aspect of the present invention includes compounds of formula (I) wherein at least one of R₂ or R₃ is different from H.

An additional aspect of the present invention includes compounds of formula (I) wherein Ar is phenyl, pyridyl, pyrazine, pyrimidinyl, pyridazine, triazinyl, preferably 2-pyridyl, 3-pyridyl, 2-pyrazinyl, 2- pyrimidinyl, 5-pyrimidinyl, 3-pyridazinyl or 3-(1,2,4-triazinyl).

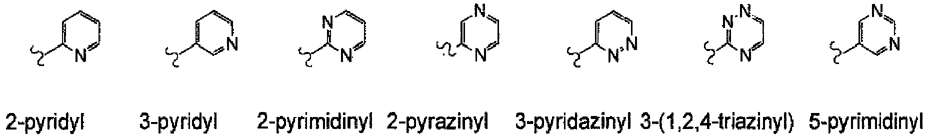

A particular aspect of the present invention includes compounds of formula (I) wherein Ar is substituted, preferably monosubstituted.

Another aspect of the present invention includes compounds of formula (I) wherein R₅ is C₁-C₆ alkyl, C₃-C₆ cycloalkyl, C₁-C₆ alkoxy F, Cl, Br, CF₂CH₃, CF₃, preferably methyl, ethyl, cyclopropyl, methoxy, F, Cl, Br, CF₂CH₃, CF₃, more preferably ethyl, cyclopropyl or methoxy.

An additional aspect of the present invention includes compounds of formula (I) wherein, Ar is phenyl or azine and at least one of R₅ is located at *para* position with regard to the pyrazole ring.

A particular aspect of the present invention includes compounds of formula (I) wherein R₁ is C₁-C₆ alkyl, C₃-C₆ cycloalkyl, C₁-C₆ alkoxy, preferably C₁-C₆ alkoxy, preferably methoxy, ethoxy or isopropyloxy, more preferably ethoxy or isopropyloxy.

In certain aspects of the present invention, there are included compounds of formula (I) wherein R₂ is C₆-C₁₀ aryloxy, (C₆-C₁₀)aryl(C₁-C₆)alkyl, preferably benzyl, PhCH(OH)-, phenoxy.

Other aspects of the present invention include compounds of formula (I) wherein R₁₀ is F, Cl, OH, methoxy, CF₃, preferably F.

Further aspects of the present invention include compounds of formula (I) wherein R₃ is H, C₁-C₆ alkyl, C₆-C₁₀ aryl, (C₆-C₁₀)aryl(C₁-C₆)alkyl, Cl, preferably methyl, benzyl or phenyl, more preferably H or methyl.

Further aspects of the present invention include compounds of formula (I) wherein R₁₂ is Cl or C₁-C₆ alkoxy, preferably Cl or methoxy.

Additional aspects of the present invention include compounds of formula (I) which are selected from:
2-((4-(2,6-difluorophenoxy)-3-isopropoxy-5-methyl-1H-pyrazol-1-yl)-5-ethylpyrimidine (example 8)
5-((4-(2,6-difluorophenoxy)-3-isopropoxy-5-methyl-1H-pyrazol-1-yl)-2-ethylpyridine (example 129)
5-cyclopropyl-2-(4-(2,6-difluorophenoxy)-3-isopropoxy-5-methyl-1H-pyrazol-1-yl)pyrimidine (example 13)
5-((4-(2,6-difluorophenoxy)-3-isopropoxy-5-methyl-1H-pyrazol-1-yl)-2-ethylpyridine (example 129)
(1-((5-cyclopropylpyrimidin-2-yl)-3-isopropoxy-5-methyl-1H-pyrazol-4-yl)(phenyl)methanol (example 176)
2-((4-benzyl-5-methyl-3-(pentan-3-yloxy)-1H-pyrazol-1-yl)-5-cyclopropylpyrimidine (example 141)
2-((4-benzyl-3-sec-butoxy-5-methyl-1H-pyrazol-1-yl)-5-cyclopropylpyrimidine (example 142)
3-cyclopropyl-6-(4-(2,6-difluorophenoxy)-3-isopropoxy-5-methyl-1H-pyrazol-1-yl)pyridazine (example 7)
2-cyclopropyl-5-(4-(2,5-difluorophenoxy)-3-isopropoxy-5-methyl-1H-pyrazol-1-yl)pyrazine (example 45)
5-cyclopropyl-2-(4-(2,6-difluorophenoxy)-3-isopropoxy-5-methyl-1H-pyrazol-1-yl)-3-fluoropyridine (example 5)
2-((4-(2,6-difluorophenoxy)-3-isopropoxy-5-methyl-1H-pyrazol-1-yl)-5-propylpyrimidine (example 6)
2-((4-benzyl-3-isopropoxy-5-methyl-1H-pyrazol-1-yl)-5-ethylpyrimidine (example 139)
5-cyclopropyl-2-(4-(2-fluoro-6-hydroxyphenoxy)-3-isopropoxy-5-methyl-1H-pyrazol-1-yl)pyridin-3-ol (example 188)

### Compounds of formulae (Ia), (Ib), (Ic), (Id), or (Ie)

In another object, the present invention relates to a compound of formula (Ia):

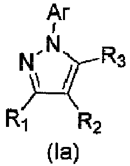

Wherein:
R₁ is independently selected from OH, C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from C₆-C₁₀ aryloxy, 5 to 7 membered heteroaryloxy, wherein said aryl groups are optionally substituted by one to three R₁₀ and wherein said alkyl groups are optionally substituted by OH;
R₃ is independently selected from C₁-C₆ alkyl, (C₁-C₆)alkoxy(C₁-C₆)alkyl, (C₆-C₁₀)aryl(C₁-C₆)alkyl, (5 to 7 membered heterocyclyl)-CH₂-, CH₂CO₂R₁₁, CH₂NR₇R₈, wherein said aryl or heterocyclyl groups are optionally substituted by one to three R₁₂,
Ar, R₇, R₈, R₁₀, R₁₁, R₁₂ being as defined in formula (I) hereabove,
and pharmaceutically acceptable salts thereof.

Further aspects of the present invention include compounds of formula (Ia) wherein R₃ is C₁-C₆ alkyl, preferably CH₃.

Compounds of formula (Ia) are notably selected from:
2-((4-(2,6-difluorophenoxy)-3-isopropoxy-5-methyl-1H-pyrazol-1-yl)-5-ethylpyrimidine
5-((4-(2,6-difluorophenoxy)-3-isopropoxy-5-methyl-1H-pyrazol-1-yl)-2-ethylpyridine
5-cyclopropyl-2-(4-(2,6-difluorophenoxy)-3-isopropoxy-5-methyl-1H-pyrazol-1-yl)pyrimidine
5-((4-(2,6-difluorophenoxy)-3-isopropoxy-5-methyl-1H-pyrazol-1-yl)-2-ethylpyridine
(1-((5-cyclopropylpyrimidin-2-yl)-3-isopropoxy-5-methyl-1H-pyrazol-4-yl)(phenyl)methanol
2-((4-benzyl-5-methyl-3-(pentan-3-yloxy)-1H-pyrazol-1-yl)-5-cyclopropylpyrimidine
2-((4-benzyl-3-sec-butoxy-5-methyl-1H-pyrazol-1-yl)-5-cyclopropylpyrimidine
3-cyclopropyl-6-(4-(2,6-difluoropbenoxy)-3-isopropoxy-5-methyl-1H-pyrazol-1-yl)pyridazine
2-cyclopropyl-5-(4-(2,6-difluorophenoxy)-3-isopropoxy-5-methyl-1 H-pyrazol-1-yl)pyrazine
5-cyclopropyl-2-(4-(2,6-difluorophenoxy)-3-isopropoxy-5-methyl-1H-pyrazol-1-yl)-3-fluoropyridine
2-((4-(2,6-difluorophenoxy)-3-isopropoxy-5-methyl-1H-pyrazol-1-yl)-5-propylpyrimidine
2-((4-benzyl-3-isopropoxy-5-methyl-1H-pyrazol-1-yl)-5-ethylpyrimidine
5-cyclopropyl-2-(4-(2-fluoro-6-hydroxyphenoxy)-3-isopropoxy-5-methyl-1H-pyrazol-1-yl)pyridin-3-ol.

In a further object, the present invention provides compounds of formula (lb):

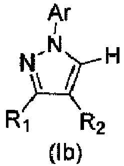

Wherein:
R₁ is independently selected from C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from (C₆-C₁₀)aryl(C₁-C₆)alkyl, C₆-C₁₀ aryloxy, 5 to 7 membered heteroaryloxy, wherein said aryl groups are optionally substituted by one to three R₁₀ and wherein said alkyl groups are optionally substituted by OH ;
Ar, R₇, R₈, R₁₀, being as defined in formula (I) hereabove,
and pharmaceutically acceptable salts thereof.

Compounds of formula (Ib) are notably selected from:
(1-((5-cyclopropylpyridin-2-yl)-3-isopropoxy-1H-pyrazol-4-yl)(phenyl)methanol
5-cyclopropyl-2-(4-(2-fluorophenoxy)-3-isopropoxy-1H-pyrazol-1-yl)pyrimidine
5-cyclopropyl-2-(3-isopropoxy-4-phenoxy-1H-pyrazol-1-yl)pyrimidine
5-cyclopropyl-3-fluoro-2-(3-isopropoxy-4-phenoxy-1H-pyrazol-1-yl)pyridine
5-cyclopropyl-2-(4-(2-fluorophenoxy)-3-isopropoxy-1H-pyrazol-1-yl)pyridine
5-cyclopropyl-2-(3-isopropoxy-4-(phenylsulfonyl)-1H-pyrazol-1-yl)pyrimidine
5-cyclopropyl-2-(3-isopropoxy-4-phenoxy-1H-pyrazol-1-yl)-3-methoxypyridine
5-cyclopropyl-3 -fluoro-2-(4-(2-fluorophenoxy)-3-isopropoxy-1H-pyrazol-1-yl)pyridine.

In a further object, the present invention provides compounds of formula (Ic):

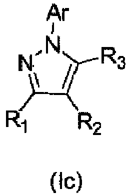

Wherein :
Ar is 2- or 3-pyridyl,
R₁ is independently selected from C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from (C₆-C₁₀)aryl(C₁-C₆)alkyl, wherein said aryl groups are optionally substituted by one to three R₁₀ and wherein said alkyl groups are optionally substituted by OH ;
R₃, R₇, R₈, being as defined in formula (I) hereabove,

With the exclusion of the following compounds :
2-((4-benzyl-3-ethoxy-5-methyl-1H-pyrazol-1-yl)pyridine,
4-benzyl-5-methyl-1-(pyridin-2-yl)-1H-pyrazol-3(2H)-one, and/or
4-benzyl-5-methyl-1-(pyridin-2-yl)-1H-pyrazol-3-ol,
and pharmaceutically acceptable salts thereof.

In a further object, the present invention provides compounds of formula (Id):

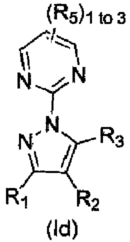

Wherein:
R₁ is independently selected from C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from C₁-C₆ alkyl, (C₆-C₁₀)aryl(C₁-C₆)alkyl, ,wherein said aryl groups are optionally substituted by one to three R₁₀ and wherein said alkyl groups are optionally substituted by OH;
R₃, R₅, R₇, R₈, R₁₀, being as defined in formula (I) hereabove,
and pharmaceutically acceptable salts thereof.

In a further object, the present invention provides compounds of formula (Ie):

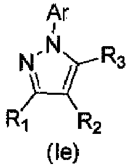

Wherein:
Ar is 2-pyrazinyl, 3-pyridazinyl, 5-pyrimidinyl, or 3-(1,2,4-triazinyl),
R₁ is independently selected from C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from C₁-C₆ alkyl, C₆-C₁₀ aryl, (C₆-C₁₀)aryl(C₁-C₆)alkyl, CN, CO₂R₉, C₆-C₁₀ aroyl, wherein said aryl groups are optionally substituted by one to three R₁₀ and wherein said alkyl groups are optionally substituted by OH;
R₅ is at each occurrence, independently selected from C₁-C₆ alkyl, C₁-C₆ alkenyl, C₃-C₆ cycloalkyl, C₁-C₆ alkoxy, (C₆-C₁₀)aryl(C₁-C₆)alkyl, (C₆-C₁₀)aryl(C₁-C₆)alkoxy, OH, F, Cl, Br, I, CF₃, CF₂CH₃, CN, C(=O)R₆, CO₂R₆, wherein said alkyl groups are optionally substituted by one or more OH, F, Cl, or Br;
R₃, R₆, R₇, R₈, R₁₀, being as defined in formula (I) hereabove, and pharmaceutically acceptable salts thereof.

### Pharmaceutical compositions

In a further object, the present invention provides a pharmaceutical composition comprising a compound of formula (I), as defined hereabove in admixture with one or more pharmaceutically acceptable excipients or carriers.

Further aspects of the present invention include compounds of formula (I) wherein the compound of formula (I) is a compound of formula (Ia), (Ib), (Ic), (Id), or (Ie) as defined hereabove.

### Method of preparation of compounds of formula (I)

The compounds of the present invention may be prepared in a number of methods well known to those skilled in the art, including, but not limited to those described below, or through modifications of these methods by applying standard techniques known to those skilled in the art of organic synthesis. The reagents and starting materials are commercially available, or readily synthesized by well-known techniques by one of ordinary skill in the arts. All substituents, unless otherwise indicated, are as previously defined. All processes disclosed in association with the present invention are contemplated to be practiced on any scale, including milligram, gram, multigram, or commercial industrial scale.

It will be appreciated that the compounds of the present invention may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from active starting materials or by deliberate chiral synthesis of target centers.

As will be readily understood, functional groups present on the compounds of Formula I may contain protecting groups. Protecting groups are known per se as chemical functional groups that can be selectively appended to and removed from functionalities, such as hydroxyl groups and carboxyl groups. These groups are present in a chemical compound to render such functionality inert to chemical reaction conditions to which the compound is exposed. Any of a variety of protecting groups may be employed with the present invention. Other preferred protecting groups according to the invention may be found in Greene, T.W. and Wuts, P.G.M., "Protective Groups in Organic Synthesis" 2d. Ed., Wiley & Sons, 1991, or in P.J. Kocienski, "Protecting Groups", 3rd. Ed., Thieme, Stuttgart, NY, 2004.

The compound thus prepared may be recovered from the reaction mixture by conventional means. For example, the compounds may be recovered by distilling off the solvent from the reaction mixture or, if necessary after distilling off the solvent from the reaction mixture, pouring the residue into water followed by extraction with a water-immiscible organic solvent and distilling off the solvent from the extract. Additionally, the product can, if desired, be further purified by various well known techniques, such as recrystallization, reprecipitation or the various chromatography techniques, notably column chromatography or preparative thin layer chromatography, in particular High Performance Liquid Chromatography (HPLC).

In another object, the present invention provides a method for preparing a compound of formula (I) as defined above, said method comprising the steps of:
i) Reacting a compound of formula (II) with R₁₄NHNH₂ thereby obtaining a compound of formula (III): 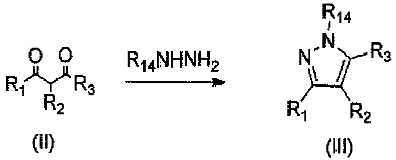 Wherein
   R₁, R₂ are as defined in formula (I)
   R₃ is C₁-C₆ alkoxy,
   R₁₄ is H or Ar, Ar being as defined hereabove,
ii) If R₁₄ is H, then reacting the compound of formula (III) with Ar-X, thereby obtaining a compound of formula (I) 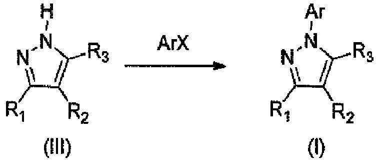 Wherein
   Ar is as defined hereabove and
   X is a halogen atom, notably F,Cl, Br or I,
iii) Recovering the obtained compound of formula (I).

The general routes to prepare the examples shown in Table 1 of the present invention are shown in schemes A to I. The reagents and starting materials are commercially available, or readily synthesized by well-known techniques by one of ordinary skill in the arts. All substituents in the synthetic Schemes, unless otherwise indicated, are not necessarily as previously defined.

### Synthesis of 3-alkoxypyrazoles.

### - Method A, from beta-carbonyl esters.

This method is based on the condensation of hydrazine hydrochloride and beta-carbonyl esters. The latter can be prepared by many types of modification of less complex beta-carbonyl esters or from malonates. The use of substituted hydrazines hydrochloride regioselectively leads to 1-substituted-5-alkoxypyrazoles. Both reactions are also leading to a proportion of the corresponding hydroxypyrazoles. Many examples of this synthetic path were reported in publications (Guillou et al. Synthesis 2008, 3504 and Guillou et al. Chem. Eur. J. 2010, 16, 4669), which also contains a review of previous work.

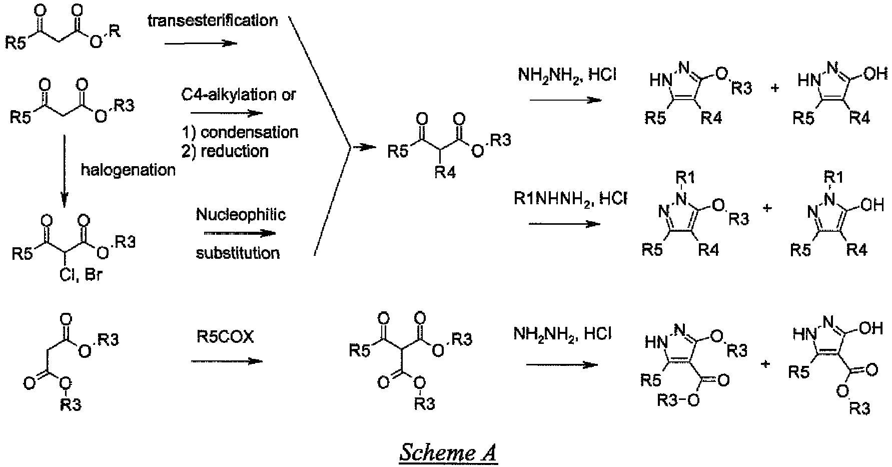

### - Method B, from dialkyl 2-(alkoxymethylene)malonate.

This method is based on the condensation of hydrazine hydrochloride and dialkyl 2-(alkoxymethylene)malonate. The use of substituted hydrazine hydrochloride, depending on the respective nucleophilic power of the two nitrogens, leads to a mixture of 1-substituted-3-alkoxypyrazoles and 1-substituted-5-alkoxypyrazoles (Guillou et al. Chem. Eur. J. 2010, 16, 4669).

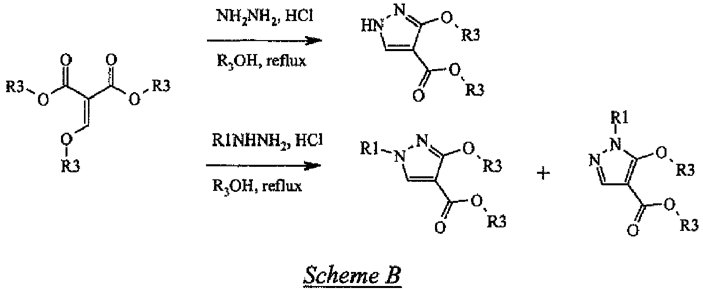

### - Method C, from 3-(dialkylamino)acrylates.

This method is based on the condensation of hydrazine dihydrochloride and 3-(dialkylamino)acrylates, the additional hydrochloride being necessary to neutralize the evolving dialkylamine (Guillou et al. Chem. Eur. J. 2010, 16, 4669).

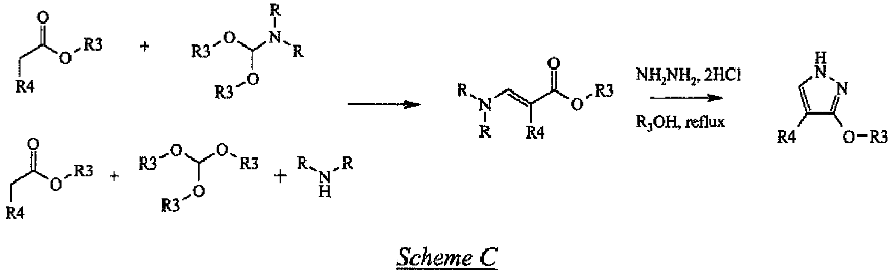

### - Method D, by alkylation of 3-hydroxypyrazoles

From 3-hydroxypyrazoles (either prepared by the Knorr reaction or resulting from the ether cleavage of 3-alkoxypyrazoles), it is possible to prepare 3-alkoxypyrazoles by alkylation. In the case of substrates free of a nitrogen substituent (R1 = H), the O-alkylation regioselectivity can sometime be improved by the use of transient N-protecting groups, as reviewed in a publication (Guillou et al. Synthesis 2008, 3504). Moreover, as further described below the bis acetylation of unsubtituted 3-hydroxypyrazoles provides an access to bis acetylated compounds. The ensuing O-alkylation of these, in the course of which the O-acetyl is preferentially cleaved, also provides a method to improve the proportion of O-akylated product.

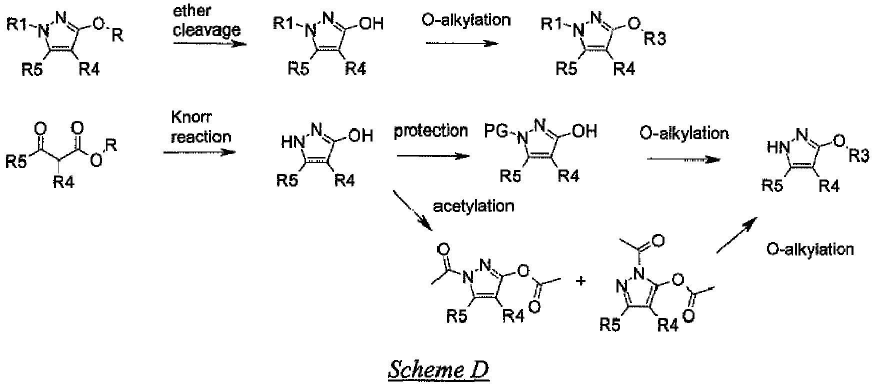

### - Method E, from 1,1-dialkoxyethenes.

This method is based on the condensation between 1,1-dialkoxyethene and activated acids (i.e.: carboxychlorides, symmetric anhydrides or pivaloyl-derived asymmetric anhydrides) which leads to beta, beta-dialkoxy-alpha, beta-unsaturated ketones. The subsequent condensation with hydrazine leads to 3-alkoxypyrazoles. The use of aryl hydrazines regioselectively leads to 1-substituted-3-alkoxypyrazoles. A recent report describes some of the scope of this approach and also contains a review of prior examples including alternative synthetic methods that can be used to prepare the key beta, beta-dialkoxy-alpha, beta-unsaturated ketones (Salanouve et al. Tetrahedron 2012, 68, 3165).

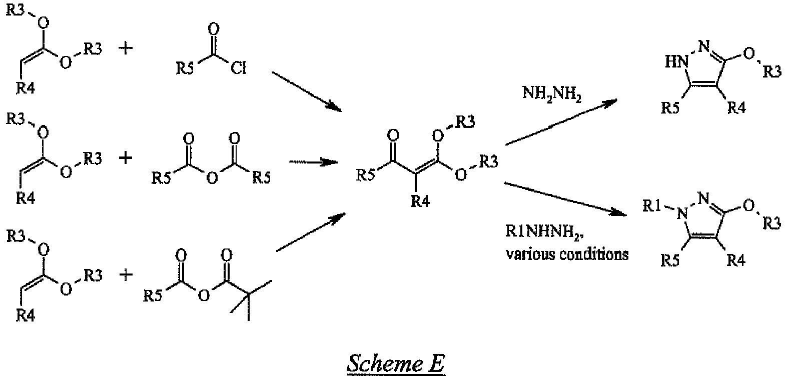

### Modifications of 3-alkoxypyrazoles.

### - Modifications on 3-alkoxypyrazoles nitrogens.

The N-arylation or N-alkylation of 3-alkoxypyrazoles can be achieved with many synthetic methods. The ease of this transformation is very much dependant on the reactivity of the halogenated heterocycle considered. From 2-halogenopyrimidines featuring electron attracting groups, the use of cesium carbonate in dry dimethylformamide and heating for one hour at 150 °C is enough. For 2-halogenopyrimidines featuring electron donating substituents a higher temperature (i.e.: 160 °C) is better. From 2-halogenopyridines featuring electron attracting substituents, these conditions are also efficient. On the other hand, from 2-halogenopyridines featuring electron donating substituents, higher temperature (with 2-fluropyridines) or, with 2-chloro or 2-bromopyridines, the use of copper-based catalysts (see Guillou et al. Tetrahedron 2010, 66, 2654 for instance) are often necessary. In the former case, because of its decomposition into dimethylamine, the use of dimethylformamide is then detrimental to the reaction yield and acetonitrile can be used instead. From other less reactive halogenated aryls, the use of copper-based catalysts is necessary. Alternatively, the use of arylboronic acids and copper catalyst at room temperature is also possible. Such N-arylation is usually leading to a mixture of 1-substituted-3-alkoxypyrazoles and 1-substituted-5-alkoxypyrazoles isomers. Studies of the influence of the reagents and reaction conditions on the ratio of these isomers were reported. Structure assignment for these isomers can be performed by control reactions between the corresponding arylhydrazine hydrochloride (or aryl hydrazine and an acid source) and beta-carbonyl ester which selectively lead to the corresponding 1-substituted-5-alkoxypyrazole isomer. Alternatively, the Knorr reaction between the corresponding arylhydrazine and beta-carbonyl ester selectively lead to the corresponding 1-substituted-5-hydroxypyrazole isomer. In this case, ether cleavage of each of the N-arylation reaction products also allows a structural assignment by comparison (for illustrations of all these points, see Guillou et al. 2009 Tetrahedron 2009, 65, 2660; Guillou et al. Tetrahedron 2009, 65, 3529; Guillou et al. Tetrahedron 2010, 66, 2654; Guillou et al. Chem. Eur. J. 2010, 16, 4669 and Salanouve et al. Tetrahedron 2012, 68,3165).

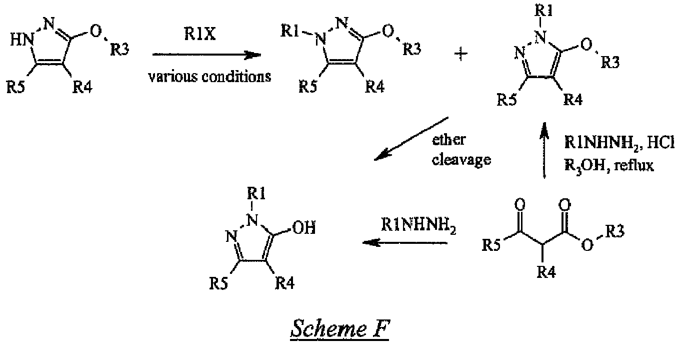

### - Modifications on 3-alkoxypyrazoles carbon 4.

Halogenation on carbon 4 of alkoxypyrazole allows the use of organometallic-based transformations such as the many process involving palladium, copper or nickel-catalyzed reactions. Alternatively, strong base displacement of the 4-halogen and thus generation of the corresponding anion can be followed by the addition of various electrophiles. Depending on the transformations planned, a pyrazole nitrogen substituent, eventually labile, can be required. Reports illustrating such modifications were published (Guillou et al. Chem. Eur. J. 2010, 16, 4669; Guillou et al, Tetrahedron 2009, 65, 3529; Janin Chem. Rev. 2012, 112, 3924).

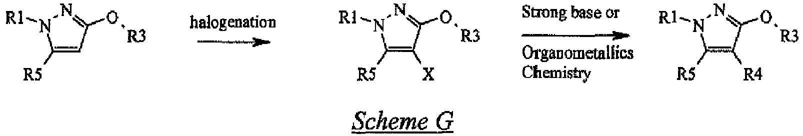

### - Modifications on 3-alkoxypyrazoles carbon 5.

Depending on the substrate, a carbon 5 halogenation (preferably iodination) of 3-alkoxypyrazoles is possible. The resulting building blocks are the amenable to the same chemistry mentioned above for 4-halogenated pyrazoles. Moreover, depending on the nature of the nitrogen substituent, a carbon 5 deprotonation followed by the addition of electrophiles is possible. Examples of both types of transformations were reported (Guillou et al. Chem. Eur. J. 2010, 16, 4669; Guillou et al. Tetrahedron 2010, 66, 2654; Salanouve et al. Tetrahedron 2012, 68, 3165; Salanouve et al. Tetrahedron 2012, 68, 2135; Cottineau et al. Synlett 2002, 769; Janin Chem. Rev. 2012, 112, 3924).

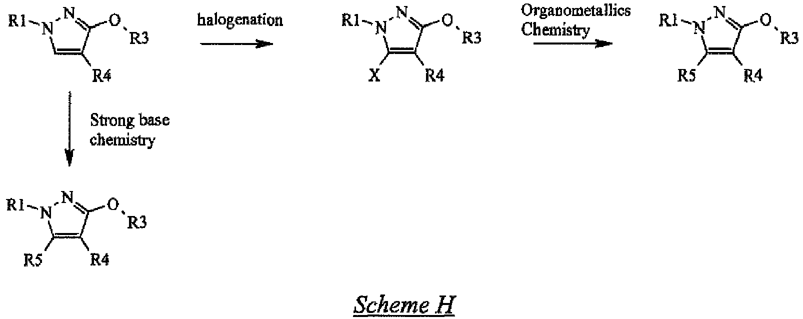

### - Modification of a methyl group on position 5 of 3-alkoxypyrazoles.

Depending on its substituents, the selective halogenation of a methyl on position 5 of a 3-alkoxypyrazole can take place via the transient occurrence of a carbon 4-halogenated specie. From these substrates, a nucleophilic displacement leads to 3-alkoxypyrazole featuring, for instance, aminated or ether side chains.

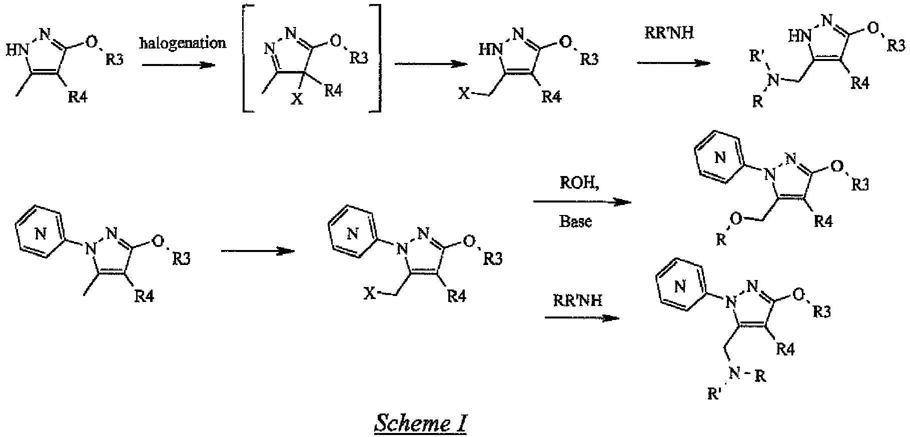

### Synthetic intermediates

In another object, the present invention provides compounds of formula (IV):

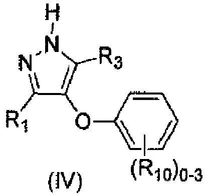

Wherein:
R₁ is C₁-C₆ alkoxy, optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₃, R₁₀ are as defined in formula (I) hereabove.

### Definitions

The following terms and expressions contained herein are defined as follows:
As used herein, the term "alkyl" refers to a straight-chain, or branched alkyl group having 1 to 8 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, octyl, etc. The alkyl moiety of alkyl-containing groups, such as alkoxy, arylalkyl has the same meaning as alkyl defined above. Lower alkyl groups, which are preferred, are alkyl groups as defined above which contain 1 to 4 carbons. A designation such as "C₁-C₆ alkyl" refers to an alkyl radical containing from 1 to 6 carbon atoms.

As used herein, the term "cycloalkyl" refers to a non-aromatic mono- or multicyclic ring system of 3 to 6 ring atoms. Exemplary monocyclic cycloalkyl include cyclopropyl, cyclopentyl, or cyclohexyl.

As used herein, the term "alkoxy" means an alkyl-O- group wherein the alkyl group is as herein described. Exemplary alkoxy groups include methoxy, ethoxy, n-propoxy, i-propoxy, and n-butoxy.

As used herein, the term "alkenyl" refers to a straight chain, or branched hydrocarbon chains of 2 to 6 carbon atoms having at least one carbon-carbon double bond. A designation "C₂-C₆ alkenyl" refers to an alkenyl radical containing from 2 to 6 carbon atoms. Examples of alkenyl groups include ethenyl, propenyl, isopropenyl, 2,4-pentadienyl. "C₂-C₄ alkenyl" are particularly preferred.

As used herein, the term "aryl" refers to a substituted or unsubstituted, mono- or bicyclic hydrocarbon aromatic ring system having 6 to 10 ring carbon atoms. Examples include phenyl and naphthyl.

As used herein, the term "arylalkyl" refers to an alkyl group substituted with an aryl group, wherein said alkyl and aryl groups are as defined herein. Examples of arylalkyl groups include benzyl.

As used herein, the term "arylalkoxy" refers to an alkoxy group substituted with an aryl group, wherein said alkoxy and aryl groups are as defined herein. Examples of arylalkoxy groups include benzyloxy.

As used herein, the term "aryloxy" refers to an aryl-O- group, wherein said aryl group is as defined herein. Examples of aryloxy groups include phenoxy.

As used herein, the term "arylthio" refers to an aryl-S- group, wherein said aryl group is as defined herein. Examples of arylthio groups include phenylthio

As used herein, the term "arylsulfinyl" refers to an aryl-SO- group, wherein said aryl group is as defined herein. Examples of arylsulfinyl groups include phenylsulfinyl

As used herein, the term "arylsulfonyl" refers to an aryl-SO₂- group, wherein said aryl group is as defined herein. Examples of arylsulfonyl groups include phenylsulfonyl

As use herein, the term "aroyl" refers to an aryl-CO- group. Examples of aroyl groups include benzoyl.

As used herein, the term "heteroaryl" refers to an aromatic group containing 5 to 7 ring carbon atoms in which one or more ring carbon atoms are replaced by at least one hetero atom such as -O-, -N-, or -S-. The nitrogen and sulfur heteroatoms may be optionally oxidized, and the nitrogen may be optionally substituted in non-aromatic rings. Examples of heteroaryl groups include pyrrolyl, furanyl, thienyl, pyrazolyl, imidazolyl, thiazolyl, isothiazolyl, isoxazolyl, oxazolyl, oxathiolyl, oxadiazolyl, triazolyl, oxatriazolyl, furazanyl, tetrazolyl, pyridyl, pyrazinyl, pyrimidinyl, pyridazinyl, triazinyl.

As used herein, the term "heterocyclyl" refers to a substituted or unsubstituted carbocyclic group in which the ring portion includes at least one heteroatom such as O, N, or S. The nitrogen and sulfur heteroatoms may be optionally oxidized, and the nitrogen may be optionally substituted in non-aromatic rings. Heterocycles are intended to include heteroaryl and heterocycloalkyl groups.

As used herein, the term "subject" refers to a warm blooded animal such as a mammal, preferably a human, or a human child, which is afflicted with, or has the potential to be afflicted with one or more diseases and conditions described herein.

As used herein, a "therapeutically effective amount" refers to an amount of a compound of the present invention effective to prevent or treat the symptoms of particular disorder. Such disorders include, but are not limited to, those pathological and neurological disorders associated with the aberrant activity of the receptors described herein, wherein the treatment or prevention comprises inhibiting, inducing, or enhancing the activity thereof by contacting the receptor with a compound of the present invention.

As used herein, the term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

The meanings of all the other terms used in the description of the present invention are well known in the art.

In another aspect, the present invention is directed to pharmaceutically acceptable salts of the compounds described above. As used herein, "pharmaceutically acceptable salts" includes salts of compounds of the present invention derived from the combination of such compounds with non-toxic acid or base addition salts.

Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, para-toluenesulfonic, succinic, and benzoic acid, and related inorganic and organic acids.

Base addition salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.

In addition to pharmaceutically acceptable salts, other salts are included in the invention. They may serve as intermediates in the purification of the compounds, in the preparation of other salts, or in the identification and characterization of the compounds or intermediates.

Other features of the invention will become apparent in the course of the following descriptions of exemplary embodiments. These examples are given for illustration of the invention and are not intended to be limiting thereof.

### Figures

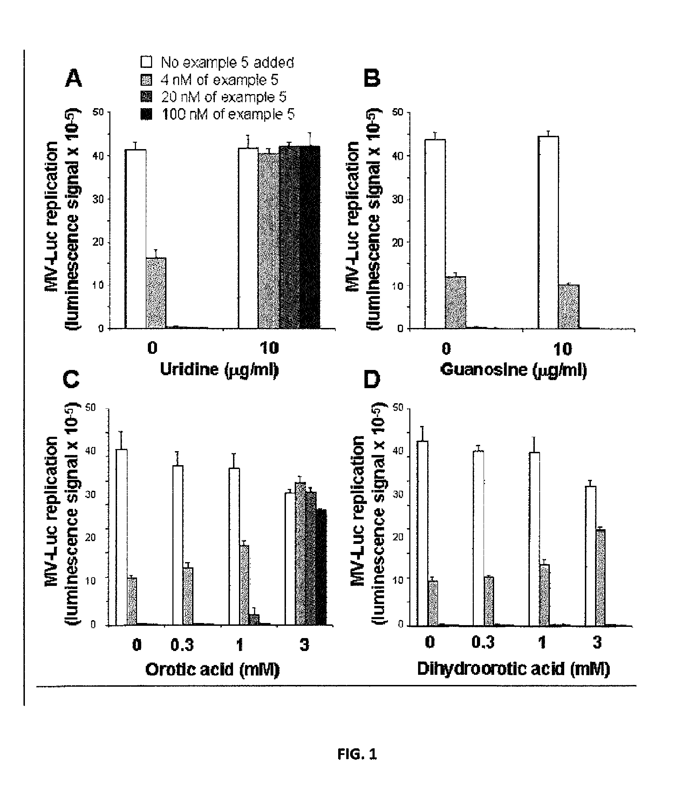

**Figure 1** **:** Mechanism of antiviral action of compounds of formula (I), illustrated with 5-cyclopropyl-2-(4-(2,6-difluorophenoxy)-3-isopropoxy-5-methyl-1H-pyrazol-1-yl)-3-fluoropyridine (example 5).

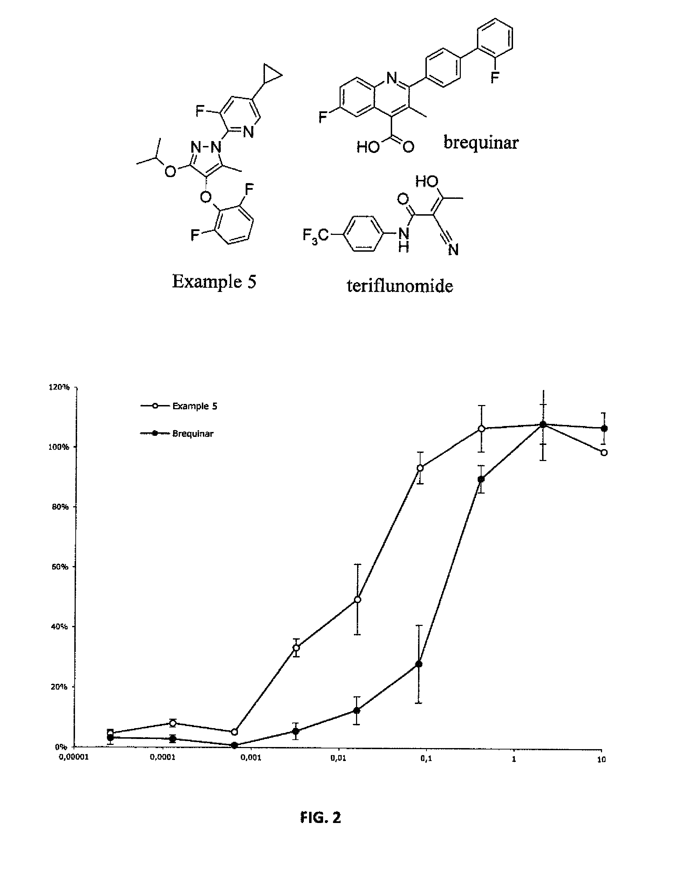

**Figure 2** **:** Inhibition of the proliferation of Jurkat cells by example 5 or brequinar. (Concentration in µM versus % growth) Jurkat cells were incubated with increasing doses of example 5 or brequinar. As a control, cells were treated with DMSO alone. At t=0 and after 72 hours of culture, the number of living cells was determined using the CellTiter-Glo reagent. The inhibition of cellular proliferation is expressed as a percentage relative to DMSO-treated control wells. Presented results correspond to the mean ± SD of two independent experiments.

### Examples

### 1. Synthesis of compounds of formula (I)

### General experimental procedure

A Biotage initiator 2 microwave oven was used for reactions mentioning such heating method. ¹H NMR and ¹³C NMR spectra were recorded on a Bruker Avance 400 spectrometer at 400 MHz and 100 MHz, respectively. Shifts (*δ*) are given in ppm with respect to the TMS signal and coupling constants (*J*) are given in Hertz. Column chromatography were performed either on Merck silica gel 60 (0.035 - 0.070 mm) or neutral alumina using a solvent pump and an automated collecting system driven by a UV detector set to 254 nm unless required otherwise. Sample deposition was carried out by absorption of the mixture to be purified on a small amount of the solid phase followed by its deposition of the top of the column. The low resolution mass spectra were obtained on an Agilent 1100 series LC/MSD system using an atmospheric electrospray ionization system and the high resolution mass spectra (HRMS) were obtained using a Waters Micromass Q-Tof with an electrospray ion source.

**- Examples of N-arylation of 3-alkoxypyrazoles with halogenoheteroaryl without copper catalyst, general method.** In a reaction vial designed for microwave heating, the considered alkoxypyrazole (2 mmol), the considered halogenated heteroaryl (2.2 mmol) and cesium carbonate (2.8 mmol) were stirred in dimethylformamide or acetonitrile (3 mL) as specified in the examples. This was heated using a microwave at a temperature between 120 °C and 180 °C for the individually specified duration. The resulting suspension was diluted in water, extracted with ethyl acetate and the organic layer was washed with brine, dried over magnesium sulfate and concentrated to dryness. The residue was further purified as described below.

**6-benzyl-3-ethoxy-1-(pyridin-2-yl)-4,5,6,7-tetrahydro-1H-pyrazolo[3,4-c]pyridine (example 1):** Obtained in 47 % yield, using 2-fluoropyridine in acetonitrile at 180 °C for three hours and a chromatography over silica gel (dichloromethane-ethanol 99/1). ¹H (CDCl₃): 1.45 (t, 3H, J = 7.0); 2.54 (m, 2H); 2.75 (m, 2H); 3.80 (s, 2H); 4.19 (s(1), 2H); 4.38 (q, 2H, J = 7.0); 6.97 (m, 1H); 7.36 (m, 2H); 7.43 (m, 2H); 7.69 (m, 1H); 7.76 (m, 1H); 8.27 (m, 1H). ¹³C (CDCl₃): 14.9; 19.6; 48.9; 53.2; 61.4; 64.3; 104.2; 112.5; 119.0; 127.1; 128.3; 129.0; 138.1; 138.7; 139.9; 147.4; 153.3; 161.3. HRMS: Calc. for C₂₀H₂₂N₄O + H: 335.1872; Found: 335.1846.

**2-(5-benzyl-3-ethoxy-1H-pyrazol-1-yl)-5-ethylpyrimidine (example 2):** Obtained in 54 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 1.26 (t, 3H, J = 7.6); 1.38 (t, 3H, J = 7.1); 2.62 (q, 2H, J = 7.6); 4.38 (q, 2H, J = 7.1); 4.47 (s, 2H); 5.54 (s, 1H); 7.17-7.29 (m, 5H); 8.52 (s, 2H). ¹³C (CDCl₃): 14.7; 15.0; 23.0; 35.3; 64.5; 98.3; 126.8; 125.3; 128.3; 129.0; 132.3; 138.4; 147.2; 155.9; 157.6; 164.0. HRMS: Calc. for C₁₈H₂₀N₄O + H: 309.1715; Found: 309.1757.

**2-(5-benzyl-3-ethoxy-1H-pyrazol-1-yl)pyridine (example 3):** Prepared as previously described (Guillou et al. *Tetrahedron,* **2010,** *66,* 2654).

**2-(4-(2,6-difluorophenoxy)-3-isopropoxy-5-methyl-1H-pyrazol-1-yl)pyridine (example 4):** Obtained in 66 % yield as an oil, using 2-fluoropyridine in acetonitrile at 180 °C for 6 hours. ¹H (CDCl₃): 1.26 (d, 6H, J = 6.2); 2.68 (s, 3H); 4.91 (sept, 1H, J = 6.2); 7.01 (m, 2H); 7.06 (m, 2H); 7.72 (m, 2H); 8.36 (m, 1H). ¹³C (CDCl₃): 11.9; 21.9; 72.0; 111.9 (6 and 17 Hz); 114.3; 119.6; 123.4 (9 Hz); 129.4; 131.3; 134.7 (14 Hz); 137.9; 147.2; 153.7; 154.1; 155.6 (5 and 250 Hz). HRMS: Calc. for C₁₈H₁₇F₂N₃O₂ + H: 346.1367; Found: 346.1409.

**5-cyclopropyl-2-(4-(2,6-difluorophenoxy)-3-isopropoxy-5-methyl-1H-pyrazol-1-yl)-3-fluoropyridine** (example **5):** Obtained in 46 % yield, using 5-cyclopropyl-2,3-difluoropyridine (intermediate 51) in acetonitrile at 180 °C for 3 hours and after a first chromatography over silica gel (cyclohexane/ethyl acetate 7/1) and a second one over silica gel (dichloromethane) as an oil. ¹H (CDCl₃): 0.76 (m, 2H); 1.11 (m, 2H); 1.22 (d, 6H, J = 6.1); 1.97 (m, 1H); 2.27 (s, 3H); 4.87 (sept, 1H, J = 6.1); 6.82 (m, 3H); 7.16 (m, 1H); 8.16 (d, 1H, J = 2.0). ¹³C (CDCl₃): 9.2; 9.6; 12.6; 21.9; 71.9; 111.9 (6 and 16 Hz); 121.9 (18 Hz); 123.5 (9 Hz); 128.3; 131.0; 134.7 (14 Hz); 138.1 (10 Hz); 141.6; 142.3; 152.9 (263 Hz); 154.1; 155.8 (5 and 250 Hz). HRMS: Calc. for C₂₁H₂₀F₃N₃O₂ + H: 404.1586; Found: 404.1527.

**2-(4-(2,6-difluorophenoxy)-3-isopropoxy-5-methyl-1H-pyrazol-1-yl)-5-ethyl-3-**fluoropyridine (example 5A): Obtained in 34 % yield as an oil, using 2,3-difluoro-5-ethylpyridine (intermediate 54B) in acetonitrile at 140 °C for 4 hours and a chromatography over silica gel (cyclohexane-ethyl acetate 97/3) followed by drying under high vacuum. ¹H (CDCl₃): 1.23 (d, 6H, J = 6.2); 1.31 (t, 3H, J = 7.6); 2.28 (s, 3H); 2.74 (q, 2H, J = 7.6); 4.88 (sept, 1H, J = 6.2); 6.91 (m, 2H); 7.01 (m, 1H); 7.41 (m, 1H); 8.20 (m, 1H). ¹³C (CDCl₃): 9.3; 15.0; 21.9; 25.3; 71.9; 111.9 (6 and 17 Hz); 123.5 (9 Hz); 124.8 (17 Hz); 128.3; 131.1; 134.7 (14 Hz); 138.5 (11 Hz); 141.0 (3 Hz); 143.5 (5 Hz); 152.8 (260 Hz); 154.1; 155.8 (4 and 250 Hz). HRMS: Calc. for C₂₀H₂₀F₃N₃O₂ + H: 392.1586; Found: 392.1572.

**2-(4-(2,6-difluorophenoxy)-3-isopropoxy-5-methyl-1H-pyrazol-1-yl)-5-propylpyrimidine (example 6):** Obtained in 67 % yield as a solid using 2-chloro-5-propylpyrimidine in acetonitrile at 160 °C for one hour after a chromatography over silica gel (cyclohexane-ethyl acetate 4/1). ¹H (CDCl₃): 0.95 (t, 3H, J = 7.4); 1.21 (d, 6H, J = 6.2); 1.63 (m, 2H); 2.56 (t, 2H, J = 7.5); 2.65 (s, 3H); 5.10 (sept, 1H, J = 6.2); 6.89 (m, 2H); 7.01 (m, 1H); 8.51 (s, 2H). ¹³C (CDCl₃): 12.3; 13.4; 21.9; 24.0; 31.7; 71.9; 111.9 (6 and 16 Hz); 123.5 (9 Hz); 130.2; 130.6; 132.2; 134.5 (13 Hz); 154.9; 155.6 (250 and 4 Hz); 155.2; 158.0. HRMS: Calc. for C₂₀H₂₂F₂N₄O₂ + H: 389.1789; Found: 389.1765.

**3-cyclopropyl-6-(4-(2,6-difluorophenoxy)-3-isopropoxy-5-methyl-1H-pyrizol-1-yl)pyridazine (example 7):** Obtained in 22 % yield as an oil, using 3-chloro-6-cyclopropylpyridazine (intermediate 54) and 4-(2,6-difluorophenoxy)-3-isopropoxy-5-methyl-1H-pyrazole (intermediate 8) in acetonitrile at 160 °C for two hour and a chromatography over silica gel (cyclohexane-ethyl acetate 6/1). ¹H (CDCl₃): 1.11 (m, 2H); 1.21 (m, 2H); 1.26 (d, 6H, J = 6.2); 2.15 (m 1H); 2.75 (s, 3H); 4.88 (sept, 1H, J = 6.2); 6.91 (m, 2H); 7.02 (m, 1H); 7.29 (d, 1H, J = 9.1); 7.88 (d, 1H, J = 9.1). ¹³C (CDCl₃): 10.2; 12.1; 15.4; 21.9; 72.1; 111.9 (6 and 17 Hz); 118.8; 123.6 (9 Hz); 126.9; 129.8; 131.8; 134.5 (14 Hz); 154.4; 155.5 (4 and 250 Hz); 155.7; 161.3. HRMS: Calc. for C₁₈H₁₈F₂N₄O₂ + H: 387.1633; Found: 387.1628.

**2-(4-(2,6-difluorophenoxy)-3-isopropoxy-5-methyl-1H-pyrazol-1-yl)-5-ethylpyrimidine (example 8):** Obtained in 56 % yield, using 2-chloro-5-ethylpyrimidine and 4-(2,6-difluorophenoxy)-3-isopropoxy-5-methyl-1H-pyrazole (intermediate 8) in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as an oil. ¹H (CDCl₃): 1.20 (d, 6H, J = 6.1); 1.27 (t, 3H, J = 7.5); 2.64 (q, 2H, J = 7.5); 2.65 (s, 3H); 5.10 (sept, 1H, J = 6.1); 6.88 (m, 2H); 7.01 (m, 1H); 8.53 (s, 2H). ¹³C (CDCl₃): 12.3; 15.0; 21.9; 23.0; 71.9; 111.9 (6 and 16 Hz); 123.5 (9 Hz); 130.2; 132.1; 132.2; 134.5 (14 Hz); 154.9; 155.6 (250 and 4 Hz); 156.2; 157.6. HRMS: Calc. for C₁₉H₂₀F₂N₄O₂ + H: 375.1633; Found: 375.1625.

**5-cyclopropyl-2-(4-(2,6-difluorophenoxy)-3-isopropoxy-5-methyl-1H-pyrazol-1-yl)pyridine (example 9):** Obtained in 53 % yield, using 5-cyclopropyl-2-fluoropyridine (intermediate 50) and 4-(2,6-difluorophenoxy)-3-isopropoxy-5-met-hyl-1H-pyrazole (intermediate 8) in acetonitrile at 180 °C for 6 hours and after a chromatography over silica gel (cyclohexane-ethyl acetate 98/2 to 97/3) followed by extensive drying under high vacuum as an oil. ¹H (CDCl₃): 0.72 (m, 2H); 1.01 (m, 2H); 1.25 (d, 6H, J = 6.2); 1.91 (m, 1H); 2.63 (s, 3H); 4.89 (sept, 1H, J = 6.2); 6.88 (m, 2H); 7.01 (m, 1H); 7.37 (m, 1H); 7.62 (d, 1H, J = 8.5); 8.18 (d, 1H, J = 2.3). ¹³C (CDCl₃): 8.6; 11.6; 12.5; 21.9; 71.9; 111.9 (6 and 16 Hz); 114.2; 123.4 (9 Hz); 129.1; 131.0; 134.7 (13 Hz); 135.0; 135.4; 145.4; 152.0; 153.4; 155.7 (4 and 250 Hz). HRMS: Calc. for C₂₁H₂₁F₂N₃O₂ + H: 386.1680; Found: 386.1664.

**2-(4-(2,6-difluorophenoxy)-3-isopropoxy-5-methyl-1H-pyrazol-1-yl)-5-methylpyridine (example 9A):** Obtained in 34 % yield as an oil, using 2-fluoro-5-methylpyridine in acetonitrile at 180 °C for 6 hours and a chromatography over silica gel (cyclohexane/ethyl acetate 97/3). ¹H (CDCl₃): 1.26 (d, 6H, J = 6.2); 2.33 (s, 3H); 2.64 (s, 3H); 4.90 (sept, 1H, J = 6.2); 6.92 (m, 2H); 7.01 (m, 1H); 7.54 (m, 1H); 7.64 (d, 1H? J = 8.4); 8.18 (m, 1H). ¹³C (CDCl₃): 11.6; 17.7; 21.9; 71.9; 111.9 (6 and 17 Hz); 114.2; 123.4 (9 Hz); 129.1; 129.2; 131.0; 134.7 (14 Hz); 138.7; 147.1; 152.0; 153.4; 155.7 (5 and 250 Hz). HRMS: Calc. for C₁₉H₁₉F₂N₃O₂ + H: 360.1524; Found: 360.1450.

**2-(4-(2,6-difluorophenoxy)-3-isopropoxy-5-methyl-1H-pyrazol-1-yl)-5-ethylpyridine (example 9B):** Obtained in 49 % yield as an oil, using 5-ethyl-2-fluoropyridine (intermediate 54A) in acetonitrile at 180 °C for 6 hours and a chromatography over silica gel (cyclohexane-ethyl acetate 97/3) followed by drying under high vacuum. ¹H (CDCl₃): 1.26 (d, 6H, J = 6.2); 1.27 (t, 3H, J = 7.6); 2.64 (s, 3H); 2.65 (q, 2H, J = 7.6); 4.90 (sept, 1H, J = 6.2); 6.92 (m, 2H); 7.00 (m, 1H); 7.56 (dd, 1H, J = 1.9 and 8.5); 7.66 (d, 1H, J = 8.5); 8.20 (d, 1H, J = 1.9). ¹³C (CDCl₃): 11.6; 15.4; 21.9; 25.5; 71.9; 111.9 (6 and 17 Hz); 114.3; 123.4 (9 Hz); 129.1; 131.0; 134.7 (14 Hz); 135.4; 137.6; 146.4; 152.2; 153.5; 155.6 (4 and 250 Hz). HRMS: Calc. for C₂₀H₂₁F₂N₃O₂ + H: 374.1680; Found: 374.1680.

**5-cyclopropyl-2-(4-(2-fluorobenzyl)-3-isopropoxy-5-methyl-1H-pyrazol-1-yl)pyrimidine (example 10):** Obtained in 41 % yield, using 2-chloro-5-cyclopropylpyrimidine (intermediate 49) in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 0.75 (m, 2H); 1.02 (m, 2H); 1.31 (d, 6H, J = 6.1); 1.84 (m, 1H); 2.54 (s, 3H); 3.73 (s, 2H); 5.22 (sept, 1H, J = 6.1); 7.00 (m, 2H); 7.20 (m, 2H); 8.44 (s, 2H). ¹³C (CDCl₃): 8.3; 10.4; 13.5; 20.6; 22.2; 71.2; 107.5; 114.9 (22 Hz); 123.7; 127.2; 127.4; 127.5; 130.5; 132.0; 140.5; 156.2; 160.9 (244 Hz); 162.6. HRMS: Calc. for C₂₁H₂₃FN₄O + H: 367.1934; Found: 367.1911.

**5-ethyl-2-(4-(3-fluorobenzyl)-3-isopropoxy-5-methyl-1H-pyrazol-1-yl)pyrimidine (example 11):** Obtained in 46 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as an oil. ¹H (CDCl₃); 1.22 (t, 3H, J = 7.6); 1.33 (d, 6H, J = 6.1); 2.55 (s, 3H); 2.67 (q, 2H, J = 7.6); 3.72 (s, 2H); 5.23 (sept, 1H, J = 6.1); 6.84 (m, 1H); 6.95 (m, 1H); 7.00 (m, 1H); 7.22 (m, 1H); 8.55 (s, 2H). ¹³C (CDCl₃): 13.7; 15.0; 22.2; 23.0; 27.6; 71.3; 108.4; 112.6 (21 Hz); 115.1 (21 Hz); 123.8; 129.5; 132.0; 140.4; 143.3; 156.2; 157.6; 162.5; 163.0 (241 Hz). HRMS: Calc. for C₂₀H₂₃FN₄O + H: 355.1934; Found: 355.1937.

**3-(4-(2,6-difluorophenoxy)-3-isopropoxy-5-methyl-1H-pyrazol-1-yl)-6-methylpyridazine (example 12):** Obtained in 29 % yield as a solid, using 3-chloro-6-methylpyridazine in acetonitrile at 140 °C for two hour and two consecutive chromatography, the first one over silica gel (cyclohexane-ethyl acetate 3/1), the second one over alumina containing 1.5 % of water (cyclohexane-dichloromethane from 1/1 to 1/2). ¹H (CDCl₃): 1.25 (d, 6H, J = 6.2); 2.69 (s, 3H); 2.75 (s, 3H); 4.88 (sept, 1H, J = 6.2); 6.89 (m, 2H); 7.00 (m, 1H); 7.35 (d, 1H, J = 9.1); 7.90 (d, 1H, J = 9.1). ¹³C (CDCl₃): 12.1; 21.6; 21.8; 72.1; 112.0 (6 and 17 Hz); 118.9; 123.6 (9 Hz); 128.8; 129.9; 131.9; 134.4 (13 Hz); 154.5; 155.6 (4 and 250 Hz); 155.8; 156.7. HRMS: Calc. for C₁₈H₁₈F₂N₄O₂ + H: 361.1476; Found: 361.1491.

**5-cyclopropyl-2-(4-(2,6-difluorophenoxy)-3-isopropoxy-5-methyl-1H-pyrazol-1-yl)pyrimidine (example 13):** Obtained in 83 % yield, using 2-chloro-5-cyclopropylpyrimidine (intermediate 49) and 4-(2,6-difluorophenoxy)-3-isopropoxy-5-methyl-1H-pyrazole (intermediate 8) in acetonitrile at 160 °C for one hour and after a chromatography over silica gel (cyclohexane-ethyl acetate 4/1) as a solid. ¹H (CDCl₃): 0.75 (m, 2H); 1.08 (m, 2H); 1.21 (d, 6H, J = 6.2); 1.85 (m, 1H); 2.64 (s, 3H); 5.10 (sept, 1H, J = 6.2); 6.90 (m, 2H); 7.00 (m, 1H); 8.44 (s, 2H). ¹³C (CDCl₃): 8.2; 10.4; 12.3; 21.9; 71.9; 111.9 (6 and 16 Hz); 123.5 (9 Hz); 130.2; 132.1; 132.3; 134.8 (13 Hz); 154.9; 155.6 (251 and 4 Hz); 155.9; 156.2. HRMS: Calc. for C₂₀H₂₀F₂N₄O₂ + H: 387.1633; Found: 387.1599.

**2-(4-(2,6-difluorophenoxy)-3-isopropoxy-5-methyl-1H-pyrazol-1-yl)-5-fluoropyrimidine (example 14):** Obtained in 18 % yield as solid, using 2-chloro-5-fluoropyrimidine in acetonitrile at 150 °C for one hour and after two consecutive chromatographies over silica gel (dichloromethane) and (cyclohexane-ethyl acetate 4/1). ¹H (CDCl₃): 1.21 (d, 6H, J = 6.2); 2.65 (s, 3H); 5.08 (sept, 1H, J = 6.2); 6.89 (m, 2H); 7.01 (m, 1H); 8.57 (s, 2H). ¹³C (CDCl₃): 12.4; 21.9; 72.1; 111.9 (6 and 16 Hz); 123.7 (9 Hz); 130.5; 132.4; 134.3 (13 Hz); 145.8 (22 Hz); 153.9 (3 Hz); 155.0 (260 Hz); 155.3; 155.7 (4 and 250 Hz). HRMS: Calc. for C₁₇H₁₅F₃N₄O₂ + H: 365.1225; Found: 365.1222.

**3-chloro-6-(4-(2,6-difluorophenoxy)-3-isopropoxy-5-methyl-1H-pyrazol-1-yl)pyridazine (example 15):** Obtained in 58 % yield as a solid, using 3,6-dichloropyridazinein acetonitrile at 140 °C for one hour and a chromatography over silica gel (cyclohexane-ethyl acetate from 97/3 to 95/5). ¹H (CDCl₃): 1.25 (d, 6H, J = 6.2); 2.75 (s, 3H); 4.88 (sept, 1H, J = 6.2); 6.89 (m, 2H); 7.01 (m, 1H); 7.50 (d, 1H, J = 9.3); 8.01 (d, 1H, J = 9.3). ¹³C (CDCl₃): 12.3; 21.8; 72.3; 112.0 (6 and 17 Hz); 121.2; 123.8 (9 Hz); 129.8; 130.5; 132.2; 134.2 (14 Hz); 152.6; 155.1; 155.6 (4 and 250 Hz); 156.4. HRMS: Calc. for C₁₇H₁₅ClF₂N₄O₂ + H: 381.0930; Found: 381.0927.

**5-cyclopropyl-2-(4-(2,3-difluorophenoxy)-3-isopropoxy-5-methyl-1H-pyrazol-1-yl)pyridine (example 16):** Obtained in 55 % yield as an oil, using 5-cyclopropyl-2-fluoropyridine (intermediate 50) in acetonitrile at 180 °C for 6 hours and after a chromatography over silica gel (cyclohexane/ethyl acetate 97/3) followed by concentration under high vacuum. ¹H (CDCl₃): 0.72 (m, 2H); 1.03 (m, 2H); 1.34 (d, 6H, J = 6.2); 1.92 (m, 1H); 2.55 (s, 3H); 4.99 (sept, 1H, J = 6.2); 6.71 (m, 1H); 6.84 (m, 1H); 6.91 (m, 1H); 7.41 (dd, 1H, J 2.3 and 8.5); 7.68 (d, 1H, J = 8.5); 8.19 (d, 1H, J = 2.3). ¹³C (CDCl₃): 8.7; 11.8; 12.5; 21.1; 72.2; 110.2 (18 Hz); 111.2 (3 Hz); 114.2; 122.8 (5 and 9 Hz); 124.9; 133.0; 135.2; 135.8; 141.1 (15 and 249 Hz); 145.5; 148.0 (3 and 8 Hz); 151.5 (10 and 247 Hz); 151.8; 154.2. HRMS: Calc. for C₂₁H₂₁F₂N₃O₂ + H: 386.1680; Found: 386.1672.

**5-cyclopropyl-2-(4-(2,4-difluorophenoxy)-3-isopropoxy-5-methyl-1H-pyrazol-1-yl)pyridine (example 17):** Obtained in 54 % yield as an oil, using 5-cyclopropyl-2-fluoropyridine (intermediate 50) in acetonitrile at 180 °C for 6 hours and after a chromatography over silica gel (cyclohexane/ethyl acetate 97/3) followed by concentration under high vacuum. ¹H (CDCl₃): 0.72 (m, 2H); 1.03 (m, 2H); 1.33 (d, 6H, J = 6.2); 1.93 (m, 1H); 2.55 (s, 3H); 4.98 (sept, 1H, J = 6.2); 6.74 (m, 1H); 6.91 (m, 2H); 7.42 (dd, 1H, J 2.3 and 8.5); 7.67 (d, 1H, J = 8.5); 8.19 (d, 1H, J = 2.3). ¹³C (CDCl₃): 8.7; 11.7; 12.5; 22.1; 72.1; 104.8 (22 and 28 Hz); 110.3 (4 and 22 Hz); 114.2; 116.9 (2 and 10 Hz); 125.4; 132.9; 135.2; 135.7; 143.0 (4 and 11 Hz); 145.4; 151.8; 151.9 (12 and 242 Hz); 154.3; 157.2 (10 and 242 Hz). HRMS: Calc. for C₂₁H₂₁F₂N₃O₂ + H: 386.1680; Found: 386.1667.

**5-cyclopropyl-2-(4-(2,5-difluorophenoxy)-3-isopropoxy-5-methyl-1H-pyrazol-1-yl)pyridine (example 18):** Obtained in 55 % yield as an oil, using 5-cyclopropyl-2-fluoropyridine (intermediate 50) in acetonitrile at 180 °C for 6 hours and after a chromatography over silica gel (cyclohexane/ethyl acetate 97/3) followed by concentration under high vacuum. ¹H (CDCl₃): 0.72 (m, 2H); 1.04 (m, 2H); 1.35 (d, 6H, J = 6.2); 1.93 (m, 1H); 2.55 (s, 3H); 5.00 (sept, 1H, J = 6.2); 6.67 (m, 2H); 7.08 (m, 1H); 7.42 (dd, 1H, J 2.3 and 8.5); 7.68 (d, 1H, J = 8.5); 8.19 (d, 1H, J = 2.3). ¹³C (CDCl₃): 8.7; 11.8; 12.5; 22.1; 72.2; 104.8 (28 Hz); 108.2 (6 and 24 Hz); 114.2; 116.9 (10 and 20 Hz); 124.5; 133.1; 135.2; 135.8; 145.4; 147.1 (22 Hz); 148.5 (4 and 243 Hz); 151.8; 154.2; 158.6 (3 and 242 Hz). HRMS: Calc. for C₂₁H₂₁F₂N₃O₂ + H: 386.1680; Found: 386.1648.

**2-(4-(2,5-difluorophenoxy)-3-isopropoxy-5-methyl-1H-pyrazol-1-yl)-5-ethylpyrimidine (example 19):** Obtained in 71 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 1.29 (t, 3H, J = 7.6); 1.32 (d, 6H, J = 6.1); 2.55 (s, 3H); 2.67 (q, 2H, J = 7.6); 5.22 (sept, 1H, J = 6.1); 6.66 (m, 2H); 7.06 (m, 1H); 8.57 (s, 2H). ¹³C (CDCl₃): 12.4; 15.0; 22.1; 23.0; 72.2; 104.1 (28 Hz); 108.4 (24 and 7 Hz); 116.6 (21 and 10 Hz); 125.9; 132.6; 134.5; 146.7 (12 and 10 Hz); 148.5 (242 and 3 Hz); 155.6; 156.0; 157.7; 158.6 (243 and 2 Hz). HRMS: Calc. for C₁₉H₂₀F₂N₄O₂ + H: 375.1633; Found: 375.1643.

**2-(4-benzyl-3-isopropoxy-5-methyl-1H-pyrazol-1-yl)-5-cyclopropylpyridine** (example 20): Obtained in 66 % yield, using 5-cyclopropyl-2-fluoropyridine (intermediate 50) in acetonitrile at 180 °C for 5 hours and after a chromatography over silica gel (cyclohexane/ethyl acetate 97/3) followed by concentration under high vacuum as an oil. ¹H (CDCl₃): 0.71 (m, 2H); 1.02 (m, 2H); 1.36 (d, 6H, J = 6.0); 1.91 (m, 1H); 2.54 (s, 3H); 3.73 (s, 2H); 5.00 (sept, 1H, J = 6.0); 7.16 (m, 1H); 7.26 (m, 4H); 7.37 (m, 1H); 7.63 (m, 1H); 8.18 (d, 1H, J = 2.3). ¹³C (CDCl₃): 8.6; 12.5; 12.9; 22.2; 27.8; 71.2; 107.1; 114.9; 125.6; 128.2; 128.3; 135.0; 135.2; 138.9; 141.2; 145.4; 152.0; 161.5. HRMS: Calc. for C₂₂H₂₅N₃O + H: 348.2076; Found: 348.2033.

**2-(4-benzyl-3-isopropoxy-5-methyl-1H-pyrazol-1-yl)-5-cyclopropylpyrimidine (example 21):** Obtained in 46 % yield, using 2-chloro-5-cyclopropylpyrimidine (intermediate 49) in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as an oil. ¹H (CDCl₃): 0.75 (m, 2H); 1.06 (m, 2H); 1.34 (d, 6H, J = 6.0); 1.87 (m, 1H); 2.53 (s, 3H); 3.73 (s, 2H); 5.22 (sept, 1H, J = 6.0); 7.26 (m, 1H); 7.24 (m, 4H); 8.44 (s, 2H). ¹³C (CDCl₃): 8.3; 10.4; 13.7; 22.3; 27.8; 71.2; 109.1; 125.7; 128.2; 128.3; 131.9; 140.3; 140.8; 156.0; 156.2; 162.6. HRMS: Calc. for C₂₁H₂₄N₄O + H: 349.2028; Found: 349.2009.

**2-(4-benzyl-5-isopropoxy-3-methyl-1H-pyrazol-1-yl)-5-cyclopropylpyrimidine (example 22):** On a large scale (20 mmol), this isomer was also isolated from the reaction described above in a 1.5 % yield after a second chromatography over alumina containing 1.5 % of water (cyclohexane-dichloromethane 1/1) as a wax. ¹H (CDCl₃): 0.79 (m, 2H); 1.11 (m, 2H); 1.28 (d, 6H, J = 6.2); 1.89 (m, 1H); 2.13 (s, 3H); 3.78 (s, 2H); 4.40 (sept, 1H, J = 6.2); 7.18-7.28 (m, 5H); 8.50 (s, 2H). ¹³C (CDCl₃): 8.6; 10.5; 13.6; 22.1; 28.5; 78.6; 108.1; 126.0; 128.2; 128.3; 133.4; 139.8; 151.0; 151.4; 154.8; 156.3. HRMS: Calc. for C₂₁H₂₄N₄O + H: 349.2028; Found: 349.1977.

**5-ethyl-2-(4-(2-fluorobenzyl)-3-isopropoxy-5-methyl-1H-pyrazol-1-yl)pyrimidine (example 23):** Obtained in 46 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as an oil. ¹H (CDCl₃): 1.27 (t, 3H, J = 7.6); 1.32 (d, 6H, J = 6.2); 2.56 (s, 3H); 2.64 (q, 2H, J = 7.6); 3.74 (s, 2H); 5.21 (sept, 1H, J = 6.2); 6.99 (m, 2H); 7.14 (m, 1H); 7.28 (m, 1H); 8.54 (s, 2H). ¹³C (CDCl₃):13.5; 15.1; 20.6; 22.2; 23.0; 71.2; 107.6; 114.8 (22 Hz); 123.7; 127.2; 127.4; 130.6; 131.9; 140.6; 156.2; 157.6; 160.8 (245 Hz); 162.6. HRMS: Calc. for C₂₀H₂₃FN₄O + H: 355.1934; Found: 355.1921.

**5-ethyl-2-(4-(4-fluorobenzyl)-3-isopropoxy-5-methyl-1H-pyrazol-1-yl)pyrimidine (example 24):** Obtained in 43 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as an oil. ¹H (CDCl₃): 1.28 (t, 3H, J = 7.6); 1.33 (d, 6H, J = 6.1); 2.54 (s, 3H); 2.64 (q, 2H, J = 7.6); 3.68 (s, 2H); 5.21 (sept, 1H, J = 6.1); 6.93 (m, 2H); 7.18 (m, 2H); 8.54 (s, 2H). ¹³C (CDCl₃): 13.6; 15.0; 22.2; 23.0; 27.1; 71.2; 109.0; 114.8 (21 Hz); 129.6; 131.9; 136.4; 140.2; 156.2; 157.6; 161.3 (243 Hz); 162.5. HRMS: Calc. for C₂₀H₂₃FN₄O + H: 355.1934; Found: 355.1900.

**2-(4-benzyl-3-ethoxy-5-methyl-1H-pyrazol-1-yl)-5-ethylpyrimidine (example 25):**

Obtained on a larger scale (6.9 mmol) in 65 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 1.29 (t, 3H, J = 7.3); 1.39 (t, 3H, J = 7.0); 2.56 (s, 3H); 2.66 (q, 2H, J = 7.3); 3.76 (s, 2H); 4.45 (q, 2H, J = 7.0); 7.25 (m, 5H); 8.57 (s, 2H). ¹³C (CDCl₃): 13.8; 14.8; 15.1; 23.0; 27.7; 64.4; 108.3; 125.8; 128.2; 128.3; 131.9; 140.5; 140.6; 156.1; 157.6; 163.4. HRMS: Calc. for C₁₉H₂₂N₄O + H: 323.1872; Found: 323.1581.

**2-(4-benzyl-5-ethoxy-3-methyl-1R-pyrazol-1-yl)-5-ethylpyrimidine (example 26**)**:** This isomer was obtained in the experiment described above in 2% yield after an additional purification (over silica gel (cyclohexane-ethyl acetate 3/1) of the corresponding fraction as an oil. ¹H (CDCl₃): 1.31 (m, 6H); 2.19 (s, 3H); 2.68 (q, 2H, J = 7.4); 3.79 (s, 2H); 4.12 (q, 2H, J = 7.0); 7.26 (m, 5H); 8.61 (s, 2H). ¹³C (CDCl₃): 13.4; 15.0; 15.2; 23.1; 28.2; 71.7; 107.4; 126.0; 128.2; 128.4; 133.2; 139.9; 150.9; 152.8; 154.9; 157.9. HRMS: Calc. for C₁₉H₂₂N₄O + H: 323.1872; Found: 323.1819.

**2-(4-benzyl-3-ethoxy-5-methyl-1H-pyrazol-1-yl)-5-propylpyrimidine (example 27):** Obtained in 45 % yield, using 2-chloro-5-propylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 2/1) as an oil. ¹H (CDCl₃): 0.98 (t, 3H, J = 7.3); 1.38 (t, 3H, J = 7.1); 1.66 (m, 2H); 2.56 (s, 3H); 2.58 (q, 2H, J = 7.4); 3.76 (s, 2H); 4.45 (q, 2H, J = 7.1); 7.17 (m, 1H); 7.24 (m, 4H); 8.53 (s, 2H). ¹³C (CDCl₃): 13.4; 13.8; 14.8; 20.1; 27.7; 31.7; 64.5; 108.4; 125.8; 128.2; 128.3; 130.4; 140.5; 140.7; 156.0; 158.0; 163.4. HRMS: Calc. for C₂₀H₂₄N₄O + H: 337.2028; Found: 337.1985.

**2-(4-benzyl-3-ethoxy-5-methyl-1H-pyrazol-1-yl)-5-pentylpyrimidine (example 28):** Obtained in 52 % yield, using 2-chloro-5-propylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 2/1) as an oil. ¹H (CDCl₃): 0.91 (t, 3H, J = 7.3); 1.35 (m, 7H); 1.63 (m, 2H); 2.57 (s, 3H); 2.59 (q, 2H, J = 7.4); 3.76 (s, 2H); 4.45 (q, 2H, J = 7.0); 7.18 (m, 1H); 7.25 (m, 4H); 8.53 (s, 2H). ¹³C (CDCl₃): 13.8; 14.0; 14.8; 22.4; 27.7; 29.7; 30.5; 31.0; 64.5; 108.4; 125.8; 128.2; 128.3; 130.6; 140.5; 140.7; 155.9; 158.0; 163.4. HRMS: Calc. for C₂₂H₂₈N₄O + H: 365.2341; Found: 365.2336.

**2-(4-benzyl-3-ethoxy-5-methyl-1H-pyrazol-1-yl)-5-cyclopropylpyrimidine (example 29):** Obtained in 64 % yield, using 2-chloro-5-cyclopropylpyrimidine (intermediate 49) in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as an oil. ¹H (CDCl₃): 0.74 (m, 2H); 1.06 (m, 2H); 1.38 (t, 3H, J = 7.1); 1.87 (m, 1H); 2.54 (s, 3H); 3.75 (s, 2H); 4.44 (q, 2H, J = 7.1); 7.17 (m, 1H); 7.27 (m, 4H); 8.45 (s, 2H). ¹³C (CDCl₃): 8.3; 10.4; 13.7; 14.8; 27.7; 64.4; 108.3; 125.8; 128.2; 128.3; 132.0; 140.5; 140.6; 155.9; 156.2; 163.4. HRMS: Calc. for C₂₀H₂₂N₄O + H: 335.1872; Found: 335.1859.

**2-(4-benzyl-3-ethoxy-5-methyl-1H-pyrazol-1-yl)pyridine (example 30):** Prepared as previously described (Guillou et al. *Tetrahedron,* **2010,** *66*, 2654)*.*

**2-(4-benzyl-3-ethoxy-5-methyl-1H-pyrazol-1-yl)-5-methylpyridine (example 31):** Obtained in 32 % yield, using 2-fluoro-5-methylpyridine in acetonitrile at 180 °C for two hours and after a chromatography over silica gel (dichloromethane) as an oil. ¹H NMR (CDCl₃): 1.40 (t, 3H, *J=* 7.0 Hz); 2.34 (s, 3H); 2.54 (s, 3H); 3.75 (s, 2H); 4.34 (q, 2H, *J=* 7.0 Hz); 7.17 (m, 1H); 7.27 (m, 4H); 7.54 (m, 1H); 7.64 (m, 1H); 8.19 (m, 1H). ¹³C NMR (CDCl₃): 12.9; 14.9; 17.8; 27.8; 64.2; 106.3; 114.9; 125.7; 128.2 (two signals); 129.1; 138.7; 139.1; 141.0; 147.2; 151.9; 162.2. HRMS: Calc. for C₁₉H₂₁N₃O + H: 308.1763; Found: 308.1751.

**2-(4-benzyl-3-ethoxy-5-methyl-1H-pyrazol-1-yl)-4-methylpyridine (example 32):** Obtained in 57 % yield, using 2-fluoro-4-methylpyridine in acetonitrile at 180 °C for two hours and after a chromatography over silica gel (dichloromethane) as a oil. ¹H NMR (CDCl₃): 1.41 (t, 3H, *J=* 7.0 Hz); 2.41 (s, 3H); 2.56 (s, 3H); 3.75 (s, 2H); 4.36 (q, 2H, *J=* 7.0 Hz); 6.90 (m, 1H); 7.18 (m, 1H); 7.28 (m, 4H); 7.59 (m, 1H); 8.22 (m, 1H). ¹³C NMR (CDCl₃): 13.2; 14.9; 21.2; 27.8; 64.2; 106.7; 115.6; 121.0; 125.7; 128.2; 128.3; 139.5; 141.0; 147.0; 149.3; 154.0; 162.3. HRMS: Calc. for C₁₉H₂₁N₃O + H: 308.1763; Found: 308.1718.

**2-(4-benzyl-3-ethoxy-5-methyl-1H-pyrazol-1-yl)-3-methylpyridine (example 33):** Obtained in 39 % yield, using 2-fluoro-3-methylpyridine in acetonitrile at 180 °C for two hours and after a chromatography over silica gel (dichloromethane) as a oil. ¹H NMR (CDCl₃): 1.41 (t, 3H, *J*= 7.0 Hz); 2.52 (s, 3H); 2.59 (s, 3H); 3.76 (s, 2H); 4.35 (q, 2H, *J=* 7.0 Hz); 6.92 (m, 1H); 7.19 (m, 1H); 7.28 (m, 4H); 7.59 (m, 2H). ¹³C NMR (CDCl₃): 13.3; 14.9; 24.1; 27.8; 64.2; 106.6; 111.8; 118.9; 125.7; 128.2; 128.3; 138.2; 139.4; 141.1; 153.3; 156.4; 162.2. HRMS: Calc. for C₁₉H₂₁N₃O+H: 308.1763; Found: 308.1747.

**2-(6-(4-benzyl-3-ethoxy-5-methyl-1H-pyrazol-1-yl)pyridin-3-yl)propan-2-ol (example 34):** Obtained in 67 % yield as an oil, using 2-(6-fluoropyridin-3-yl)propan-2-ol (intermediate 55) at 180 °C for 6 hours in acetonitrile and after a chromatography over silica gel (cyclohexane/ethyl acetate 4/1). ¹H (CDCl₃): 1.41 (t, 3H, J = 7.1); 1.60 (s, 6H); 2.12 (s, 1H); 2.57 (s, 3H); 3.76 (s, 2H); 4.36 (q, 2H, J = 7.1); 7.25 (m, 1H); 7.28 (m, 4H); 7.71 (dd, 1H, J = 0.8 and 8.7 Hz); 7.85 (dd, 1H, J = 2.5 and 8.7 Hz); 8.47 (dd, 1H, J = 0.8 and 2.5 Hz). ¹³C (CDCl₃): 13.1; 14.9; 27.7; 31.6; 64.2; 71.2; 106.7; 114.6; 125.8; 128.2; 128.3; 134.8; 139.4; 140.2; 140.9; 143.7; 152.6; 163.2. HRMS: Calc. for C₂₁H₂₅N₃O₂ + H: 352.2520; Found: 352.2022.

**2-(4-benzyl-3-ethoxy-5-methyl-1H-pyrazol-1-yl)-5-cyclopropyl-3-fluoropyridine (example 35):** Obtained in 17 % yield as an oil using 5-cyclopropyl-2,3-difluoropyridine (intermediate 51) at 140 °C for 4 hours in acetonitrile after a chromatography over silica gel (cyclohexane/ethyl acetate from 97/3 to 4/1) followed by concentration under high vacuum. ¹H (CDCl₃): 0.76 (m, 2H); 1.11 (m, 2H); 1.38 (t, 3H, J = 7.2); 1.98 (m, 1H); 2.14 (s, 3H); 3.76 (s, 2H); 4.33 (q, 2H, J = 7.2); 7.18 (m, 2H); 7.29 (m, 4H); 8.18 (d, 1H, J = 1.7). ¹³C (CDCl₃): 9.6; 10.5 (2 Hz); 12.6; 14.9; 27.9; 64.9; 105.0; 121.8 (19 Hz); 125.7; 128.2; 128.3; 138.3 (11 Hz); 139.4; 140.8; 141.6 (6 Hz); 142.4 (5 Hz); 153.0 (260 Hz); 162.9. HRMS: Calc. for C₂₁H₂₂FN₃O + H: 352.1825; Found: 352.1827.

**2-(4-benzyl-3-ethoxy-5-methyl-1H-pyrazol-1-yl)-5-cyclopropylnicotinonitrile (example 36):** Obtained in 18 % yield as a solid using 2-chloro-5-cyclopropylnicotinonitrile (intermediate 52) at 160 °C for 4 hours in acetonitrile after a chromatography over silica gel (cyclohexane/dichloromethane from 2/1 to 0/1). ¹H (CDCl₃): 0.76 (m, 2H); 1.11 (m, 2H); 1.43 (t, 3H, J = 7.2); 1.92 (m, 1H); 2.47 (s, 3H); 3.74 (s, 2H); 4.41 (q, 2H, J = 7.2); 7.18 (m, 1H); 7.27 (m, 4H); 7.67 (d, 1H, J = 2.5); 8.34 (d, 1H, J = 2.5). ¹³C (CDCl₃): 9.1; 12.2; 12.6; 14.8; 27.7; 64.9; 101.3; 108.1; 116.8; 125.9; 128.2; 128.3; 135.7; 139.5; 140.4; 140.9; 149.3; 150.7; 162.4. HRMS: Calc. for C₂₂H₂₂N₄O + H: 359.1872; Found: 359.1841.

**2-(4-benzyl-3-ethoxy-5-methyl-1R-pyrazol-1-yl)-3-fluoropyridine (example 37):** Obtained in 74 % yield, using 2,3-difluoropyridine in acetonitrile at 180 °C for two hours and after a chromatography over silica gel (dichloromethane - ethanol from 100/0 to 98/2) as an oil. ¹H NMR (CDCl₃): 1.40 (t, 3H, *J=* 7.1 Hz); 2.20 (s, 3H); 3.77 (s, 2H); 4.35 (q, 2H, *J=* 7.1 Hz); 7.19 (m, 1H); 7.28 (m, 5H); 7.57 (m, 1H); 8.37 (m, 1H). ¹³C NMR (CDCl₃): 10.7; 14.9; 27.9; 64.3; 105.6; 123.7; 125.5 (J = 18 Hz); 125.8; 128.3; 128.32; 139.5; 140.7; 141.1; 144.1; 152.8 (J = 273 Hz); 163.1. HRMS: Calc. for C₁₈H₁₈FN₃O + H: 312.1512; Found: 312.1503.

**2-(4-benzyl-3-ethoxy-5-methyl-1H-pyrazol-1-yl)-5-(trifluoromethyl)pyridine (example 38):** Obtained in 47 % yield, using 2-chloro-5-(trifluoromethyl)pyridine in acetonitrile at 180 °C for two hours and after a chromatography over silica gel (cyclohexane-dichloromethane 4/1 to 2/1) as a white powder. ¹H NMR (CDCl₃): 1.43 (t, 3H, *J* = 7.1 Hz); 2.65 (s, 3H); 3.75 (s, 2H); 4.37 (q, 2H, *J*= 7.1 Hz); 7.22 (m, 1H); 7.28 (m, 4H); 7.93 (m, 2H); 8.61 (m, 1H). ¹³C NMR (CDCl₃): 13.9; 14.8; 27.6; 64.3; 108.7; 113.6; 121.7 (J = 33 Hz); 123.9 (J = 271 Hz); 125.9; 128.2; 128.4; 135.0; 140.4; 140.5; 144.6; 156.2; 163.0. HRMS: Calc. for C₁₉H₁₈F₃N₃O + H: 362.1480; Found: 362.1449.

**2-(4-benzyl-3-ethoxy-5-methyl-1H-pyrazol-1-yl)-5-chloropyridine (example 39):** Obtained in 40 % yield, using 2,5-dichloropyridine in acetonitrile at 180 °C for two hours and after a chromatography over silica gel (cyclohexane - dichloromethane 4/1) as a white powder. ¹H NMR (CDCl₃): 1.42 (t, 3H, *J*= 7.1 Hz); 2.57 (s, 3H); 3.74 (s, 2H); 4.34 (q, 2H, *J* = 7.1 Hz); 7.19 (m, 1H); 7.27 (m, 4H); 7.68 (dd, 1H, J = 2.5 and 8.8 Hz); 7.76 (d, 1H, J = 8.8 Hz); 8.30 (d, 1H, J = 2.5). ¹³C NMR (CDCl₃): 13.4; 14.8; 27.7; 64.2; 107.5; 115.5; 125.8; 126.9; 128.2; 128.3; 137.7; 139.7; 140.7; 145.7; 152.2; 162.5. HRMS: Calc. for C₁₈H₁₈ClN₃O + H: 328.1217; Found: 328.1186.

**2-(4-benzyl-3-ethoxy-5-methyl-1H-pyrazol-1-yl)-5-bromo-3-fluoropyridine (example 40):** Obtained in a 33 % yield using 5-bromo-2,3-difluoropyridine in acetonitrile at 180 °C for 2 hours and after a chromatography over silica gel (cyclohexane/ethyl acetate from 97/3) as an oil. ¹H (CDCl₃): 1.40 (t, 3H, J = 7.1); 2.22 (s, 3H); 3.76 (s, 2H); 4.34 (q, 2H, J = 7.1); 7.20 (m, 1H); 7.28 (m, 4H); 7.74 (m, 1H); 8.40 (d, 1H, J = 2.0). ¹³C (CDCl₃): 10.9; 14.8; 27.9; 64.3; 106.3; 117.1; 125.9; 128.3; 128.31; 128.5 (21 Hz); 139.6; 140.0; 140.5; 144.8; 151.9 (270 Hz); 163.2. HRMS: Calc. for C₁₈H₁₇⁷⁹BrFF₃O + H: 390.0617; Found: 390.0621.

**2-(4-benzyl-3-ethoxy-5-methyl-1H-pyrazol-1-yl)-5-bromopyridine (example 41):** Obtained in 44 % yield, using 5-bromo-2-fluoropyridine in dimethylformamide at 130 °C for 12 hours and after a chromatography over silica gel (cyclohexane/dichloromethane 2/1) followed by concentration under high vacuum as a solid. ¹H (CDCl₃): 1.41 (t, 3H, J = 7.0); 2.57 (s, 3H); 3.74 (s, 2H); 4.34 (q, 2H, J = 7.0); 7.19 (m, 1H); 7.28 (m, 4H); 7.71 (m, 1H); 7.81 (m, 1H); 8.38 (m, 1H). ¹³C (CDCl₃): 13.4; 14.8; 27.7; 64.3; 107.6; 115.0; 116.0; 125.8; 128.2; 128.3; 139.7; 140.5; 140.7; 147.9; 152.6; 162.5. HRMS: Calc. for C₁₈H₁₈BrN₃O + H: 372.0711; Found: 372.0684.

**2-(4-benzyl-3-ethoxy-5-methyl-1H-pyrazol-1-yl)-5-bromopyrimidine (example 42):** Obtained in 58 % yield on a large scale (13 mmol) using 2-chloro-5-bromopyrimidine in dimethylformamide at 150 °C for one hour and a chromatography over silica gel (cyclohexane-dichloromethane 1/2) as a solid. ¹H (CDCl₃): 1.40 (t, 3H, J = 7.0); 2.56 (s, 3H); 3.75 (s, 2H); 4.44 (q, 2H, J = 7.0); 7.24 (m, 5H); 8.71 (s, 2H). ¹³C (CDCl₃): 14.1; 14.7; 27.7; 64.6; 109.7; 114.0; 125.9; 128.2; 128.3; 140.2; 141.2; 155.8; 158.7; 163.9. HRMS: Calc. for C₁₇H₁₇BrN₄O + H: 373.0664; Found: 373.0788.

**2-(4-benzyl-5-ethoxy-3-methyl-1H-pyrazol-1-yl)-5-bromopyrimidine (example 43):** From the reaction described above, further purification of the corresponding chromatographic fraction (two consecutive chromatography, the first one over alumina containing 1.5 % of water (cyclohexane-dichloromethane 1/2) the second over alumina containing 1.5 % of water (cyclohexane-ethyl acetate from 4/1 to 2/1)), this isomer was obtained in 1 % yield as a solid. ¹H (CDCl₃): 1.35 (t, 3H, J = 7.0); 2.16 (s, 3H); 3.79 (s, 2H); 4.11 (q, 2H, J = 7.0); 7.21 (m, 3H); 7.28 (m, 2H); 8.79 (s, 2H). ¹³C (CDCl₃): 13.5; 15.2; 28.2; 71.9; 108.2; 115.5; 126.2; 128.1; 128.5; 139.6; 152.1; 153.1; 154.6; 159.1. HRMS: Calc. for C₁₇H₁₇BrN₄O + H: 373.0664; Found: 373.0568.

**4-benzyl-1,2-bis(5-bromopyrimidin-2-yl)-5-methyl-1H-pyrazol-3(2H)-one (example 44):** From the purification procedure described above this bis adduct was also obtained in 0.8 % yield as a solid. ¹H (CDCl₃): 2.68 (s, 3H); 3.73 (s, 2H); 7.16 (m, 5H); 8.43 (s, 2H); 8.76 (s, 2H). ¹³C (CDCl₃):14.2; 28.2; 112.7; 113.7; 115.8; 126.1; 128.3; 128.4; 138.8; 142.0; 155.7; 158.1; 158.9; 159.9; 162.9. HRMS: Calc. for C₁₉H₁₄Br₂N₆O + H: 500.9674; Found: 500.9636.

**2-cyclopropyl-5-(4-(2,6-difluorophenoxy)-3-isopropoxy-5-methyl-1H-pyrazol-1-yl)pyrazine (example 45):** Obtained in 45 % yield as an oil using 2-bromo-5-cyclopropylpyrazine (intermediate 53) in acetonitrile at 160 °C for two hours and a chromatography over silica gel (cyclohexane-dichloromethane from 3/2 to 1/2). ¹H (CDCl₃): 1.04 (m, 4H); 1.25 (d, 6H, J = 6.2); 2.07 (m, 1H); 2.61 (s, 3H); 4.88 (sept, 1H, J = 6.2); 6.91 (m, 2H); 7.02 (m, 1H); 8.17 (d, 1H, J = 1.4); 8.90 (d, 1H, J = 1.4). ¹³C (CDCl₃): 9.6; 11.4; 14.1; 28.1; 72.1; 111.9 (6 and 17 Hz); 123.5 (9 Hz); 129.5; 131.2; 134.5 (14 Hz); 136.4; 138.7; 147.8; 153.2; 154.1; 155.6 (4 and 250 Hz). HRMS: Calc. for C₂₀H₂₀F₂N₄O₂ + H: 387.1633; Found: 387.1718.

**5-cyclopropyl-2-(4-(2,6-difluorophenoxy)-3-isopropoxy-5-methyl-1H-pyrazol-1-yl)nicotinonitrile (example 46**)**:** Obtained in 96 % yield, using 2-chloro-5-cyclopropylnicotinonitrile (intermediate 52) in acetonitrile at 150 °C for 40 minutes and after a chromatography over silica gel (cyclohexane-ethyl acetate 97/3) as a white solid. ¹H (CDCl₃): 0.76 (m, 2H); 1.12 (m, 2H); 1.28 (d, 6H, J = 6.2); 1.94 (m, 1H); 2.58 (s, 3H); 4.94 (sept, 1H, J = 6.2); 6.94 (m, 2H); 7.05 (m, 1H); 7.67 (d, 1H, J = 2.4); 8.34 (d, 1H, J = 2.4). ¹³C (CDCl₃): 9.0; 11.4; 12.2; 21.8; 72.8; 100.6; 111.9 (6 and 16 Hz); 116.7; 123.7 (9 Hz); 130.0; 131.1; 134.3 (14 Hz); 135.7; 141.1; 149.3; 152.7; 153.9; 155.7 (4 and 249 Hz). HRMS: Calc. for C₂₂H₂₀F₂N₄O₂ + H: 411.1633; Found: 411.1630.

**2-(4-(2,6-difluorophenoxy)-3-isopropoxy-5-methyl-1H-pyrazol-1-yl)-5-methylpyrimidine (example 47):** Obtained in 77 % yield as a solid, using 2-chloro-5-methylpyrimidine in acetonitrile at 160 °C for 1 hour after a chromatography over silica gel (cyclohexane-ethyl acetate 3/2). ¹H (CDCl₃): 1.19 (d, 6H, J = 6.2); 2.29 (s, 3H); 2.64 (s, 3H); 5.09 (sept, 1H, J = 6.2); 6.87 (m, 2H); 7.01 (m, 1H); 8.51 (s, 2H). ¹³C (CDCl₃): 12.2; 14.9; 21.9; 71.9; 111.9 (6 and 17 Hz); 123.5 (9 Hz); 126.1; 130.2; 132.2; 134.5 (14 Hz); 154.9; 155.6 (250 and 4 Hz); 156.1; 158.2. HRMS: Calc. for C₁₈H₁₈F₂N₄O₂ + H: 361.1476; Found: 361.1467.

**2-bromo-5-(4-(2,6-difluorophenoxy)-3-isopropoxy-5-methyl-1H-pyrazol-1-yl)pyrazine (example 48):** Obtained in 59 % yield as a white powder using 2,5-dibromopyrazine in acetonitrile at 120 °C for five hours and after a chromatography over silica gel (cyclohexane-dichloromethane from 2/1 to 1/1). ¹H (CDCl₃): 1.26 (d, 6H, J = 6.2); 2.64 (s, 3H); 4.91 (sept, 1H, J = 6.2); 6.91 (m, 2H); 7.05 (m, 1H); 8.35 (d, 1H, J = 1.3); 8.87 (d, 1H, J = 1.3). ¹³C (CDCl₃): 11.8; 21.8; 72.4; 111.9 (6 and 17 Hz); 123.8 (9 Hz); 130.4; 131.8; 133.4; 134.2 (13 Hz); 134.6; 143.1; 149.1; 154.9; 155.6 (4 and 250 Hz). HRMS: Calc. for C₁₇H₁₅BrF₂N₄O₂ + H: 425.0425; Found: 425.0418.

**5-cyclopropyl-2-(4-(2,3-dichlorophenoxy)-3-ethoxy-5-methyl-1H-pyrazol-1-yl)pyridine (example 49):** Obtained in 50 % yield as a solid using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.72 (m, 2H); 1.03 (m, 2H); 1.37 (t, 3H, J = 7.2); 1.93 (m, 1H); 2.52 (s, 3H); 4.34 (q, 2H, J = 7.2); 6.82 (dd, 1H, J = 1.5 and 8.1); 7.08 (t, 1H, J = 8.1); 7.15 (dd, 1H, = 1.5 and 8.1); 7.40 (dd, 1H, J = 2.5 and 8.5); 7.68 (d, 1H, J = 8.5); 8.20 (d, 1H, J = 2.5). ¹³C (CDCl₃): 8.7; 11.8; 12.5; 14.8; 64.9; 112.9; 114.2; 121.5; 123.6; 124.3; 127.1; 133.3; 133.9; 135.2; 135.9; 145.5; 151.7; 154.9; 155.5. HRMS: Calc. for C₂₀H₁₉Cl₂N₃O₂ + H: 404.0933; Found: 404.0918.

**5-ethyl-2-(4-(2-fluorophenoxy)-3-isopropoxy-5-methyl-1H-pyrazol-1-yl)pyrimidine (example 50):** Obtained in 55 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 1.29 (t, 3H, J = 7.6); 1.31 (d, 6H, J = 6.1); 2.55 (s, 3H); 2.66 (q, 2H, J = 7.6); 5.20 (sept, 1H, J = 6.1); 6.96 (m, 3H); 7.13 (m, 1H); 8.56 (s, 2H). ¹³C (CDCl₃): 12.4; 15.0; 22.1; 23.0; 72.1; 116.3; 116.4 (18 Hz); 122.5; 124.0; 126.5; 132.4; 132.4; 146.2; 152.2 (246 Hz); 156.0; 156.1; 157.7. HRMS: Calc. for C₁₉H₂₁FN₄O₂ + H: 357.1727; Found: 357.1715.

**5-cyclopropyl-2-(4-(2-fluorophenoxy)-3-isopropoxy-5-methyl-1H-pyrazol-1-yl)pyrimidine (example 51):** Obtained in 68 % yield, using 2-chloro-5-cyclopropylpyrimidine (intermediate 49) in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 0.75 (m, 2H); 1.08 (m, 2H); 1.29 (d, 6H, J = 6.1); 1.87 (m, 1H); 2.53 (s, 3H); 5.19 (sept, 1H, J = 6.1); 6.92 (m, 3H); 7.13 (m, 1H); 8.45 (s, 2H). ¹³C (CDCl₃): 8.3; 10.4; 12.4; 22.1; 72.1; 116.3; 116.5; 122.5 (7 Hz); 124.0; 126.4; 132.5; 134.3; 146.2 (11 Hz); 152.3 (247 Hz); 155.8; 156.0; 156.3. HRMS: Calc. for C₂₀H₂₁FN₄O₂ + H: 369.1727; Found: 369.1704.

**5-cyclopropyl-2-(4-(2-fluorophenoxy)-3-isopropoxy-5-methyl-1H-pyrazol-1-yl)nicotinonitrile (example 52):** Obtained in 80 % yield, using 2-chloro-5-cyclopropylnicotinonitrile (intermediate 52) in acetonitrile at 150 °C for 2 hours and after a chromatography over silica gel (cyclohexane-ethyl acetate 93/7) as a white solid. ¹H (CDCl₃): 0.77 (m, 2H); 1.12 (m, 2H); 1.37 (d, 6H, J = 6.1); 1.95 (m, 1H); 2.49 (s, 3H); 5.07 (sept, 1H, J = 6.1); 6.99 (m, 3H); 7.12 (m, 1H); 7.70 (d, 1H, J = 2.4); 8.35 (d, 1H, J = 2.4). ¹³C (CDCl₃): 9.1; 11.5; 12.2; 20.0; 73.0; 100.7; 116.4; 116.5; 116.6 (8 Hz); 122.7 (6 Hz); 124.1 (4 Hz); 126.5; 133.3; 136.0; 141.1; 146.0 (10 Hz); 149.3; 150.6; 152.2 (247 Hz); 155.0. HRMS: Calc. for C₂₂H₂₁FN₄O₂ + H: 393.1727; Found: 393.1725.

**5-cyclopropyl-3-fluoro-2-(4-(2-fluorophenoxy)-3-isopropoxy-5-methyl-1H-pyrazol**-**1**-**yl)pyridine (example 53):** Obtained in 47 % yield, using 5-cyclopropyl-2,3-difluoropyridine (intermediate 51) in acetonitrile at 180 °C for 3 hours and after a chromatography over silica gel (cyclohexane/ethyl acetate 7/1) as a solid. ¹H (CDCl₃): 0.79 (m, 2H); 1.14 (m, 2H); 1.32 (d, 6H, J = 6.2); 1.99 (m, 1H); 2.19 (s, 3H); 4.97 (sept, 1H, J = 6.2); 6.98 (m, 3H); 7.14 (m, 2H); 8.19 (d, 1H, J = 1.9). ¹³C (CDCl₃): 9.3; 9.7; 12.6; 22.1; 72.1; 116.4 (18 Hz); 116.5; 122.0 (18 Hz); 122.4 (7 Hz); 124.0; 124.4; 133.0; 138.0; 142.0; 142.4; 146.3 (11 Hz); 152.2 (247 Hz); 153.0 (261 Hz); 155.2. HRMS: Calc. for C₂₁H₂₁F₂N₃O₂ + H: 386.1680; Found: 386.1660.

**5-ethyl-2-(4-(3-fluorophenoxy)-3-isopropoxy-5-methyl-1H-pyrazol-1-yl)pyrimidine (example 54):** Obtained in 78 % yield as a solid, using 2-chloro-5-ethylpyrimidine in acetonitrile at 160 °C for one hour and after a chromatography over silica gel (cyclohexane-ethyl acetate 4/1). ¹H (CDCl₃): 1.30 (m, 9H); 2.52 (s, 3H); 2.66 (q, 2H, J = 7.6); 5.22 (sept, 1H, J = 6.1); 6.70 (m, 1H); 6.77 (m, 1H); 7.23 (m, 1H); 8.56 (s, 2H). ¹³C (CDCl₃): 12.5; 15.1; 22.1; 23.0; 72.1; 103.1 (25 Hz); 108.9 (21 Hz); 110.9 (3 Hz); 126.1; 130.1 (10 Hz); 132.4; 134.6; 156.0; 156.1; 157.7; 159.7 (10 Hz); 163.5 (245 Hz). HRMS: Calc. for C₁₉H₂₁FN₄O₂ + H: 357.1727; Found: 357.1687.

**2-(4-(2-bromophenoxy)-3-ethoxy-5-methyl-1H-pyrazol-1-yl)-5-cyclopropylpyridine (example 55):** Obtained in 50 % yield as an oil using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.72 (m, 2H); 1.03 (m, 2H); 1.37 (t, 3H, J = 7.2); 1.92 (m, 1H); 2.53 (s, 3H); 4.35 (q, 2H, J = 7.2); 6.89 (m, 2H); 7.19 (m, 1H); 7.41 (dd, 1H, = 2.5 and 8.5); 7.59 (dd, 1H, J = 1.6 and 7.8); 7.68 (d, 1H, J = 8.5); 8.19 (d, 1H, J = 2.5). ¹³C (CDCl₃): 8.7; 11.9; 12.6; 14.8; 64.8; 111.2; 114.1; 114.9; 123.2; 124.6; 128.3; 133.3; 133.4; 135.2; 135.8; 145.5; 151.8; 155.1; 155.2. HRMS: Calc. for C₂₀H₂₀BrN₃O₂ + H: 414.0817; Found: 414.0807.

**5-cyclopropyl-2-(3-ethoxy-5-methyl-4-(2-(trifluoromethyl)phenoxy)-1H-pyrazol-1-yl)pyridine (example 56):** Obtained in 44 % yield as an oil using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.74 (m, 2H); 1.04 (m, 2H); 1.36 (t, 3H, J = 7.2); 1.91 (m, 1H); 2.51 (s, 3H); 4.35 (q, 2H, J = 7.2); 6.96 (m, 1H); 7.08 (m, 1H); 7.43 (m, 2H); 7.65 (m, 2H); 8.20 (d, 1H, J = 2.5). ¹³C (CDCl₃): 8.7; 11.6; 12.5; 14.8; 64.9; 114.2; 114.6; 118.6 (31 Hz); 121.4; 123.6 (272 Hz); 123.9; 127.0 (5 Hz); 133.1; 133.4; 135.2; 135.9; 145.5; 151.7; 155.1; 153.3 (2 Hz). HRMS: Calc. for C₂₁H₂₀F₃N₃O₂ + H: 404.1586; Found: 404.1602.

**2-(3-ethoxy-5-methyl-4-(3-(trifluoromethyl)phenoxy)-1H-pyrazol-1-yl)-5-ethylpyrimidine (example 57):** Obtained in 54 % yield as a solid, using 2-chloro-5-ethylpyrimidine in acetonitrile at 160 °C for one hour and after two consecutive chromatography, the first one over silica gel (cyclohexane-ethyl acetate 4/1), the second one over alumina containing 1.5 % of water (cyclohexane-dichloromethane 1/1). ¹H (CDCl₃): 1.32 (m, 6H); 2.55 (s, 3H); 2.68 (q, 2H, J = 7.0); 4.46 (q, 2H, J = 7.7); 7.17 (m, 1H); 7.24 (s, 1H); 7.28 (m, 1H); 7.41 (m, 1H); 8.58 (s, 2H). ¹³C (CDCl₃): 12.6; 14.6; 15.1; 23.0; 65.0; 112.2 (4 Hz); 118.5; 119.9 (4 Hz); 123.8 (272 Hz); 125.2; 130.0; 131.9 (33 Hz); 132.6; 134.7; 156.0; 156.5; 157.8; 158.3. HRMS: Calc. for C₁₉H₁₉F₃N₄O₂ + H: 393.1538; Found: 393.1521.

**5-bromo-2-(4-(2,6-difluorophenoxy)-3-isopropoxy-5-methyl-1H-pyrazol-yl**)**pyrimidine (example 58):** Obtained in 69 % yield as a solid using 5-bromo-2-chloropyrimidine in acetonitrile at 150 °C for one hour after a chromatography over silica gel (cyclohexane-dichloromethane from 1/2 to 2/1). ¹H (CDCl₃): 1.20 (d, 6H, J = 6.2); 2.65 (s, 3H); 5.08 (sept, 1H, J = 6.2); 6.90 (m, 2H); 7.00 (m, 1H); 8.70 (s, 2H). ¹³C (CDCl₃): 12.6; 21.8; 72.1; 111.8 (6 and 17 Hz); 114.2; 123.7 (9 Hz); 130.9; 132.6; 134.2 (13 Hz); 155.6; 155.6 (4 and 250 Hz); 155.9; 158.7. HRMS: Calc. for C₁₇H₁₅BrF₂N₄O₂ + H: 425.0425; Found: 425.0381.

**5-bromo-2-(4-(2,6-difluorophenoxy)-3-isopropoxy-5-methyl-1H-pyrazol-1-yl)pyridine (example 59):** Obtained in 79 % yield as a solid, using 2-fluoro-5-bromopyridine in acetonitrile at 160 °C for three hours and after a chromatography over silica gel (cyclohexane-ethyl acetate 98/2 to 9/1). ¹H (CDCl₃): 1.25 (d, 6H, J = 6.2); 2.65 (s, 3H); 4.89 (sept, 1H, J = 6.2); 6.94 (m, 2H); 7.02 (m, 1H); 7.69 (d, 1H, J = 8.8); 7.80 (dd, 1H, J = 2.6 and 8.8); 8.39 (d, 1H, J = 2.6). ¹³C (CDCl₃): 12.0; 21.9; 72.1; 111.9 (6 and 16 Hz); 115.1; 115.4; 123.5 (9 Hz); 129.7; 131.5; 134.5 (13 Hz); 140.4; 147.9; 152.8; 154.0; 155.6 (5 and 250 Hz). HRMS: Calc. for C₁₈H₁₆BrF₂N₃O₂ + H: 424.0472; Found: 424.0443.

**5-bromo-2-(4-(2,6-difluorophenoxy)-5-isopropoxy-3-methyl-1H-pyrazol-1-yl)pyridine (example 60):** On a large scale, a second fraction of the chromatography described above led to 4 % of this minor isomer as a white powder. ¹H (CDCl₃): 1.27 (d, 6H, J = 6.2); 2.21 (s, 3H); 4.75 (sept, 1H, J = 6.2); 6.94 (m, 2H); 7.03 (m, 1H); 7.64 (d, 1H, J = 8.8); 7.87 (dd, 1H, J = 2.5 and 8.8); 8.57 (d, 1H, J = 2.5). ¹³C (CDCl₃): 11.7; 22.1; 78.1; 112.4 (6 and 17 Hz); 116.8; 117.1; 123.6 (9 Hz); 128.4; 134.2 (13 Hz); 140.5; 142.3; 142.4; 149.5; 149.9; 155.1 (4 and 250 Hz). HRMS: Calc. for C₁₈H₁₆BrF₂N₃O₂ + H: 424.0472; Found: 424.0473.

**2-(4-(2,4-difluorophenoxy)-3-isopropoxy-5-methyl-1H-pyrazol-1-yl)-5-ethylpyrimidine (example 61):** Obtained in 57 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 1.29 (t, 3H, J = 7.6); 1.31 (d, 6H, J = 6.1); 2.56 (s, 3H); 2.66 (q, 2H, J = 7.6); 5.19 (sept, 1H, J = 6.1); 6.73 (m, 1H); 6.90 (m, 2H); 8.56 (s, 2H). ¹³C (CDCl₃): 12.4; 15.0; 22.1; 23.0; 72.1; 104.8 (22 and 27 Hz); 110.2 (23 Hz); 117.0 (10 Hz); 126.8; 132.5; 134.3; 142.6(11 Hz); 152.0 (250 and 12 Hz); 155.8; 156.1; 157.3 (243 and 10 Hz); 157.7. HRMS: Calc. for C₁₉H₂₀F₂N₄O₂ + H: 375.1633; Found: 375.1639.

**2-(4-(2,6-difluorophenoxy)-3-isopropoxy-5-methyl-1H-pyrazol-1-yl)-5-methoxypyrimidine (example 62):** Obtained in 45 % yield as a solid using 2-chloro-5-methoxypyrimidine in acetonitrile at 160 °C for one 90 minutes after a chromatography over silica gel (cyclohexane-ethyl acetate 2/1). ¹H (CDCl₃): 1.20 (d, 6H, J = 6.2); 2.61 (s, 3H); 3.92 (s, 3H); 5.07 (sept, 1H, J = 6.2); 6.88 (m, 2H); 7.02 (m, 1H); 8.38 (s, 2H). ¹³C (CDCl₃): 12.0; 21.9; 56.3; 71.9; 111.8 (6 and 16 Hz); 123.5 (8 Hz); 129.8; 131.8; 134.5 (13 Hz); 144.4; 150.6; 151.8; 154.6; 155.6 (4 and 250 Hz). HRMS: Calc. for C₁₈H₁₈F₂N₄O₃ + H: 377.1425; Found: 377.1370.

**2-(4-(2-chlorophenoxy)-3-ethoxy-5-methyl-1H-pyrazol-1-yl)-5-cyclopropylpyridine (example** 63): Obtained in 70 % yield as an oil using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.72 (m, 2H); 1.03 (m, 2H); 1.37 (t, 3H, J = 7.2); 1.92 (m, 1H); 2.54 (s, 3H); 4.35 (q, 2H, J = 7.2); 6.89 (m, 1H); 6.96 (m, 2H); 7.15 (m, 1H); 7.42 (m, 2H); 7.69 (d, 1H, J = 8.5); 8.19 (d, 1H, J = 2.4). ¹³C (CDCl₃): 8.7; 11.8; 12.6; 14.8; 64.8; 114.1; 115.0; 122.3; 122.7; 124.5; 127.5; 130.4; 133.3; 135.2; 135.8; 145.5; 151.8; 154.1; 155.1. HRMS: Calc. for C₂₀H₂₀ClN₃O₂ + H: 370.1322; Found: 370.1280.

**2-(4-(2,5-dichlorophenoxy)-3-ethoxy-5-methyl-1H-pyrazol-1-yl)-5-ethylpyrimidine (example 64):** Obtained in 46 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white solid. ¹H (CDCl₃): 1.31 (t, 3H, J = 7.6); 1.36 (t, 3H, J = 7.0); 2.56 (s, 3H); 2.68 (q, 2H, J = 7.6); 4.46 (q, 2H, J = 7.0); 6.86 (d, 1H, J = 2.3); 6.96 (dd, 1H, J = 2.3 and 8.5); 7.34 (d, 1H, J = 8.5); 8.58 (s, 2H). ¹³C (CDCl₃): 12.5; 14.7; 15.0; 23.0; 65.1; 115.5; 120.9; 123.0; 125.2; 131.0; 132.7; 133.1; 134.8; 154.3; 156.0; 156.3; 157.8. HRMS: Calc. for C₁₈H₁₈Cl₂N₄O₂ + H: 393.0885; Found: 393.0875.

**5-cyclopropyl-2-(4-(2-fluorophenoxy)-3-isopropoxy-1H-pyrazol-1-yl)pyridine (example 65**)**:** Obtained in 57 % yield, using 5-cyclopropyl-2-fluoropyridine (intermediate 50) in acetonitrile at 180 °C for 6 hours and after a chromatography over silica gel (cyclohexane/ethyl acetate 95/5) followed by concentration under high vacuum as an oil. ¹H (CDCl₃): 0.69 (m, 2H); 1.00 (m, 2H); 1.39 (d, 6H, J = 6.1); 1.89 (m, 1H); 5.04 (sept, 1H, J = 6.1); 7.03 (m, 2H); 7.13 (m, 2H); 7.38 (dd, 1H, J = 2.3 and 8.5 Hz); 7.68 (d, 1H, J = 8.5); 8.11 (d, 1H, J=2.3); 8.20 (s, 1H). ¹³C (CDCl₃): 8.6; 12.5; 22.1; 72.7; 110.3; 116.6 (18 Hz); 117.6; 118.3; 123.5 (7 Hz); 124.2 (4 Hz); 129.4; 135.4; 135.6; 145.8; 146.0; 149.5; 152.9 (248 Hz); 154.7. HRMS: Calc. for C₂₀H₂₀FN₃O₂ + H: 354.1618; Found: 354.1603.

**5-cyclopropyl-3-fluoro-2-(4-(2-fluorophenoxy)-3-isopropoxy-1H-pyrazol-1-yl)pyridine (example 66):** Obtained in 56 % yield, using 5-cyclopropyl-2,3-difluoropyridine (intermediate 51) in acetonitrile at 150 °C for 1 hours and after a chromatography over silica gel (cyclohexane/ethyl acetate 95/5) as an oil. ¹H (CDCl₃): 0.74 (m, 2H); 1.09 (m, 2H); 1.37 (d, 6H, J = 6.2); 1.96 (m, 1H); 5.09 (sept, 1H, J = 6.2); 7.02-7.18 (m, 5H); 7.96 (m, 1H); 8.07 (s, 1H). ¹³C (CDCl₃), two unexpected additional signals: 9.2; 12.3; 22.1; 72.7; 116.7 (J = 17 Hz); 118.1; 120.4 (J = 5 Hz); 122.5 (J = 19 Hz); 123.5 (J = 6 Hz); 124.2 (J = 4 Hz); 129.3 (J = 2 Hz); 137.7 (J = 8 Hz); 138.8 (J = 3Hz); 141.7 (J = 5 Hz); 145.9; 146.9 (J = 227 Hz); 150.7; 152_{.}8 (J = 248 Hz); 155.2; 155.3. HRMS: Calc. for C₂₀H₁₉F₂N₃O₂ + H: 372.1524; Found: 372.1468.

**5-cyclopropyl-2-(4-(2-fluorophenoxy)-3-isopropoxy-1H-pyrazol-1-yl)pyrimidine (example 67):** Obtained in 66 % yield, using 2-chloro-5-cyclopropylpyrimidine (intermediate 49) in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 0.75 (m, 2H); 1.07 (m, 2H); 1.37 (d, 6H, J = 6.2); 1.88 (m, 1H); 5.26 (sept, 1H, J = 6.2); 7.04 (m, 2H); 7.13 (m, 2H); 8.21 (s, 1H); 8.40 (s, 2H). ¹³C (CDCl₃): 8.2; 10.4; 22.1; 72.7; 116.8 (18 Hz); 118.6; 123.8 (7 Hz); 124.2 (4 Hz); 130.7; 132.4; 145.5 (11 Hz); 153.1 (248 Hz); 154.0; 156.3; 156.6. HRMS: Calc. for C₁₉H₁₉FN₄O₂ + H: 355.1570; Found: 355.1555.

**5-cyclopropyl-2-(3-ethoxy-4-(2-fluorophenoxy)-5-methyl-1H-pyrazol-1-yl)pyridine (example 68):** Obtained in 41 % yield as an oil using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.73 (m, 2H); 1.03 (m, 2H); 1.37 (t, 3H, J = 7.2); 1.92 (m, 1H); 2.55 (s, 3H); 4.35 (q, 2H, J = 7.2); 6.97 (m, 3H); 7.12 (m, 1H); 7.41 (dd, 1H, J = 2.5 and 8.6); 7.68 (d, 1H, J = 8.6); 8.19 (d, 1H, J = 2.5). ¹³C (CDCl₃): 8.7; 11.8; 12.5; 14.8; 64.8; 114.1; 116.1; 116.4 (18 Hz); 123.3 (6 Hz); 124.0 (3 Hz); 124.4; 133.3; 135.2; 135.7; 145.5; 146.4 (11 Hz); 151.8; 152.1 (248 Hz); 155.1. HRMS: Calc. for C₂₀H₂₀FN₃O₂ + H: 354.1618; Found: 354.1563.

**5-cyclopropyl-2-(4-(2-fluorophenoxy)-3-isopropoxy-5-methyl-1H-pyrazol-1-yl)pyridine (example 69):** Obtained in 58 % yield as a solid using 5-cyclopropyl-2-fluoropyridine (intermediate 50) in acetonitrile at 180 °C for 6 hours and after a chromatography over silica gel (cyclohexane/ethyl acetate 97/3) followed by concentration under high vacuum. ¹H (CDCl₃): 0.75 (m, 2H); 1.03 (m, 2H); 1.33 (d, 6H, J = 6.0); 1.92 (m, 1H); 2.54 (s, 3H); 4.98 (sept, 1H, J = 6.0); 6.98 (m, 3H); 7.12 (m, 1H); 7.40 (m, 1H); 7.68 (m, 1H); 8.18 (d, 1H, J = 2.4). ¹³C (CDCl₃): 8.6; 11.8; 12.5; 22.1; 72.1; 114.2; 116.3; 116.4 (18 Hz); 122.3; 124.0; 125.1; 133.0; 135.1; 135.6; 145.4; 146.4 (10 Hz); 151.9; 152.3 (248 Hz); 154.5. HRMS: Calc. for C₂₁H₂₂FN₃O₂ + H: 368.1774; Found: 368.1742.

**5-cyclopropyl-3-fluoro-2-(3-isopropoxy-4-phenoxy-1H-pyrazol-1-yl)pyridine (example 70):** Obtained in 60 % yield, using 5-cyclopropyl-2,3-difluoropyridine (intermediate 51) in acetonitrile at 160 °C for 2 hours and after a chromatography over silica gel (cyclohexane/ethyl acetate 9/1) as a solid. ¹H (CDCl₃): 0.74 (m, 2H); 1.08 (m, 2H); 1.37 (d, 6H, J = 6.1); 1.93 (m, 1H); 5.10 (sept, 1H, J = 6.1); 7.10 (m, 3H); 7.15 (m, 1H); 7.31 (m, 2H); 7.97 (m, 1H); 8.08 (s,1H). ¹³C (CDCl₃): 9.2; 12.3; 22.1; 72.6; 116.2 120.9 (5 Hz); 122.4; 122.6; 128.9; 129.4; 137.7 (7 Hz); 138.8; 141.8; 149.4 (262 Hz); 155.8; 158.4. HRMS: Calc. for C₂₀H₂₀FN₃O₂ + H: 354.1618; Found: 354.1641.

**5-cyclopropyl-2-(3-Isopropoxy-4-phenoxy-1H-pyrazol-1-yl)pyridine (example 71):** Obtained in 72 % yield, using 5-cyclopropyl-2-fluoropyridine (intermediate 50) in acetonitrile at 180 °C for 6 hours and after a chromatography over silica gel (cyclohexane /ethyl acetate 95/5) as an oil. ¹H (CDCl₃): 0.72 (m, 2H); 1.02 (m, 2H); 1.39 (d, 6H, J = 6.2); 1.92 (m, 1H); 5.04 (sept, 1H, J = 6.2); 7.12 (m, 3H); 7.30 (m, 2H); 7.38 (m, 1H); 7.71 (d, 1H, J = 8.5); 8.13 (, 1H, J = 2.3); 8.22 (s, 1H). ¹³C (CDCl₃): 8.5; 12.5; 22.1; 72.6; 110.3; 116.3; 118.1; 122.6; 129.0; 129.4; 135.4; 135.5; 146.0; 149.6; 152.3; 158.4. HRMS: Calc. for C₂₀H₂₁N₃O₂ + H: 336.1712; Found: 336.1714.

**2-(3-ethoxy-5-methyl-4-phenoxy-1H-pyrazol-1-yl)-5-ethylpyrimidine (example 72):** Obtained in 63 % yield, using 2-chloro-5-ethylpyrimidine in dimethylformamide at 160 °C for one hour and after two consecutive chromatography, the first one over silica gel (cyclohexane-ethyl acetate from 9/1 to 3/1), the second one over alumina containing 1.5 % of water (cyclohexane-dichloromethane 1/1) as a solid. ¹H (CDCl₃): 1.29 (t, 3H, J = 7.3); 1.34 (t, 3H, J = 7.0); 2.53 (s, 3H); 2.66 (q, 2H, J = 7.3); 4.45 (q, 2H, J = 7.0); 7.01 (m, 3H); 7.29 (m, 2H); 8.57 (s, 2H). ¹³C (CDCl₃): 12.6; 14.7; 15.1; 23.0; 64.4; 115.1; 122.1; 125.8; 129.4; 132.3; 134.8; 156.1; 157.0; 157.7; 158.3. HRMS: Calc. for C₁₈H₂₀N₄O₂ + H: 325.1665; Found: 325.1698.

**2-(3-ethoxy-4-(2-fluorophenoxy)-5-methyl-1H-pyrazol-1-yl)-5-ethylpyrimidine (example 73):** Obtained in 46 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white solid. ¹H (CDCl₃): 1.21 (t, 3H, J = 7.5); 1.25 (t, 3H, J = 7.0); 2.57 (s, 3H); 2.67 (q, 2H, J = 7.5); 4.45 (q, 2H, J = 7.0); 6.89-7.00 (m, 3H); 7.16 (m, 1H); 8.57 (s, 2H). ¹³C (CDCl₃): 12.5; 14.7; 15.0; 23.0; 65.0; 116.1; 116.5 (18 Hz); 122.6; 124.1; 125.8; 132.4; 132.6; 146.1; 152.2 (248 Hz); 156.1; 156.7; 157.7. HRMS: Calc. for C₁₈H₁₉FN₄O₂ + H: 343.1570; Found: 343.1551. 2-(4-(2,6-difluorophenoxy)-3-isopropoxy-5-methyl-1H-pyrazol-1-yl)pyrimidine (example 74): Obtained in 78 % yield as an oil, using 2-chloropyrimidine in acetonitrile at 160 °C for 1 hour after a chromatography over silica gel (cyclohexane-ethyl acetate 2/3) ¹H (CDCl₃); 1.20 (d, 6H, J = 6.2); 2.65 (s, 3H); 5.11 (sept, 1H, J = 6.2); 6.87 (m, 2H); 7.04 (m, 2H); 8.68 (d, 2H, J = 4.7). ¹³C (CDCl₃): 12.6; 21.9; 72.0; 111.9 (6 and 17 Hz); 116.8; 123.6 (9 Hz); 130.5; 134.4 (13 Hz); 155.2; 155.7; (250 and 4 Hz); 157.7; 158.3; 158.4. HRMS: Calc. for C₁₇H₁₆F₂N₄O₂ + H: 347.1320; Found: 347.1333.

**5-cyclopropyl-2-(3-isopropoxy-4-phenoxy-1H-pyrazol-1-yl)nicotinonitrile (example 75):** Obtained in 94 % yield, using 2-chloro-5-cyclopropylnicotinonitrile (intermediate 52) in acetonitrile at 150 °C for 40 minutes and after a chromatography over silica gel (cyclohexane-ethyl acetate 94/6) as a white solid. ¹H (CDCl₃): 0.74 (m, 2H); 1.10 (m, 2H); 1.44 (d, 6H, J = 6.1); 1.92 (m, 1H); 5.13 (sept, 1H, J = 6.1); 7.11 (m, 2H); 7.73 (m, 2H); 7.66 (d, 1H, J = 2.3); 7.67 (s, 1H); 8.28 (d, 1H, J = 2.3). ¹³C (CDCl₃): 8.9; 12.1; 21.9; 73.6; 96.4; 116.6; 116.7; 118.2; 123.0; 129.5; 130.6; 135.3; 141.5; 148.3; 150.1; 155.7; 157.9. HRMS: Calc. for C₂₁H₂₀N₄O₂ + H: 361.1665; Found: 361.1649.

**5-ethyl-2-(4-(4-fluorophenoxy)-3-isopropoxy-5-methyl-1H-pyrazol-1-yl**)**pyrimidine (example 76):** Obtained in 70 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 1.30 (t, 3H, J = 7.6); 1.32 (d, 6H, J = 6.0); 2.53 (s, 3H); 2.67 (q, 2H, J = 7.6); 5.21 (sept, 1H, J = 6.0); 6.96 (m, 4H); 8.57 (s, 2H). ¹³C (CDCl₃): 12.5; 15.0; 22.1; 23.0; 72.1; 115.7 (23 Hz); 116.3 (8 Hz); 126.9; 132.4; 134.4; 154.4; 155.1; 156.2; 157.7; 158.1 (240 Hz). HRMS: Calc. for C₁₉H₂₁FN₄O₂ + H: 357.1727; Found: 357.1690.

**2-(4-(2,3-difluorophenoxy)-3-isopropoxy-5-methyl-1H-pyrazol-1-yl)-5-ethylpyrimidine (example 77):** Obtained in 58 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a solid. ¹H (CDCl₃): 1.28 (t, 3H, J = 7.6); 1.30 (d, 6H, J = 6.1); 2.54 (s, 3H); 2.65 (q, 2H, J = 7.6); 5.19 (sept, 1H, J = 6.1); 6.67 (m, 1H); 6.82 (m, 1H); 6.89 (m, 1H); 8.55 (s, 2H). ¹³C (CDCl₃): 12.4; 15.0; 22.1; 23.0; 72.2; 104.8 (18 Hz); 111.3 (3 Hz); 122.8 (8 and 5 Hz); 126.2; 132.5; 134.4; 141.2 (250 and 15 Hz); 147.7 (8 Hz); 151.5 (247 and 10 Hz); 155.7; 156.0; 157.7. HRMS: Calc. for C₁₉H₂₀F₂N₄O₂ + H: 375.1633; Found: 375.1609.

**2-(4-(2-chlorophenoxy)-3-ethoxy-5-methyl-1H-pyrazol-1-yl)-5-ethylpyrimidine (example 78):** Obtained in 49 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white solid. ¹H (CDCl₃): 1.30 (t, 3H, J = 7.6); 1.34 (t, 3H, J = 7.0); 2.55 (s, 3H); 2.66 (q, 2H, J = 7.6); 4.46 (q, 2H, J = 7.0); 6.85 (m, 1H); 6.97 (m, 1H); 7.15 (m, 1H); 7.42 (m, 1H); 8.57 (s, 2H). ¹³C (CDCl₃): 12.5; 14.7; 15.0; 23.0; 65.0; 115.0; 122.4; 122.8; 125.8; 127.5; 130.4; 132.5; 134.7; 153.9; 156.1; 156.7; 157.7. HRMS: Calc. for C₁₈H₁₉ClN₄O₂ + H: 359.1275; Found: 359.1260.

**2-(4-(3,5-dichlorophenoxy)-3-ethoxy-5-methyl-1H-pyrazol-1-yl)-5-ethylpyrimidine (example 79):** Obtained in 49 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white solid. ¹H (CDCl₃): 1.22 (t, 3H, J = 7.6); 1.27 (t, 3H, J = 7.0); 2.54 (s, 3H); 2.68 (q, 2H, J = 7.6); 4.46 (q, 2H, J = 7.0); 6.82 (d, 1H, J = 1.8); 6.95 (d, 1H, J = 1.8); 8.58 (s, 2H). ¹³C (CDCl₃): 12.5; 14.7; 15.0; 23.0; 65.1; 114.3; 122.6; 124.9; 132.7; 134.8; 135.5; 156.0; 156.3; 157.7; 159.3. HRMS: Calc. for C₁₈H₁₈Cl₂N₄O₂ + H: 393.0885; Found: 393.0858.

**2-(4-(2,3-dichlorophenoxy)-3-ethoxy-5-methyl-1H-pyrazol-1-yl)-5-ethylpyrimidine (example 80):** Obtained in 62 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white solid. ¹H (CDCl₃): 1.30 (t, 3H, J = 7.6); 1.34 (t, 3H, J = 7.0); 2.55 (s, 3H); 2.67 (q, 2H, J = 7.6); 4.46 (q, 2H, J = 7.0); 6.78 (dd, 1H, J = 1.4 and 8.3); 7.08 (t, 1H, J = 8.3); 7.16 (dd, 1H, J = 1.4 and 8.3); 8.58 (s, 2H). ¹³C (CDCl₃): 12.5; 14.7; 15.0; 23.0; 65.1; 112.9; 121.6; 123.7; 125.6; 127.1; 132.6; 134.0; 134.7; 155.3; 156.0; 156.4; 157.7. HRMS: Calc. for C₁₈H₁₈N₄O₂Cl₂ + H: 393.0885; Found: 393.0867.

**2-(4-(2-bromophenoxy)-3-ethoxy-5-methyl-1H-pyrazol-1-yl)-5-ethylpyrimidine (example 81):** Obtained in 66 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white solid. ¹H (CDCl₃): 1.30 (t, 3H, J = 7.6); 1.35 (t, 3H, J = 7.0); 2.55 (s, 3H); 2.67 (q, 2H, J = 7.6); 4.46 (q, 2H, J = 7.0); 6.84 (m, 1H); 6.91 (m, 1H); 7.19 (m, 1H); 7.60 (m, 1H); 8.57 (s, 2H). ¹³C (CDCl₃): 12.6; 14.7; 15.0; 23.0; 65.0; 111.2; 114.9; 123.3; 125.9; 128.3; 132.5; 133.4; 134.7; 154.9; 156.1; 156.7; 157.7. HRMS: Calc. for C₁₈H₁₉BrN₄O₂ + H: 403.0770; Found: 403.0780.

**2-(3-ethoxy-5-methyl-4-(2-(trifluoromethyl)phenoxy)-1H-pyrazol-1-yl)-5-ethylpyrimidine (example 82):** Obtained in 49 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white solid. ¹H (CDCl₃): 1.31 (t, 3H, J = 7.6); 1.33 (t, 3H, J = 7.0); 2.54 (s, 3H); 2.68 (q, 2H, J = 7.6); 4.45 (q, 2H, J = 7.0); 6.94 (m, 1H); 7.10 (m, 1H); 7.42 (m, 1H); 7.65 (m, 1H); 8.58 (s, 2H). ¹³C (CDCl₃): (one signal missing) 12.3; 14.7; 15.0; 26.9; 65.1; 114.6; 118.7 (31 Hz); 121.6; 123.6 (273 Hz); 125.2; 127.0; 132.5; 133.1; 134.8; 156.1; 156.7; 157.7. HRMS: Calc. for C₁₉H₁₉F₃N₄O₂ + H: 393.1538; Found: 393.1541.

**2-(3-ethoxy-5-methyl-4-(4-(trifluoromethyl)phenoxy)-1H-pyrazol-1-yl)-5-ethylpyrimidine (example 83):** Obtained in 27 % yield as a solid, using 2-chloro-5-ethylpyrimidine in acetonitrile at 160 °C for one hour and after two consecutive chromatography, the first one over silica gel (cyclohexane-ethyl acetate 4/1), the second one over alumina containing 1.5 % of water (cyclohexane-dichloromethane 1/1). ¹H (CDCl₃): 1.30 (t, 3H, J = 7.7); 1.34 (t, 3H, J = 7.0); 2.53 (s, 3H); 2.68 (q, 2H, J = 7.0); 4.46 (q, 2H, J = 7.7); 7.07 (d, 2H, J = 8.7); 7.55 (d, 2H, J = 8.7); 8.58 (s, 2H). ¹³C (CDCl₃): 12.5; 14.7; 15.1; 23.0; 65.0; 115.2 124.2 (270 Hz); 124.4 (33 Hz); 125.1; 126.9 (4 Hz); 132.6; 134.8; 156.0; 156.5; 157.8; 160.7. HRMS: Calc. for C₁₉H₁₉F₃N₄O₂ + H: 393.1538; Found: 393.1483.

**2-(6-(4-(2,6-difluorophenoxy)-3-isopropoxy-5-methyl-1H-pyrazol-1-yl)pyridin-3-yl)propan-2-ol (example 84):** Obtained in 68 % yield as an oil using 2-(6-fluoropyridin-3-yl)propan-2-ol (intermediate 55) in acetonitrile at 180 °C for six hours and after a chromatography over silica gel (cyclohexane-ethyl acetate 4/1). ¹H (CDCl₃): 1.25 (d, 6H, J = 6.2); 1.63 (s, 6H); 1.86 (s, 1H); 2.65 (s, 3H); 4.90 (sept, 1H, J = 6.2); 6.90 (m, 2H); 6.99 (m, 1H); 7.70 (dd, 1H, J = 0.7 and 8.6); 8.25 (dd, 1H, J = 2.5 and 8.6); 8.48 (dd, 1H, J = 0.7 and 2.5). ¹³C (CDCl₃): 11.8; 21.9; 31.7; 71.3; 72.0; 111.9 (6 and 17 Hz); 113.8; 123.4 (9 Hz); 129.3; 131.2; 134.7 (14 Hz); 134.8; 140.2; 143.7; 152.9; 153.6; 155.6 (4 and 250 Hz). HRMS: Calc. for C₂₁H₂₃F₂N₃O₃ + H: 404.1786; Found: 404.1767.

**5-bromo-2-(4-(2,6-difluorophenoxy)-3-isopropoxy-5-methyl-1H-pyrazol-1-yl)-3-methylpyridine (example 85):** Obtained in 84 % yield as an oil, using 5-bromo-2-fluoro-3-methylpyridine in acetonitrile at 180 °C for three hours and a chromatography over silica gel (cyclohexane-ethyl acetate 98/2). ¹H (CDCl₃): 1.23 (d, 6H, J = 6.2); 2.25 (s, 3H); 2.32 (s, 3H); 4.79 (sept, 1H, J = 6.2); 6.90 (m, 2H); 7.01 (m, 1H); 7.78 (d, 1H, J = 2.3); 8.39 (d, 1H, J = 2.3). ¹³C (CDCl₃): 9.5; 18.2; 21.9; 72.0; 111.9 (6 and 17 Hz); 119.0; 123.5 (9 Hz); 128.0; 130.7; 132.1; 134.7 (14 Hz); 142.7; 146.7; 149.7; 153.3; 155.7 (4 and 250 Hz). HRMS: Calc. for C₁₉H₁₈BrF₂N₃O₂ + H: 438.0629; Found: 438.0589.

**5-bromo-2-(4-(2,6-difluorophenoxy)-3-isopropoxy-5-methyl-1H-pyrazol-1-yl)-3-fluoropyridine (example 86):** Obtained in 73 % yield as a solid, using 5-bromo-2,3-difluoropyridine in acetonitrile at 180 °C for three hours and a chromatography over silica gel (cyclohexane-ethyl acetate 98/2). ¹H (CDCl₃): 1.23 (d, 6H, J = 6.2); 2.35 (s, 3H); 4.86 (sept, 1H, J = 6.2); 6.91 (m, 2H); 7.02 (m, 1H); 7.75 (d, 1H, J = 2.1 and 9.1); 8.38 (d, 1H, J = 2.1). ¹³C (CDCl₃): 9.6; 21.8; 72.1; 111.9 (6 and 17 Hz); 117.6 (2 Hz); 123.7 (9 Hz); 128.6 (21 Hz); 129.0; 131.2; 134.5 (14 Hz); 139.9 (9 Hz); 144.7 (5 Hz); 151.7 (270 Hz); 154.5; 155.7 (4 and 250 Hz). HRMS: Calc. for C₁₈H₁₅BrF₃N₃O₂ + H: 442.0378; Found: 442.0346.

**1-(6-(4-(2,6-difluorophenoxy)-3-isopropoxy-5-methyl-1H-pyrazol-1-yl)pyridin-3-yl)ethanone (example 87):** Obtained in 52 % yield as a solid, using 1-(6-bromopyridin-3-yl)ethanone in acetonitrile at 130 °C for three hours and after a chromatography over silica gel (cyclohexane-ethyl acetate 95/5 to 9/1). ¹H (CDCl₃): 1.27 (d, 6H, J = 6.1); 2.63 (s, 3H); 2.74 (s, 3H); 4.93 (sept, 1H, J = 6.1); 6.92 (m, 2H); 7.03 (m, 1H); 7.87 (d, 1H, J = 8.7); 8.25 (dd, 1H, J = 2.5 and 8.7); 8.92 (m, 1H). ¹³C (CDCl₃): 12.5; 21.8; 26.5; 72.2; 112.0 (6 and 16 Hz); 113.0; 123.7 (10 Hz); 128.2; 130.4; 132.1; 134.3 (13 Hz); 137.4; 148.8; 154.7; 155.6 (4 and 250 Hz); 156.7; 195.7. HRMS: Calc. for C₂₀H₁₉F₂N₃O₃ + H: 388.1473; Found: 388.1447.

**1-(6-(4-benzyl-3-ethoxy-5-methyl-1H-pyrazol-1-yl)pyridin-3-yl)ethanone (example 88):** Obtained in 15 % yield, using methyl 1-(6-bromopyridin-3-yl)ethanone at 150 °C for 30 minutes and after a chromatography over silica gel (cyclohexane/ethyl acetate 9/1) as a solid. ¹H (CDCl₃): 1.42 (t, 3H, J = 7.1); 2.63 (s, 3H); 2.66 (s, 3H); 3.75 (s, 2H); 4.37 (q, 2H, J = 7.1); 7.19 (m, 1H); 7.27 (m, 4H); 7.89 (m, 1H); 8.26 (m, 1H); 8.93 (m, 1H). ¹³C (CDCl₃): 14.0; 14.8; 26.5; 27.7; 64.3; 109.0; 113.5; 125.9; 128.0; 128.2; 128.3; 137.5; 140.5; 140.6; 148.8; 156.5; 163.1; 195.7. HRMS: Calc. for C₂₀H₂₁N₃O₂ + H: 336.1712; Found: 336.1638.

**2-(4-benzyl-3-isopropoxy-5-methyl-1H-pyrazol-1-yl)nicotinonitrile (example 89):** Obtained in 73 % yield using 2-fluoronicotinonitrile at 140 °C for one hour in acetonitrile after a chromatography over silica gel (dichloromethane) as a white powder. ¹H (CDCl₃): 1.41 (d, 6H, J = 6.2); 2.52 (s, 3H); 3.72 (s, 2H); 5.08 (sept, 1H, J = 6.2); 7.12-7.27 (m, 6H); 8.04 (m, 1H); 8.51 (m, 1H). ¹³C (CDCl₃): 13.1; 22.2; 27.8; 72.3; 100.9; 109.6; 116.8; 118.9; 125.9; 128.3; 139.7; 140.4; 144.4; 144.6; 150.7; 152.7; 162.0. HRMS: Calc. for C₂₀H₂₀N₄O + H: 333.1715; Found: 333.1681.

**5-cyclopropyl-2-(4-(2-fluorobenzyl)-3-isopropoxy-5-methyl-1H-pyrazol-1-yl)pyridine (example 90):** Obtained in 27 % yield, using 5-cyclopropyl-2-fluoropyridine (intermediate 50) in acetonitrile at 180 °C for 3 hours and after a chromatography over silica gel (cyclohexane/ethyl acetate 97/3) followed by concentration under high vacuum as an oil. ¹H (CDCl₃): 0.71 (m, 2H); 1.02 (m, 2H); 1.36 (d, 6H, J = 6.0); 1.91 (m, 1H); 2.54 (s, 3H); 3.71 (s, 2H); 5.01 (sept, 1H, J = 6.0); 6.84 (m, 1H); 6.97 (m, 1H); 7.03 (m, 1H); 7.21 (m, 1H); 7.37 (m, 1H); 7.64 (m, 1H); 8.18 (m, 1H). ¹³C (CDCl₃): 8.6; 12.5; 12.9; 22.2; 27.6; 71.2; 106.3; 112.4; 112.6; 114.9; 115.1 (21 Hz); 123.8; 129.5; 135.0; 135.3; 138.9; 143.8; 145.4; 151.9; 161.4; 163.0 (244 Hz). HRMS: Calc. for C₂₂H₂₄FN₃O + H: 366.1982; Found: 366.1941.

**5-(1,1-difluoroethyl)-2-(4-(2,6-difluorophenoxy)-3-isopropoxy-5-methyl-1H-pyrazol-1-yl)pyridine (example 91):** Obtained in 12 % yield as an oil using 2-bromo-5-(1,1-difluoroethyl)pyridine (intermediate 56) in acetonitrile at 140 °C for six hours and after a chromatography over silica gel (cyclohexane-ethyl acetate from 98.5/1.5 to 9/1). ¹H (CDCl₃): 1.26 (d, 6H, J = 6.2); 1.98 (t, 3H, J = 18); 2.70 (s, 3H); 4.92 (sept, 1H, J = 6.2); 6.93 (m, 2H); 7.02 (m, 1H); 7.84 (m, 2H); 8.50 (s, 1H). ¹³C (CDCl₃): 12.1; 21.9; 25.7 (30 Hz); 72.1; 111.9 (6 and 16 Hz); 113.3; 121.0 (239 Hz); 123.5 (9 Hz); 129.3 (27 Hz); 129.8; 131.6; 134.5 (14 Hz); 134.6 (5 Hz); 144.0 (6 Hz); 154.2; 155.1; 155.6 (4 and 250 Hz). HRMS: Calc. for C₂₀H₁₉F₄N₃O₂ + H: 410.1492; Found: 410.1469.

**5-cyclopropyl-2-(3-ethoxy-5-methyl-4-phenoxy-1H-pyrazol-1-yl)pyridine (example 92):** Obtained in 17 % yield as an oil using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.73 (m, 2H); 1.04 (m, 2H); 1.38 (t, 3H, J = 7.2); 1.93 (m, 1H); 2.54 (s, 3H); 4.35 (q, 2H, J = 7.2); 7.04 (m, 3H); 7.30 (m, 2H); 7.42 (dd, 1H, J = 2.4 and 8.5); 7.69 (d, 1H, J = 8.5); 8.19 (d, 1H, J = 2.4). ¹³C (CDCl₃): 8.6; 11.9; 12.5; 14.8; 64.8; 114.1; 115.1; 121.9; 124.8; 129.4; 133.3; 135.2; 135.6; 145.4; 151.8; 155.4; 158.6. HRMS: Calc. for C₂₀H₂₁N₃O₂ + H: 336.1712; Found: 336.1791.

**5-cyclopropyl-2-(3-ethoxy-5-methyl-4-(3-(trifluoromethyl)phenoxy)-1H-pyrazol-1-yl)pyridine (example 93):** Obtained in 55 % yield as an oil using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.73 (m, 2H); 1.03 (m, 2H); 1.36 (t, 3H, J = 7.2); 1.93 (m, 1H); 2.54 (s, 3H); 4.36 (q, 2H, J = 7.2); 7.18 (m, 1H); 7.20 (m, 2H); 7.40 (m, 2H); 7.70 (d, 1H; J = 8.5); 8.20 (d, 1H, J = 2.3). ¹³C (CDCl₃): 8.7; 11.8; 12.5; 14.7; 64.8; 112.2 (4 Hz); 114.1; 118.4; 118.7 (4 Hz); 123.9 (273 Hz); 124.0; 130.0; 131.9 (33 Hz); 133.3; 135.2; 135.8; 145.5; 151.8; 155.0; 158.6. HRMS: Calc. for C₂₁H₂₀F₃N₃O₂ + H: 404.1586; Found: 404.1596.

**5-cyclopropyl-2-(3-ethoxy-5-methyl-4-(4-(trifluoromethyl)phenoxy)-1H-pyrazol-1**-**yl)pyridine (example 94):** Obtained in 53 % yield as a solid using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.73 (m, 2H); 1.04 (m, 2H); 1.37 (t, 3H, J = 7.2); 1.94 (m, 1H); 2.52 (s, 3H); 4.35 (q, 2H, J = 7.2); 6.9 (d, 2H, J = 8.6); 7.42 (dd, 1H, J = 2.5 and 8.5); 7.57 (d, 2H, J = 8.6); 7.69 (d, 2H, J = 8.5); 8.20 (d, 1H, J = 2.5). ¹³C (CDCl₃): 8.7; 11.8; 12.5; 14.7; 64.8; 114.1; 115.2; 123.9 124.3; (38 Hz); 124.4 (272 Hz); 126.9 (4 Hz); 131.3; 135.2; 135.9; 145.5; 151.7; 155.0; 161.0. HRMS: Calc. for C₂₁H₂₀F₃N₃O₂ + H: 404.1586; Found: 404.1638.

**5-cyclopropyl-2-(4-(2,5-dichlorophenoxy)-3-ethoxy-5-methyl-1H-pyrazol-1-yl)pyridine (example 95):** Obtained in 43 % yield as a solid using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.73 (m, 2H); 1.04 (m, 2H); 1.37 (t, 3H, J = 7.1); 1.93 (m, 1H); 2.54 (s, 3H); 4.34 (q, 2H, J = 7.1); 6.89 (d, 1H, J = 2.3); 6.96 (dd, 1H, = 2.3 and 8.5); 7.33 (d, 1H, J = 8.5); 7.42 (dd, 1H, = 2.5 and 8.5); 7.69 (d, 1H, J = 8.5); 8.20 (d, 1H, J = 2.5). ¹³C (CDCl₃): 8.7; 11.8; 12.6; 14.8; 64.9; 114.1; 115.6; 120.8; 122.8; 124.0; 130.9; 133.1; 133.3; 135.2; 135.9; 145.5; 151.7; 154.5; 154.7. HRMS: Calc. for C₂₀H₁₉Cl₂N₃O₂ + H: 404.0933; Found: 404.0927.

**5-cyclopropyl-2-(4-(3,5-dichlorophenoxy)-3-ethoxy-5-methyl-1H-pyrazol-1-yl)pyridine (example 96):** Obtained in 48 % yield as an oil using 5-cyclopropyl-2-fluoropyridine (intermediate 50) in acetonitrile at 180 °C for six hour and a chromatography over silica gel (cyclohexane-ethyl acetate 97/3) followed by concentration under high vacuum. ¹H (CDCl₃): 0.74 (m, 2H); 1.05 (m, 2H); 1.38 (t, 3H, J = 7.4); 1.93 (m, 1H); 2.52 (s, 3H); 4.34 (q, 2H, J = 7.4); 6.92 (d, 1H, J = 1.8); 7.03 (t, 1H, = 1.8); 7.42 (dd, 1H, = 2.3 and 8.5); 7.69 (d, 1H, J = 8.5); 8.20 (d, 1H, J = 2.3). ¹³C (CDCl₃): 8.7; 11.8; 12.6; 14.7; 64.8; 114.1; 114.3; 1.22.4; 123.7; 133.3; 135.2; 135.4; 135.9; 145.5; 151.7; 154.7; 159.5. HRMS: Calc. for C₂₀H₁₉Cl₂N₃O₂ + H: 404.0933; Found: 404.0961.

**5-cyclopropyl-2-(4-(3-fluorophenoxy)-3-isopropoxy-5-methyl-1H-pyrazol-1-yl)pyridine (example 97):** Obtained in 57 % yield as an oil, using 5-cyclopropyl-2-fluoropyridine (intermediate 50) in acetonitrile at 180 °C for 6 hours and after a chromatography over silica gel (cyclohexane/ethyl acetate 97/3) followed by concentration under high vacuum. ¹H (CDCl₃): 0.72 (m, 2H); 1.03 (m, 2H); 1.35 (d, 6H, J = 6.2); 1.92 (m, 1H); 2.52 (s, 3H); 5.00 (sept, 1H, J = 6.2); 6.73 (m, 2H); 6.82 (m, 1H); 7.22 (m, 1H); 7.41 (dd, 1H, J 2.4 and 8.5); 7.70 (d, 1H, J = 8.5); 8.19 (d, 1H, J = 2.4). ¹³C (CDCl₃): 8.7; 11.9; 12.5; 21.1; 72.1; 103.1 (25 Hz); 108.8 (21 Hz); 111.0 (3 Hz); 114.1; 124.9; 130.1 (10 Hz); 133.1; 135.1; 135.7; 145.4; 151.9; 154.5; 160.0 (10 Hz); 163.6 (245 Hz). HRMS: Calc. for C₂₁H₂₂FN₃O₂ + H: 368.1774; Found: 368.1670.

**5-cyclopropyl-2-(4-(4-fluorophenoxy)-3-isopropoxy-5-methyl-1H-pyrazol-1-yl)pyridine (example 98):** Obtained in 50 % yield as a solid, using 5-cyclopropyl-2-fluoropyridine (intermediate 50) in acetonitrile at 180 °C for 6 hours and after a chromatography over silica gel (cyclohexane/ethyl acetate 97/3) followed by concentration under high vacuum. ¹H (CDCl₃): 0.72 (m, 2H); 1.03 (m, 2H); 1.34 (d, 6H, J = 6.2); 1.93 (m, 1H); 2.52 (s, 3H); 4.98 (sept, 1H, J = 6.2); 6.97 (m, 4H); 7.40 (dd, 1H, J 2.3 and 8.5); 7.68 (d, 1H, J = 8.5); 8.19 (d, 1H, J = 2.3). ¹³C (CDCl₃): 8.6; 11.9; 12.5; 21.1; 72.0; 114.1; 115.6 (23 Hz); 116.2 (8 Hz); 125.6; 133.0; 135.1; 135.6; 145.4; 151.9; 154.6; 154.7 (3 Hz); 158.0 (239 Hz). HRMS: Calc. for C₂₁H₂₂FN₃O₂ + H: 368.1774; Found: 368.1759.

**2-(4-benzyl-3-ethoxy-5-methyl-1H-pyrazol-1-yl)-5-(1,1-difluoroethyl)pyridine (example 99):** Obtained in 10 % yield as an oil using 2-bromo-5-(1,1-difluoroethyl)pyridine (intermediate 56) in acetonitrile at 170 °C for six hour and two consecutive chromatography over silica gel (cyclohexane-dichloromethane from 9/1 to 2/1) and (cyclohexane-ethyl acetate 97/3). ¹H (CDCl₃): 1.42 (t, 3H, J = 7.0); 1.98 (t, 3H, J = 18); 2.62 (s, 3H); 3.75 (s, 2H); 4.36 (q, 2H, J = 7.0); 7.19 (m, 1H); 7.27 (m, 4H); 7.85 (m, 2H); 8.51 (m, 1H). ¹³C (CDCl₃): 13.6; 14.8; 25.7 (30 Hz); 27.7; 64.2; 107.8; 113.9; 121.0 (238 Hz); 125.8; 128.2; 128.3; 129.1 (27 Hz); 134.7 (5 Hz); 140.0; 140.7; 154.9; 162.7; one signal missing. HRMS: Calc. for C₂₀H₂₁F₂N₃O + H: 358.1731; Found: 358.1681.

**5-cyclopropyl-2-(3-isopropoxy-4-phenoxy-1H-pyrazol-1-yl)pyrimidine (example 100):** Obtained in 81 % yield, using 2-chloro-5-cyclopropylpyrimidine (intermediate 49) in dimethylformamide at 160 °C for one hour and after a chromatography over silica gel (cyclohexane-ethyl acetate 3/1) as an oil. ¹H (CDCl₃): 0.73 (m, 2H); 1.05 (m, 2H); 1.36 (d, 6H, J = 6.2); 1.86 (m, 1H); 5.26 (sept, 1H, J = 6.2); 7.08 (m, 3H); 7.31 (m, 2H); 8.23 (s, 1H); 8.40 (s, 2H). ¹³C (CDCl₃): 8.2; 10.4; 21.1; 72.6; 116.5; 120.0; 122.8; 129.5; 130.3; 132.3; 154.1; 156.6; 156.9; 158.1. HRMS: Calc. for C₁₉H₂₀N₄O₂ + H: 337.1665; Found: 337.1630.

**5-ethyl-2-(3-isopropoxy-4-(2-methoxybenzyl)-5-methyl-1H-pyrazol-1-yl)pyrimidine (example 101):** Obtained in 36 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white powder. ¹H (CDCl₃): 1.28 (t, 3H, J = 7.6); 1.32 (d, 6H, J = 6.2); 2.55 (s, 3H); 2.63 (q, 2H, J = 7.6); 3.70 (s, 2H); 3.86 (s, 3H); 5.21 (sept, 1H, J = 6.2); 6.85 (m, 2H); 7.14 (m, 1H); 8.54 (s, 2H). ¹³C (CDCl₃): 13.6; 15.1; 21.7; 22.3; 23.0; 55.2; 71.0; 108.3; 109.8; 120.2; 126.9; 128.7; 129.5; 131.7; 140.7; 156.4; 157.1; 157.5; 163.0. HRMS: Calc. for C₂₁H₂₀N₄O₂ + H: 367.2134; Found: 367.2143.

**5-ethyl-2-(3-isopropoxy-4-(3-methoxybenzyl)-5-methyl-1H-pyrazol-1-yl)pyrimidine (example 102):** Obtained in 38 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as an oil. ¹H (CDCl₃): 1.26 (t, 3H, J = 7.6); 1.34 (d, 6H, J = 6.1); 2.53 (s, 3H); 2.62 (q, 2H, J = 7.6); 3.65 (s, 2H); 3.76 (s, 3H); 5.22 (sept, 1H, J = 6.1); 6.80 (m, 2H); 7.15 (m, 2H); 8.52 (s, 2H). ¹³C (CDCl₃): 13.7; 15.0; 22.3; 23.0; 26.9; 51.2; 71.2; 109.5; 113.6; 129.1; 131.8; 132.9; 140.2; 157.3; 157.5; 157.8; 162.6. HRMS: Calc. for C₂₁H₂₀N₄O₂ + H: 367.2134; Found: 367.2134.

**2-(4-benzyl-3-(2-(benzyloxy)ethoxy)-5-methyl-1H-pyrazol-1-yl)-5-ethylpyrimidine (example 103):** Obtained in 81 % yield, using 2-chloro-5-ethylpyrimidine in dimethylformamide at 160 °C for one hour and after a chromatography over silica gel (cyclohexane-ethyl acetate 2/1), as an oil. ¹H (CDCl₃): 1.29 (t, 3H, J = 7.3); 2.58 (s, 3H); 2.65 (q, 2H, J = 7.3); 3.79 (s, 2H); 3.85 (m, 2H); 4.60 (s, 2H); 4.61 (m, 2H); 7.15-7.36 (m, 10H); 8.56 (s, 2H). ¹³C (CDCl₃): 13.8; 15.1; 23.0; 28.7; 68.2; 68.6; 73.0; 108.3; 125.8; 127.5; 127.6; 128.2; 128.3; 128.4; 132.0; 138.4; 140.5; 140.8; 156.2; 157.6; 163.1. HRMS: Calc. for C₂₆H₂₈N₄O₂ + H: 429.2291; Found: 429.2293.

**5-cyclopropyl-2-(3-isopropoxy-4-(phenylthio)-1H-pyrazol-1-yl)pyrimidine (example 104):** Obtained in 92 % yield, using 2-chloro-5-cyclopropylpyrimidine (intermediate 49) in acetonitrile at 160 °C for one hour and after a chromatography over silica gel (cyclohexane-ethyl acetate 4/1) as a solid. ¹H (CDCl₃): 0.75 (m, 2H); 1.08 (m, 2H); 1.33 (d, 6H, J = 6.2); 1.87 (m, 1H); 5.27 (sept, 1H, J = 6.2); 7.13 (m, 1H); 7.23 (m, 4H); 8.43 (s, 2H); 8.58 (s, 1H). ¹³C (CDCl₃): 8.4; 10.5; 22.0; 72.6; 100.1; 125.6; 127.2; 128.7; 133.3; 135.1; 137.2; 153.7; 156.6; 164.7. HRMS: Calc. for C₁₉H₂₀N₄OS + H: 353.1436; Found: 353.1414.

**5-cyclopropyl-2-(3-isopropoxy-4-(phenylthio)-1H-pyrazol-1-yl)pyridine (example 105):** Obtained in 97 % yield, using 5-cyclopropyl-2-fluoropyridine (intermediate 50) in acetonitrile at 180 °C for 6 hours and after a chromatography over silica gel (cyclohexane/ethyl acetate 95/5) followed by concentration under high vacuum as an oil. ¹H (CDCl₃): 0.73 (m, 2H); 1.07 (m, 2H); 1.40 (d, 6H, J = 6.1); 1.95 (m, 1H); 5.12 (sept, 1H, J = 6.1); 7.16 (m, 1H); 7.27 (m, 4H); 7.45 (dd, 1H, J = 2.4 and 8.5); 7.74 (d, 1H, J = 8.5); 8.2 (d, 1H, J = 2.4); 8.56 (s, 1H). ¹³C (CDCl₃): 8.7; 12.6; 22.0; 72.6; 97.6; 111.0; 125.3; 126.9; 128.7; 132.7; 135.5; 136.5; 138.0; 146.2; 149.1; 163.8. HRMS: Calc. for C₂₀H₂₁N₃OS + H: 352.1484; Found: 352.1459.

**2-(3-ethoxy-4-iodo-5-methyl-1H-pyrazol-1-yl)-5-ethylpyrimidine (example 106):** Obtained in 52 % yield, using 2-chloro-5-ethylpyrimidine in dimethylformamide at 160 °C for one hour and after a chromatography over silica gel (cyclohexane-ethyl acetate from 4/1 to 8/3) as a solid. ¹H (CDCl₃): 1.30 (t, 3H, J = 7.4); 1.45 (t, 3H, J = 7.0); 2.67 (q, 2H, J = 7.4); 2.71 (s, 3H); 4.46 (q, 2H, J = 7.0); 8.58 (s, 2H). ¹³C (CDCl₃): 14.7; 15.0; 16.3; 23.0; 55.8; 65.3; 132.8; 144.7; 155.6; 157.3; 163.3. HRMS: Calc. for C₁₂H₁₅IN₄O + H: 359.0369; Found: 359.0305.

**2-(3-ethoxy-4-iodo-1H-pyrazol-1-yl)-5-ethylpyrimidine (example 107):** Obtained in 45 % yield, using 2-chloro-5-ethylpyrimidine in dimethylformamide at 160 °C for 90 minutes and after a chromatography over silica gel (cyclohexane-ethyl acetate from 2/1) as a solid. ¹H (CDCl₃): 1.29 (t, 3H, J = 7.3); 1.47 (t, 3H, J = 7.0); 2.66 (q, 2H, J = 7.3); 4.49 (q, 2H, J = 7.0); 8.50 (s, 1H); 8.53 (s, 2H). ¹³C (CDCl₃): 14.6; 15.1; 23.0; 50.9; 65.9; 133.1; 134.9; 153.7; 158.0; 164.8. HRMS: Calc. for C₁₁H₁₃IN₄O + H: 345.0212; Found: 345.0151.

**5-cyclopropyl-2-(3-ethoxy-4-iodo-5-methyl-1H-pyrazol-1-yl)pyridine (example 108):** In a 20 mL Biotage tube, 3-ethoxy-4-iodo-5-methyl-1H-pyrazole (1.53 g, 6.07 mmol), cesium carbonate (2.2 g, 6.98 mmol and 5-cyclopropyl-2-fluoropyridine (intermediate 50) (0.87 g, 6.37 mmol) were dispersed in acetonitrile (14 mL, dried over 4Å molecular sieves). This was heated at 180 °C for 12 hours in the microwave oven. The resulting suspension was adsorbed over silica gel and purified by a chromatography over silica gel (cyclohexane-dichloromethane from 97.5/2.5 to 96.5/3.5) to yield 5-cyclopropyl-2-(3-ethoxy-4-iodo-5-methyl-1H-pyrazol-1-yl)pyridine (0.3 g, 13 %) as a solid. ¹H (CDCl₃): 0.73 (m, 2H); 1.03 (m, 2H); 1.45 (t, 3H, J = 7.2); 1.92 (m, 1H); 2.66 (s, 3H); 4.36 (q, 2H, J = 7.2); 7.39 (dd, 1H, J = 2.4 and 8.5); 7.62 (d, 1H, J = 8.5); 8.20 (d, 1H, J = 2.4). ¹³C (CDCl₃): 8.8; 12.6; 14.7; 15.2; 52.8; 65.0; 114.7; 135.1; 136.3; 143.0; 145.5; 151.3; 162.4. HRMS: Calc. for C₁₄H₁₆IN₃O + H: 370.0416; Found: 370.0441.

**(1-(5-cyclopropylpyridin-2-yl)-3-isopropoxy-1H-pyrazol4-yl)(phenyl)methanone (example 109):** Obtained in 46 % yield, using 5-cyclopropyl-2-fluoropyridine (intermediate 50) and (3-isopropoxy-1H-pyrazol-4-yl)(phenyl)methanone (intermediate 60) in acetonitrile at 180 °C for 6 hours and after a chromatography over silica gel (dichloromethane) as a solid. ¹H (CDCl₃): 0.75 (m, 2H); 1.07 (m, 2H); 1.44 (d, 6H, J = 6.2); 1.96 (m, 1H); 5.14 (sept, 1H, J = 6.2); 7.49 (m, 3H); 7.55 (m, 1H); 7.77 (m, 1H); 7.86 (m, 2H); 8.18 (m, 1H); 8.74 (s, 1H). ¹³C (CDCl₃): 8.9; 12.6; 21.9; 72.8; 110.8; 111.8; 127.8; 128.1; 129.0; 131.9; 135.5; 137.4; 139.5; 146.3; 148.6; 162.3; 188.6.

**5-cyclopropyl-2-(3-isopropoxy-5-methyl-4-(pyridin-3-yloxy)-1H-pyrazol-1-yl)pyrimidine (example 110):** Obtained in 84 % yield, using 2-chloro-5-cyclopropylpyrimidine (intermediate 49) in acetonitrile at 160 °C for one hour and after a chromatography over silica gel (cyclohexane-ethyl acetate 1/1) as a solid. ¹H (CDCl₃): 0.72 (m, 2H); 1.05 (m, 2H); 1.26 (d, 6H, J = 6.2); 1.85 (m, 1H); 2.48 (s, 3H); 5.17 (sept, 1H, J = 6.2); 7.17 (m, 1H); 7.22 (m, 1H); 8.24 (dd, 1H, J = 1.5 and 4.5); 8.39 (d, 1H, J = 2.7); 8.42 (s, 2H). ¹³C (CDCl₃): 8.4; 10.4; 12.4; 22.1; 72.1; 121.9; 123.7; 125.8; 132.7; 134.3; 138.7; 143.4; 154.6; 155.6; 155.7; 156.2. HRMS: Calc. for C₁₉H₂₁N₅O₂ + H: 352.1774; Found: 352.1754.

**5-ethyl-2-(3-isopropoxy-4-(4-methoxybenzyl)-5-methyl-1*H*-pyrazol-1-yl)pyrimidine (example 111):** Obtained in 38 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 1/1) as a white powder. ¹H (CDCl₃): 1.28 (t, 3H, J = 7.6); 1.32 (d, 6H, J = 6.2); 2.55 (s, 3H); 2.63 (q, 2H, J = 7.6); 3.70 (s, 2H); 3.86 (s, 3H); 5.21 (sept, 1H, J = 6.2); 6.85 (m, 2H); 7.14 (m, 2H); 8.54 (s, 2H). ¹³C (CDCl₃): 13.6; 15.1; 21.7; 22.3; 23.0; 55.2; 71.0; 108.3; 109.8; 120.2; 126.9; 128.7; 129.5; 131.7; 140.7; 156.4; 157.1; 157.5; 163.0. HRMS: Calc. for C₂₁H₂₀N₄O₂ + H: 367.2134; Found: 367.2143.

**2-(4-benzyl-3-ethoxy-5-methyl-1H-pyrazol-1-yl)-5-methoxypyrimidine (example 112):** Obtained in 44 % yield, using 2-chloro-5-methoxypyrimidine in dimethylformamide at 160 °C for one hour and after two consecutive chromatography, the first one over silica gel (cyclohexane-ethyl acetate 2/1), the second one over alumina containing 1.5 % of water (cyclohexane-dichloromethane from 1/1 to 2/3) as a solid. ¹H (CDCl₃): 1.38 (t, 3H, J = 7.0); 2.51 (s, 3H); 3.75 (s, 2H); 3.92 (s, 3H); 4.42 (q, 2H, J = 7.0); 7.18 (m, 1H); 7.25 (m, 4H); 8.39 (s, 2H). ¹³C (CDCl₃): 13.4; 14.8; 27.8; 56.3; 64.4; 107.6; 125.8; 128.2; 128.3; 140.1; 140.7; 144.4; 150.5; 151.9; 163.2. HRMS: Calc. for C₁₈H₂₀N₄O₂ + H: 325.1665; Found: 325.1673.

**2-(4-benzyl-3-ethoxy-5-methyl-1H-pyrazol-1-yl)-4-methylpyrimidine (example 113):** Obtained in 40 % yield, using 2-chloro-4-methylpyrimidine in dimethylformamide at 160 °C for one hour and after two consecutive chromatography, the first one over silica gel (cyclohexane-ethyl acetate 1/1), the second one over alumina containing 1.5 % of water (cyclohexane-dichloromethane 2/1) as an oil. ¹H (CDCl₃): 1.38 (t, 3H, J = 7.0); 2.53 (s, 3H); 2.57 (s, 3H); 3.75 (s, 2H); 4.45 (q, 2H, J = 7.0); 6.92 (d, 1H, J = 5.1); 7.16 (m, 1H); 7.24 (m, 4H); 8.56 (d, 1H, J = 5.1). ¹³C (CDCl₃): 14.0; 14.8; 24.3; 27.7; 64.4; 108.6; 116.4; 125.8; 128.2; 128.3; 140.5; 140.9; 157.4; 158.1; 163.5; 168.7. HRMS: Calc. for C₁₈H₂₀N₄O + H: 309.1715; Found: 309.1711.

**2-(4-butyl-3-ethoxy-5-methyl-1H-pyrazol-1-yl)-5-ethylpyrimidine (example 114):** Obtained in 43 % yield, using 2-chloro-5-ethylpyrimidine in dimethylformamide at 160 °C for one hour and after two consecutive chromatography, the first one over silica gel (cyclohexane-ethyl acetate 3/1), the second one over alumina containing 1.5 % of water (cyclohexane-dichloromethane 2/1) as a solid. ¹H (CDCl₃): 0.92 (t, 3H, J = 7.2); 1.27 (t, 3H, J = 7.8); 1.35 (m, 2H); 1.39 (t, 3H, J = 7.1); 1.49 (m, 2H); 2.35 (m, 2H); 2.54 (s, 3H); 2.63 (t, 2H, J = 7.4); 4.42 (q, 2H, J = 7.1); 8.53 (s, 2H). ¹³C (CDCl₃): 13.6; 13.9; 14.8; 15.1; 21.5; 22.2; 23.0; 31.9; 64.2; 109.8; 131.6; 139.8; 156.2; 157.6; 163.6. HRMS: Calc. for C₁₆H₂₄N₄O + H: 289.1916; Found: 289.1968.

**4-((4-benzyl-3-ethoxy-1-(5-ethylpyrimidin-2-yl)-1H-pyrazol-5-yl)methyl)morpholine (example 115):** Obtained in 46 % yield, using 2-chloro-5-ethylpyrimidine in dimethylformamide at 160 °C for one hour and after a chromatography over silica gel (dichloromethane-ethanol from 97/3 to 95/5) as an oil. ¹H (CDCl₃): 1.29 (t, 3H, J = 7.3); 1.39 (t, 3H, J = 7.0); 2.28 (m, 4H); 2.66 (q, 2H, J = 7.3); 3.38 (m, 4H); 3.83 (s, 2H); 3.90 (s(l), 2H); 4.43 (q, 2H, J = 7.0); 7.15 (m, 1H); 7.23 (m, 4H); 8.55 (s, 2H). ¹³C (CDCl₃): 14.9; 15.2; 23.0; 27.7; 52.5; 53.0; 64.5; 66.9; 109.8; 125.9; 128.2; 128.4; 132.7; 139.8; 140.6; 156.3; 157.4; 163.0. HRMS: Calc. for C₂₃H₂₉N₅O₂ + H: 408.2400; Found: 408.2393.

**- Examples of N-arylation of 3-alkoxypyrazoles with thiomethylated heteroaryl without copper catalyst, general method.** In a 4 ml tube sealed with a cap, the considered alkoxypyrazole (1.09 mmol) and the considered thiomethylated heteroaryl (1.09 mmol) were heated at 200 °C for 12 hours. The resulting tarry solid was dispersed in ethanol, adsorbed over silica and purified as described below for the specific examples

**2-(4-(2,6-difluorophenoxy)-3-isopropoxy-5-methyl-1H-pyrazol-1-yl)-5-methylpyrimidin**-**4**-**ol (example 116):** By following the general procedure described above using 5-methyl-2-(methylthio)pyrimidin-4-ol and 4-(2,6-difluorophenoxy)-3-isopropoxy-5-methyl-1H-pyrazole (intermediate 8), this compound was obtained as a white solid in 23 % yield after two chromatography over silica gel (dichlommethane - ethanol 99/1) and (cyclohexane/ethylacetate 2/1). ¹H (CDCl₃): 1.22 (d, 6H, J = 6.2); 2.05 (d, 3H, J = 1.0); 2.67 (s, 3H); 4.86 (sept, 1H, J = 6.2); 6.89 (m, 2H); 7.03 (m, 1H); 7.61 (q, 1H, J = 1.0); 9.95 (s(l), 1H). ¹³C (CDCl₃): 11.9; 12.6; 21.7; 73.8; 112.0 (6 and 16 Hz); 119.7; 124.0 (9 Hz); 130.8; 132.5; 133.9 (14 Hz); 147.0; 150.8; 154.8; 155.4 (4 and 250 Hz); 162.2. HRMS: Calc. for C₁₈H₁₈F₂N₄O₃ + H: 377.1425; Found: 377.1443.

**2-(4-(2,6-difluorophenoxy)-3-isopropoxy-5-methyl-1H-pyrazol-1-yl)-5-ethylpyrimidin-4-ol (example 117):** By following the procedure described above using 5-ethyl-2-(methylthio)pyrimidin-4-ol (intermediate 57) and 4-(2,6-difluorophenoxy)-3-isopropoxy-5-methyl-1H-pyrazole (intermediate 8), this compound was obtained in 10 % yield as white crystals after a chromatography over silica gel (cyclohexane/ethylacetate 3/1) and a recrystallisation in cyclohexane. ¹H (CDCl₃): 1.21 (t, 3H, J = 7.4); 1.24 (d, 6H, J = 6.2); 2.51 (dt, 2H, J = 1.0 and 7.4); 2.66 (s, 3H); 4.86 (sept, 1H, J = 6.2); 6.90 (m, 2H); 7.05 (m, 1H); 7.66 (s(l), 1H); 10.14 (s(l), 1H). ¹³C (CDCl₃): 12.0; 12.9; 20.4; 21.7; 72.8; 111.9 (6 and 16 Hz); 124.0 (9 Hz); 125.4; 130.8; 132.5; 133.9 (14 Hz); 146.8; 149.8; 154.8; 155.5 (4 and 250 Hz); 161.7. HRMS: Calc. for C₁₉H₂₀F₂N₄O₃ + H: 391.1582; Found: 391.1610.

**2-(4-(2,6-difluorophenoxy)-3-isopropoxy-5-methyl-1H-pyrazol-1-yl)-5-propylpyrimidin-4-ol (example 117A):** By following the procedure described above for the preparation of example 117, using 2-(methylthio)-5-propylpyrimidin-4-ol (intermediate 57A) and 4-(2,6-difluorophenoxy)-3-isopropoxy-5-methyl-1H-pyrazole (intermediate 8), this compound was obtained in 17 % yield as white crystals after a chromatography over silica gel (cyclohexane - ethylacetate 3/1) and a recrystallisation (two crops) in cyclohexane. ¹H (CDCl₃): 0.99 (t, 3H, J = 7.3); 1.23 (d, 6H, J = 6.2); 1.63 (m, 2H); 2.44 (m, 2H); 2.67 (s, 3H); 4.86 (sept, 1H, J = 6.2); 6.92 (m, 2H); 7.04 (m, 1H); 7.64 (s(l), 1H); 10.13 (s(l), 1H). ¹³C (CDCl₃): 12.0; 13.7; 21.6; 21.7; 29.1; 72.8; 112.0 (6 and 17 Hz); 123.8; 124.0 (9 Hz); 130.8; 132.5; 133.9 (14 Hz); 146.9; 150.5; 154.8; 155.5 (4 and 250 Hz); 161.8. HRMS: Calc. for C₂₀H₂₂F₂N₄O₃ + H: 405.1738; Found: 405.1739.

**3-(4-(2,6-difluorophenoxy)-3-isopropoxy-5-methyl-1H-pyrazol-1-yl)-6-ethyl-1,2,4-triazin-5-ol (example 118):** By following the procedure described above using 6-ethyl-3-(methylthio)-1,2,4-triazin-5-ol (intermediate 58) and 4-(2,6-difluorophenoxy)-3-isopropoxy-5-methyl-1H-pyrazole (intermediate 8) this compound was obtained in 10 % yield as a glass after two consecutive chromatography over silica gel (dichloromethane - ethanol 98/2) and (cyclohexane - ethyl acetate from 2/1 to 1/1). ¹H (CDCl₃): 1.23 (t, 3H, J = 7.5); 1.25 (d, 6H, J = 6.2); 2.71 (s, 3H); 2.76 (q, 2H, J = 7.5); 4.86 (sept, 1H, J = 6.2); 6.90 (m, 2H); 7.04 (m, 1H); 10.89 (s(l), 1H). ¹³C (CDCl₃): 10.0; 12.1; 21.6; 24.0; 73.2; 111.9 (6 and 17 Hz); 124.2 (9 Hz); 131.4; 133.7 (14 Hz); 133.8; 150.4; 154.4; 155.4 (4 and 250 Hz); 156.1; 162.7. HRMS: Calc. for C₁₈H₁₉F₂N₅O₃ + H: 392.1534; Found: 392.1538.

### - Examples of N-arylation of pyrazoles with halogenoheteroaryl without copper catalyst

**5-cyclopropyl-2-(3-cyclopropyl-4-phenoxy-1H-pyrazol-1-yl)pyrimidine (example 119):** In a reaction vial designed for microwave heating, 3-cyclopropyl-4-phenoxy-1H-pyrazole (intermediate 45) (0.16 g, 0.799 mmol), 2-chloro-5-cyclopropylpyrimidine (intermediate 49) (0.13 g, 0.838 mmol) and cesium carbonate (0.28 g, 0.878 mmol) were heated in dry acetonitrile (4 mL, dried over 4A molecular sieves) at 160 °C for one hour. The resulting was adsorbed over silica and purified by chromatography over silica gel (cyclohexane - ethyl acetate from 3/1 to 2/1) to yield the expected compound as a solid (0.22 g, 86 %). ¹H (CDCl₃): 0.76 (m, 2H); 0.89 (m, 2H); 1.10 (m, 4H); 1.94 (m, 2H); 7.08 (m, 3H); 7.32 (m, 2H); 8.25 (s, 1H); 8.42 (s, 2H). ¹³C (CDCl₃): 7.3; 7.4; 8.4; 10.4; 116.3; 119.3; 122.8; 129.6; 133.3; 140.7; 151.0; 154.0; 156.6; 158.4. HRMS: Calc. for C₁₉H₁₈N₄O + H: 319.1559; Found: 319.1494.

**5-cyclopropyl-2-(3-cyclopropyl-4-phenoxy-1H-pyrazol-1-yl)pyridine (example 120):** In a reaction vial designed for microwave heating, 3-cyclopropyl-4-phenoxy-1H-pyrazole (0.16 g, 0.799 mmol), 5-cyclopropyl-2-fluoropyridine (intermediate 50) (0.11 g, 0.799 mmol) and cesium carbonate (0.28 g, 0.878 mmol) were heated in dry acetonitrile (4 mL, dried over 4A molecular sieves) at 160 °C for one hour. The resulting was adsorbed over silica and purified by chromatography over silica gel (cyclohexane - ethyl acetate 95/5) to yield the expected compound as an oil (0.2 g, 78 %). ¹H (CDCl₃): 0.72 (m, 2H); 0.85 (m, 2H); 1.03 (m, 4H); 1.93 (m, 2H); 7.09 (m, 3H); 7.31 (m, 2H); 7.40 (dd, 1H, J = 2.4 and 8.5); 7.80 (d, 1H, J = 8.5); 8.15 (d, 1H, J = 2.4); 8.27 (s, 1H). ¹³C (CDCl₃): 7.0; 7.3; 8.7; 12.6; 111.0; 116.2; 117.6; 122.5; 129.6; 135.4; 136.3; 140.1; 146.0; 148.5; 149.7; 158.7. HRMS: Calc. for C₂₀H₁₉N₃O + H: 318.1606; Found: 318.1562.

**2-(4-benzyl-3-isobutyl-5-methyl-1H-pyrazol-1-yl)-5-ethylpyrimidine (example 121):** Obtained as a solid using 2-chloro-5-ethylpyrimidine and 4-benzyl-3-isobutyl-5-methyl-IH-pyrazole (intermediate 46) in dimethylformamide at 160 °C for one hour and after two consecutive chromatography, the first one over silica gel (cyclohexane-ethyl acetate from 3/1 to 2/1), the second one over alumina containing 1.5 % of water (cyclohexane-dichloromethane 1/1), followed by removing a volatile side compound under high vacuum. ¹H (CDCl₃): 0.90 (d, 6H, J = 6.6); 1.31 (t, 3H, J = 7.6); 2.00 (m, 1H, J = 6.8); 2.49 (d, 2H, J = 6.8); 2.56 (s, 3H); 2.68 (q, 2H, J = 7.6); 3.85 (s, 2H); 7.66 (m, 3H); 7.27 (m, 2H); 8.60 (s, 2H). ¹³C (CDCl₃): 13.2; 15.1; 22.6; 23.1; 28.6; 29.1; 36.0; 118.7; 125.9; 128.1; 128.3; 132.9; 139.5; 140.3; 154.2; 156.3; 157.7. HRMS: Calc. for C₂₁H₂₆N₄ + H: 335.2236; Found: 335.2257.

**5-cyclopropyl-2-(3-phenyl-1H-pyrazol-1-yl)pyrimidine** (example **122):** Obtained in 32 % yield, using 2-chloro-5-cyclopropylpyrimidine (intermediate 49) in dimethylformamide at 160 °C for one hour and after a chromatography over silica gel (dichloromethane) and a recrystallisation in cyclohexane. ¹H (CDCl₃): 0.80 (m, 2H); 1.112 (m, 2H); 1.92 (m, 1H); 6.82 (d, 1H, J = 2.7); 7.37 (m, 1H); 7.45 (m, 2H); 8.01 (m, 2H); 8.51 (s, 1H); 8.62 (d, 1H, J = 2.7). ¹³C (CDCl₃): 8.5; 10.5; 106.1; 126.4; 128.4; 128.5; 130.4; 132.5; 134.0; 154.5; 155.2; 156.6. HRMS: Calc. for C₁₆H₁₄N₄ + H: 263.1297; Found: 263.1282.

### - Examples of N-arylation of 3-alkoxypyrazoles with halogenoheteroaryl using a copper catalyst

**2-(4-benzyl-3-ethoxy-5-methyl-1H-pyrozol-1-yl)pyrimidine (example 123):** In a 20 mL Biotage tube 4-benzyl-3-ethoxy-5-methyl-1H-pyrazole (0.62 g, 2.87 mol), 2-chloropyrimidine (0.34 g, 3.01 mmol), cesium carbonate (0.98 g, 3.01 mmol), 4Å molecular sieves (0.3 g, 3.2 mm pellets) [*N,N*'-bis-((2'-pyridine)-methylene)]-1,2-diaminocyclohexane (Cristau et al. Org. Lett. 2004, 6, 913) (0.084 g, 0.28 mmol) were dispersed in acetonitrile (10 mL, dried over 4Å molecular sieves). This was degassed using a slow stream of argon bubbling in the suspension. Copper oxide (0.02 g, 0.14 mmol) was then added and the tube was sealed. This was shaken thoroughly, heated for 30 seconds in the microwave oven at 100 °C and shaken again. At this stage the pink copper oxide is well dissolved in the reaction mixture; if not, another 30 seconds heating at 100 °C is required. The heating is then resumed at 180 °C for two hours. The resulting suspension was dispersed in ethyl acetate, directly adsorbed over a small amount of silica purified by a chromatography over silica gel (cyclohexane - ethyl acetate 1/2) to yield the pyrimidine derivative as a solid (0.62 g, 73 %). ¹H NMR (CDCl₃): 1.39 (t, 3H, *J* = 7.0 Hz); 2.59 (s, 3H); 3.76 (s, 2H); 4.46 (q, 2H, *J* = 7.0 Hz); 7.05 (t, 1H, J = 4.8); 7.24 (m, 5H); 8.71 (d, 2H, J = 4.8). ¹³C NMR (CDCl₃): 14.0; 14.8; 27.7; 64.5; 109.1; 116.6; 125.9; 128.2; 128.3; 140.4; 141.0; 157.5; 158.3; 163.7. HRMS: Calc. for C₁₇H₁₈N₄O + H: 295.1559; Found: 295.1544.

**5-(4-benzyl-3-ethoxy-5-methyl-1H-pyrazol-1-yl)-2-methylpyridine (example 124):** In a 10 mL tube adapted for the microwave oven 4-benzyl-3-ethoxy-5-methyl-1H-pyrazole (0.4 g, 1.84 mmol), 5-bromo-2-methylpyridine (0.35 2.03 mmol), cesium carbonate (0.66 g, 2.03 mmol, 3.42 g), 4Å molecular sieves (0.1 g, 3.2 mm pellets) [*N,N*'-bis-((2'-pyridine)-methylene)]-1,2-diaminocyclohexane (Cristau et al. Org. Lett. 2004, 6, 913; 0.1 mmol, 0.29 g) were dispersed in acetonitrile (3 mL, dried over 4Å molecular sieves). This was degassed using a slow stream of argon bubbling in the suspension. Copper oxide (0.013 g, 0.090 mmol) was then added and the tube was sealed. This was shaken thoroughly, heated for 30 seconds in the microwave oven at 100 °C and shaken again. At this stage the pink copper oxide is well dissolved in the reaction mixture; if not, another 30 seconds heating at 100 °C is required. The heating is then resumed at 170 °C for 5 hours. The resulting suspension was dispersed in ethyl acetate, directly adsorbed over a small amount of silica and purified by a chromatography over silica gel (cyclohexane-ethyl acetate 2/1) and the relevant fraction further purified by a chromatography over alumina containing 1.5 % water (cyclohexane-dichloromethane from 2/3 to 1/2) to yield the 5-substitued pyridine as a solid (0.08 g, 14 %). ¹H (CDCl₃): 1.41 (t, 3H, J = 7.1); 2.20 (s, 3H); 2.61 (s, 3H); 3.74 (s, 2H); 4.31 (q, 2H, J = 7.1); 7.18 (m, 1H); 7.27 (m, 5H); 7.68 (m, 1H); 8.58 (s(l), 1H). ¹³C (CDCl₃): 11.3; 15.0; 24.0; 28.1; 64.3; 105.3; 123.4; 125.9; 128.3; 128.4; 132.1; 137.7; 140.9; 144.3; 156.2; 162.5. HRMS: Calc. for C₁₉H₂₀N₃O + H: 308.1763; Found: 308.1757.

**2-(4-benzyl-3-ethoxy-5-methyl-1H-pyrazol-1-yl)-5-methoxypyridine (example 125):** In a 10 mL Biotage tube 4-benzyl-3-ethoxy-5-methyl-1H-pyrazole (0.14 g, 6.33 mmol), 5-methoxy-2-bromopyrimidine (0.12 g, 6.65 mmol), cesium carbonate (0.22 g, 6.96 mmol), 4Å molecular sieves (0.1 g, 3.2 mm pellets) [*N,N*'-bis-((2'-pyridine)-methylene)]-1,2-diaminocyclohexane (Cristau et al. Org. Lett. 2004, 6, 913; 0.018 g, 0.63 mmol) were dispersed in acetonitrile (4.5 mL, dried over 4Å molecular sieves). This was degassed using a slow stream of argon bubbling in the suspension. Copper oxide (0.004 g, 0.031 mmol) was then added and the tube was sealed. This was shaken thoroughly, heated for 30 seconds in the microwave oven at 100 °C and shaken again. At this stage the pink copper oxide is well dissolved in the reaction mixture; if not, another 30 seconds heating at 100 °C is required. The heating was then resumed at 180 °C for 6 hours. The resulting suspension was directly adsorbed over a small amount of silica gel and this was subjected to a chromatography over silica gel (cyclohexane/ethyl acetate 9/1) to give the 5-methoxypyridine derivative (0.11 g, 53 %) as a white solid. ¹H (CDCl₃): 1.42 (t, 3H, J = 7.2); 2.51 (s, 3H); 3.76 (s, 2H); 3.88 (s, 3H); 4.36 (q, 2H, J = 7.2); 7.19 (m, 1H); 7.30 (m, 5H); 7.67 (d, 1H, J = 8.5); 8.09 (d, 1H, J = 2.3). ¹³C (CDCl₃): 12.6; 14.9; 27.8; 55.9; 64.4; 105.9; 116.4; 123.9; 125.7; 128.2 (two signals); 133.4; 138.8; 141.1; 147.6; 153.2; 162.0. HRMS: Calc. for C₁₉H₂₁N₃O₂ + H: 324.1712; Found: 324.1678.

**2-(4-(2,6-difluorophenoxy)-3-isopropoxy-5-methyl-1H-pyrazol-1-yl)-5-methoxypyridine (example 126):** By using the same procedure described above for the preparation of 2-(4-benzyl-3-ethoxy-5-methyl-1H-pyrazol-1-yl)-5-methoxypyrdine (example 125), this compound was obtained as a solid in 64 % yield after a chromatography over silica gel (cyclohexane/ethyl acetate from 97/3 to 95/5). ¹H (CDCl₃): 1.25 (d, 6H, J = 6.2); 2.60 (s, 3H); 3.87 (s, 3H); 4.88 (sept, 1H, J = 6.2); 6.90 (m, 2H); 6.98 (m, 1H); 7.30 (dd, 1H, J = 2.4 and 8.5); 7.65 (d, 1H, J = 8.5); 8.06 (d, 1H, J = 2.4). ¹³C (CDCl₃): 11.3; 21.9; 55.9; 71.9; 112.0 (6 and 17 Hz); 115.7; 123.4 (9 Hz); 124.0; 128.8; 133.2; 134.8 (13 Hz); 147.7; 153.3; 155.7 (4 and 250 Hz). HRMS: Calc. for C₁₉H₁₉F₂N₃O₃ + H: 376.1473; Found: 376.1510.

**2-(3-ethoxy-5-methyl-4-phenoxy-1H-pyrazol-1-yl)-5-methylpyridine (example 127):** Obtained in 70 % yield as a solid, using 5-methyl-2-bromopyridine, 3-ethoxy-5-methyl-4-phenoxy-1H-pyrazole and the protocol described above for the preparation of 5-(4-benzyl-3-ethoxy-5-methyl-1H-pyrazol-1-yl)-2-methylpyridine and after a chromatography over silica gel (cyclohexane/ethyl acetate 95/5) as a solid. ¹H (CDCl₃): 1.37 (t, 3H, J = 7.1); 2.35 (s, 3H); 2.52 (s, 3H); 4.36 (q, 2H, J = 7.1); 7.02 (m, 3H); 7.30 (m, 2H); 7.59 (dd, 1H, J = 8.4 and 2.2); 7.70 (d, 1H, J = 8.4); 8.20 (d, 1H, J = 2.2). ¹³C (CDCl₃): 11.9; 14.8; 17.8; 64.8; 114.1; 115.1; 121.9; 124.6; 129.4 (two signals); 133.4; 138.9; 147.3; 151.9; 155.4; 158.6. HRMS: Calc. for C₁₈H₁₉N₃O₂ + H: 310.1556; Found: 310.1524.

**5-(4-(2,6-difluorophenoxy)-3-isopropoxy-5-methyl-1H-pyrazol-1-yl)-2-methylpyridine (example 128):** By using the procedure described above for the preparation of 2-(4-benzyl-3-ethoxy-5-methyl-1H-pyrazol-1-yl)-5-methoxypyridine (example 125), this compound was obtained from 5-bromo-2-methylpyridine as a solid in 34 % yield after two consecutive chromatography, the first one over silica gel (cyclohexane - ethyl acetate 3/1), the second one over alumina containing 1.5 % of water (cyclohexane - dichloromethane from 2/3 to 1/1). ¹H (CDCl₃): 1.23 (d, 6H, J = 6.2); 2.32 (s, 3H); 2.59 (s, 3H); 4.83 (sept, 1H, J = 6.2); 6.92 (m, 2H); 7.02 (m, 1H); 7.22 (d, 1H, J = 8.4); 7.66 (dd, 1H, J = 2.5 and 8.4); 8.58 (d, 1H, J = 2.5). ¹³C (CDCl₃): 10.0; 21.9; 23.9; 72.1; 112.0 (6 and 17 Hz); 123.2; 123.6 (9 Hz); 128.4; 129.6; 131.5; 134.4; 134.6 (14 Hz); 144.0; 153.8; 155.7 (4 and 250 Hz); 156.4. HRMS: Calc. for C₁₉H₁₉F₂N₃O₂ + H: 360.1524; Found: 360.1515.

**5-(4-(2,6-difluorophenoxy)-3-isopropoxy-5-methyl-1H-pyrazol-1-yl)-2-ethylpyridine (example 129):** By using the procedure described above for the preparation of 2-(4-benzyl-3-ethoxy-5-methyl-1H-pyrazol-1-yl)-5-methoxypyridine (example 125), this compound was obtained from 5-bromo-2-ethylpyridine as an oil in 42 % yield after two consecutive chromatography, the first one over silica gel (cyclohexane - ethyl acetate 5/1), the second one over alumina containing 1.5 % of water (cyclohexane - dichloromethane 3/2). ¹H (CDCl₃): 1.23 (d, 6H, J = 6.2); 1.33 (t, 3H, J = 7.6); 2.33 (s, 3H); 2.87 (q, 2H, J = 7.6); 4.83 (sept, 1H, J = 6.2); 6.91 (m, 2H); 7.02 (m, 1H); 7.23 (d, 1H, J = 8.5); 7.68 (dd, 1H, J = 2.4 and 8.5); 8.58 (d, 1H, J = 2.4). ¹³C (CDCl₃): 10.0; 13.8; 21.9; 30.9; 72.1; 112.0 (6 and 17 Hz); 122.0; 123.6 (10 Hz); 128.4; 129.6; 131.6; 134.5; 134.6 (14 Hz); 144.1; 153.8; 155.7 (4 and 250 Hz); 161.5. HRMS: Calc. for C₂₀H₂₁F₂N₃O₂ + H: 374.1680; Found: 374.1667.

**5-(4-(2,6-difluorophenoxy)-3-isopropoxy-5-methyl-1H-pyrazol-1-yl)-2-methoxypyridine (example 130):** By using the procedure described above for the preparation of 2-(4-benzyl-3-ethoxy-5-methyl-1H-pyrazol-1-yl)-5-methoxypyridine (example 125), this compound was obtained from 5-bromo-2-methoxypyridine as a solid in 16 % yield after a chromatography over silica gel (cyclohexane - ethyl acetate 9/1). ¹H (CDCl₃): 1.23 (d, 6H, J = 6.2); 2.27 (s, 3H); 3.97 (s, 3H); 4.81 (sept, 1H, J = 6.2); 6.82 (d, 1H, J = 8.7); 6.91 (m, 2H); 7.02 (m, 1H); 7.65 (dd, 1H, J = 2.7 and 8.7); 8.20 (d, 1H, J = 2.7). ¹³C (CDCl₃): 9.7; 21.9; 53.8; 72.0; 111.9; 112.9 (6 and 17 Hz); 123.6 (9 Hz); 127.9; 129.8; 130.9; 134.7 (14 Hz); 135.4; 142.3; 153.5; 155.8 (4 and 250 Hz); 162.6. HRMS: Calc. for C₁₉H₁₉F₂N₃O₃ + H: 376.1473; Found: 376.1446.

**2-tert-butyl-5-(4-(2,6-difluorophenoxy)-3-isopropoxy-5-methyl-1H-pyrazol-1-yl)pyrimidine (example 131):** By using the procedure described above for the preparation of 2-(4-benzyl-3-ethoxy-5-methyl-1H-pyrazol-1-yl)-5-methoxypyridine (example 125), this compound was obtained from 5-bromo-2-tert-butylpyrimidine as a solid in 24 % yield after two consecutive chromatography, the first one over silica gel (cyclohexane - ethyl acetate 95/5), the second one over alumina containing 1.5 % of water (cyclohexane-dichloromethane 4/1). ¹H (CDCl₃): 1.24 (d, 6H, J = 6.2); 1.46 (s, 9H); 2.36 (s, 3H); 4.82 (sept, 1H, J = 6.2); 6.92 (m, 2H); 7.03 (m, 1H); 8.82 (s, 2H). ¹³C (CDCl₃): 10.0; 21.9; 29.6; 39.3; 72.2; 112.0 (6 and 16 Hz); 123.8; (10 Hz); 128.9; 129.6; 132.5; 134.4 (14 Hz); 150.4; 155.6 (4 and 250 Hz); 174.6. HRMS: Calc. for C₂₁H₂₄F₂N₄O₂ + H: 403.1946; Found: 403.1900.

**4-benzyl-1-(4-ethylphenyl)-3-isopropoxy-5-methyl-1H-pyrazole (example 132):** In an open flask, 4-benzyl-3-isopropoxy-5-methyl-1H-pyrazole (intermediate 11) (0.24 g, 1.0 mmol), 4-ethylphenylboronic acid (0.17 g, 1.1 mmol), pyridine (0.17 mL, 2.1 mmol, dried over 4Å molecular sieves), 4Å molecular sieves (0.6 g) and copper (II) acetate hydrate (0.31 g, 1.5 mmol) were dispersed in dichloromethane (20 mL). The reaction was stirred in open air for 48 hours. The suspension was absorbed on a small amount of silica gel and purified by a chromatography over silica gel (cyclohexane - ethyl acetate 97/3) to give the N-arylated compound mentioned above (0.22 g, 63 %) as an oil. ¹H (CDCl₃): 1.27 (t, 3H, J = 7.6); 1.36 (d, 6H, J = 6.1); 2.17 (s, 3H); 2.69 (q, 2H, J = 7.6); 3.73 (s, 2H); 4.98 (sept, 1H, J = 6.1); 7.25 (m, 5H). ¹³C (CDCl₃): 11.3; 15.6; 22.3; 28.2; 28.4; 71.1; 104.9; 124.6; 126.7; 128.2; 128.3; 128.35; 137.1; 138.0; 141.5; 142.6; 161.2. HRMS: Calc. for C₂₂H₂₆N₂O + H: 335.2123; Found: 335.2105.

**2-cyclopropyl-5-(4-(2,6-difluorophenoxy)-3-isopropoxy-5-methyl-1H-pyrazol-1-yl)pyrimidine (example 133):** By following the procedure described for the preparation of 4-benzyl-1-(4-ethylphenyl)-3-isopropoxy-5-methyl-1H-pyrazole (example 132) using a very aged sample of 2-cyclopropylpyrimidin-5-ylboronic acid the N-arylated compound was obtained in 1.8 % yield as an oil. ¹H (CDCl₃): 1.11 (m, 2H); 1.15 (m, 2H); 1.24 (d, 6H, J = 6.1); 2.30 (m, 1H); 2.35 (s, 3H); 4.82 (sept, 1H, J = 6.1); 6.93 (m, 2H); 7.05 (m, 1H); 8.68 (s, 2H). ¹³C (CDCl₃): 9.9; 11.0; 17.9; 21.9; 72.2; 112.0 (6 and 16 Hz); 123.8; (9 Hz); 128.8; 129.6; 132.5; 134.4 (14 Hz); 151.1; 155.4; 155.7 (4 and 250 Hz). HRMS: Calc. for C₂₀H₂₀F₂N₄O₂ + H: 387.1633; Found: 387.1643.

**4-benzyl-3-ethoxy-5-methyl-1-phenyl-1H-pyrazole (example 134):** Prepared using phenylboronic acid as previously described (Guillou et al. Tetrahedron 2009, 65, 2660).

### - Examples of preparations of 3-alkoxypyrazoles by alkylation of 3-hydroxypyrazoles

### 3-alkopyrazole ether cleavage.

**4-benzyl-1-(5-ethylpyrimidin-2-yl)-5-methyl-1H-pyrazol-3(2H)-one (example 135):** In a 100 mL tube fitted with a Teflon-coated screw cap, 2-(4-benzyl-3-ethoxy-5-methyl-1H-pyrazol-1-yl)-5-ethylpyrimidine (example 25) (0.58 g, 1.79 mmol) and 1M boron tribromide in dichloromethane (2 mL) were stirred for 4 days at room temperature. Ethanol was cautiously added and the resulting solution concentrated to dryness. The residue was made basic with 2 N ethanolic ammonia and this was concentrated to dryness. The residue was purified by a chromatography over silica gel (dichloromethane/ethanol from 98/2 to 96/4) to yield unreacted material (0.16 g, 27 %) and the deprotected pyrazol-3-ol (0.25 g, 47 %) as a white powder. ¹H (DMSO-*d6*): 1.21 (t, 3H, J = 7.2); 2.61 (q, 2H, J = 7.2); 3.33 (s, 3H); 3.65 (s, 2H); 7.14 (m, 1H); 7.25 (m, 4H); 8.63 (s, 2H); 10.63 (s(l), 1H). ¹³C (DMSO-*d6*): 13.7; 15.4; 22.6; 27.7; 108.0; 126.2; 128.5; 128.7; 132.7; 139.6; 141.3; 155.9; 158.1; 161.8. HRMS: Calc. for C₁₇H₁₈N₄O + H: 295.1559; Found: 295.1459.

**4-benzyl-1-(5-cyclopropylpyrimidin-2-yl)-5-methyl-1H-pyrazol-3-ol (example 136):** By using the same procedure described for the preparation of 4-benzyl-1-(5-ethylpyrimidin-2-yl)-5-methyl-1H-pyrazol-3(2H)-one (example 135), this compound was obtained from 2-(4-benzyl-3-isopropoxy-5-methyl-1H-pyrazol-1-yl)-5-cyclopropylpyrimidine (example 21) as a light yellow powder (0.14 g, 51 %). ¹H (DMSO-*d6*): 0.83 (m, 2H); 1.02 (m, 2H); 1.94 (m, 1H); 3.47 (s, 3H); 3.65 (s, 2H); 7.13 (m, 1H); 7.24 (m, 4H); 8.52 (s, 2H); 10.57 (s(l), 1H). ¹³C (DMSO-*d6*): 9.2; 10.5; 13.6; 27.7; 107.9; 126.2; 128.4; 128.7; 133.0; 139.5; 141.3; 155.6; 156.2; 161.8. HRMS: Calc. for C₁₈H₁₈N₄O + H: 307.1559; Found: 307.1556.

### Alkylations of 3-hydroxypyrazoles

**2-(4-benzyl-3-methoxy-5-methyl-1H-pyrazol-1-yl)-5-methylpyridine (example 137):** 4-benzyl-5-methyl-1-(5-methylpyridin-2-yl)-1H-pyrazol-3-ol (intermediate 59) (0.23 g, 0.82 mmol), trimethyl orthoacetate (2 mL) were refluxed for 8 hours. This was concentrated to dryness and the residue purified by a chromatography over silica gel (cyclohexane/dichloromethane 1/5 to 0/1) to yield the 3-methoxypyrazole (0.11 g, 45 %) as an oil. ¹H NMR (CDCl₃): 2.35 (s, 3H); 2.56 (s, 3H); 3.77 (s, 2H); 4.01 (s, 3H); 7.19 (m, 1H); 7.28 (m, 4H); 7.57 (dd, 1H, J = 2.2 and 8.5); 7.68 (d, 1H, J = 8.5); 8.22 (d, 1H, J = 2.2). ¹³C NMR (CDCl₃): 13.0; 17.8; 27.7; 55.8; 105.9; 114.9; 125.8; 128.2; 128.3; 129.2; 138.8; 139.4; 140.9; 147.3; 151.8; 162.7. HRMS: Calc. for C₁₈H₁₉N₃O + H: 316.1426; Found: 316.1424.

**2-(4-benzyl-3-methoxy-5-methyl-1H-pyrazol-l-yl)-5-cyclopropylpyrimidine (example 138):** Under a moisture-protected atmosphere, 4-benzyl-1-(5-cyclopropylpyrimidin-2-yl)-5-methyl-1H-pyrazol-3-ol (example 136) (0.11 g, 0.359 mmol), methyl iodide (0.076 g, 0.535 mmol) and potassium carbonate (0.074 g, 0.535 mmol) were stirred overnight at 20 °C in dimethylformamide (3 ml, dried over 4A molecular sieves). The solution was concentrated to dryness. The residue was purified by a chromatography over silica gel (cyclohexane/ethyl acetate from 2/1 to 1/2) to yield the 3-methoxypyrazole as a solid (0.05 g, 43 %). ¹H (CDCl₃): 0.76 (m, 2H); 1.08 (m, 2H); 1.89 (m, 1H); 2.55 (s, 3H); 3.75 (s, 2H); 4.07 (s, 3H); 7.17-7.29 (m, 5H); 8.46 (s, 2H). ¹³C (CDCl₃): 8.3; 10.4; 13.7; 27.7; 56.1; 107.9; 125.9; 128.1; 128.3; 132.1; 140.4; 140.9; 155.9; 156.2; 163.8. HRMS: Calc. for C₁₉H₂₀N₄O + H: 321.1715; Found: 321.1660.

**2-(4-benzyl-3-isopropoxy-5-methyl-1H-pyrazol-1-yl)-5-ethylpyrimidine (example 139):** Under a moisture-protected atmosphere, 4-benzyl-1-(5-ethylpyrimidin-2-yl)-5-methyl-1H-pyrazol-3(2H)-one (example 135) (0.1 g, 0.339 mmol), isopropyl bromide (0.04 mL, 0.40 mmol) and potassium carbonate (0.1 g, 6.79 mmol) were heated at 80 °C in dimethylformamide (3 ml, dried over 4A molecular sieves) for one hour. The solution was diluted in water and extracted with ethyl acetate. The organic layer was washed with water four times with brine once, dried over magnesium sulfate and concentrated to dryness. The residue was purified by a chromatography over silica gel (cyclohexane/ethyl acetate from 2/1 to 1/1) to yield the 3-pyrazole isopropyl ether (0.04 g, 34 %). ¹H (CDCl₃): 1.27 (t, 3H, J = 7.3); 1.34 (d, 6H, J = 6.1); 2.55 (s, 3H); 2.62 (q, 2H, J = 7.3); 3.73 (s, 2H); 5.23 (sept, 1H, J = 6.1); 7.17 (m, 1H); 7.25 (m, 4H); 8.54 (s, 2H). ¹³C (CDCl₃): 13.8; 15.1; 22.3; 23.0; 27.8; 71.2; 109.1; 125.7; 128.2; 128.3; 131.8; 140.4; 140.8; 156.2; 157.6; 162.6. HRMS: Calc. for C₂₀H₂₄N₄O + H: 337.2028; Found: 337.2006.

**3-(4-benzyl-1-(5-ethylpyrimidin-2-yl)-5-methyl-1H-pyrazol-3-yloxy)-N,N-dimethylpropan-1-amine (example 140):** From 4-benzyl-1-(5-ethylpyrimidin-2-yl)-5-methyl-1H-pyrazol-3(2H)-one (example 135), following a the same protocol using the hydrochloride of 3-chloro-N,N-dimethylpropan-1-amine, the title compound was obtained after a purification by a chromatography over alumina oxide containing 1.5 % of water (dichloromethane/ethanol from 99/1 to 98/2) to yield the 3-pyrazole isopropyl ether (0.04 g, 34 %). ¹H (CDCl₃): 1.25 (t, 3H, J = 7.6); 1.92 (m, 2H); 2.35 (s, 6H); 2.37 (m, 2H); 2.55 (s, 3H); 2.62 (q, 2H, J = 7.6); 3.72 (s, 2H); 4.39 (t, 2H, J = 6.2); 7.15 (m, 1H); 7.24 (m, 4H); 8.52 (s, 2H). ¹³C (CDCl₃): 13.7; 15.1; 23.0; 27.3; 27.8; 45.2; 56.2; 66.7; 108.2; 125.8; 128.2; 128.3; 132.0; 140.5; 140.6; 156.2; 157.6; 163.2. HRMS: Calc. for C₂₂H₂₉N₅O + H: 380.2450; Found: 380.2395.

**2-(4-benzyl-5-methyl-3-(pentan-3-yloxy)-1H-pyrazol-1-yl)-5-cyclopropylpyrimidine (example 141):** From 4-benzyl-1-(5-cyclopropylpyrimidin-2-yl)-5-methyl-1H-pyrazol-3-ol (example 136), following a similar protocol using 3-bromopentane, the corresponding ether was obtained (0.03 g, 40 %) as an oil after a chromatography over silica gel (cyclohexane/ethyl acetate 3/1 to 1/1). ¹H (CDCl₃): 0.76 (m, 2H); 0.89 (t, 6H, J = 7.4); 1.07 (m, 2H); 1.70 (m, 4H); 1.84 (m, 1H); 2.54 (s, 3H); 3.74 (s, 2H); 4.94 (pent, 1H, J = 5.8); 7.16 (m, 1H); 7.25 (m, 4H); 8.44 (s, 2H). ¹³C (CDCl₃): 8.2; 9.1; 10.4; 13.7; 25.8; 27.9; 79.7; 108.9; 125.7; 128.2; 128.3; 131.8; 140.2; 140.8; 156.0; 156.2; 163.1. HRMS: Calc. for C₂₃H₂₈N₄O + H: 377.2341; Found: 377.2303.

**2-(4-benzyl-3-sec-butoxy-5-methyl-1H-pyrazol-1-yl)-5-cyclopropylpyrimidine (example 142):** From 4-benzyl-1-(5-cyclopropylpyrimidin-2-yl)-5-methyl-1H-pyrazol-3-ol (example 136), following a similar protocol using 2-bromobutane, the corresponding ether was obtained (0.04 g, 37 %) as an oil after a chromatography over silica gel (cyclohexane/ethyl acetate 3/1 to 1/1). ¹H (CDCl₃): 0.75 (m, 2H); 0.92 (t, 6H, J = 7.5); 1.08 (m, 2H); 1.32 (d, 3H, J 6.1); 1.68 (m, 2H); 1.87 (m, 1H); 2.54 (s, 3H); 3.73 (s, 2H); 5.04 (m, 1H); 7.16 (m, 1H); 7.25 (m, 4H); 8.44 (s, 2H). ¹³C (CDCl₃): 8.2; 9.4; 10.4; 13.7; 19.5; 27.9; 29.2; 75.6; 109.0; 125.7; 128.2; 128.3; 131.9; 140.3; 140.8; 156.0; 156.2; 162.8. HRMS: Calc. for C₂₂H₂₆N₄O + H: 363.2185; Found: 363.2129.

- **Preparation of N-arylated 3-methoxypyrazoles from 1,1-dimethoxyethene and carboxychlorides.** In a 60 mL tube fitted with a Teflon-covered screw cap, 1,1-dimethoxyethene (0,78 g, 8.85 mmol) was added cautiously to the considered carboxyl chloride (2.97 mmol). An exothermic reaction took place, the tube was closed and this was stirred at room temperature for one hour. The 2-pyridylhydrazine (0.65 g, 5.95 mmol) was added, the tube was closed again and the suspension was stirred for 15 minutes. The resulting oil was diluted in dichloromethane (40 mL), acidic alumina, from Merck, ref 1.01078, containing 1.5% water (6 g) was added and the suspension was concentrated to dryness. The resulting powder was boiled in toluene (50 mL) for 15 minutes, this was concentrated to dryness and the residue was subjected to purification procedure as described below.

**2-(5-(3,4-dichlorophenyl)-3-methoxy-1H-pyrazol-1-yl)pyridine (example 143):** This compound was obtained as a solid in 25 % yield after a chromatography over silica gel (dichloromethane - ethanol 92/8) followed by a chromatography over alumina containing 1.5 % water (cyclohexane - ethyl acetate 95/5). ¹H NMR (CDCl₃): 4.02 (s, 3H); 5.99 (s, 1H); 7.11 (m, 1H); 7.16 (m, 2H); 7.38 (d, 1H, J = 8.3 Hz); 7.44 (d, 1H, J = 2.0 Hz); 7.77 (m, 1H); 8.25 (m, 1H). ¹³C NMR (CDCl₃): 56.3; 96.4; 117.2; 121.6; 128.1; 129.9; 130.5; 131.5; 132.2; 132.4; 138.3; 142.8; 147.8; 152.2; 164.2. HRMS: Calc. for C₁₅H₁₁Cl₂N₃O + H: 320.0357; Found: 320.0346.

**2-(5-(2,4-dichlorophenyl)-3-methoxy-1H-pyrazol-1-yl)pyridine (example 144):** This compound was obtained as a solid in 25 % yield after a chromatography over silica gel (dichloromethane - ethanol 92/8) followed by a chromatography over alumina containing 1.5 % water (cyclohexane - ethyl acetate 95/5). ¹H NMR (CDCl₃): 4.04 (s, 3H); 5.94 (s, 1H); 7.04 (m, 1H); 7.29 (m, 2H); 7.39 (d, 1H, J = 1.9 Hz); 7.71 (m, 2H); 8.07 (m, 1H). ¹³C NMR (CDCl₃): 56.3; 97.3; 114.9; 120.7; 127.8; 129.1; 130.8; 131.8; 134.6; 134.8; 138.1; 140.6; 147.5; 152.5; 164.0. HRMS: Calc. for C₁₅H₁₁Cl₂N₃O + H: 320.0357; Found: 320.0344.

**2-(3-ethoxy-5-(4-methoxyphenyl)-1H-pyrazol-1-yl)pyridine (example 145):** Obtained as a white solid in 87 % yield after a chromatography over silica gel (dichloromethane - ethanol 99/1 to 95/5) and a second over alumina containing 1.5 % water (cyclohexane - dichloromethane 1/2). ¹H NMR (CDCl₃): 1.44 (t, 3H, *J =* 7.1 Hz); 3.81 (s, 3H); 4.34 (q, 2H, J = 7.1 Hz); 5.94 (s, 1H); 6.84 (m, 2H); 7.12 (m, 1H); 7.20 (m, 2H); 7.39 (m, 1H); 7.67 (m, 1H); 8.33 (m, 1H). ¹³C NMR (CDCl₃): 14.9; 55.2; 64.7; 95.3; 113.6; 118.2; 121.4; 123.7; 130.0; 137.9; 145.0; 148.3; 152.6; 159.6; 163.8. HRMS: Calc. for C₁₇H₁₇N₃O₂ + H: 296.1399; Found: 296.1366.

**2-(5-(4-tert-butylphenyl)-3-methoxy-1H-pyrazol-1-yl)pyridine (example 146):** This compound was obtained as a solid in 26 % yield after a chromatography over silica gel (dichloromethane - ethanol 95/5) followed by a chromatography over alumina containing 1.5 % water (cyclohexane - ethyl acetate 95/5). ¹H NMR (CDCl₃): 1.33 (s, 9H); 4.04 (s, 3H); 5.99 (s, 1H); 7.21 (m, 3H); 7.34 (m, 3H); 7.69 (m, 1H); 8.29 (m, 1H). ¹³C NMR (CDCl₃): 31.2; 34.6; 56.2; 95.3; 118.4.; 121.5; 125.2; 128.1; 128.3; 137.9; 145.3; 148.5; 151.4; 152.5; 164.5. HRMS: Calc. for C₁₉H₂₁N₃O + H: 308.1763; Found: 308.1746.

**2-(5-(4-chlorophenyl)-3-ethoxy-1H-pyrazol-1-yl)pyridine (example 147):** Obtained as a white solid in 38 % yield after a chromatography over silica gel (cyclohexane - ethyl acetate 95/5) and evaporation under high vacuum to remove some reduced material. ¹H NMR (CDCl₃): 1.42 (t, 3H, *J=* 7.1 Hz); 4.34 (q, 2H, *J=* 7.1 Hz); 5.96 (s, 1H); 7.12 (m, 1H); 7.22 (m, 2H); 7.31 (m, 2H); 7.52 (m, 1H); 7.73 (m, 1H); 8.25 (m, 1H). ¹³C NMR (CDCl₃): 14.8; 64.8; 96.2; 117.6; 121.4; 128.3; 130.0 (two signals); 134.2; 138.1; 143.9; 148.0; 152.4; 163.8. HRMS: Calc. for C₁₆H₁₄ClN₃O + H: 300.0904; Found: 300.0872.

**- Preparation of 3-methoxypyrazoles from 1,1-dimethoxyethene and unsymmetrical anhydrides.** The considered acid (6 mmol) and poly(4-vinylpyridine) (3.7 g, dried using a Dean-Stark apparatus and boiling toluene) were dispersed in dry dichloromethane (40 mL, dried over 4A molecular sieves, stabilized with amylene). Pivaloyl chloride (6.3 mmol) was added and the moisture-protected suspension was stirred for one hours. This was filtered and the solution concentrated to dryness before using it in the next step. In a 60 mL tube fitted with a Teflon-covered screw cap, the considered unsymmetrical anhydride (6 mmol) and 1,1-dimethoxyethene (1.3 mL, 12.6 mmol) were heated at 110 °C for 40 mn. 2-Pyridylhydrazine (0.65 g) was then added and this was heated at 110 °C for ten minutes. The resulting oil was further purified as described below.

**2-(3-methoxy-5-(3-methoxybenzyl)-1H-pyrazol-1-yl)pyridine (example 148):** This compound was obtained as oil in 25 % yield after a chromatography over alumina containing 1.5 % water (cyclohexane - dichloromethane 80/20). ¹H NMR (CDCl₃): 3.78 (s, 3H); 3.94 (s, 3H); 4.53 (s, 2H); 5.55 (s, 1H); 6.76 (m, 1H); 6.82 (m, 2H); 7.10 (m, 1H); 7.20 (t, 1H, J = 7.8 Hz); 7.72 (m, 1H); 7.82 (m, 1H); 8.38 (m, 1H). ¹³C NMR (CDCl₃): 34.8; 55.1; 56.0; 95.9; 111.8; 114.8; 115.0; 120.1; 121.5; 129.3; 138.1; 140.3; 146.1; 147.2; 153.7; 159.6; 163.9. HRMS: Calc. for C₁₇H₁₇N₃O₂ + H: 296.1399; Found: 296.1394.

### - Examples of modification on carbon 5 of 3-alkoxypyrazoles, anion-based reaction

**(4-chlorophenyl)(3-ethoxy-1-(pyridin-2-yl)-1H-pyrazol-5-yl)methanone (example 149):** Under an argon atmosphere, 2-(3-ethoxy-1H-pyrazol-1-yl)pyridine (0.5 g, 2.64 mmol) was dissolved in dry tetrahydrofuran (6 mL, purchased) and the solution was cooled to -78 °C using a dry ice bath. A 2M solution of n-butyl lithium in cyclohexane (1.45 mL, 2.91 mmol) was added via a syringe. The solution was then stirred at -78 °C for 40 min, quenched with 4-chlorobenzoyl chloride (0.47 mL, 3.69 mmol) and then allowed to warm to room temperature. The resulting yellow mixture was extracted with ethyl acetate, washed with a saturated solution of sodium hydrogenocarbonate, water, dried over magnesium sulfate and concentrated to dryness. The residue was purified by chromatography over silica gel (dichloromethane) to yield compound the 5-benzol derivative as a light yellow solid (0.5 g, 58 %). ¹H NMR (CDCl₃): 1.47 (t, 3H); 4.38 (q, 2H); 6.13 (s, 1H); 7.28 (m, 1H); 7.38 (m, 1H); 7.74 (m, 1H); 7.97 (m, 1H). ¹³C NMR (CDCl₃): 14.7; 65.3; 97.5; 113.2; 120.8; 128.8; 130.5; 135.9; 138.4; 139.4; 140.7; 147.0; 150.6; 163.7; 186.5. HRMS: Calc. for C₁₇H₁₄N₃O₂Cl + H: 328.0853; Found: 328.0817.

### - Examples of modifications on carbon 4 of 3-alkoxypyrazoles, anion-based chemistry

**(3-ethoxy-1-(5-ethylpyrimidin-2-yl)-5-methyl-1H-pyrazol-4-yl)(phenyl)methanone (example 150**)**:** Under an inert atmosphere, a solution of 2-(3-ethoxy-4-iodo-5-methyl-1H-pyrazol-1-yl)-5-ethylpyrimidine (example 106) (0.27 g, 0.75 mmol) in dry tetrahydrofuran (5 mL) was cooled to -78 °C. A 2N solution of butyllithium in cyclohexane (0.4 mL, 0.79 mmol) was added, the resulting precipitate was stirred for three minutes and benzoyl chloride (0.09 mL, 0.83 mmol) was added. The resulting suspension was diluted in water, extracted with ethyl acetate and the organic layer was washed with brine, dried over magnesium sulfate and concentrated to dryness. The resulting residue was purified first by a chromatography over silica gel (cyclohexane-ethyl acetate from 2/1) followed by a second chromatography over alumina oxide containing 1.5 % of water (cyclohexane-dichloromethane from 1/1 to 1/2) to yield the 4-benzoyl derivative (0.06 g, 23 %) as a solid. ¹H (CDCl₃): 1.19 (t, 3H, J = 7.2); 1.32 (t, 3H, J = 7.0); 2.70 (q, 2H, J = 7.2); 2.79 (s, 3H); 4.37 (q, 2H, J = 7.0); 7.42 (m, 2H); 7.54 (m, 1H); 7.85 (m, 2H); 8.65 (s, 2H). ¹³C (CDCl₃): 14.3; 14.4; 14.9; 23.1; 64.8; 110.2; 127.8; 129.5; 132.3; 133.9; 139.0; 147.7; 155.6; 157.9; 161.9; 161.9; 190.7. HRMS: Calc. for C₁₉H₂₀N₄O₂ + H: 337.1665; Found: 337.1632.

**(1-(5-cyclopropylpyridin-2-yl)-3-ethoxy-5-methyl-1H-pyrazol-4-yl)(phenyl)methanone (example 151):** By using the protocol described for the preparation of (3-ethoxy-1-(5-ethylpyrimidin-2-yl)-5-methyl-1H-pyrazol-4-yl)(phenyl)methanone, this ketone was obtained from as a glass (0.13 g, 44 %). ¹H (CDCl₃): 0.78 (m, 2H); 1.09 (m, 2H); 1.23 (t, 3H, J = 7.2); 1.92 (m, 1H); 2.75 (s, 3H); 4.28 (q, 2H, J = 7.2); 7.47 (m, 4H); 7.53 (m, 1H); 7.64 (d, 1H, J = 8.5); 7.84 (dd, 1H, J = 8.5 and 2.3); 8.29 (d, 1H, J = 2.3). ¹³C (CDCl₃): 9.1; 12.7; 13.8; 14.4; 64.6; 108.8; 116.9; 127.7; 129.4; 132.0; 135.1; 137.7; 139.4; 145.9; 146.2; 150.5; 161.3; 190.7. HRMS: Calc. for C₂₁H₂₁N₃O₂ + H: 348.1712; Found: 348.1738.

2-(3-ethoxy-5-methyl-4-(phenylthio)-1H-pyrazol-1-yl)-5-ethylpyrimidine (example 152): Under an argon atmosphere, 2-(3-ethoxy-4-iodo-5-methyl-1H-pyrazol-1-yl)pyridine (0.23 g, 0.64 mmol) was dissolved in dry tetrahydrofuran (5 mL, purchased). This was cooled to -78 °C with a dry ice bath and 2M butyl lithium in cyclohexane (0.34 mL, 0.69 mmol) was added. A solution of 1,2-diphenyldisulfane (0.15 g, 0.69 mmol) in dry THF ( mL) was added after ten seconds of stirring and the solution was stirred an additional 10 minutes at -78 °C before allowing it to warm to room temperature. The resulting solution was adsorbed on silica and purified by two consecutive chromatographies, the first one over silica gel (dichloromethane) and the second also over silica gel (cyclohexane - ethyl acetate 3/1) to yield the target thioether (0.01 g, 4.5 %). ¹H (CDCl₃): 1.32 (t, 3H, J = 7.3); 1.39 (t, 3H, J = 7.7); 2.70 (q, 2H, J = 7.7); 2.72 (s, 3H); 4.49 (q, 2H, J = 7.3); 7.11 (m, 1H); 7.17 (m, 2H); 7.22 (m, 2H); 8.62 (s, 2H). ¹³C (CDCl₃): 14.1; 14.7; 15.0; 23.0; 65.0; 97.8; 125.1; 126.2; 128.7; 133.1; 137.6; 148.4; 155.9; 157.8; 164.6. HRMS: Calc. for C₁₈H₂₀N₄OS + H: 341.1436; Found: 341.1404.

### - Examples of modifications on carbon 4 of 3-alkoxypyrazoles, Suzuki-Miyaura reactions

**2-(3-ethoxy-5-methyl-4-phenyl-1H-pyrazol-1-yl)-5-ethylpyrimidine (example 153):** In a vial adapted for microwave heating, 2-(3- ethoxy-4-iodo-5-methyl-1H-pyrazol-1-yl)-5-ethylpyrimidine (0.23 g, 0.64 mmol), phenylboronic acid (0.086 g, 0.70 mmol) and cesium carbonate (0.62 g, 1.93 mmol) were dissolved in a 2/3 mixture of propanol and water (3 mL). This was degassed by a gentle steam of argon, [1,1'-bis(diphenylphosphino)ferrocene] dichloropalladium complexed with dichloromethane (0.026 g, 0.032 mmol) was added and the sealed tube heated at 120 °C for 30 minutes. The resulting solution was diluted in water, extracted with ethyl acetate. The organic layer was washed with brine, dried over sodium sulfate and concentrated to dryness. The residue was purified first by a chromatography over alumina containing 1.5 % water (cyclohexane-dichloromethane 1/2) to yield the 4-phenyl derivative as a solid (0.12 g, 60 %). ¹H (CDCl₃): 1.31 (t, 3H, J = 7.4); 1.42 (t, 3H, J = 7.0); 2.68 (q, 2H, J = 7.4); 2.70 (s, 3H); 4.51 (q, 2H, J = 7.0); 7.33 (m, 1H); 7.45 (m, 4H); 8.61 (s, 2H). ¹³C (CDCl₃): 14.5; 14.8; 15.1; 23.1; 64.7; 111.2; 126.6; 128.3; 129.4; 131.4; 132.4; 140.0; 156.1; 157.7; 162.1. HRMS: Calc. for C₁₈H₂₀N₄O + H: 309.1715; Found: 309.1681.

**2-(4-(2,4-difluorophenyl)-3-ethoxy-5-methyl-1H-pyrazol-1-yl)-5-ethylpyrimidine (example 154):** By using the 4-arylation protocol described above, with 2,4-difluorophenylboronic acid, this compound was obtained 62 % yield as a solid. ¹H (CDCl₃): 1.29 (t, 3H, J = 7.3); 1.36 (t, 3H, J = 7.0); 2.54 (s, 3H); 2.67 (q, 2H, J = 7.3); 4.47 (q, 2H, J = 7.0); 6.92 (m, 2H); 7.37 (m, 1H); 8.59 (s, 2H). ¹³C (CDCl₃): 14.4; 14.7; 15.0; 23.0; 64.7; 104.1 (J = 51); 104.3 (J = 43); 111.2 (J = 21); 115.1 (J = 16); 132.5; 133.1; 141.6; 156.1; 157.7; 160.2 (J = 12 and 250); 161.9; 162.4 (J = 11 and 249). HRMS: Calc. for C₁₈H₁₈F₂N₄O + H: 345.1527; Found: 345.1538.

**2-(4-(2-chlorophenyl)-3-ethoxy-5-methyl-1H-pyrazol-1-yl)pyridine (example 155):** By using the 4-arylation protocol described above with 2-chlorophenylboronic acid, followed by drying the corresponding fraction under high vacuum to remove some reduced material, this compound was obtained in 18 % yield as a solid. ¹H NMR (CDCl₃): 1.40 (t, 3H, *J =* 7.0 Hz); 2.56 (s, 3H); 4.38 (q, 2H, *J* = 7.0 Hz); 7.12 (m, 1H); 7.33 (m, 3H); 7.50 (m, 1H); 7.79 (m, 1H); 7.88 (m, 1H); 8.41 (m, 1H). ¹³C NMR (CDCl₃): 14.1; 14.8; 64.4; 107.9; 115.1; 120.1; 126.6; 128.7; 129.6; 130.5; 132.9; 134.8; 138.2; 140.2; 147.3; 153.7; 160.8. HRMS: Calc. for C₁₇H₁₆ClN₃O + H: 314.1060; Found: 314.1041.

**2-(4-(3-chlorophenyl)-3-ethoxy-5-methyl-1H-pyrazol-1-yl)pyridine (example 156):** By using the 4-arylation protocol described above with 3-chlorophenylboronic acid, this compound was obtained in 71 % yield as a solid. ¹H NMR (CDCl₃): 1.46 (t, 3H, *J =* 7.0 Hz); 2.73 (s, 3H); 4.42 (q, 2H, *J* = 7.0 Hz); 7.15 (m, 1H); 7.27 (m, 1H); 7.37 (m, 2H); 7.50 (m, 1H); 7.82 (m, 2H); 8.44 (m, 1H). ¹³C NMR (CDCl₃): 14.0; 14.8; 64.5; 108.6; 115.7; 120.3; 126.3; 127.3; 129.1; 129.5; 133.7; 134.1; 138.2; 139.2; 147.4; 153.7; 160.9. HRMS: Calc. for C₁₇H₁₆ClN₃O + H: 314.1060; Found: 314.1041.

**2-(4-(4-chlorophenyl)-3-ethoxy-1H-pyrazol-1-yl)pyridine (example 157):** By using the 4-arylation protocol described above with 4-chlorophenylboronic acid, this compound was obtained in 61 % yield as a solid. ¹H NMR (CDCl₃): 1.52 (t, 3H, *J* = 7.0 Hz); 4.49 (q, 2H, J= 7.0 Hz); 7.10 (m, 1H); 7.35 (m, 2H); 7.70 (m, 2H); 7.77 (m, 1H); 7.82 (m, 1H); 8.37 (m, 1H). 8.66 (s, 1H). ¹³C NMR (CDCl₃): 14.9; 65.0; 109.6; 111.3; 120.2; 124.7; 127.3; 128.9; 130.1; 131.8; 138.5; 147.9; 151.1; 161.7. HRMS: Calc. for C₁₆H₁₄ClN₃O + H: 300.0904; Found: 300.0889.

**2-(3-ethoxy-5-methyl-4-(3-(trifluoromethyl)phenyl)-1H-pyrazol-1-yl)pyridine (example 158):** By using the 4-arylation protocol described above with 3-trifluoromethylphenylboronic acid, this compound was obtained in 47 % yield as a solid. ¹H NMR (CDCl₃): 1.45 (t, *3H, J =* 7.0 Hz); 2.74 (s, 3H); 4.43 (q, 2H, *J =* 7.0 Hz); 7.16 (m, 1H); 7.55 (m, 2H); 7.68 (m, 1H); 7.81 (m, 3H); 8.44 (m, 1H). ¹³C NMR (CDCl₃): 13.9; 14.8; 64.6; 108.6; 115.7; 120.4; 122.9; 124.3 (J = 273 Hz); 125.8; 128.7; 130.7 (J = 32 Hz); 132.3; 132.7; 138.2; 139.2; 147.4; 153.6; 160.9. HRMS: Calc. for C₁₈H₁₆F₃N₃O + H: 348.1324; Found: 348.1303.

**2-(3-ethoxy-5-methyl-4-(4-(trifluoromethyl)phenyl)-1H-pyrazol-1-yl)pyridine (example 159):** By using the 4-arylation protocol described above with 4-trifluoromethylphenylboronic acid, this compound was obtained in 76 % yield as a solid. ¹H NMR (CDCl₃): 1.46 (t, 3H, *J* = 7.0 Hz); 2.75 (s, 3H); 4.43 (q, 2H, *J=* 7.0 Hz); 7.16 (m, 1H); 7.64 (m, 4H); 7.83 (m, 2H); 8.43 (m, 1H). ¹³C NMR (CDCl₃): 14.0; 14.8; 64.6; 108.6; 115.8; 120.4; 124.4 (J = 270 Hz); 125.2; 128.2 (J = 32 Hz); 129.2; 135.7; 138.2; 139.4; 147.4; 153.6; 160.9. HRMS: Calc. for C₁₈H₁₆F₃N₃O + H: 348.1324; Found: 348.1294.

**2-(3-ethoxy-4-(4-methoxyphenyl)-5-methyl-1H-pyrazol-1-yl)pyridine (example** 160): By using the 4-arylation protocol described above with 4-methoxyphenylboronic acid, this compound was obtained in 72 % yield as a solid. ¹H NMR (CDCl₃): 1.35 (t, 3H, *J* = 7.0 Hz); 2.61 (s, 3H); 3.77 (s, 3H); 4.31 (q, 2H, *J=* 7.0 Hz); 6.89 (m, 2H); 7.01 (m, 1H); 7.31 (m, 2H); 7.70 (m, 2H); 8.32 (m, 1H). ¹³C NMR (CDCl₃): 14.0; 14.3; 55.3; 64.4; 109.6; 113.9; 115.5; 117.5; 119.9; 124.1; 130.4; 138.1; 138.6; 147.4; 158.2; 161.2. HRMS: Calc. for C₁₈H₁₉N₃O₂ + H: 310.1556; Found: 310.1537.

**2-(3-ethoxy-4-(4-methoxyphenyl)-5-methyl-1H-pyrazol-1-yl)-5-ethylpyrimidine (example 161):** By using the 4-arylation protocol described above with 4-methoxyphenylboronic acid, and 2-(3-ethoxy-4-iodo-5-methyl-1H-pyrazol-1-yl)-5-ethylpyrimidine (example 106), this compound was obtained in 52 % yield as a solid. ¹H NMR (CDCl₃): 1.30 (t, 3H, *J =* 7.4 Hz); 1.41 (t, 3H, *J* = 7.0 Hz); 2.67 (m, 5H); 3.85 (s, 3H); 4.49 (q, 2H, J= 7.0 Hz); 6.98 (m, 2H); 7.40 (m, 2H); 8.59 (s, 2H). ¹³C NMR (CDCl₃): 14.5; 14.8; 15.1; 23.0; 55.3; 64.6; 110.8; 113.8; 123.7; 130.6; 132.2; 139.6; 156.2; 157.7; 158.4; 162.1. HRMS: Calc. for C₁₉H₂₂N₄O₂ + H: 339.1821; Found: 339.1795.

**5-cyclopropyl-2-(3-ethoxy-5-methyl-4-phenyl-1H-pyrazol-1-yl)pyridine (example 161A):** In a vial adapted for microwave heating, 2-(4-bromo-3-ethoxy-5-methyl-1H-pyrazol-1-yl)-5-cyclopropylpyridine (intermediate 63) (0.21 g, 0.65 mmol), phenylboronic acid (0.087 g, 0.71 mmol) and cesium carbonate (0.53 g, 1.69 mmol) were dissolved in a 2/3 mixture of propanol and water (5 mL). This was degassed by a gentle steam of argon, [1,1'-bis(diphenylphosphino)ferrocene] dichloropalladium complexed with dichloromethane (0.026 g, 0.032 mmol) was added and the sealed tube heated at 120 °C for 30 minutes. The resulting solution was diluted in water, extracted with ethyl acetate. The organic layer was washed with brine, dried over sodium sulfate and concentrated to dryness. The residue was purified first by a chromatography over alumina containing 1.5 % water (cyclohexane-dichloromethane from 1/0 to 1/1) to yield the 4-phenyl derivative as a solid (0.07 g, 33 %). ¹H (CDCl₃): 0.74 (m, 2H); 1.05 (m, 2H); 1.44 (t, 3H, J = 7.2); 1.93 (m, 1H); 2.68 (s, 3H); 4.40 (q, 2H, J = 7.2); 7.27 (m, 1H); 7.43 (m, 3H); 7.51 (m, 2H); 7.68 (d, 1H, J = 8.4); 8.24 (d, 1H, J = 2.3). ¹³C (CDCl₃): 8.7; 12.6; 13.7; 14.9; 64.4; 109.3; 115.6; 126.2; 128.3; 129.2; 131.9; 135.1; 135.9; 138.9; 145.6; 151.6; 160.9. HRMS: Calc. for C₂₀H₂₁N₃O + H: 320.1763; Found: 320.1747.

- **Examples of modifications on carbon 4 of 3-alkoxypyrazoles, Negishi reactions** General procedure: in a tube adapted for microwave oven, 2-(3-ethoxy-4-iodo-5-methyl-1H-pyrazol-1-yl)pyridine (0.3 g, 0.91 mmol), or the corresponding halogenated substrate, and [1,1'-bis(diphenylphosphino)ferrocene] dichloropalladium complexed with dichloromethane (0.037 g, 0.045 mmol) were degassed with argon. A 0.5 M solution of the corresponding benzylzinc halide in tetrahydrofuran (5.4 mL, 2.73 mmol) was added; the tube was sealed and heated in the microwave oven at 110 °C for 1 hour. The resulting solution was dispersed in a mixture of water and ethyl acetate, the organic layer was washed with a 0.1 M solution of Rochelle salt (potassium and sodium tartrate), brine, dried over magnesium sulfate and concentrated to dryness. The resulting residue was further purified as described below for each cases.

**2-(4-(2-chlorobenzyl)-3-ethoxy-5-methyl-1H-pyrazol-1-yl)pyridine (example 162):** Obtained in 36 % yield as an oil after two chromatography the first over silica gel (cyclohexane - dichloromethane 1/1), the second over alumina containing 1.5 % water (cyclohexane - dichloromethane 9/1). ¹H NMR (CDCl₃): 1.38 (t, 3H, *J* = 7.1 Hz); 2.58 (s, 3H); 3.86 (s, 2H); 4.35 (q, 2H, *J=* 7.1 Hz); 7.07 (m, 1H); 7.17 (m, 3H); 7.38 (m, 1H); 7.74 (m, 1H); 7.82 (m, 1H); 8.39 (m, 1H). ¹³C NMR (CDCl₃): 13.3; 14.8; 25.3; 64.2; 105.0; 115.0; 119.7; 126.6; 127.2; 129.1; 129.9; 133.8; 137.9; 138.0; 140.1; 147.2; 153.9; 162.5. HRMS: Calc. for C₁₈H₁₈ClN₃O + H: 328.1217; Found: 328.1169.

**2-(4-(3-chlorobenzyl)-3-ethoxy-5-methyl-1H-pyrazol-1-yl)pyridine (example 163):** Obtained in 51 % yield as an oil after two chromatography the first over silica gel (cyclohexane - dichloromethane 2/1), the second over alumina containing 1.5 % water (cyclohexane - dichloromethane 9/1). ¹H NMR (CDCl₃): 1.41 (t, 3H, *J=* 7.0 Hz); 2.60 (s, 3H); 3.73 (s, 2H); 4.35 (q, 2H, *J* = 7.0 Hz); 7.06; (m, 1H); 7.17 (m, 3H); 7.22 (m, 1H); 7.72 (m, 1H); 7.78 (m, 1H); 8.38 (m, 1H). ¹³C NMR (CDCl₃): 13.2; 14.9; 27.5; 64.2; 106.1; 115.0; 119.7; 126.0; 126.4; 128.4; 129.5; 134.1; 138.0; 139.6; 143.0; 147.2; 153.9; 162.2. HRMS: Calc. for C₁₈H₁₈ClN₃O + H: 328.1217; Found: 328.1206.

**4-benzyl-3-ethoxy-1-(4-methoxyphenyl)-1H-pyrazole (example 164):** Obtained in 42 % yield from 3-ethoxy-4-iodo-1-(4-methoxyphenyl)-1H-pyrazole (intermediate 48) and benzylzinc chloride as an oil after a chromatography over silica (cyclohexane-dichloromethane 1/1). ¹H NMR (CDCl₃): 1.45 (t, 3H, J = 7.0); 3.78 (s, 2H); 3.83 (s, 3H); 4.38 (q, 2H, J = 7.0); 6.91 (m, 2H); 7.25 (m, 1H); 7.30 (m, 5H); 7.45 (m, 2H). ¹³C NMR (CDCl₃): 15.0; 28.6; 55.5; 64.7; 107.9; 114.4; 119.1; 126.0; 126.3; 128.4; 128.6; 134.3; 140.8; 157.1; 162.0. HRMS: Calc. for C₁₉H₂₁N₂O₂ + H: 309.1603; Found: 309.1600.

**2-(4-benzyl-3-isopropoxy-5-methyl-1H-pyrazol-1-yl)pyridine (example 165):** By using the procedure described above, from 2-(4-bromo-3-isopropoxy-5-methyl-1H-pyrazol-1-yl)pyridine (intermediate 47) and benzylzinc bromide but heating only at 85 °C for 4 h, the compound was obtained in 30 % yield after a chromatography over silica gel (dichloromethane - cyclohexane 9/1). ¹H NMR (CDCl₃): 1.39 (d, 6H, J = 6.0); 2.60 (s, 3H); 3.74 (s, 2H); 5.03 (sept, 1H, J = 6.0); 7.05 (m, 1H); 7.18 (m, 1H); 7.27 (m, 4H); 7.75 (m, 2H); 8.37 (m, 1H). ¹³C NMR (CDCl₃): 13.2; 22.2; 27.8; 71.2; 107.7; 115.0; 119.4; 125.7; 128.2; 128.3; 138.0; 139.3; 141.0; 147.1; 154.0; 161.7. HRMS: Calc. for C₁₉H₂₁N₃O + H: 308.1763; Found: 308.1773.

**Attempt to achieve a Negishi reaction with 2-(3-ethoxy-4-iodo-1H-pyrazol-1-yl)-5-ethylpyrimidine; preparation of 2-(3-ethoxy-1H-pyrazol-1-yl)-5-ethylpyrimidine (example 166) and 4-benzyl-2-(3-ethoxy-4-iodo-1H-pyrazol-1-yl)-5-ethylpyrimidine (example 167).** By using the procedure described above, from 2-(3-ethoxy-4-iodo-1H-pyrazol-1-yl)-5-ethylpyrimidine (example 107) and benzylzinc bromide but heating at 60 °C for 16 hours, these two compounds were isolated from the reaction mixture as described here. The crude residue was subjected to an initial chromatography over silica gel (cyclohexane - ethylacetate from 9/1 to 3/1). The fraction containing the reduced material was further purified by a chromatography over alumina containing 1.5 % water (cyclohexane - dichloromethane from 2/1 to 1/1) to yield the 2-(3-ethoxy-1H-pyrazol-1-yl)-5-ethylpyrimidine as a solid (15 %) (example 166). ¹H NMR (CDCl₃): 1.27 (t, 3H, J = 7.4); 1.42 (t, 3H, J = 7.1); 2.65 (q, 2H, J = 7.4); 4.45 (q, 2H, J = 7.1); 5.96 (d, 1H, J = 2.8); 8.39 (d, 1H, J = 2.8); 8.52 (s, 2H). ¹³C NMR (CDCl₃): 14.5; 15.1; 23.0; 65.0; 96.7; 130.6; 132.5; 154.3; 157.9; 165.9. HRMS: Calc. for C₁₁H₁₄N₄O + H: 219.1246; Found: 219.1208. The fraction containing the benzylated material was further purified by a chromatography over alumina containing 1.5 % water (cyclohexane - dichloromethane from 95/5) and a recrystallisation in cyclohexane to yield the 4-benzyl-2-(3-ethoxy-4-iodo-1H-pyrazol-1-yl)-5-ethylpyrimidine (7%) (example 167). ¹H NMR (CDCl₃): 1.18 (t, 3H, J = 7.3); 1.48 (t, 3H, J = 7.1); 2.65 (q, 2H, J = 7.3); 4.16 (s, 2H); 4.50 (q, 2H, J = 7.1); 7.30 (m, 5H); 8.49 (s, 1H); 8.50 (s, 1H). ¹³C NMR (CDCl₃): 14.3; 14.7; 22.1; 41.0; 50.7; 65.9; 126.8; 128.6; 128.8; 131.3; 131.4; 137.1; 153.2; 158.5; 164.7; 168.3. HRMS: Calc. for C₁₈H₁₉IN₄O + H: 435.0682; Found: 435.0686.

- **Examples of modification of a methyl group on position 5 of 3-alkoxypyrazole. 2-(4-henzyl-3-isopropoxy-5-(methoxymethyl)-1H-pyrazol-1-yl)-5-cyclopropylpyrimidine (example 168):** Under an inert atmosphere, 2-(4-benzyl-5-(bromomethyl)-3-isopropoxy-1H-pyrazol-1-yl)-5-cyclopropylpyrimidine (intermediate 43) (0.28 g, 0.65 mmol) and a 30 % solution of sodium methanolate in methanol (1 mL) were stirred overnight. This was dispersed in water and ethyl acetate, the organic layer was washed with water, dried over magnesium sulfate and concentrated to dryness. The resulting residue was purified by two consecutive chromatography, the first one over silica gel (cyclohexane-ethyl acetate 3/1), the second one over alumina containing 1.5 % of water (cyclohexane-dichloromethane 1/2) followed by the use of high vacuum to yield the methoxy ether as an oil (0.07 g, 23 %). ¹H (CDCl₃): 0.76 (m, 2H); 1.08 (m, 2H); 1.32 (d, 6H, J = 6.1); 1.89 (m, 1H); 3.26 (s, 3H); 3.83 (s, 2H); 4.87 (s, 2H); 5.22 (sept, 1H, J = 6.1); 7.18 (m, 1H); 7.27 (m, 4H); 8.46 (s, 2H). ¹³C (CDCl₃): 8.3; 10.4; 22.2; 27.8; 58.0; 64.8; 71.5; 111.8; 125.8; 128.2; 128.4; 132.4; 139.2; 140.6; 155.6; 156.2; 162.3. HRMS: Calc. for C₂₂H₂₆FN₄O₂ + H: 379.2134; Found: 379.2084.

**N-((4-benzyl-1-(5-cyclopropylpyrimidin-2-yl)-3-isopropoxy-1H-pyrazol-5-yl)methyl)-N-ethylethanamine (example 169):** Under an inert atmosphere, 2-(4-benzyl-5-(bromomethyl)-3-isopropoxy-1H-pyrazol-1-yl)-5-cyclopropylpyrimidine (intermediate 43) (0.18 g, 0.43 mmol) and diethylamine (1 mL) were stirred overnight. This was dispersed in water and ethyl acetate and made basic with a saturated solution of sodium bicarbonate. The organic layer was washed with water, dried over magnesium sulfate and concentrated to dryness. The resulting residue was purified by a chromatography over silica gel (dichloromethane -ethanol 95/5 to yield the diethylamine as an oil (0.07 g, 39 %). ¹H (CDCl₃): 0.75 (m, 8H); 1.07 (m, 2H); 1.32 (d, 6H, J = 6.1); 1.88 (m, 1H); 2.35 (q, 4H, J = 6.9); 3.82 (s, 2H); 3.94 (s, 2H); 5.18 (sept, 1H, J = 6.1); 7.16 (m, 1H); 7.25 (m, 4H); 8.44 (s, 2H). ¹³C (CDCl₃): 8.4; 10.5; 11.3; 22.3; 27.8; 46.2; 48.4; 71.2; 110.2; 125.6; 128.0; 128.4; 132.7; 141.0; 141.2; 155.9; 156.1; 162.3. HRMS: Calc. for C₂₅H₃₃N₅O + H: 420.2763; Found: 420.2853.

### Other type of modifications of 3-alkoxypyrazoles

**2-(4-benzyl-3-ethoxy-5-methyl-1H-pyrazol-1-yl)-5-isopropylpyridine (example 170):** The 2-(6-(4-benzyl-3-ethoxy-5-methyl-1H-pyrazol-1-yl)pyridin-3-yl)propan-2-ol (example 34) (0.17 g, 0.48 mmol) was dissolved in dichloromethane (10 mL) and trifluoroacetic acid (2 mL). Triethylsilane (0.28 mL, 1.75 mmol) was added and the reaction was stirred at room temperature for four hours. An LC/MS pointed out a very slow reaction. Trifluoromethane sulfonic acid (0.2 mL, 2.26 mmol) was added followed by some more triethylsilane (0.2 mL, 1.25 mmol). A hydrogen evolution was observed and LC/MS monitoring pointed out the occurrence of the reduced derivative. More triethylsilane (0.2 mL, 1.25 mmol) was added and this was stirred 24 hours. The resulting solution was diluted in ethyl acetate, washed until neutrality with saturated sodium hydrogenocarbonate, brine, dried over magnesium sulfate and concentrated to dryness. The residue was purified by two consecutive chromatography, the first one over silica gel (cyclohexane-ethyl acetate from 97/3), the second one over silica gel (toluene), to yield the title compound (0.04 g, 24 %) as an oil. ¹H (CDCl₃): 1.30 (t, 6H, J = 6.8); 1.41 (t, 3H, J = 7.1); 2.55 (s, 3H); 2.96 (sept, 1H, J = 6.8); 3.75 (s, 2H); 4.35 (q, 2H, J = 7.1); 7.18 (m, 1H); 7.28 (m, 4H); 7.61 (dd, 1H, J = 2.4 and 8.6); 7.67 (d, 1H, J = 8.6); 8.25 (d, 1H, J = 2.4). ¹³C (CDCl₃): 11.9; 13.9; 22.7; 26.7; 30.2; 63.1; 105.2; 114.1; 124.7; 127.2 (two signals); 135.0; 138.1; 138.8; 140.0; 144.5; 151.1; 161.1. HRMS: Calc. for C₂₁H₂₅N₃O + H: 336.2076; Found: 336.2051.

**2-(4-benzyl-3-ethoxy-5-methyl-1H-pyrazol-1-yl)-5-methylpyrimidine (example 171):** In a tube adapted for microwave oven, 2-(4-benzyl-3-ethoxy-5-methyl-1H-pyrazol-1-yl)-5-bromopyrimidine (example 42) (0.24 g, 0.64 mmol), cesium carbonate (1.04 g, 3.21 mmol), methylboronic acid (0.11 g, 1.93 mmol) in dimethylformamide (4 ml, dried over 4A molecular sieves) were mixed. This suspension was degassed by a gentle stream of argon, [1,1'-bis(diphenylphosphino)ferrocene dichloropalladium complexed with dichloromethane (0.026 g, 0.032 mmol) was added, the tube was sealed and heated in a microwave oven at 100 °C for one hour. The resulting suspension was diluted in water, extracted with ethyl acetate. The organic layer was washed with brine, dried over sodium sulfate and concentrated to dryness. The residue was purified first by a chromatography over silica gel (cyclohexane-ethyl acetate from 2/1 to 3/2) followed by a second chromatography over alumina oxide containing 1.5 % of water (cyclohexane-dichloromethane from 2/1 to 1/1) to yield the 5-methyl derivative as a white powder (0.06 g, 30 %). ¹H (CDCl₃): 1.38 (t, 3H, J = 7.0); 2.30 (s, 3H); 2.55 (s, 3H); 3.75 (s, 2H); 4.44 (q, 2H, J = 7.0); 7.17 (m, 1H); 7.24 (m, 4H); 8.53 (s, 2H). ¹³C (CDCl₃): 13.8; 14.9; 15.0; 27.7; 64.4; 108.3; 125.8; 125.9; 128.2; 128.3; 140.5; 140.6; 156.0; 158.3; 163.4. HRMS: Calc. for C₁₈H₂₀N₄O + H: 309.1715; Found: 309.1705.

**2-(4-benzyl-3-ethoxy-5-methyl-1H-pyrazol-1-yl)-5-cyclopropylpyridine (example 1**.**72**)**:** In a tube adapted for microwave oven, 22-(4-benzyl-3-ethoxy-5-methyl-1H-pyrazol-1-yl)-5-bromopyridine (example 41) (0.16 g, 0.43 mmol), cesium carbonate (0.7 g, 2.14 mmol), cyclopropylboronic acid (0.11 g, 1.28 mmol) in dimethylformamide (4 ml, dried over 4A molecular sieves) were mixed. This suspension was degassed by a gentle stream of argon, [1,1'-bis(diphenylphosphino)ferrocene] dichloropalladium complexed with dichloromethane (0.017 g, 0.021 mmol) was added, the tube was sealed and heated in a microwave oven at 110 °C for one hour. The resulting suspension was diluted in water, extracted with ethyl acetate. The organic layer was washed with brine, dried over sodium sulfate and concentrated to dryness. The residue was purified first by a chromatography over silica gel (cyclohexane-ethyl acetate from 2/1 to 3/2) to yield the 5-cyclopropyl derivative as a white powder (0.07 g, 48 %). ¹H (CDCl₃): 0.78 (m, 2H); 1.03 (m, 2H); 1.41 (t, 3H, J = 7.0); 1.91 (m, 1H); 2.54 (s, 3H); 3.76 (s, 2H); 4.35 (q, 2H, J = 7.0); 7.18 (m, 1H); 7.28 (m, 4H); 7.38 (m, 1H); 7.65 (m, 1H); 8.20 (m, 1H). ¹³C (CDCl₃): 8.7; 12.5; 12.9; 14.9; 27.8; 64.2; 106.3; 115.0; 125.7; 128.2; 128.3; 135.1; 135.3; 139.1; 141.0; 145.4; 151.8; 162.2. HRMS: Calc. for C₂₁H₂₃N₃O + H: 334.1919; Found: 334.1931.

**2-(4-(2,6-difluorophenoxy)-3-isopropoxy-5-methyl-1H-pyrazol-1-yl)-5-methoxypyrazine (example 173):** 2-bromo-5-(4-(2,6-difluorophenoxy)-3-isopropoxy-5-methyl-1H-pyrazol-1-yl)pyrazine (example 48) (0.076 g, 0.17 mmol),cesium carbonate (0.087 g, 0.26 mmol) dissolved in methanol (4 mL) are heated in a microwave oven at 140 °C for 60 minutes. This was concentrated to dryness and the residue purified by a chromatography over silica gel (cyclohexane - dichloromethane from 3/2 to 2/1) to yield the methoxy ether (0.04 g, 59 %) as a white powder. ¹H (CDCl₃): 1.25 (d, 6H, J = 6.2); 2.55 (s, 3H); 3.99 (s, 3H); 4.88 (sept, 1H, J = 6.2); 6.89 (m, 2H); 7.00 (m, 1H); 7.98 (d, 1H, J = 1.3); 8.54 (d, 1H, J = 1.3). ¹³C (CDCl₃): 11.0; 21.9; 53.9; 72.1; 112.0 (6 and 17 Hz); 123.5 (9 Hz); 129.0; 130.8; 130.9; 133.1; 134.5 (14 Hz); 144.4; 153.8; 155.6 (4 and 249 Hz); 157.8. HRMS: Calc. for C₁₈H₁₈F₂N₄O₃ + H: 377.1425; Found: 377.1372.

**3-(4-(2,6-difluorophenoxy)-3-isopropoxy-5-methyl-1H-pyrazol-1-yl)-6-methoxypyridazine (example 174):** By using the procedure described for the preparation of 2-(4-(2,6-difluorophenoxy)-3-isopropoxy-5-methyl-1H-pyrazol-1-yl)-5-methoxypyrazine described above this compound was obtained from as a white powder in 80 % after a chromatography over silica gel (cyclohexane - dichloromethane from 9/1). ¹H (CDCl₃): 1.24 (d, 6H, J = 6.2); 2.72 (s, 3H); 4.14 (s, 3H); 4.86 (sept, 1H, J = 6.2); 6.91 (m, 2H); 7.03 (m, 2H); 7.96 (d, 1H, J = 9.4). ¹³C (CDCl₃): 12.0; 21.9; 54.9; 72.1; 111.9 (6 and 17 Hz); 119.8; 123.2; 123.6 (9 Hz); 129.6; 131.5; 134.5 (14 Hz); 153.8; 154.2; 155.6 (4 and 249 Hz); 163.0. HRMS: Calc. for C₁₈H₁₈F₂N₄O₃ + H: 377.1425; Found: 377.1364.

**3-(4-(2,6-difluorophenoxy)-3-isopropoxy-5-methyl-1H-pyrazol-1-yl)-6-ethoxypyridazine (example 174A):** By using the procedure described for the preparation of example 174, using ethanol, this compound was obtained as a solid in 71 % after a chromatography over silica gel (cyclohexane - dichloromethane from 9/1). ¹H (CDCl₃): 1.24 (d, 6H, J = 6.2); 1.47 (d, 6H, J = 7.1); 2.72 (s, 3H); 4.58 (q, 2H, J = 7.1); 4.86 (sept, 1H, J = 6.2); 6.92 (m, 2H); 7.03 (m, 2H); 7.96 (d, 1H, J = 9.5). ¹³C (CDCl₃): 11.9; 14.5; 21.9; 63.4; 72.1; 120.0 (6 and 17 Hz); 119.8; 123.2; 123.5 (9 Hz); 129.6; 131.5; 134.5 (14 Hz); 153.6; 154.1; 155.6 (4 and 249 Hz); 162.8. HRMS: Calc. for C₁₉H₂₀F₂N₄O₃ + H: 391.1582; Found: 391.1577.

**2-(4-benzyl-3-ethoxy-5-methyl-1H-pyrazol-1-yl)-5-ethyl-4-methylpyrimidine (example 175):** Under an inert atmosphere, 2-(4-benzyl-3-ethoxy-5-methyl-1H-pyrazol-1-yl)-5-ethylpyrimidine (example 25) (0.17 g, 0.52 mmol) and a 3N solution of methyl magnesium chloride in THF (3 mL, 2.95 mmol) were stirred overnight at room temperature. The resulting solution was cautiously quenched with ethanol and dichlorodicyanoquinone (0.14 g, 0.63 mmol) was added at room temperature. After 30 minutes, the solution was diluted in ethyl acetate, washed with water, dried over magnesium sulfate and concentrated to dryness. The resulting residue was purified by a chromatography over silica gel (cyclohexane/ethyl acetate 2/1) to yield the 4-methylated derivative as an oil (0.05 g, 28 %). ¹H (CDCl₃): 1.24 (t, 3H, J = 7.3); 1.37 (t, 3H, J = 7.0); 2.52 (s, 3H); 2.55 (s, 3H); 2.63 (q, 2H, J = 7.3); 3.75 (s, 2H); 4.44 (q, 2H, J = 7.0); 7.16 (m, 1H); 7.24 (m, 4H); 8.41 (s, 1H). ¹³C (CDCl₃): 13.7; 14.0; 14.9; 21.8; 22.5; 27.7; 64.3; 107.9; 125.8; 128.2; 128.3; 130.2; 140.5; 140.7; 155.8; 157.1; 163.2; 166.5. HRMS: Calc. for C₂₀H₂₄N₄O + H: 337.2028; Found: 337.2025.

(1-(**5**-**cyclopropylpyrimidin**-**2**-**yl**)-**3**-**isopropoxy**-**5**-**methyl**-1**H**-**pyrazol**-**4-yl)(phenyl)methanol (example 176):** In a 1/1 mixture of water and acetonitrile (6 mL), 2-(4-benzyl-3-isopropoxy-5-methyl-1H-pyrazol-1-yl)-5-cyclopropylpyrimidine (example 21) (0.12 g, 0.34 mmol) and cerium ammonium nitrate (0.56 g, 1.02 mmol) were stirred overnight. This was diluted in water, extracted with ethyl acetate, the organic layer was washed with water, brine, dried over magnesium sulfate and concentrated to dryness. The crude residue was dissolved in ethanol (10 mL) sodium borohydride (0.033 g, 0.88 mmol) was added followed by one drop of acetic acid. The suspension was stirred overnight diluted in ethyl acetate, the organic layer was washed with water, brine, dried over magnesium sulfate and concentrated to dryness. The resulting residue was purified by a chromatography over silica gel (dichloromethane - ethanol from 99/1 to 98/2) to yield the alcohol (0.03 g, 24 %) as a solid. ¹H (CDCl₃): 0.77 (m, 2H); 1.01 (m, 2H); 1.27 (d, 3H, J = 6.1); 1.37 (d, 3H, J = 6.1); 1.89 (m, 1H); 2.54 (s, 3H); 3.03 (d, 1H, J = 7.5); 5.24 (sept, 1H, J = 6.1); 5.79 (d, 1H, J = 7.5); 7.25 (m, 1H); 7.33 (m, 2H); 7.45 (m, 1H); 8.46 (s, 2H). ¹³C (CDCl₃): 8.4; 10.4; 13.6; 22.1; 22.3; 67.9; 71.9; 111.6; 126.8; 127.1; 128.2; 132.7; 140.1; 143.5; 155.8; 156.2; 161.3. HRMS: Calc. for C₂₁H₂₄N₃O₂ + H: 365.1978; Found: 365.1951.

**(1-(5-cyclopropylpyridin-2-yl)-3-ethoxy-5-methyl-1H-pyrazol-4-yl)(phenyl)methanol (example 177):** (1-(5-cyclopropylpyridin-2-yl)-3-ethoxy-5-methyl-1H-pyrazol-4-yl)(phenyl)methanone (0.07 g, 0.2 mmol) and sodium borohydride (0.074 g, 2.01 mmol) were stirred overnight in methanol (15 mL) at room temperature. This was neutralized with acetic acid, concentrated to dryness and diluted in ethyl acetate. The organic layer was washed with water and brine, dried over magnesium sulfate and concentrated to dryness. The residue was purified by a chromatography over silica gel (dichloromethane - ethanol from 99.5/0.5 to 98/2) to yield the alcohol as a glass (0.05 g, 71 %). ¹H (CDCl₃): 0.72 (m, 2H); 1.03 (m, 2H); 1.38 (t, 3H, J = 7.2); 1.92 (m, 1H); 2.51 (s, 3H); 4.35 (q, 2H, J = 7.2); 5.82 (s, 1H); 7.25 (m, 1H); 7.37 (m, 3H); 7.47 (m, 2H); 7.60 (d, 1H, J = 8.5); 8.20 (d, 1H, J = 2.3). ¹³C (CDCl₃): 8.7; 12.6; 12.9; 14.8; 64.5; 67.8; 109.2; 115.6; 126.0; 127.0; 128.2; 129.8; 135.1; 138.9; 143.6; 145.6; 151.4; 160.9. HRMS: Calc. for C₂₁H₂₃N₃O₂ + H: 350.1869; Found: 350.1829.

**1-(1-(5-cyclopropylpyridin-2-yl)-3-ethoxy-5-methyl-1H-pyrazol-4-yl)-1-phenylethanol (example 178):** Under an argon atmosphere(1-(5-cyclopropylpyridin-2-yl)-3-ethoxy-5-methyl-1H-pyrazol-4-yl)(phenyl)methanone (0.05 g, 0.14 mmol) was dissolved in dry THF (5 mL) at room temperature. A 1.6 M solution of methyllithium in ether (0.5 mL, 0.84 mmol) was added and the solution stirred for 5 minutes. This was diluted in water, extracted with ethyl acetate, the organic layer was washed with water, brine, dried over magnesium sulfate and concentrated to dryness. The residue was further purified by a chromatography over silica gel (dichloromethane - ethanol from 98/2) to yield the alcohol as a glass (0.04 g, 76 %). ¹H (CDCl₃): 0.72 (m, 2H); 1.04 (m, 2H); 1.38 (t, 3H, J = 7.2); 1.91 (m, 1H); 1.95 (s, 3H); 2.31 (s, 3H); 3.52 (s, 1H); 4.32 (m, 2H); 7.25-7.55 (m, 7H); 8.21 (d, 1H, J = 2.3). ¹³C (CDCl₃): 8.8; 12.6; 13.3; 14.8; 31.2; 64.6; 73.3; 112.6; 116.9; 125.5; 126.7; 126.9; 127.9; 135.0; 136.6; 138.5; 145.7; 151.2; 161.6. HRMS: Calc. for C₂₂H₂₅N₃O₂ + H: 364.2025; Found: 364.1948.

**(1-(5-cyclopropylpyridin-2-yl)-3-isopropoxy-1H-pyrazol-4-yl)(phenyl)methanol (example 179):** (1-(5-cyclopropylpyridin-2-yl)-3-isopropoxy-1H-pyrazol-4-yl)(phenyl)methanone (0.14 g, 0.402 mmol) and sodium borohydride (45 mg, 1.2 mmol) were stirred overnight in ethanol (20 mL). This was concentrated; the residue dissolved in water and ethyl acetate, the organic layer was washed with brine, dried over magnesium sulfate and concentrated to dryness. The residue was purified by a chromatography over alumina containing 1.5 % of water (cyclohexane - dichloromethane from 1/2 to 0/1) to yield the alcohol (0.1 g, 71 %) as an oil. ¹H (CDCl₃): 0.69 (m, 2H); 1.01 (m, 2H); 1.41 (d, 3H, J = 6.2); 1.42 (d, 3H, J = 6.2); 1.84 (m, 1H); 2.72 (d(1), 1H, J = 3.5); 5.07 (sept, 1H, J = 6.2); 5.84 (m, 1H); 7.31 (m, 1H); 7.38 (m, 3H); 7.50 (m, 2H); 7.63 (d, 1H, J = 8.5); 7.98 (d, 1H, J = 0.7); 8.09 (m, 1H). ¹³C (CDCl₃): 8.5; 12.5; 22.1; 67.8; 72.2; 110.9; 113.9; 125.8; 126.3; 127.5; 128.3; 135.4; 135.6; 142.8; 146.0; 149.5; 161.1. HRMS: Calc. for C₂₁H₂₃N₃O₂ + H: 350.1869; Found: 350.1833.

**5-cyclopropyl-2-(3-isopropoxy-4-(phenylsulfonyl)-1H-pyrazol-1-yl)pyridine and 5-cyclopropyl-2-(3-isopropoxy-4-(phenylsulfinyl)-1H-pyrazol-1-yl)pyridine (Example 180) :** 5-cyclopropyl-2-(3-isopropoxy-4-(phenylthio)-1H-pyrazol-1-yl)pyridine (0.18 g, 0.0005 mol) was dissolved in acetic acid (10 mL) hydrogen peroxide complexed with urea (0.24 g, 0.0025 mol) was added followed by sulfuric acid (94 %, 0.6 mL). This was stirred 5 minutes, the solution was diluted in water and extracted with ethyl acetate. The organic layer was washed with water, a saturated solution of sodium hydrogenocarbonate, brine, dried over magnesium sulfate and concentrated to dryness. The residue was purified by a chromatography over silica gel (cyclohexane - ethylacetate from 5/1 to 3/1) to yield the corresponding sulfone (0.09 g, 45 %) followed by the sulfoxide (0.09 g, 47 %) as described below. 5-cyclopropyl-2-(3-isopropoxy-4-(phenylsulfonyl)-1H-pyrazol-1-yl)pyridine (example 180): obtained as an oil. ¹H (CDCl₃): 0.73 (m, 2H); 1.05 (m, 2H); 1.36 (d, 6H, J = 6.2); 1.92 (m, 1H); 4.99 (sept, 1H, J = 6.2); 7.40 (dd, 1H, J = 2.3 and 8.4); 7.50 (m, 2H); 7.58 (m, 1H); 7.63 (d, 1H, J = 8.4); 8.08 (m, 2H); 8.19 (d, 1H, J = 2.3); 8.86 (s, 1H). ¹³C (CDCl₃): 9.0; 12.6; 21.8; 73.4; 111.5; 112.3; 127.6; 128.7; 130.1; 132.9; 135.5; 137.9; 142.2; 146.4; 148.2; 158.8. HRMS: Calc. for C₂₀H₂₁N₃O₃S+ H: 384.1382; Found: 384.1383. 5-cyclopropyl-2-(3-isopropoxy-4-(phenylsulfinyl)-1H-pyrazol-1-yl)pyridine (example 181): obtained as an oil. ¹H (CDCl₃): 0.69 (m, 2H); 1.02 (m, 2H); 1.22 (d, 3H, J = 6.2); 1.33 (d, 3H, J = 6.2); 1.92 (m, 1H); 4.96 (sept, 1H, J = 6.2); 7.37 (dd, 1H, J = 2.4 and 8.4); 7.48 (m, 3H); 7.63 (d, 1H, J = 8.4); 7.74 (m, 2H); 8.12 (d, 1H, J = 2.4); 8.48 (s, 1H). ¹³C (CDCl₃): 8.8; 12.6; 21.7; 73.0; 111.3; 113.9; 124.7; 128.8; 128.9; 130.4; 135.5; 137.3; 143.9; 146.2; 148.5; 160.4. HRMS: Calc. for C₂₀H₂₁N₃O₂S+ H: 368.1433; Found: 368.1433.

**5-cyclopropyl-2-(3-isopropoxy-4-(phenylsulfinyl)-1H-pyrizol-1-yl)pyrimidine (example 182):** 5-cyclopropyl-2-(3-isopropoxy-4-(phenylthio)-1H-pyrazol-1-yl)pyrimidine (0.093 g, 0.26 mmol) was dissolved in acetic acid (5 mL) hydrogen peroxide complexed with urea (0.05 g, 0.52 mol) was added followed by sulfuric acid (94 %, 0.5 mL). This was stirred 10 minutes, the solution was diluted in water and extracted with ethyl acetate. The organic layer was washed with water, a saturated solution of sodium hydrogenocarbonate, brine, dried over magnesium sulfate and concentrated to dryness. The residue was purified by a chromatography over silica gel (cyclohexane - ethylacetate from 3/2 to 1/1) to yield the sulfoxide (0.07 g, 72 %) as an oil. ¹H (CDCl₃): 0.73 (m, 2H); 1.06 (m, 2H); 1.19 (d, 3H, J = 6.2); 1.27 (d, 3H, J = 6.2); 1.85 (m, 1H); 5.14 (sept, 1H, J = 6.2); 7.49 (m, 3H); 7.73 (m, 2H); 8.40 (s, 2H); 8.60 (s, 1H). ¹³C (CDCl₃): 8.6; 10.5; 21.8; 73.0; 115.8; 124.7; 128.8; 130.7; 131.4; 134.1; 143.7; 153.4; 156.6; 160.8. HRMS: Calc. for C₁₉H₂₀N₄O₂S+ H: 369.1385; Found: 369.1353.

**5-cyclopropyl-2-(3-isopropoxy-4-(phenylsulfonyl)-1H-pyrazol-1-yl)pyrimidine (example 183):** 5-cyclopropyl-2-(3-isopropoxy-4-(phenylthio)-1H-pyrazol-1-yl)pyrimidine (0.13 g, 0.36 mmol) was dissolved in acetic acid (5 mL) hydrogen peroxide complexed with urea (0.4 g, 4.25 mol) was added followed by sulfuric acid (94 %, 0.5 mL). This was stirred 10 minutes, the solution was diluted in water and extracted with ethyl acetate. The organic layer was washed with water, a saturated solution of sodium hydrogenocarbonate, brine, dried over magnesium sulfate and concentrated to dryness. The residue was purified by a chromatography over silica gel (cyclohexane - ethylacetate from 3/2 to 1/1) to yield the sulfone (0.12 g, 88 %) as a solid. ¹H (CDCl₃): 0.78 (m, 2H); 1.11 (m, 2H); 1.32 (d, 6H, J = 6.2); 1.91 (m, 1H); 5.17 (sept, 1H, J = 6.2); 7.49 (m, 2H); 7.58 (m, 1H); 8.06 (m, 2H); 8.45 (s, 2H); 8.95 (s, 1H). ¹³C (CDCl₃): 8.8; 10.5; 21.9; 73.6; 114.1; 127.7; 128.7; 133.0; 132.2; 134.8; 141.7; 153.2; 156.6; 159.5. HRMS: Calc. for C₁₉H₂₀N₄O₃S+ H: 385.1334; Found: 385.1335.

**5-eyclopropyl-2-(3-isopropoxy-4-phenoxy-1H-pyrazol-1-yl)-3-methoxypyridine (example 184):** 5-cyclopropyl-3-fluoro-2-(3-isopropoxy-4-phenoxy-1H-pyrazol-1-yl)pyridine (0.063 g, 0.17 mmol) and cesium carbonate (0.08 g, 0.26 mmol) dissolved in methanol (2 mL) were heated at 140 °C in a microwave oven for 2 hour. This was concentrated to dryness and the residue was purified by a chromatography over silica gel (cyclohexane - ethylacetate 4/1) to yield the methoxy ether (0.05 g, 76 %) as an oil. ¹H (CDCl₃): 0.74 (m, 2H); 1.05 (m, 2H); 1.34 (d, 6H, J = 6.2); 1.94 (m, 1H); 3.88 (s, 3H); 5.15 (sept, 1H, J = 6.2); 6.99-7.04 (m, 4H); 7.29 (m, 2H); 7.94 (s, 1H); 7.96 (d, 1H, J = 2.0); 8.86 (s, 1H). ¹³C (CDCl₃): 8.9; 12.8; 22.2; 56.1; 72.4; 115.8; 118.2; 122.2; 123.4; 127.3; 129.3; 138.2; 138.4; 139.3; 146.6; 155.4; 158.7. HRMS: Calc. for C₂₁H₂₃N₃O₃+ H: 366.1818; Found: 366.1794.

**5-cyclopropyl-2-(4-(2-fluorophenoxy)-3-isopropoxy-1H-pyrazol-1-yl)-3-methoxypyridine (example 185)** : By using the protocol described for the preparation of 5-cyclopropyl-2-(3-isopropoxy-4-phenoxy-1H-pyrazol-1-yl)-3-methoxypyridine, this compound was obtained from 5-cyclopropyl-3-fluoro-2-(4-(2-fluorophenoxy)-3-isopropoxy-1H-pyrazol-1-yl)pyridine in a 83 % yield as an oil. ¹H (CDCl₃): 0.75 (m, 2H); 1.06 (m, 2H); 1.34 (d, 6H, J = 6.2); 1.94 (m, 1H); 3.89 (s, 3H); 5.14 (sept, 1H, J = 6.2); 6.98-7.16 (m, 5H); 7.95 (m, 2H). ¹³C (CDCl₃): 8.9; 12.8; 22.2; 56.1; 72.4; 116.4 (J = 17 Hz); 117.4; 118.2; 122.9 (J = 6 Hz); 123.2; 124.1 (J = 4 Hz); 127.3; 138.2; 138.5; 139.3; 146.3 (J = 11 Hz); 146.6; 152.0 (J = 246 Hz); 155.0. HRMS: Calc. for C₂₁H₂₂FN₃O₃+ H: 384.1723; Found: 384.1669.

**3-(benzyloxy)-5-cyclopropyl-2-(4-(2,6-difluorophenoxy)-3-isopropoxy-5-methyl-1H-pyrazol-1-yl)pyridine (example 186):** 5-cyclopropyl-2-(4-(2,6-difluorophenoxy)-3-isopropoxy-5-methyl-1H-pyrazol-1-yl)-3-fluoropyridine (example 5) (0.38 g, 0.94 mmol), cesium carbonate (0.34 g, 1.03 mmol) dissolved in benzylalcohol (2 mL) are heated in a microwave oven at 150 °C for 90 minutes. This is concentrated to dryness and the residue purified by two consecutive chromatographies, the first one over silica gel (cyclohexane-ethyl acetate 4/1), the second one over alumina containing 1.5 % of water (cyclohexane-dichloromethane from 1/1) to yield the benzyl ether (0.14 g, 29 %) as an oil. ¹H (CDCl₃): 0.74 (m, 2H); 1.08 (m, 2H); 1.22 (d, 6H, J = 6.1); 1.92 (m, 1H); 2.13 (s, 3H); 4.89 (sept, 1H, J = 6.1); 5.02 (s, 2H); 6.97 (m, 2H); 7.02 (m, 2H); 7.31 (m, 5H); 8.01 (d, 1H, J = 2.0). ¹³C (CDCl₃): 9.1; 9.4; 12.9; 22.0; 71.1; 71.8; 111.9 (6 and 17 Hz); 123.4 (9 Hz); 127.1; 127.5; 128.0; 128.5; 131.2; 134.9 (14 Hz); 135.9; 139.3; 139.9; 141.4; 149.8; 153.6; 155.8 (4 and 249 Hz). HRMS: Calc. for C₂₉H₂₇F₂N₃O₃ + H: 492.2099; Found: 492.2076.

**5-cyclopropyl-2-(4-(2,6-difluorophenoxy)-3-isopropoxy-5-methyl-1H-pyrazol-1-yl)pyridin-3-ol (example 187):** 3-(benzyloxy)-5-cyclopropyl-2-(4-(2,6-difluorophenoxy)-3-isopropoxy-5-methyl-1H-pyrazol-1-yl)pyridine (0.22 g, 0.44 mmol), ammonium formate (0.11 g, 1.79 mmol) and 10 % palladium over charcoal (0.023 g, 0.021 mmol) were heated to reflux in ethanol (50 mL) for 45 minutes. This was adsorbed over silica gel and purified by a chromatography over silica gel (cyclohexane-ethyl acetate 95/5) to yield the hydroxyl derivative (0.13 g, 72 %) as an oil. ¹H (CDCl₃): 0.71 (m, 2H); 1.01 (m, 2H); 1.25 (d, 6H, J = 6.1); 1.89 (m, 1H); 2.69 (s, 3H); 4.75 (sept, 1H, J = 6.1); 6.92 (m, 3H); 7.04 (m, 1H); 7.75 (d, 1H, J = 2.0); 10.98 (s, 1H). ¹³C (CDCl₃): 8.9; 11.8; 12.4; 21.9; 72.9; 112.0 (6 and 17 Hz); 121.9; 123.7 (9 Hz); 128.0; 132.6; 134.5 (14 Hz); 135.8; 137.5; 138.1; 144.9; 151.2; 155.6 (5 and 250 Hz). HRMS: Calc. for C₂₁H₂₁F₂N₃O₃ + H: 402.1629; Found: 402.1642.

**5-cyclopropyl-2-(4-(2-fluoro-6-hydroxyphenoxy)-3-isopropoxy-5-methyl-1H-pyrazol-1-yl)pyridin-3-ol (example 188):** When using the protocol used for the preparation of 3-(benzyloxy)-5-cyclopropyl-2-(4-(2,6-difluorophenoxy)-3-isopropoxy-5-methyl-1H-pyrazol-1-yl)pyridine but heating at 160 h for 3 hours, an inseparable mixture of this compound and the bis benzylated derivative 3-(benzyloxy)-2-(4-(2-(benzyloxy)-6-fluorophenoxy)-3-isopropoxy-5-methyl-1H-pyrazol-1-yl)-5-cyclopropylpyridine was obtained. Hydrogenation of this mixture as described for the preparation of 5-cyclopropyl-2-(4-(2,6-difluorophenoxy)-3-isopropoxy-5-methyl-1H-pyrazol-1-yl)pyridin-3-ol allowed the purification of the bis hydroxylated derivative 5-cyclopropyl-2-(4-(2-fluoro-6-hydroxyphenoxy)-3-isopropoxy-5-methyl-1H-pyrazol-1-yl)pyridin-3-ol which was obtained as a solid. ¹H (CDCl₃): 0.72 (m, 2H); 1.02 (m, 2H); 1.35 (d, 6H, J = 6.1); 1.88 (m, 1H); 2.73 (s, 3H); 4.85 (sept, 1H, J = 6.1); 6.61 (s, 1H); 6.64 (m, 1H); 6.93 (d, 1H, J = 2.05); 6.98 (m, 1H); 7.76 (d, 1H, J = 2.05); 10.71 (s, 1H). ¹³C (CDCl₃): 9.0; 11.9; 12.4; 21.9; 73.8; 108.1 (19 Hz); 112.3 (3 Hz); 122.0; 124.9 (9 Hz); 128.1; 134.0 (13 Hz); 134.1; 136.0; 137.3; 138.5; 145.0; 150.1 (3 Hz); 151.4; 155.2 (248 Hz). HRMS: Calc. for C₂₁H₂₂FN₃O₃ + H: 400.1673; Found: 400.1688.

**5-cyclopropyl-2-(4-(2,6-difluorophenoxy)-3-isopropoxy-5-methyl-1H-pyrazol-1-yl)-3-methoxypyridine (example 189):** 5-cyclopropyl-2-(4-(2,6-difluorophenoxy)-3-isopropoxy-5-methyl-1H-pyrazol-1-yl)-3-fluoropyridine (example 5) (0.11 g, 0.272 mmol), cesium carbonate (0.13 g, 0.409 mmol) dissolved in methanol (2 mL) are heated in a microwave oven at 150 °C for 90 minutes. This is concentrated to dryness and the residue purified by a chromatography over silica gel (cyclohexane - ethyl acetate 4/1) to yield the methoxy ether (0.08 g, 70 %) as an oil. ¹H (CDCl₃): 0.77 (m, 2H); 1.08 (m, 2H); 1.21 (d, 6H, J = 6.2); 1.96 (m, 1H); 2.11 (s, 3H); 3.79 (s, 3H); 4.87 (sept, 1H, J = 6.2); 6.87 (m, 2H); 7.00 (m, 2H); 7.97 (d, 1H, J = 2.0). ¹³C (CDCl₃): 9.0; 9.3; 13.0; 22.0; 56.0; 71.7; 111.9 (6 and 16 Hz); 118.2; 123.4 (9 Hz); 127.4; 131.2; 134.9 (14 Hz); 138.7; 139.2; 141.4; 150.7; 153.5; 155.8 (4 and 249 Hz). HRMS: Calc. for C₂₂H₂₃F₂N₃O₃ + H: 416.1786; Found: 416.1779.

**5-cyclopropyl-2-(4-(2,6-difluorophenoxy)-3-isopropoxy-5-methyl-1H-pyrazol-1-yl)-N,N-dimethylpyridin-3-amine (example 190):** 5-cyclopropyl-2-(4-(2,6-difluorophenoxy)-3-isopropoxy-5-methyl-1H-pyrazol-1-yl)-3-fluoropyridine (example 5) (0.06 g, 0.148 mmol), cesium and a 2N solution of dimethylamine in tetrahydrofuran (0.5 mL) in tetrahydrofuran (2 mL) are heated in a microwave oven at 180 °C for 9 hours. This is concentrated to dryness and the residue purified by a chromatography over silica gel (cyclohexane - ethyl acetate 9/1) to yield the N-dimethylamine (0.04 g, 62 %) as an oil. ¹H (CDCl₃): 0.75 (m, 2H); 1.03 (m, 2H); 1.20 (d, 6H, J = 6.2); 1.91 (m, 1H); 2.09 (s, 3H); 2.56 (s, 6H); 4.89 (sept, 1H, J = 6.2); 6.91 (m, 3H); 5.98 (m, 1H); 7.86 (d, 1H, J = 2). ¹³C (CDCl₃): (one signal missing) 8.8; 9.0; 13.0; 21.9; 41.3; 71.8; 111.9 (6 and 16 Hz); 122.8; 123.3 (9 Hz); 127.5; 130.9; 135.0 (13 Hz); 139.7; 140.4; 144.3; 153.4; 155.7 (4 and 249 Hz). HRMS: Calc. for C₂₃H₂₆F₂N₄O₃ + H: 429.2102; Found: 429.2023.

**2-(4-(2,6-difluorophenoxy)-3-isopropoxy-5-methyl-1H-pyrazol-1-yl)-3-fluoro-5-propylpyridine (example 190A):** The 5-cyclopropyl-2-(4-(2,6-difluorophenoxy)-3-isopropoxy-5-methyl-1H-pyrazol-1-yl)-3-fluoropyridine (example 5) (0.17 g, 0.42 mmol) and 10 % palladium over charcoal (0.066 g, 0.062 mmol) were dispersed in ethanol (20 mL). This was charge with hydrogen at 1 atmosphere and stirred at room temperature for 5 days. The suspension was filtered, the filtrate concentrated to dryness and the residue purified by a chromatography over silica gel (cyclohexane-ethyl acetate 97/3) followed by drying under high vacuum to give the n-propyl derivative as an oil (0.05 g, 30 %). ¹H (CDCl₃): 1.00 (t, 3H, J = 7.3); 1.23 (d, 6H, J = 6.2); 1.69 (m, 2H); 2.28 (s, 3H); 2.66 (t, 2H, J = 7.4); 4.88 (sept, 1H, J = 6.2); 6.89 (m, 2H); 7.01 (m, 1H); 7.39 (m, 1H); 8.18 (m, 1H). ¹³C (CDCl₃): 9.3; 13.5; 21.9; 24.0; 34.2; 71.9; 111.9 (6 and 17 Hz); 123.5 (9 Hz); 125.2 (17 Hz); 128.3; 131.1; 134.7 (14 Hz); 138.5 (11 Hz); 139.5 (3 Hz); 144.0 (5 Hz); 152.7 (260 Hz); 154.1; 155.7 (4 and 250 Hz). HRMS: Calc. for C₂₁H₂₂F₃N₃O₂ + H: 406.1742; Found: 406.1713.

**2**-(**5**-**chloro**-**3**-**isopropoxy**-**4**-**phenoxy**-1**H**-**pyrazol**-1-**yl**)-**5**-**cyclopropylpyridine (example 191):** 5-cyclopropyl-2-(3-isopropoxy-4-phenoxy-1H-pyrazol-1-yl)pyridine (0.031 g, 0.092 mol) and N-chlorosuccinimide (0.018 g, 0.138 mmol) in dry acetonitrile (0.5 mL, dried over 4A molecular sieves) were heated at 130 °C in a microwave oven for 30 minutes. The resulting solution was concentrated to dryness and the residue purified by a chromatography over silica gel (cyclohexane - ethyl acetate 9/1) to yield the 5-chloropyrazole (0.02 g, 58 %) as an oil. ¹H (CDCl₃): 0.77 (m, 2H); 1.07 (m, 2H); 1.34 (d, 6H, J = 6.2); 1.96 (m, 1H); 5.02 (sept, 1H, J = 6.2); 7.06 (m, 3H); 7.31 (m, 2H); 7.45 (dd, 1H, J = 2.4 and 8.4); 7.56 (d, 1H, J = 8.4); 8.32 (d, 1H, J = 2.4). ¹³C (CDCl₃): 9.0; 12.7; 22.1; 72.3; 115.6; 116.4; 119.9; 122.5; 124.1; 129.4; 135.1; 137.8; 146.2; 149.5; 154.7; 157.7. HRMS: Calc. for C₂₀H₂₀N₃ClO₂+ H: 370.1322; Found: 370.1298.

**2-(5-chloro-4-(2-fluorophenoxy)-3-isopropoxy-1H-pyrazol-1-yl)-5-cyclopropylpyrimidine (example 192):** by using the protocol described for the preparation of 2-(5-chloro-3-isopropoxy-4-phenoxy-1H-pyrazol-1-yl)-5-cyclopropylpyridine, this compound was obtained from 5-cyclopropyl-2-(4-(2-fluorophenoxy)-3-isopropoxy-1H-pyrazol-1-yl)pyrimidine (example 67) as a solid in a 91 % yield after a chromatography over silica gel (cyclohexane - ethyl acetate 3/1). ¹H (CDCl₃): 0.82 (m, 2H); 1.13 (m, 2H); 1.32 (d, 6H, J = 6.2); 1.94 (m, 1H); 5.18 (sept, 1H, J = 6.2); 7.02 (m, 3H); 7.18 (m, 1H); 8.53 (s, 2H). ¹³C (CDCl₃): 8.7; 10.5; 22.0; 72.5; 116.6 (J = 18 Hz); 116.8; 120.9; 123.3 (J = 6 Hz); 124.0 (J = 4 Hz); 125.5; 134.3; 145.1 (J = 11 Hz); 152.0 (J = 245 Hz); 154.4; 155.3; 156.3. HRMS: Calc. for C₁₉H₁₈N₄ClFO₂+ H: 389.1181; Found: 389.1262.

**2-(4-benzyl-1-(5-ethylpyrimidin-2-yl)-5-methyl-1H-pyrazol-3-yloxy)ethanol (example 193):** A suspension of 2-(4-benzyl-3-(2-(benzyloxy)ethoxy)-5-methyl-1H-pyrazol-1-yl)-5-ethylpyrimidine (0.45 g, 1.05 mmol), ammonium formate (0.40 g, 6.3 mmol) and 10 % palladium over charcoal (0.05 g, 0.052 mmol) were boiled in ethanol (20 mL) for 4 hours. The resulting suspension was adsorbed over silica and purified by a chromatography over silica gel (dichloromethane-ethanol, from 98/2 to 97/3) to yield the corresponding alcohol as a white powder (0.23 g, 64 %). ¹H (CDCl₃): 1.29 (t, 3H, J = 7.3); 2.60 (s, 3H); 2.66 (q, 2H, J = 7.3); 3.77 (s, 2H); 3.89 (m, 2H); 4.49 (m, 2H); 7.18-7.30 (m, 5H); 8.55 (s, 2H). ¹³C (CDCl₃): 13.7; 15.0; 23.0; 27.9; 62.3; 71.3; 108.1; 126.0; 128.1; 128.4; 132.2; 140.2; 141.0; 155.9; 157.6; 162.9. HRMS: Calc. for C₁₉H₂₂N₄O₂ + H: 339.1821; Found: 339.1766.

### Intermediates

### - Methods used for the preparation of beta-carbonyl esters.

- Representative preparation of beta-carbonyl esters by transesterification, synthesis of 2-(benzyloxy)ethyl 2-benzyl-3-oxobutanoate (intermediate 1). Ethyl 2-benzyl-3-oxobutanoate (18 g, 0.081 mol), and 2-(benzyloxy)ethanol (13.09 g, 0.085 mol) were dissolved in toluene (200 mL). Sodium hydride (60 % in oil, 0.34 g, 0.008 mol) was added and the solution was heated to reflux for 24 hours. The resulting solution was washed with water, brine, dried over magnesium sulfate concentrated to dryness and used directly in the next synthetic step.
- Representative preparation of 4-substituted beta-carbonyl esters by condensations followed by reductions, synthesis of isopropyl 2-benzyl-3-oxobutanoate (intermediate 2). Isopropyl 3-oxobutanoate (21.55 g, 0.15 mol), benzaldehyde (15.86 g, 0.15 mol), acetic acid (0.9 g, 0.015 mol) and piperidine (1.3 g, 0.015 mol) were stirred in isopropanol (200 mL) overnight. To the resulting solution was added 10 % palladium on charcoal (3.9 g, 0.0037 mol), the flask was charged with hydrogen and the suspension was stirred for 3 hours. This was filtered, concentrated to dryness, the residue was dissolved in ethyl acetate, the solution was washed with water, brine, dried over magnesium sulfate and concentrated to dryness to yield the corresponding beta ketoester as an oil (32.17 g, 91.8 %) which was used directly in the next step.
- Representative preparation of 4-aryloxy beta-carbonyl esters by nucleophilic reactions synthesis of ethyl 2-(2-chlorophenoxy)-3-oxobutanoate (intermediate 3). Under an inert atmosphere, 2-Chlorophenol (3.58 g, 0.028 mol) was dissolved in dry DMF (100 mL, dried over 4A molecular sieves). To this solution sodium hydride (60 % in oil, 1.11 g, 0.028 mol) was added and, at the end of the hydrogen evolution, ethyl 2-chloro-3-oxobutanoate (3.85 mL, 0.028 mol) was added. This was heated at 85 °C under an inert atmosphere for 5 hours and the suspension was concentrated to dryness. The residue could be used directly in the next step or, in some cases, further purified by a partition between water and ethyl acetate.
- Representative formylation of phenoxyacetates, preparation of isopropyl 2-(2-fluorophenoxy)-3-oxopropanoate (intermediate 4): Isopropyl 2-(2-fluorophenoxy)acetate (2.15 g; see Tetrahedron 2005, 61, 10061 fro a preparation of such compounds) was dissolved in isopropyl formate (14 g). The solution was cooled to 0°C and sodium hydride (60 % in oil, 1.64 g) was added in portions. The resulting suspension was stirred for 1h30 at 20 °C and dispersed in ice. This was made acid with 2N hydrochloric acid and extracted with ethyl acetate. The organic layer was washed with saturated sodium hydrogeno carbonate, brine dried over magnesium sulfate and concentrated to dryness under vacuum. The resulting oil, containing extensive decomposition products, along with the 3-oxopropanoate was directly used in the next step.

### - Method used for the preparation of 3-(dialkylamino)acrylates

**isopropyl 3-(dimethylamino)-2-phenoxyacrylate (intermediate 5):** In a flask fitted with a vigreux column an air condenser and a calcium guard, a mixture of isopropyl 2-phenoxyacetate (3.86 g, 0.0198 mol) and 1,1-diisopropoxy-N,N-dimethylmethanamine (6.96 g, 0.0397 mol) was heated at 200 °C while allowing the isopropanol distillation. At the end of the isopropanol evolution (12-24 hours), the resulting mixture was concentrated to dry and purified by chromatography over silica gel (cyclohexane - dichloromethane) to yield the acrylate as a solid (4.32 g, 87 %). ¹H (CDCl₃): 1.13 (d, 6H, J = 6.1); 2.98 (s, 6H); 4.99 (sept, 1H, J = 6.1); 6.97 (m, 3H); 7.12 (m, 1H); 7.29 (m, 2H). ¹³C (CDCl₃): 21.9; 42.1; 66.8; 115.1; 115.9; 121.3; 129.3; 139.1; 159.4; 165.6. HRMS: Calc. for C₁₄H₁₉NO₃ + H: 250.1443; Found: 250.1373.

**isopropyl 3-(dimethylamino)-2-(2-fluorophenoxy)acrylate (intermediate 6):** From isopropyl 2-(2-fluorophenoxy)acetate, by using the procedure described for the preparation of isopropyl 3-(dimethylamino)-2-phenoxyacrylate, this compound was obtained as a solid (72 %). ¹H (CDCl₃): 1.10 (d, 6H, J = 6.2); 3.00 (s, 6H); 4.98 (sept, 1H, J = 6.2); 6.90-7.12 (m, 4H); 7.13 (s, 1H). ¹³C (CDCl₃): 21.9; 42.1; 66.9; 115.9 (8 Hz); 116.0; 116.2 (18 Hz); 139.1; 147.3 (10 Hz); 152.2 (246 Hz); 165.1.

**isopropyl 3-morpholino-2-(phenylthio)acrylate (Intermediate 7):** isopropyl 2-(phenylthio)acetate (17.09 g, 0.081 mol) isopropylorthoformate (23.2 g, 0.12 mol) and morpholine (7.45 g, 0.085 mol) were heated to reflux overnight. The flask was then fitted with a vigreux column, an air condenser and a calcium guard and heated to 200 °C for 15 hours. The resulting black oil was concentrated to dryness and used in the next without further purification. On a smaller run, the residue was purified by chromatography over silica gel (dichloromethane/ethanol 100/0 to 99/1) to yield the expected acrylate which also contained traces of isopropyl 3-morpholino-2-(phenylthio)-3-(piperidin-1-yl)propanoate. ¹H (CDCl₃): 1.17 (d, 6H, J = 6.2); 3.64 (m, 4H); 3.82 (m, 4H); 5.01 (sept, 1H, J = 6.2); 7.08 (m, 1H); 7.16 (m, 2H); 7.26 (m, 2H); 7.99 (s, 1H). ¹³C (CDCl₃): 21.9; 60.0; 66.8; 67.7; 84.4; 124.6; 125.3; 128.7; 139.4; 153.2; 169.0. HRMS: Calc. for C₁₆H₂₂NO₃S + H: 308.1320; Found: 308.1281.

### - Examples of preparations of 3-alkoxypyrazole from beta-carbonyl esters.

General procedure: One equivalent of a beta-carbonyl ester (commercially available or made by a number of methods as described above) and hydrazine monohydrochloride (1.05 equivalents) are boiled in the relevant alcohol (to avoid any transesterification reaction) or stirred at room temperature in isopropanol until disappearance of the starting material. After concentration to dryness, the crude mixture of hydroxy and alkoxy pyrazoles was purified by filtration, extraction and/or chromatography as described in the following examples.

**4-(2,6-difluorophenoxy)-3-isopropoxy-5-methyl-1H-pyrazole (intermediate 8):** The crude residue was directly purified by a chromatography over silica gel (dichloromethane - ethanol 99/1 to 95/5), the alkoxypyrazole was obtained as an oil after a second chromatography over neutral alumina containing 1.5 % water (cyclohexane - dichloromethane 1/4). ¹H (CDCl₃): 1.22 (d, 6H, J = 6.0); 2.24 (s, 3H); 4.73 (sept, 1H, J = 6.0); 6.90 (m, 2H); 7.02 (m, 1H); 8.6 (s(1), 1H). ¹³C (CDCl₃): 9.0; 22.0; 72.1; 111.9 (16 Hz); 123.4 (9 Hz); 126.6; 129.5; 134.8; 153.4; 155.7 (249 Hz). HRMS: Calc. for C₁₃H₁₄F₂N₂O₂ + H: 269.1102; Found: 269.1034.

**4-(2,4-difluorophenoxy)-3-isopropoxy-5-methyl-1H-pyrazole (intermediate 9):** The crude residue was directly purified by a chromatography over silica gel (dichloromethane - ethanol 99/1 to 95/5), the alkoxypyrazole was obtained as a solid after a second chromatography over neutral alumina containing 1.5 % water (cyclohexane - dichloromethane 1/4). ¹H (CDCl₃): 1.28 (d, 6H, J = 6.1); 2.16 (s, 3H); 4.76 (sept, 1H, J = 6.1); 6.73 (m, 1H); 6.87 (m, 2H); 9.90 (s(1), 1H). ¹³C (CDCl₃): 9.1; 22.2; 72.5; 104.9 (26 and 21 Hz); 110.3 (23 Hz); 116.9 (10 Hz); 122.9; 131.5; 143.0; 152.0 (250 and 12 Hz); 153.9; 157.2 (243 and 10 Hz). HRMS: Calc. for C₁₃H₁₄F₂N₂O₂ + H: 269.1102; Found: 269.1012.

**4-(2-chlorophenoxy)-3-ethoxy-5-methyl-1H-pyrazole (intermediate 10):** The crude residue was dispersed in water neutralized with solid sodium hydrogenocarbonate, and extracted with dichloromethane. The filtered organic layer was washed with a saturated solution of sodium hydrogenocarbonate, water, brine, dried over magnesium sulfate and concentrated to dryness. The resulting residue was further purified by a chromatography over silica gel (dichloromethane - ethanol 99/1 to 98/2) to yield the alkoxypyrazole as a solid. ¹H (CDCl₃): 1.34 (t, 3H, J = 7.0); 2.15 (s, 3H); 4.26 (q, 2H, J = 7.0); 6.84 (m, 1H); 6.97 (m, 1H); 7.15 (m, 1H); 7.40 (m, 1H); 9.70 (s(1), 1H). ¹³C (CDCl₃): 9.0; 14.8; 64.8; 115.0; 121.9; 122.4; 122.6; 127.5; 130.3; 131.7; 154.2; 155.1. HRMS: Calc. for C₁₂H₁₃ClN₂O₂ + H: 253.0744; Found: 253.0667.

**4-benzyl-3-isopropoxy-5-methyl-1H-pyrazole (intermediate 11):** The crude residue was dispersed in water neutralized with solid sodium hydrogenocarbonate, and extracted with dichloromethane. The filtered organic layer was washed with a saturated solution of sodium hydrogenocarbonate, water, brine, dried over magnesium sulfate and concentrated to dryness. The resulting residue was further purified by a chromatography over silica gel (cyclohexane - ethyl acetate 2/1) to yield the isopropoxypyrazole as a solid. ¹H (CDCl₃): 1.33 (d, 6H, J = 6.0); 2.10 (s, 3H); 3.68 (s, 2H); 4.84 (sept, 1H, J = 6.0); 7.23 (m, 5H); 8.0 (s(1), 1H). ¹³C (CDCl₃): 10.3; 22.3; 27.7; 71.4; 102.8; 125.7; 128.2; 128.3; 137.8; 141.4; 161.4. HRMS: Calc. for C₁₄H₁₈N₂O + H: 231.1497; Found: 231.1481.

**4**-(**2**-**fluorobenzyl**)-**3**-**isopropoxy**-**5**-**methyl**-**1H**-**pyrazole (intermediate 12):** The crude residue was dispersed in water neutralized with solid sodium hydrogenocarbonate, and extracted with dichloromethane. The insoluble material was filtered and the organic layer was washed with a saturated solution of sodium hydrogenocarbonate, water, brine, dried over magnesium sulfate and concentrated to dryness to yield this compound as an oil. ¹H (CDCl₃): 1.32 (d, 6H, J = 6.2); 2.14 (s, 3H); 3.67 (s, 2H); 4.83 (sept, 1H, J = 6.2); 7.05 (m, 2H); 7.14-7.21 (m, 2H); 8.70 (s(1), 1H). ¹³C (CDCl₃): 10.2; 20.5; 22.3; 71.2; 101.4; 114.9 (J = 23 Hz); 123.7; 127.3; 127.9 (J = 15); 130.7; 137.8; 160.8 (J = 244 Hz); 161.8. HRMS: Calc. for C₁₄H₁₇FN₂O + H: 249.1403; Found: 249.1337.

**4-(3-fluorobenzyl)-3-isopropoxy-5-methyl-1H-pyrazole (intermediate 13):** The crude residue was dispersed in water neutralized with solid sodium hydrogenocarbonate, and extracted with dichloromethane. The insoluble material was filtered and the organic layer was washed with a saturated solution of sodium hydrogenocarbonate, water, brine, dried over magnesium sulfate and concentrated to dryness to yield this compound as an oil which slowly solidified. ¹H (CDCl₃): 1.33 (d, 6H, J = 6.2); 2.11 (s, 3H); 3.65 (s, 2H); 4.84 (sept, 1H, J = 6.2); 6.86-6.92 (m, 2H); 6.98 (m, 1H); 7.20 (m, 1H); 8.62 (s(1), 1H). ¹³C (CDCl₃): 10.3; 22.3; 27.5; 71.3; 102.1; 112.5 (J = 21 Hz); 115.1 (J = 21 Hz); 123.8; 129.5; 137.7; 144.0; 161.5; 163.0 (J = 244 Hz). HRMS: Calc. for C₁₄H₁₇FN₂O + H: 249.1403; Found: 249.1321.

**4-(4-fluorobenzyl)-3-isopropoxy-5-methyl-1H-pyrazole (intermediate 14):** The crude residue was dispersed in water neutralized with solid sodium hydrogenocarbonate, and extracted with dichloromethane. The insoluble material was filtered and the organic layer was washed with a saturated solution of sodium hydrogenocarbonate, water, brine, dried over magnesium sulfate and concentrated to dryness to yield this compound as an oil. ¹H (CDCl₃): 1.34 (d, 6H, J = 6.2); 2.10 (s, 3H); 3.62 (s, 2H); 4.83 (sept, 1H, J = 6.2); 6.95 (m, 2H); 7.15 (m, 2H); 8.79 (s(1), 1H). ¹³C (CDCl₃): 10.3; 22.3; 26.9; 71.2; 102.7; 114.8 (J = 21 Hz); 129.5; 137.0; 137.5; 161.2 (J = 243 Hz); 161.6. HRMS: Calc. for C₁₄H₁₇FN₂O + H: 249.1403; Found: 249.1327.

**3-isopropoxy-4-(2-methoxybenzyl)-5-methyl-1H-pyrazole (intermediate 15):** The crude residue was dispersed in water neutralized with solid sodium hydrogenocarbonate, and extracted with dichloromethane. The insoluble material was filtered and the organic layer was washed with a saturated solution of sodium hydrogenocarbonate, water, brine, dried over magnesium sulfate and concentrated to dryness to yield this compound as a yellow solid. ¹H (CDCl₃): 1.32 (d, 6H, J = 6.0); 2.12 (s, 3H); 3.65 (s, 2H); 3.81 (s, 3H); 4.83 (sept, 1H, J = 6.0); 6.84 (m, 2H); 7.13 (m, 2H); 9.1 (s(1), 1H). ¹³C (CDCl₃): 10.2; 21.5; 22.3; 55.2; 71.1; 102.0; 109.9; 120.2; 126.8; 129.3; 129.6; 138.0; 157.1; 161.9. HRMS: Calc. for C₁₅H₂₀N₂O₂ + H: 261.1603; Found: 261.1571.

**3-isopropoxy-4-(3-methoxybenzyl)-5-methyl-1H-pyrazole (intermediate 16):** The crude residue was dispersed in water neutralized with solid sodium hydrogenocarbonate, and extracted with dichloromethane. The insoluble material was filtered and the organic layer was washed with a saturated solution of sodium hydrogenocarbonate, water, brine, dried over magnesium sulfate and concentrated to dryness to yield this compound as an oil. ¹H (CDCl₃): 1.36 (d, 6H, J = 6.2); 2.11 (s, 3H); 3.64 (s, 3H); 3.79 (s, 2H); 4.85 (sept, 1H, J = 6.2); 6.79 (m, 2H); 7.18 (m, 1H); 8.88 (s(1), 1H). ¹³C (CDCl₃): 10.3; 22.3; 27.7; 55.1; 71.2; 102.6; 111.1; 114.0; 120.7; 129.1; 137.8; 143.1; 159.6; 161.6. HRMS: Calc. for C₁₅H₂₀N₂O₂ + H: 261.1603; Found: 261.1541.

**3-ethoxy-5-methyl-4-phenoxy-1H-pyrazole (intermediate 17):** The crude residue was dispersed in water neutralized with solid sodium hydrogenocarbonate, and extracted with dichloromethane. The filtered organic layer was washed with a saturated solution of sodium hydrogenocarbonate, water, brine, dried over magnesium sulfate and concentrated to dryness. The resulting residue was further purified by a chromatography over silica gel (dichloromethane - ethanol 99/1) to yield the alkoxypyrazole as a solid. ¹H (CDCl₃): 1.34 (t, 3H, J = 7.1); 2.14 (s, 3H); 4.26 (q, 2H, J = 7.1); 6.96 (m, 2H); 7.00 (m, 1H); 7.30 (m, 2H); 9.40 (s(1), 1H). ¹³C (CDCl₃): 9.1; 14.9; 64.9; 115.0; 121.8; 121.9; 129.4; 132.0; 155.1; 158.7. HRMS: Calc. for C₁₂H₁₄N₂O₂ + H: 219.1134; Found: 219.1059.

**4-(2-fluorophenoxy)-3-isopropoxy-5-methyl-1H-pyrazole (intermediate 18):** The crude residue was directly purified by a chromatography over silica gel (dichloromethane-ethanol 99/1 to 95/5 the alkoxypyrazole was obtained as a solid after a second chromatography over neutral alumina containing 1.5 % water (cyclohexane - dichloromethane 1/4). ¹H (CDCl₃): 1.29 (d, 6H, J = 6.1); 2.16 (s, 3H); 4.79 (sept, 1H, J = 6.1); 6.88 (m, 1H); 6.97 (m, 2H); 7.13 (m, 1H); 9.3 (s(1), 1H). ¹³C (CDCl₃): 9.0; 22.1; 72.4; 116.3; 116.4 (18 Hz); 122.3; 122.6; 124.1; 131.6; 146.5; 152.3 (246 Hz); 154.2. HRMS: Calc. for C₁₃H₁₅FN₂O₂ + H: 251.1196; Found: 251.1110.

**3-ethoxy-4-(2-fluorophenoxy)-5-methyl-1H-pyrazole (intermediate 19):** The crude residue was directly purified by a chromatography over silica gel (dichloromethane - ethanol 99/1 to 95/5) to yield the alkoxypyrazole as an orange solid. ¹H (CDCl₃): 1.33 (t, 3H, J = 7.1); 2.16 (s, 3H); 4.26 (q, 2H, J = 7.1); 6.90 (m, 1H); 6.99 (m, 2H); 7.15 (m, 1H); 8.90 (s(1), 1H). ¹³C (CDCl₃): 9.0; 14.8; 64.8; 116.1; 116.2 (24 Hz); 121.8; 122.3; 124.1; 131.7; 146.5; 152.3 (245 Hz); 155.0. HRMS: Calc. for C₁₃H₁₅FN₂O₂ + H: 237.1039; Found: 237.0945.

**4-(2-fluorophenoxy)-5-methyl-1H-pyrazol-3-ol (intermediate 20):** A second fraction of the chromatography described above gave this compound as a white solid after the adsorption of a sample on a small amount of neutral alumina, containing 1.5 % of water, followed by a continuous extraction with ethyl acetate. ¹H (DMSO-d6): 2.00 (s, 3H); 6.78 (m, 1H); 6.98 (m, 1H); 7.05 (m, 1H); 7.26 (m, 1H); 10.40 (s(1), 2H). ¹³C (DMSO-d6): 9.2; 116.1; 116.8 (17 Hz); 120.3; 122.6; 125.2; 130.9; 146.8; 151.8 (244 Hz); 152.6. HRMS: Calc. for C₁₀H₉FN₂O₂ + H: 209.0726; Found: 209.0629.

**4-(2-bromophenoxy)-3-ethoxy-5-methyl-1H-pyrazol (intermediate 21):** The crude residue was directly purified by a chromatography over silica gel (dichloromethane - ethanol 99/1 to 95/5), the alkoxypyrazole as a solid. ¹H (CDCl₃): 1.33 (t, 3H, J = 7.1); 2.14 (s, 3H); 4.25 (q, 2H, J = 7.1); 6.80 (m, 1H); 6.90 (m, 1H); 7.19 (m, 1H); 7.57 (m, 1H); 9.40 (s(1), 1H). ¹³C (CDCl₃): 9.1; 14.9; 65.0; 111.2; 114.9; 122.0; 123.1; 128.3; 131.9; 133.4; 154.9; 155.2. HRMS: Calc. for C₁₂H₁₃BrN₂O₂ + H: 297.0239; Found: 297.0209.

**4-(2,3-dichlorophenoxy)-3-ethoxy-5-methyl-1H-pyrazole (intermediate 22):** The crude residue was directly purified by a chromatography over silica gel (dichloromethane-ethanol 98/2 to 9/1), the alkoxypyrazole as a solid. ¹H (DMSO-d6): 1.20 (t, 3H, J = 7.0); 2.02 (s, 3H); 4.13 (q, 2H, J = 7.0); 6.76 (m, 1H); 7.27 (m, 2H); 11.76 (s(1), 1H). ¹³C (DMSO-d6): 8.9; 15.2; 64.4; 113.6; 120.1; 120.3; 123.9; 129.0; 131.1; 133.0; 153.6; 155.8. HRMS: Calc. for C₁₂H₁₂Cl₂N₂O₂ + H: 287.0354; Found: 287.0323.

**4-(2,5-dichlorophenoxy)-3-ethoxy-5-methyl-1H-pyrazole (intermediate 23):** The crude residue was directly purified by a chromatography over silica gel (dichloromethane-ethanol 98/2 to 9/1), the alkoxypyrazole as a solid. ¹H (CDCl₃): 1.35 (t, 3H, J = 7.1); 2.16 (s, 3H); 4.27 (q, 2H, J = 7.1); 6.82 (d, 1H, J = 2.3); 6.95 (m, 2H); 7.32 (d, 1H, J = 8.4); 9.4 (s(1), 1H). ¹³C (CDCl₃): 9.0; 14.8; 65.0; 115.4; 120.9; 121.3; 122.8; 130.9; 131.8; 133.1; 154.7; 159.8. HRMS: Calc. for C₁₂H₁₂Cl₂N₂O₂ + H: 287.0354; Found: 287.0298.

**4-(2-fluorophenoxy)-3-isopropoxy-1H-pyrazole (intermediate 24):** The crude isopropyl 2-(2-fluorophenoxy)-3-oxopropanoate (3 g) described above was dissolved in isopropanol, hydrazine mono hydrochloride (0.85 g) was added and the suspension was heated to reflux overnight. This was concentrated to dryness and the residue purified by a chromatography over silica gel (dichloromethane - ethanol 98/2 to 94/6) followed by a second chromatography over neutral alumina containing 1.5 % of water (dichloromethane-ethanol 100/0 to 99/1) gave this compound as an oil. ¹H (CDCl₃): 1.32 (d, 6H, J = 6.2); 4.85 (sept, 1H, J = 6.2); 6.99 (m, 3H); 7.02 (m, 1H); 7.32 (s, 1H); 8.95 (s(1), 1H). ¹³C (CDCl₃): 22.1; 72.7; 116.5 (17 Hz); 117.3; 121.0; 122.9 (7 Hz); 124.1 (4 Hz); 126.2; 146.4 (10 Hz); 152.6 (245 Hz); 153.8. HRMS: Calc. for C₁₂H₁₃FN₂O₂ + H: 237.1039; Found: 237.0996.

**4-(2,3-difluorophenoxy)-3-isopropoxy-5-methyl-1H-pyrazole (intermediate 25):** The crude residue was directly purified by a chromatography over silica gel (dichloromethane - ethanol 99/1 to 95/5), the alkoxypyrazole was obtained as a solid after a second chromatography over neutral alumina containing 1.5 % water (cyclohexane - dichloromethane 1/4). ¹H (CDCl₃): 1.29 (d, 6H, J = 6.1); 2.16 (s, 3H); 4.79 (sept, 1H, J = 6.1); 6.65 (m, 1H); 6.83 (m, 1H); 6.92 (m, 1H); 9.5 (s(1), 1H). ¹³C (CDCl₃): 9.0; 22.1; 72.4; 110.2 (18 Hz); 111.2; 122.4; 122.8; 131.6; 141.2 (248 and 15 Hz); 148.1; 151.5 (247 and 10 Hz); 154.0. HRMS: Calc. for C₁₃H₁₄F₂N₂O₂ + H: 269.1102; Found: 269.1071.

**4-(2,5-difluorophenoxy)-3-isopropoxy-5-methyl-1H-pyrazole (intermediate 26):** The crude residue was directly purified by a chromatography over silica gel (dichloromethane - ethanol 99/1 to 95/5), the alkoxypyrazole was obtained as a solid after a second chromatography over neutral alumina containing 1.5 % water (cyclohexane - dichloromethane 1/4). ¹H (CDCl₃): 1.31 (d, 6H, J = 6.1); 2.17 (s, 3H); 4.82 (sept, 1H, J = 6.1); 6.63 (m, 2H); 7.08 (m, 1H); 9.40 (s(1), 1H). ¹³C (CDCl₃): 9.0; 21.2; 72.4; 104.0 (28 Hz); 108.2 (24 and 6 Hz); 116.6 (20 and 10 Hz); 122.0; 131.6; 147.2 (13 and 7 Hz); 148.5 (243 Hz); 154.0; 158.6 (243 Hz). HRMS: Calc. for C₁₃H₁₄F₂N₂O₂ + H: 269.1102; Found: 269.1028.

**4-(3-fluorophenoxy)-3-isopropoxy-5-methyl-1H-pyrazole (intermediate 27):** The crude residue was directly purified by a chromatography over silica gel (dichloromethane-ethanol 99/1 to 95/5 and the alkoxypyrazole was obtained as an oil after a second chromatography over neutral alumina containing 1.5 % water (cyclohexane - dichloromethane 4/1). ¹H (CDCl₃): 1.30 (d, 6H, *J* = 6.2); 2.14 (s, 3H); 4.80 (sept, 1H, J = 6.2); 6.00 (m, 3H); 7.20 (m, 1H); 9.60 (s(l), 1H). ¹³C (CDCl₃): 9.1; 22.2; 72.3; 102.9 (25 Hz); 108.7 (21 Hz); 110.9 (3 Hz); 122.1; 130.2 (9 Hz); 131.6; 154.2; 160.0 (10 Hz); 163.5 (245 Hz). HRMS: Calc. for C₁₃H₁₅FN₂O₂ + H: 251.1196; Found: 251.1165.

**3-ethoxy-5-methyl-4-(2-(trifluoromethyl)phenoxy)-1H-pyrazole** (intermediate 28): The crude residue was directly purified by a chromatography over silica gel (dichloromethane - ethanol 98/2 to 9/1), and the alkoxypyrazole obtained as a solid. ¹H (CDCl₃): 1.32 (t, 3H, J = 7.0); 2.13 (s, 3H); 4.25 (q, 2H, J = 7.0); 6.91 (m, 1H); 7.06 (m, 1H); 7.43 (m, 1H); 7.63 (m, 1H); 9.3 (s(l), 1H). ¹³C (CDCl₃): 8.9; 14.8; 65.1; 114.6; 118.7 (31 Hz); 121.4 (two signals); 123.6 (273 Hz); 126.9; 132.0; 133.1; 155.0; 156.5. HRMS: Calc. for C₁₃H₁₃F₃N₂O₂ + H: 287.1007; Found: 287.0924.

**3-ethoxy-5-methyl-4-(3-(trifluoromethyl)phenoxy)-1H-pyrazole (intermediate 29):** The crude residue was directly purified by a chromatography over silica gel (dichloromethane - ethanol 98/2 to 93/7) and the alkoxypyrazole was obtained as a solid. ¹H (CDCl₃): 1.33 (t, 3H, J = 7.04); 2.15 (s, 3H); 4.26 (q, 2H, J = 7.04); 7.13 (s, 1H); 7.20 (m, 1H); 7.29 (m, 1H); 7.40 (m, 1H); 8.70 (s(l), 1H). ¹³C (CDCl₃): 9.1; 14.8; 64.8; 112.1 (4 Hz); 118.4; 118.6 (4Hz); 121.1; 123.8 (272 Hz); 130.0; 131.8; 131.9 (32 Hz); 154.9; 158.7. HRMS: Calc. for C₁₃H₁₃F₃N₂O₂ + H: 287.1007; Found: 287.0966.

**3**-**isopropoxy**-**4**-(**4**-**methoxybenzyl**)-**5**-**methyl**-1**H**-**pyrazole (intermediate 30):** The crude residue was dispersed in water neutralized with solid sodium hydrogenocarbonate, and extracted with dichloromethane. The insoluble material was filtered and the organic layer was washed with a saturated solution of sodium hydrogenocarbonate, water, brine, dried over magnesium sulfate and concentrated to dryness to yield this compound as an oil. ¹H (CDCl₃): 1.34 (d, 6H, J = 6.1); 2.09 (s, 3H); 3.60 (s, 2H); 3.79 (s, 3H); 4.83 (sept, 1H, J = 6.1); 6.81 (m, 2H); 7.13 (m, 2H); 8.70 (s(l), 1H). ¹³C (CDCl₃): 10.3; 22.3; 26.8; 55.2; 71.2; 103.2; 113.6; 129.1; 135.5; 137.6; 157.7; 161.6. HRMS: Calc. for C₁₅H₂₀N₂O₂ + H: 261.1603; Found: 261.1533.

**4-(4-fluorophenoxy)-3-isopropoxy-5-methyl-1H-pyrazole (intermediate 31):** The crude residue was directly purified by a chromatography over silica gel (dichloromethane - ethanol 99/1 to 95/5), the alkoxypyrazole was obtained as an oil after a second chromatography over neutral alumina containing 1.5% water (cyclohexane - dichloromethane 1/9). ¹H (CDCl₃): 1.28 (d, 6H, J = 6.1); 2.12 (s, 3H); 4.77 (sept, 1H, J = 6.1); 6.89 (m, 2H); 6.96 (m, 2H); 10.1 (s(l), 1H). ¹³C (CDCl₃): 9.1; 22.2; 72.4; 115.7 (23 Hz); 116.1 (8 Hz); 122.9; 131.8; 154.1; 154.8; 157.9 (239 Hz). HRMS: Calc. for C₁₃H₁₅FN₂O₂ + H: 251.1196; Found: 251.1136.

**4-(3,5-dichlorophenoxy)-3-ethoxy-5-methyl-1H-pyrazole (intermediate 32):** The crude residue was directly purified by a chromatography over silica gel (dichloromethane-ethanol 98/2 to 9/1), the alkoxypyrazole as a solid. ¹H (CDCl₃): 1.35 (t, 3H, J = 7.1); 2.14 (s, 3H); 4.27 (q, 2H, J = 7.1); 6.86 (m, 2H); 7.03 (m, 1H); 9.2 (s(l), 1H). ¹³C (CDCl₃): 9.1; 14.8; 64.9; 114.2; 121.0; 122.3; 131.8; 135.4; 154.8; 159.7. HRMS: Calc. for C₁₂H₁₂Cl₂N₂O₂ + H: 287.0354; Found: 287.0302.

**3-ethoxy-5-methyl-4-(4-(trifluoromethyl)phenoxy)-1H-pyrazole (intermediate 33):** The crude residue was directly purified by a chromatography over silica gel (dichloromethane - ethanol 98/2 to 9/1) and the alkoxypyrazole was obtained as a solid. ¹H (CDCl₃): 1.34 (t, 3H, J = 7.0); 2.14 (s, 3H); 4.26 (q, 2H, J = 7.0); 6.91 (d, 2H, J = 9.1); 7.59 (d, 2H, J = 9.1); 9.30 (s(l), 1H). ¹³C (CDCl₃): 9.1; 14.8; 64.9; 115.1; 121.1; 124.0 (270 Hz); 124.2 (33 Hz); 129.6 (4 Hz); 131.8; 154.9; 161.1. HRMS: Calc. for C₁₃H₁₃F₃N₂O₂ + H: 287.1007; Found: 287.0968.

**3-(3-isopropoxy-5-methyl-1H-pyrazol-4-yloxy)pyridine (intermediate 34):** The crude residue was dispersed in water neutralized with solid sodium hydrogenocarbonate, and extracted with dichloromethane. The organic layer was washed with a saturated solution of sodium hydrogenocarbonate, water, brine, dried over magnesium sulfate and concentrated to dryness. The residue was purified by a chromatography over silica gel (dichloromethane-ethanol 97/3 to 9/1) and the alkoxypyrazole was obtained as a solid after a second chromatography over neutral alumina containing 1.5 % water (dichloromethane - ethanol 99/1 to 98/2). ¹H (CDCl₃): 1.29 (d, 6H, J = 6.2); 4.81 (sept, 1H, J = 6.2); 7.23 (m, 2H); 8.29 (dd, 1H, J = 1.9 and 4.2); 8.39 (m, 1H); 9.7 (s(1), 1H). ¹³C (CDCl₃): 9.1; 22.2; 72.2; 121.9; 122.1; 123.8; 131.4; 138.6; 143.1; 154.1; 155.1. HRMS: Calc. for C₁₂H₁₅N₃O₂ + H: 234.1243; Found: 234.1163.

**4-benzyl-3-(2-(benzyloxy)ethoxy)-5-methyl-1H-pyrazole (intermediate 35):** The crude residue was dispersed in water neutralized with solid sodium hydrogenocarbonate, and extracted with dichloromethane. The filtered organic layer was washed with a saturated solution of sodium hydrogenocarbonate, water, brine, dried over magnesium sulfate and concentrated to dryness. The resulting residue was further purified by a chromatography over silica gel (dichloromethane - ethanol 97/3) to yield the alkoxypyrazole as a white solid. ¹H (CDCl₃): 2.18 (s, 3H); 3.69 (s, 2H); 3.82 (m, 2H); 4.40 (m, 2H); 4.60 (s, 2H); 7.15-7.36 (m, 10H). ¹³C (CDCl₃): 10.3; 27.7; 68.0; 68.8; 73.2; 102.1; 125.7; 127.5; 127.6; 128.2 (two signals); 128.3; 138.0; 138.4; 141.2; 161.2. HRMS: Calc. for C₂₀H₂₃N₂O₂ + H: 323.1760; Found: 323.1732.

**4-butyl-3-ethoxy-5-methyl-1H-pyrazole (intermediate 36):** The crude residue was dispersed in water neutralized with solid sodium hydrogenocarbonate, and extracted with dichloromethane. The organic layer was washed with a saturated solution of sodium hydrogenocarbonate, water, brine, dried over magnesium sulfate and concentrated to dryness to yield this compound as a solid. ¹H (CDCl₃): 0.92 (t, 3H, J = 7.2); 1.31 (m, 2H); 1.37 (t, 3H, J = 7.1); 1.47 (m, 2H); 2.18 (s, 3H); 2.30 (t, 2H, J = 7.4); 4.25 (q, 2H, J = 7.1); 6.4 (s(l), 1H). ¹³C (CDCl₃): 10.2; 13.9; 15.0; 21.2; 22.3; 30.0; 64.7; 103.4; 138.0; 161.6. HRMS: Calc. for C₁₀H₁₈N₂O + H: 183.1497; Found: 183.1456.

**6-benzyl-3-ethoxy-4,5,6,7-tetrahydro-1H-pyrazolo[3,4-c]pyridine (intermediate 37):** Obtained from ethyl 1-benzyl-3-oxopiperidine-4-carboxylate as a solid by following the protocol described for the synthesis of 5-benzyl-3-ethoxy-4,5,6,7-tetrahydro-1H-pyrazolo[4,3-c]pyridine as described in Guillou et al. Tetrahedron 2010, 66, 2654. M.p. = 111 °C.¹H (CDCl₃): 1.38 (t, 3H, J = 7.0); 2.52 (t, 2H, J = 5.7); 2.77 (t, 2H, J = 5.7); 3.45 (s, 2H); 3.7 (s, 2H); 4.23 (q, 2H, J = 7.0); 7.34 (m, 5H); ¹³C (CDCl₃): 15.3; 19.7; 49.2; 50.9; 62.0; 64.7; 99.2; 127.7; 128.7; 129.4; 138.5; 139.6; 160.8. Calc. for C₁₅H₁₉N₃O + H : 258.1606. Found: m/z, 258.1620.

### - Examples of preparations of 3-alkoxypyrazole from 3-(dialkylamino)acrylates

**3-isopropoxy-4-phenoxy-1H-pyrazole (intermediate 38):** In isopropanol (40 mL), isopropyl 3-(dimethylamino)-2-phenoxyacrylate (0.83 g, 0.0033 mol) and hydrazine dihydrochloride (0.37 g, 0.0034 mol) were heated to reflux overnight. The resulting solution was concentrated to dryness, the residue dispersed in ethyl acetate and an excess of a saturated solution of sodium hydrogenocarbonate. The organic layer was washed once with a saturated solution of sodium hydrogenocarbonate, brine, dried over magnesium sulfate and concentrated to dryness. The residue was purified by two consecutive chromatography, the first one over silica gel (dichloromethane - ethanol 98/2), the second one over alumina containing 1.5 % of water (dichloromethane - ethanol 99/1) to yield the isopropyloxypyrazole as a light yellow solid (0.08 g, 11 %). ¹H (CDCl₃): 1.32 (d, 6H, J = 6.1); 4.84 (sept, 1H, J = 6.1); 7.03 (m, 3H); 7.30 (m, 2H); 7.32 (s, 1H); 8.91 (s(l), 1H). ¹³C (CDCl₃): 22.2; 72.6; 115.7; 121.4; 122.2; 125.9; 129.3; 154.0; 158.8. HRMS: Calc. for C₁₂H₁₄N₂O₂ + H: 219.1134; Found: 219.1056. On a larger scale, the residue described above was, before the purification by chromatography, dispersed in a mixture of water and ethylacetate, the insoluble material was filtered, washed with ethyl acetate and dried under vacuum to yield the 4-phenoxy-1H-pyrazol-3-ol (intermediate 39). ¹H (DMSO-*d6*): 6.90 (m, 2H); 6.98 (m, 1H); 7.29 (m, 2H); 7.50 (s, 1H); 9.8 (s(l), 1H); 11.5 (s(l), 1H). ¹³C (DMSO-*d6*): 115.4; 122.1; 123.2; 129.2 (two signals); 152.3; 159.4. HRMS: Calc. for C₉H₈N₂O₂+ H: 177.0664; Found: 177.0636.

**4-(2-fluorophenoxy)-3-isopropoxy-1H-pyrazole (intermediate 24):** From isopropyl 3-(dimethylamino)-2-(2-fluorophenoxy)acrylate, by using the procedure described for the preparation of 3-isopropoxy-4-phenoxy-1H-pyrazole, the compound obtained (10 %) was identical with the sample obtained from isopropyl 2-(2-fluorophenoxy)-3-oxopropanoate as described above.

**3-isopropoxy-4-(phenylthio)-1H-pyrazole (intermediate 40):** In a 1 liter flask, hydrazine dihydrochloride (9.38 g, 0.089 mol) dispersed in isopropanol (300 mL) and water (14 mL) were heated to reflux. The black oil containing isopropyl 3-morpholino-2-(phenylthio)acrylate described above was dissolved in isopropanol (200 mL) and added dropwise to this boiling solution in the course of 7 hours. The resulting solution was heated to reflux overnight and concentrated to dryness. The residue was dispersed in a mixture of water and ethyl acetate, the organic layer was washed with a saturated solution of sodium hydrogenocarbonate, brine, dried over magnesium sulfate and concentrated to dryness. The resulting residue was dispersed in dichloromethane, the insoluble material filtered and the filtrate concentrated to dryness. This residue was dissolved in water and ethanol (200 mL 1/1 v/v), sodium hydroxide (14 G) was added and this was heated to reflux for 30 mn. The ethanol was removed in vacuum, the residue was diluted in more water, extracted with ethyl acetate, the organic layer was washed with a saturated solution of sodium hydrogenocarbonate, brine, dried over magnesium sulfate and concentrated to dryness. The residue was recrystallized in cyclohexane to yield the thioether (4.6 g, 26 % from the starting isopropyl 2-(phenylthio)acetate). ¹H (CDCl₃): 1.36 (d, 6H, J = 6.2); 4.94 (sept, 1H, J = 6.2); 7.13 (m, 3H); 7.22 (m, 2H); 7.56 (s, 1H); 9.75 (s(l), 1H). ¹³C (CDCl₃): 22.1; 72.6; 93.7; 125.1; 126.3; 128.6; 135.4; 138.4; 163.4. HRMS: Calc. for C₁₂H₁₄N₂OS+ H: 235.0905; Found: 235.0812. *Nota:* the insoluble material is pure 4-(phenylthio)-1H-pyrazol-3-ol (which readily decomposes upon strong basic treatment, with release of thiophenol!). ¹H (DMSO-*d6*): 7.07 (m, 3H); 7.22 (m, 2H); 7.73 (s, 1H); 10.4 (s(l), 1H); 11.9 (s(l), 1H). ¹³C (DMSO-*d6*): 88.8; 125.2; 125.5; 129.2; 136.4; 139.7; 162.3. HRMS: Calc. for C₉H₈N₂OS+ H: 193.0436; Found: 193.0487.

### - Examples of preparations of 3-alkoxypyrazoles by alkylation of 3-hydroxypyrazoles

**3-isopropoxy-4-phenoxy-1H-pyrazole (intermediate 38):** This compound was prepared from 4-phenoxy-1H-pyrazol-3-ol (intermediate 39) in four stages. Stage 1: preparation of 1-acetyl-4-phenoxy-1H-pyrazol-3-yl acetate (intermediate 41): 4-phenoxy-1H-pyrazol-3-ol (0.54 g, 0.0030 mol) was dispersed in acetic anhydride (5 mL). Sulfuric acid (94 %, 5 drops) was added leading to an immediate dissolution. The solution was stirred for 20 mn, cooled with water and ice and water (30 mL) was added. The resulting solution was extracted with ethyl acetate, the organic layer was washed with water, a satured solution of sodium hydrogenocarbonate, brine, dried over magnesium sulfate and concentrated to dryness. The residue was purified by a chromatography over silica gel (cyclohexane-dichloromethane 1/1) to yield 1-acetyl-4-phenoxy-1H-pyrazol-3-yl acetate as an oil (0.56 g, 69 %). ¹H (CDCl₃): 2.23 (s, 3H); 2.63 (s, 3H); 7.09 (m, 1H); 7.14 (m, 1H); 7.36 (m, 2H); 7.92 (s, 1H). ¹³C (CDCl₃): 20.1; 20.6; 117.2; 117.6; 124.2; 129.8; 135.8; 149.7; 156.7; 167.1; 168.6. HRMS: Calc. for C₁₃H₁₂N₂O₄ + Na: 283.0695; Found: 283.0659. Stage 2: preparation of 1-(3-hydroxy-4-phenoxy-1H-pyrazol-1-yl)ethanone (intermediate 42): The crude 1-acetyl-4-phenoxy-1H-pyrazol-3-yl acetate was heated in ethanol (4 mL) in a microwave oven at 150 °C for one hour. The mixture was concentrated to dryness and purified by a chromatography over silica gel (dichloromethane - ethanol 98/2) to yield the 1-acetyl-4-phenoxy-1H-pyrazol-3-yl acetate as a white powder (0.14 g, 21 %). ¹H (DMSO-*d6*): 2.48 (s, 3H); 7.03 (m, 2H); 7.11 (m, 1H); 7.33 (m, 2H); 8.12 (s, 1H); 11.56 (s(l), 1H). ¹³C (DMSO-*d6*): 21.1; 116.4; 119.4; 123.5; 130.2; 131.7; 157.0; 157.7; 168.3. HRMS: Calc. for C₁₁H₁₀N₂O₃ + H: 219.0770; Found: 219.0711. Stage 3: preparation of 1-(3-isopropoxy-4-phenoxy-1H-pyrazol-1-yl)ethanone (intermediate 38): 1-acetyl-4-phenoxy-1H-pyrazol-3-yl acetate (0.11 g, 0.0005 mol), isopropylbromide (0.068 g, 0.00055 mol) and cesium carbonate (0.18 g, 0.00055 mol) were dispersed in dimethylformamide (10 mL, dried over 4A molecular sieve). This was stirred at 80 °C under a moisture-protected atmosphere for 4 hours. The resulting mixture was diluted in water, this was extracted with ethyl acetate, the organic layer was washed with water, brine, dried over magnesium sulfate and concentrated to dryness. The residue was purified by a chromatography over silica gel (dichloromethane - ethanol 98/2) to yield the 1-(3-isopropoxy-4-phenoxy-1H-pyrazol-1-yl)ethanone (0.07 g, 53 %) as an oil. ¹H (CDCl₃): 1.39 (d, 6H, J = 6.2); 2.56 (s, 3H); 5.05 (t, 1H, J = 6.2); 7.09 (m, 3H); 7.33 (m, 2H); 7.80 (s, 1H). ¹³C (CDCl₃): 20.6; 21.8; 73.0; 116.8; 117.2; 123.6; 129.6; 133.7; 156.6; 157.2; 158.6. HRMS: Calc. for C₁₄H₁₆N₂O₃ + H: 261.1239; Found: 261.1195. Stage 4: preparation of 3-isopropoxy-4-phenoxy-1H-pyrazole: Treatment of the 1-(3-isopropoxy-4-phenoxy-1H-pyrazol-1-yl)ethanone with a 2N solution of ethanolic ammonia for 30 minutes led to quantitative yield of acetamide and 3-isopropoxy-4-phenoxy-1H-pyrazole, identical with the compound obtained from isopropyl 3-(dimethylamino)-2-phenoxyacrylate (intermediate 5).

**4-(2-fluorophenoxy)-3-isopropoxy-5-methyl-1H-pyrazole (intermediate 18):** This compound was alternatively prepared from 4-(2-fluorophenoxy)-5-methyl-1H-pyrazol-3-ol in two stages. Stage 1: preparation of 1-acetyl-4-(2-fluorophenoxy)-5-methyl-1H-pyrazol-3-yl acetate (YJ 29071-067-1): 4-(2-fluorophenoxy)-5-methyl-1H-pyrazol-3-ol (intermediate 20) (10.72 g, 0.0514 mol) was dispersed in acetic anhydride (60 mL) and sulfuric acid (94 %, 1.5 mL) was added. The resulting solution was stirred for 30 mn and concentrated to dryness. The residue was cooled with water and ice, cold water (300 mL) was added and the solution was stirred for 30 mn. This was extracted with ethylacetate, the organic layer was washed with water, a satured solution of sodium hydrogenocarbonate, brine, dried over magnesium sulfate and concentrated to dryness to yield the crude 1-acetyl-4-(2-fluorophenoxy)-5-methyl-1H-pyrazol-3-yl acetate (13.96 g). %). ¹H (CDCl₃): 2.14 (s, 3H); 2.51 (s, 3H); 2.65 (s, 3H); 6.93 (m, 1H); 7.05 (m, 2H); 7.15 (m, 1H). ¹³C (CDCl₃): 11.8; 20.1; 22.6; 116.9 (J = 18 Hz); 117.4; 123.9 (J = 7 Hz); 124.3 (J = 3 Hz); 130.8; 134.9; 144.7 (J = 11 Hz); 149.0; 152.4 (J = 248 Hz); 167.2; 171.2. Stage 2: In dry dimethylformamide (150 mL, dried over molecular sieve), the crude 1-acetyl-4-(2-fluorophenoxy)-5-methyl-1H-pyrazol-3-yl acetate (10.65 g), isopropyl bromide (4.95 g, 0.040 mol) and cesium carbonate (25 g, 0.0767 mol) were heated under a moisture-protected atmosphere at 80 °C for 4 hours. The solvent was removed under vacuum and a 2N solution of ethanolic ammonia (100 mL) was added. This was stirred for 30 mn and concentrated to dryness. This residue was dispersed in water and ethyl acetate, the organic layer was washed with water and brine, dried over magnesium sulfate and concentrated to dryness. The crude compound was further purified by a chromatography over silica gel (dichloromethane - ethanol 97.5/2.5) to yield the 4-(2-fluorophenoxy)-3-isopropoxy-5-methyl-1H-pyrazole (6.28 g, 63 % from 4-(2-fluorophenoxy)-5-methyl-1H-pyrazol-3-ol) identical with the compound described above.

### Preparations of other pyrazoles

**2-(4-benzyl-5-(bromomethyl)-3-isopropoxy-1H-pyrazol-1-yl)-5-**cyclopropylpyrimidine (intermediate 43): In acetonitrile (5 mL, dried over 4A molecular sieves) 2-(4-benzyl-3-isopropoxy-5-methyl-1H-pyrazol-1-yl)-5-cyclopropylpyrimidine (0.28 g, 8.0 mmol) and N-bromosuccinimide (0.17 g, 9.6 mmol) were stirred overnight at room temperature. The resulting solution was concentrated to dryness and redissolved in ethyl acetate. The organic phase was washed with a saturated solution of sodium hydrogenocarbonate, water, brine, dried over magnesium sulfate and concentrated to dryness to yield the 2-(4-benzyl-5-(bromomethyl)-3-isopropoxy-1H-pyrazol-1-yl)-5-cyclopropylpyrimidine (0.28 g) as glass. ¹H (CDCl₃): 0.78 (m, 2H); 1.10 (m, 2H); 1.33 (d, 6H, J = 6.1); 1.87 (m, 1H); 3.81 (s, 2H); 5.05 (s, 2H); 5.23 (sept, 1H, J = 6.1); 7.19 (m, 1H); 7.27 (m, 4H); 8.48 (s, 2H). ¹³C (CDCl₃): 8.4; 10.5; 22.2; 22.7; 27.7; 71.7; 111.8; 126.1; 128.3; 128.5; 132.6; 138.8; 139.5; 154.4; 156.3; 162.1.

**4-((4-benzyl-3-ethoxy-1H-pyrazol-5-yl)methyl)morpholine (intermediate 44):** In dichloromethane (15 mL), 4-benzyl-3-ethoxy-5-methyl-1H-pyrazole (0.37 g, 1.71 mmol) and N-chlorosuccinimide (0.24 g, 1.79 mmol) were stirred at room temperature for 70 minutes. Morpholine (0.45 mL, 5.13 mmol) was added, the solution was concentrated to dryness and the residue heated at 70 °C overnight under a moisture-protected atmosphere. This was dispersed in ethyl acetate and a saturated solution of sodium hydrogenocarbonate, the organic layer was washed with a saturated solution of sodium hydrogenocarbonate, brine, dried over magnesium sulfate and concentrated to dryness. The residue was purified by chromatography over silica gel (dichloromethane - ethanol from 98/2 to 9/1) to yield the amine as an oil (0.1 g, 19 %). ¹H (CDCl₃): 1.39 (t, 3H, J = 7.1); 2.36 (m, 4H); 3.36 (s, 2H); 3.68 (m, 4H); 3.72 (s, 2H); 4.28 (q, 2H, J = 7.1); 7.21 (m, 5H); 9.80 (s(l), 1H). ¹³C (CDCl₃): 15.0; 27.5; 52.7; 53.5; 64.2; 66.7; 103.3; 125.8; 128.2; 128.3; 137.6; 141.2; 166.2. HRMS: Calc. for C₁₇H₂₃N₃O₂ + H: 302.1869; Found: 302.1858.

**3-cyclopropyl-4-phenoxy-1H-pyrazole (intermediate 45):** This compound was prepared in two stage. 1-cyclopropyl-2-phenoxyethanone (intermediate 61) (2.25 g, 0.0127 mol) and dimethylformamide dimethylacetal (3.8 g, 0.032 mol) were heated to reflux overnight. The resulting black solution was concentrated to dryness. An ¹H NMR spectrum pointed out the presence of traces of DMF and the expected 1-cyclopropyl-3-(dimethylamino)-2-phenoxyprop-2-en-1-one; ¹H (CDCl₃): 0.66 (m, 2H); 0.96 (m, 2H); 2.10 (m, 1H); 3.01 (s, 6H); 7.00 (m, 3H); 7.32 (m, 3H). This residue and hydrazine hydrate (1.3 mL, 0.025 mol) were heated to reflux in acetic acid (20 mL) for one hour. The acetic acid was removed under vacuum, the residue was dispersed in water, and this was extracted with ethyl acetate. The organic layer was washed with water, saturated solution of sodium hydrogenocarbonate, brine and dried over magnesium sulfate before concentration to dryness. The residue was purified by a chromatography over silica gel (dichloromethane - ethanol 97.5/2.5) to yield the pyrazole as a solid (2.08 g, 81 %). ¹H (CDCl₃): 0.87 (m, 4H); 1.78 (m, 1H); 7.01 (m, 3H); 7.31 (m, 2H); 7.40 (s, 1H); 10.0 (s(l), 1H). ¹³C (CDCl₃): 5.7; 6.4; 115.6; 122.1; 129.5; 137.2; 159.2. HRMS: Calc. for C₁₂H₁₂N₂O + H: 201.1028; Found: 201.0952.

**4-benzyl-3-isobutyl-5-methyl-1H-pyrazole (intermediate 46):** This compound was made from 6-methylheptane-2,4-dione in two steps. In a tube for microwave oven, 6-methylheptane-2,4-dione (0.78 g, 5.48 mmol), benzyl bromide (0.94 g, 5.48 mmol) and potassium carbonate (1.13 g, 8.22 mmol) were dispersed in dimethylformamide (10 mL, dried over 4A molecular sieves). This was heated at 130 °C for 30 minutes before diluting it in ethyl acetate and water. The organic layer was washed with water three times, brine once, dried over magnesium sulfate and concentrated to dryness. The resulting crude residue was dissolved in ethanol, hydrazine hydrate (0.29 mL, 6.03 mmol) was added and the solution was stirred overnight at room temperature. This was concentrated to dryness and partially purified by a chromatography over silica gel (cyclohexane - ethyl acetate from 3/2 to 1/1) to yield the pyrazole as an oil (0.75 g) pure enough for the N-arylation step. ¹H (CDCl₃): 0.90 (d, 6H, J = 6.7); 1.88 (m, 1H); 2.15 (s, 3H); 2.49 (d, 2H, J = 7.3); 3.77 (s, 2H); 7.11-7.29 (m, 5H); 8.30 (s(l), 1H).

### Miscellaneous

**2-(4-bromo-3-isopropoxy-5-methyl-1H-pyrazol-1-yl)pyridine (intermediate 47):** 2-(3-isopropoxy-5-methyl-1H-pyrazol-1-yl)pyridine (preparation provided in Tetrahedron, 2010, 66, 2654; 0.85 g, 3.91 mmol) and N-bromosuccinimide (0.77 g, 4.30 mmol) were heated to reflux in cyclohexane (50 mL) for one hour. This was concentrated to dryness and purified by a chromatography over silica gel (cyclohexane - dichloromethane from 8/1 to 1/1) to give the 4-brominated derivative as an oil (1 g, 86 %). ¹H (CDCl₃): 1.47 (d, 3H, J = 6.0); 2.54 (s, 3H); 5.04 (sept, 1H, J = 6.0); 7.12 (m, 1H); 7.77 (m, 2H); 8.38 (m, 1H). ¹³C (CDCl₃): 13.9; 22.1; 72.4; 85.9; 114.6; 120.3; 138.2; 140.1; 147.3; 153.5; 159.3. HRMS: Calc. for C₁₂H₁₄BrN₃O + H: 296.0398; Found: 296.0409.

**3-ethoxy-4-iodo-1-(4-methoxyphenyl)-1H-pyrazole (intermediate 48):** By following the procedure described for the preparation of 4-benzyl-1-(4-ethylphenyl)-3-isopropoxy-5-methyl-1H-pyrazole (example 132), from 3-ethoxy-4-iodopyrazole and 4-methoxybenzeneboronic acid, this compound was obtained as solid in a 85 % yield after a chromatography over silica gel (cyclohexane - dichloromethane 1/1). ¹H (CDCl₃): 1.47 (t, 3H, J = 7.1); 3.84 (s, 3H); 4.38 (q, 2H, J = 7.1); 6.95 (d, 2H, J = 7.2); 7.48 (d, 2H, J = 7.2); 7.67 (s, 1H). ¹³C (CDCl₃): 14.7; 45.8; 55.5; 65.6; 114.5; 119.6; 131.8; 133.8; 157.8; 163.5. HRMS: Calc. for C₁₂H₁₃IN₂O₂ + H: 345.0100; Found: 345.0096.

**2-chloro-5-cyclopropylpyrimidine (intermediate 49):** (see US 20090023702 for a related procedure).. Under an inert atmosphere, 5-bromo-2-chloropyrimidine (10 g, 0.051 mol), and cesium carbonate (60 g, 0.18 mol) were dispersed in a 95/5 vv mixture of toluene and water (400 mL). This was degassed by gently bubbling argon in the reaction and [1,1'-bis(diphenylphosphino)ferrocene] dichloropalladium complexed with dichloromethane (2.1 g, 0.0025 mol) and cyclopropylboronic acid (5.3 g, 0.062 mol) were added. The flask was heated to reflux for 20 minutes, upon cooling the suspension was diluted in ethyl acetate, the filtered organic layer was washed with water, brine, dried over magnesium sulfate and concentrated to dryness. The residue was purified by a chromatography over silica gel (cyclohexane - ethyl acetate 6/1) to yield the pyrimidine as a white powder (5.3 g, 69 %; stored at -20 °C). ¹H (CDCl₃): 0.79 (m, 2H); 1.13 (m, 2H); 1.88 (m, 1H); 8.36 (s, 2H). ¹³C (CDCl₃): 9.0; 10.3; 135.7; 157.3; 158.5. HRMS: Calc. for C₇H₇ClN₂ + H: 155.0376; Found: 155.0370.

**5-cyclopropyl-2-fluoropyridine (intermediate 50):** (see WO 2008076705 for an alternative procedure). By using the same procedure used for the preparation of 2-chloro-5-cyclopropylpyrimidine, this compound was obtained in 72 % yield from 5-bromo-2-fluoropyrimidine after a chromatography over silica gel (cyclohexane - dichloromethane 1/1) as a volatile oil. ¹H (CDCl₃): 0.68 (m, 2H); 1.02 (m, 2H); 1.91 (m, 1H); 6.81 (m, 1H); 7.42 (m, 1H); 8.02 (m, 1H). ¹³C (CDCl₃): 8.6; 12.2; 108.9 (38 Hz); 136.7 (4 Hz); 138.1 (7 Hz); 145.5 (14 Hz); 162.2 (236 Hz).

**5-cyclopropyl-2,3-difluoropyridine (intermediate 51):** By using the same procedure used for the preparation of 2-chloro-5-cyclopropylpyrimidine, this compound was obtained in 63 % yield from 5-bromo-2,3-difluoropyridine after a chromatography over silica gel (cyclohexane - dichloromethane 2/1) as a volatile oil. ¹H (CDCl₃): 0.71 (m, 2H); 1.06 (m, 2H); 1.94 (m, 1H); 7.18 (m, 1H); 7.78 (m, 1H). ¹³C (CDCl₃): 9.0; 12.2; 123.5 (3 and 15 Hz); 139.3; 139.4 (5 and 13 Hz); 145.4 (29 and 260 Hz); 150.4 (15 and 236 Hz).

**2-chloro-5-cyclopropylnicotinonitrile (intermediate 52):** By using the same procedure used for the preparation of 2-chloro-5-cyclopropylpyrimidine, this compound was obtained in 53 % yield from 5-bromo-2-chloronicotinonitrile after a chromatography over silica gel (cyclohexane - dichloromethane 1/1) as a solid. ¹H (CDCl₃): 0.79 (m, 2H); 1.17 (m, 2H); 1.97 (m, 1H); 7.59 (d, 1H, J = 2.5); 8.38 (d, 1H, J = 2.5). ¹³C (CDCl₃): 9.8; 12.3; 110.2; 114.8; 139.1; 139.3; 149.4. 151.3. HRMS: Calc. for C₉H₇BrN₂ + H: 222.9871; Found: 222.9840.

**2-bromo-5-cyclopropylpyrazine (intermediate 53):** By using the same procedure used for the preparation of 2-chloro-5-cyclopropylpyrimidine, this compound was obtained in 45 % yield from 2,5-dibromopyrazine after a chromatography over silica gel (cyclohexane-ethylacetate 97/3) as a solid. ¹H (CDCl₃): 1.09 (m, 4H); 2.03 (m, 1H); 8.26 (d, 1H, J = 1.4); 8.48 (d, 1H, J = 1.4). ¹³C (CDCl₃): 10.6; 14.1; 136.8; 143.0; 146.5. 157.3.

**3-chloro-6-cyclopropylpyridazine (intermediate 54):** By using the same procedure used for the preparation of 2-chloro-5-cyclopropylpyrimidine, this compound was obtained in 28 % yield as a solid, from 3,6-dichloropyridazine after a chromatography over silica gel (dichloromethane - ethanol from 100/0 to 98/2). ¹H (CDCl₃): 1.20 (m, 4H); 2.15 (m, 1H); 7.21 (d, 1H, J = 8.8); 7.35 (d, 1H, J = 8.8). ¹³C (CDCl₃): 10.9; 15.4; 127.0; 127.6; 154.2; 164.0.

**2-fluoro-5-ethylpyridine (intermediate 54A):** (see EP 2390254). Under an inert atmosphere, 5-bromo-2-fluoropyridine (5.35 g, 30.39 mmol), potassium carbonate (16.8 g, 121.59 mmol) were dissolved in dry dimethylformamide (75 mL, dried over 4Å molecular sieves). Oxygen was removed from this solution by a slow stream of argon and [1,1'-bis(diphenylphosphino)ferrocene] dichloropalladium complexed with dichloromethane (0.62 g, 1.52 mmol) was added, followed by a 1M solution of triethylborane (40 mL, 40.4 mmol) which was slowly added with a syringe. This darkening suspension was heated at 85 °C for 4 hours using an oil bath. The resulting black suspension was diluted in diethyl ether and water, the organic layer was washed with water 5 times, brine, dried over magnesium sulfate and cautiously concentrated to dryness to take into account the volatility of the reaction product. The residue was purified by a chromatography over silica gel (cyclohexane-dichloromethane from 2/3 to 1/4) to yield the 5-ethyl derivative as a volatile oil (1.52 g, 40 %). ¹H (CDCl₃): 1.25 (t, 3H, J = 7.6); 2.65 (q, 2H, J = 7.6); 6.84 (dd, 1H, J = 3.0 and 8.4); 7.61 (m, 1H); 8.03 (m, 1H). ¹³C (CDCl₃): 15.3; 25.1; 108.9 (37 Hz); 136.7 (4 Hz); 140.5 (8 Hz); 145.6 (14 Hz); 162.2 (236 Hz).

**2,3-difluoro-5-ethylpyridine (intermediate 54B):** By using the protocol described above for the synthesis 2-fluoro-5-ethylpyridine (intermediate 54A) this compound was obtained in 17 % yield, from 5-bromo-2,3-difluoropyridine, as a volatile oil after a chromatography over silica gel (cyclohexane - dichloromethane from 1/1 to 1/4). ¹H (CDCl₃): 1.27 (t, 3H, J = 7.6); 2.68 (q, 2H, J = 7.6); 7.40 (m, 1H); 7.80 (m, 1H). ¹³C (CDCl₃): 15.1; 25.0; 125.9 (3 and 14 Hz); 138.9 (4 Hz); 140.3 (5 and 12 Hz); 145.2 (28 and 260 Hz); 150.4 (14 and 235 Hz).

**2-(6-fluoropyridin-3-yl)propan-2-ol (intermediate 55):** Under an inert atmosphere, 5-bromo-2-fluoropyridine (3.12 g, 17.7 mmol) was dissolved in dry ether (100 mL) and the solution cooled to -78)C. Butyl lithium (9.3 mL, 18.6 mmol, 2N in cyclohexane) was added and the resulting precipitate was stirred at -78 °C for 15 mn. Acetone (0.4 mL, 90 mmol, dried over 4A molecular sieves) was added and the reaction was allowed to warm back to room temperature for 30 min. This was diluted with a saturated solution of ammonium chloride and extracted with ethyl acetate. The organic layer was washed with saturated solution of ammonium chloride, brine, dried over magnesium sulfate and concentrated to dryness. The residue was further purified by a chromatography over silica gel (dichloromethane - ethanol 98.5/1.5) to yield the fluropyridine as an oil (0.57 g, 20 %). ¹H (CDCl₃, slight difference with the reported one; see WO 2005028444): 1.63 (s, 6H); 1.89 (s, 1H); 6.91 (dd, 1H, J = 2.8 and 8.4 Hz); 7.94 (m, 1H); 8.33 (m, 1H). ¹³C (CDCl₃): 31.8; 71.1; 108.8 (37 Hz); 138.0 (8 Hz); 142.0 (4 Hz); 144.0 (14 Hz); 162.6 (238 Hz).

**2-bromo-5-(1,1-difluoroethyl)pyridine (intermediate 56):** In a closed Teflon flask, 1-(6-bromopyridin-3-yl)ethanone (0.95 g, 4.75 mmol) and bis(2-methoxyethyl)aminosulfur trifluoride *(*J. Org. Chem. 1999, 64, 7048) (3.6 g, 11.4 mmol) were stirred for seven days (as only a 78 % conversion was monitored by ¹HNMR, a longer reaction time could provide a better yield). This was diluted in water, solid calcium chloride was added and the solution was extracted with ethyl acetate. The organic layer was washed with water, brine, dried over magnesium sulfate and concentrated to dryness (not for too long, compound 15g is volatile). The residue was purified by a chromatography over silica gel (cyclohexane -dichloromethane 1/1) to yield the difluorinated pyridine as a colorless and volatile oil (0.57 g, 54 %). ¹H (CDCl₃): 1.96 (t, 3H, J = 18 Hz); 7.57 (d, 1H, J = 8.3); 7.69 (dd, 1H, J = 2.3 and 8.3); 8.20 (m, 1H). ¹³C (CDCl₃): 25.7 (29 Hz); 120.5 (240 Hz); 128.0; 133.2 (27 Hz); 135.0 (5 Hz); 143.7 (2 Hz); 146.9 (6 Hz). HRMS: Calc. for C₇H₆BrF₂N + H: 221.9730; Found: 221.9667.

**5-ethyl-2-(methylthio)pyrimidin-4-ol (intermediate 57**)**:** First step: preparation of methyl 2-((dimethylamino)methylene)butanoate (US 5446192). Methylbutyrate (3 g, 0.029 mol) and 1-methoxy-N,N,N',N'-tetramethylmethanediamine (2.59 g, 0.0196 mol) were diluted in dry dimethylformamide (8 mL, dried over 4A molecular sieve). This was heated in a microwave oven at 150 °C for 3 hours. Concentration to dryness gave the expected aminoacrylate (2.2 g) still containing a small proportion of dimethylformamide and this was used directly in the next step. ¹H (CDCl₃): 1.05 (t, 3H, J = 7.3); 2.42 (q, 2H, J = 7.3); 3.02 (s, 6H); 3.67 (s, 3H); 7.29 (s, 1H). ¹³C (CDCl₃): 16.5; 18.3; 42.9; 50.8; 99.3; 148.5; 171.2. Second step: preparation of 5-ethyl-2-mercaptopyrimidin-4-ol. Under an inert atmosphere, the crude acrylate described above (2.2 g), and thiourea (4.26 g, 0.056 mol) were dissolved in methanol (40 mL, dried over dried over 4A molecular sieve). A 25 % solution of sodium methanolate in methanol (13.3 mL, 0.056 mol) was added and this was heated at reflux for 17 hours. 2-(4-benzyl-3-isoThe methanol was removed under vacuum, the residue was dispersed in water and ethylacetate, made acid with acetic acid, the organic phase was washed with water, brine and and dried over magnesium sulfate and concentrated to dryness to yield the mercaptopyridine (0.42 g). ¹H NMR (DMSO-*d6*): 1.01 (t, 3H, J = 7.3); 2.21 (q, 2H, J = 7.3); 7.21 (s, 1H); 12.13 (s, 1H); 12.34 (s, 1H). ¹³C NMR (DMSO-*d6*): ¹³C NMR (DMSO-*d6*): 13.1; 19.9; 119.9; 137.7; 161.9; 175.1. Third step: preparation of 5-ethyl-2-(methylthio)pyrimidin-4-ol. The mercaptopyridine (0.4 g, 2.56 mmol) described above was dissolved in water (5 mL) containing sodium hydroxide (0.2 g, 5.12 mmol). Methyliodide (0.4 g, 2.8 mmol) dissolved in tetrahydrofuran (1 mL) was added and the solution was stirred for one hour at room temperature. This was diluted in water made acid with acetic acid, the resulting precipitate was filtered, washed with water and dried under vacuum to yield 5-ethyl-2-(methylthio)pyrimidin-4-ol (0.26 g, 7.8 % from methyl 2-((dimethylamino)methylene)butanoate). ¹H NMR (DMSO-*d6*): 1.06 (t, 3H, J = 7.4); 2.29 (q, 2H, J = 7.4); 2.45 (s, 3H); 7.69 (s, 1H); 12.61 (s, 1H). ¹³C NMR (DMSO-*d6*): ¹³C NMR (DMSO-*d6*): 12.7; 12.8; 19.9; 124.2; 149.3; 159.5; 162.5.

**2-(methylthio)-5-propylpyrimidin-4-ol (intermediate 57A):** First step: preparation of methyl 2-((dimethylamino)methylene)pentanoate (US 5446192). Prepared from methylvalerate and 1-methoxy-N,N,N',N'-tetramethylmethanediamine in DMF as described above ¹H (CDCl₃): 0.91 (t, 3H, J = 7.4); 1.42 (m, 2H); 2.34 (m, 2H); 3.00 (s, 6H); 3.65 (s, 3H); 7.29 (s, 1H). ¹³C (CDCl₃): 13.9; 25.1; 27.1; 42.9; 50.8; 97.9; 148.7; 171.4.
Second step: preparation of 2-mercapto-5-propylpyrimidin-4-ol. By using the procedure described above starting with methyl 2-((dimethylamino)methylene)pentanoate, this mercaptopyridine was obtained as a solid. ¹H NMR (DMSO-*d6*): 0.85 (t, 3H, J = 7.4); 1.43 (m, 2H); 2.17 (m, 2H); 7.23 (s, 1H); 12.12 (s, 1H); 12.33 (s, 1H). ¹³C NMR (DMSO-*d6*): 13.9; 21.3; 28.5; 118;1. 138.4; 162.0; 175.1. Third step: preparation of 2-(methylthio)-5-propylpyrimidin-4-ol: by using the procedure described above, starting with 2-mercapto-5-propylpyrimidin-4-ol, this compound was obtained as a solid in a 34 % overall yield from methyl 2-((dimethylamino)methylene)butanoate. ¹H NMR (DMSO-*d6*): 0.86 (t, 3H, J = 7.3); 1.48 (m, 2H); 2.25 (m, 2H); 2.45 (s, 3H); 7.70 (s, 1H); 12.60 (s, 1H). ¹³C NMR (DMSO-*d6*): 13.1; 14.1; 22.5; 29.1; 123.2; 150.9; 159.8; 162.9. HRMS: Calc. for C₈H₁₂N₂OS + H: 185.0749; Found: 185.0665.

**6-ethyl-3-(methylthio)-1,2,4-triazin-5-ol (intermediate 58):** In water (30 mL) sodium hydroxide (1.25 g, 0.031 mol) was dissolved and after cooling, 6-ethyl-3-mercapto-1,2,4-triazin-5-ol (WO 2007017114) (2.46 g, 0.015 mol) was dissolved. Methyl iodide (1.07 mL, 0.017 mmol) diluted in and tetrahydrofuran (2 mL) was then slowly added. The solution was stirred at room temperature for 4 hours, this was diluted with water, made acid with acetic acid and extracted with ethyl acetate. The organic layer was washed with brine, dried over magnesium sulfate and concentrated to dryness to yield pure thioether as a white powder (2.28 g, 85 %). ¹H (DMSO-*d6*): 1.07 (t, 3H, J = 7.6); 2.50 (q, 2H, J = 7.6); 12.99 (s, H); 13.28 (s, 1H). ¹³C (DMSO-*d₆*): 10.5; 12.5; 23.6; 153.6; 160.7; 164.4.

**4-benzyl-5-methyl-1-(5-methylpyridin-2-yl)-1H-pyrazol-3-ol (intermediate 59):** In a 100 mL tube fitted with a Teflon-coated screw cap, 2-(4-benzyl-3-ethoxy-5-methyl-1H-pyrazol-1-yl)-5-methylpyridine (1.54 g, 5 mmol) and 33 % hydrobromic acid in acetic acid (5 mL) were heated at 140 °C for 4 hours. The resulting suspension was concentrated to dryness, diluted in ethanol and made basic with 2 N ethanolic ammonia. This was concentrated to dryness and the residue was purified by a chromatography over silica gel (dichloromethane/ethanol from 99/1) to yield the deprotected pyrazol-3-ol (0.96 g, 68 %) as a white powder. ¹H NMR (DMSO-*d6*): 2.29 (s, 3H); 2.50 (s, 3H); 3.66 (s, 2H); 7.22 (m, 5H); 7.53 (m, 1H); 7.69 (m, 1H); 8.21 (m, 1H); 10.10 (s(br), 1H). ¹³C NMR (DMSO-*d6*): 13.4; 17.7; 27.7; 106.7; 114.5; 126.1; 128.4; 128.7; 129.5; 138.5; 139.6; 141.5; 147.5; 151.8; 161.0. HRMS: Calc. for C₁₇H₁₇N₃O + H: 280.1450; Found: 280.1407.

**(3-isopropoxy-1H-pyrazol-4-yl)(phenyl)methanone (intermediate 60):** Under an argon atmosphere, 4-iodo-3-isopropoxy-1H-pyrazole (2.73 g, 0.0108 mol) was dissolved in dry tetrahydrofuran (50 mL, purchased). This was cooled to -78 °C with a dry ice bath and 2M butyl lithium in cyclohexane (12.4 mL, 0.0249 mol) was added. This was stirred one hour at -78 °C and benzoylchloride (2.8 mL, 0.0108 mol) was added to the suspension. The solution was stirred 10 minutes at -78 °C and stirred for 20 minutes while allowing it to warm to room temperature. This was diluted in water, extracted with ethyl acetate; the organic layer was washed with brine, dried over magnesium sulfate and concentrated to dryness. The residue was dissolved in a 1/1 v/v mixture water and ethanol (80 mL), sodium hydroxide (5 g) was added and the solution stirred overnight at room temperature. This was diluted in water, extracted with ethyl acetate; the organic layer was washed with brine, dried over magnesium sulfate and concentrated to dryness. The resulting residue was purified by two consecutive chromatographies, the first over silica gel (dichloromethane - ethanol from 98.5/1.5 to 97.5/2.5), the second also over silica gel (cyclohexane - ethyl acetate from 9/1 to 1/3) to yield a mixture of 3-isopropoxy-1H-pyrazole and the target ketone (0.55 g). Their separation was achieved by bromination of the mixture using N-bromosuccinimide (0.9 g) in acetonitrile (20 mL) an extraction and a purification by chromatography over silica gel (dichloromethane-ethanol from 98/2) to yield the pure ketone as a solid (0.13 g, 5 %). ¹H NMR (CDCl₃): 1.34 (t, 6H, *J=* 6.2); 4.97 (sept, 1H, J= 6.2); 7.43 (m, 2H); 7.53 (m, 1H); 7.79 (m, 2H); 7.82 (s, 1H); 10.6 (s(l), 1H). ¹³C NMR (CDCl₃): 21.9; 72.8; 108.3; 128.0; 129.0; 131.9; 135.1; 139.4; 161.5; 189.4. HRMS: Calc. for C₁₃H₁₄N₂O₂ + Na: 253.0953; Found: 253.0980.

**1-cyclopropyl-2-phenoxyethanone (intermediate 61):** Under a moisture-protected atmosphere, 2-bromo-1-cyclopropylethanone (27.15 g, 0.16 mol, see US 20060241125 for a preparation), phenol (16.4 g, 0.175 mol) and potassium carbonate (34.5 g, 0.25 mol) were heated at 85 °C in dry dimethylformamide (200 mL, dried over 4A molecular sieves) overnight. The dimethylformamide was removed under vacuum and the residue diluted in ethyl acetate. This was washed with a 1N solution of sodium hydroxide, water, brine and dried over magnesium sulfate before concentration to dryness to yield the ether as an oil (24.99 g, 85 %). ¹H (CDCl₃): 0.99 (m, 2H); 1.17 (m, 2H); 2.33 (m, 1H); 4.70 (s, 2H); 6.93 (m, 2H); 7.02 (m, 1H); 7.30 (m, 2H). ¹³C (CDCl₃): 11.8; 17.1; 73.1; 114.6; 121.6; 129.6; 158.0; 207.5.

**3-ethoxy-4-bromo-5-methyl-1H-pyrazole (intermediate 62):** 3-ethoxy-5-methyl-IH-pyrazole (6.64 g, 52.63 mmol) was dissolved in dry acetonitrile (200 mL), N-bromosuccinimide (9.83 g, 55.26 mmol) was added and the solution was stirred at room temperature overnight. The acetonitrile was then removed under vacuum, this was dissolved in water and ethyl acetate and the organic layer was washed six times with water once with brine and dried over magnesium sulfate. Removal of the solvent under vacuum allowed the isolation of pure 3-ethoxy-4-bromo-5-methyl-1H-pyrazole as a white powder (9.83 g, 91 %). ¹H (CDCl₃): 1.42 (t, 3H, J = 7.0); 2.21 (s, 3H); 4.28 (q, 2H, J = 7.0); 9.40 (s(l), 1H). ¹³C (CDCl₃): 10.6; 14.8; 65.1; 79.7; 139.2; 160.2.

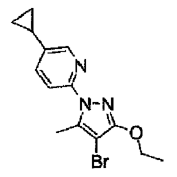

**2-(4-bromo-3-ethoxy-5-methyl-1H-pyrazol-1-yl)-5-cyclopropylpyridine (intermediate 63)** From 3-ethoxy-4-bromo-5-methyl-1H-pyrazole (intermediate 62), using the protocol described for the preparation of example 108, this compound was obtained in a 48 % yield as an oil. ¹H (CDCl₃): 0.72 (m, 2H); 1.04 (m, 2H); 1.46 (t, 3H, J = 7.2); 1.93 (m, 1H); 2.63 (s, 3H); 4.38 (q, 2H, J = 7.2); 7.40 (dd, 1H, J = 2.3 and 8.5); 7.62 (d, 1H, J = 8.5); 8.21 (d, 1H, J = 2.3). ¹³C (CDCl₃): 8.7; 12.6; 13.5; 14.7; 65.0; 84.4; 114.6; 135.2; 136.2; 140.0; 145.5; 151.4; 159.7. HRMS: Calc. for C₁₄H₁₆BrN₃O + H: 322.0555; Found: 322.0517.

### Biological assays

Few experiments were performed to elucidate the mechanism of antiviral action of this series of compounds. As depicted, the measles virus replication in cells was affected by the addition of 5-cyclopropyl-2-(4-(2,6-difluorophenoxy)-3-isopropoxy-5-methyl-1H-pyrazol-1-yl)-3-fluoropyridine (example 5) at concentration between 4 and 100 nM (Figure 1). The addition of uridine at 10 µg/mL (graphic A) restored the virus replication, whereas the addition of guanosine at 10 µg/mL did not (graphic B). A restored virus replication was also seen with the addition of orotic acid at 3 mM in shown in graphic C and the lack of effect of adding dihydroorotic acid at 3 mM is illustrated in graphic D.

As depicted in the *de novo* synthetic pathway of uridine below, these results hinted at the cellular dihydroorotate dehydrogenase (DHODH) as the biochemical target of this series of antiviral compounds.

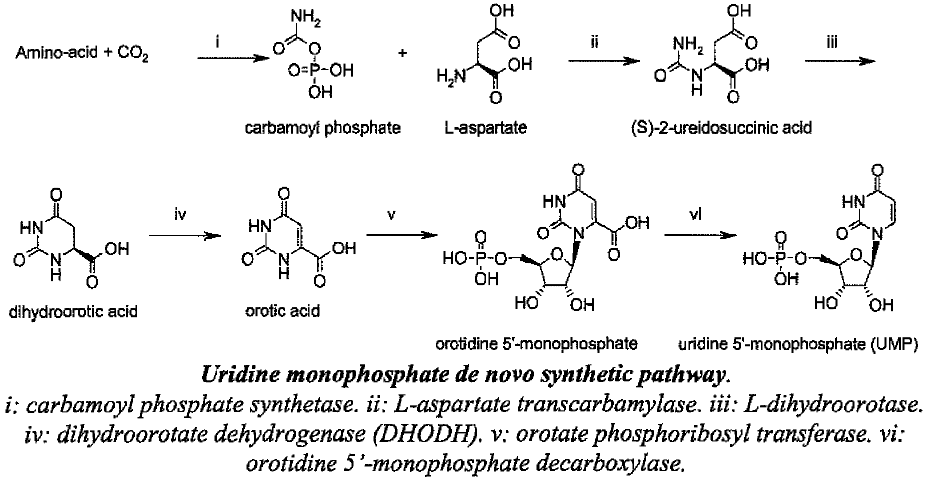

By using a metabolite analysis, the content in adenosine triphosphate (ATP), guanosine triphosphate (GTP), cytidine triphosphate (CTP) and uridine triphosphate (UTP) of HEK-293 cells treated with various concentration of example 5 could be determined. As shown in the table I below, a drastic reduction was observed in the cellular content of CTP and UTP both pyrimidine nucleotide even at the lowest dose whereas much less effect was seen in the cellular content of ATP and GTP, both purine nucleotides which biosyntheses are, contrary to CTP and UTP, not dependant on the enzymatic activity of DHODH.

**Table I**

| Table I. Cellular nucleotides content in the presence of example 5 at concentrations of 0.8, 4, 20 and 100 nM | | | | | |
|---|---|---|---|---|---|
| | DMSO | 0.8 | 4 | 20 | 100 |
| ATP | 100 ± 13 % | 65 ± 19 % | 60 ± 5 % | 111 ± 9 % | 93 ± 7 % |
| GTP | 100 ± 6 % | 64 ± 19 % | 60 ± 5 % | 128 ± 8 % | 100 ± 4 % |
| CTP | 100 ± 13 % | 15 ± 1 % | 3 ± 1 % | 2 ± 0 % | 4 ± 1 % |
| UTP | 100 ± 8 % | 23 ± 1 % | 14 ± 1 % | 3 ± 0 % | 6 ± 1 % |

Material and method for the metabolite analyses. HEK-293T cells were plated in 6-well plates (16106 cells per well). One day later, culture medium was supplemented with increasing doses of example 5 or DMSO alone. After an additional 24 hours of culture, cells were harvested, carefully counted and monitored for viability by trypan blue exclusion, and washed with ice-cold phosphate-buffered saline (PBS). All the extraction steps were performed on ice. Cellular pellets were deproteinized with an equal volume of 6% trichloroacetic acid (TCA), vortex-mixed for 20 seconds, ice-bathed for 10 min, and vortex-mixed again for 20 seconds. Acid cell extracts were centrifuged at 13,000 rpm for 10 min at 4uC. The resulting supernatants were supplemented with an equal volume of bi-distilled water, vortex-mixed for 60 seconds, and neutralized by the addition of 5M potassium carbonate. Cells extracts were subjected to an optimized SPE procedure as previously described *(*Purinergic Signal 2012, 8, 741). All fractions were injected into the HPLC system separately and the results pooled to calculate the total amount of nucleotides and nucleosides present in the original samples. HPLC analysis was performed with a Shimadzu HPLC system interfaced to the LabSolution software. Samples were injected onto an ABZ Supelco 5 mm (15064.6 mm) column (Sigma). The HPLC columns were kept at 40°C in a column oven. The mobile phase was delivered at a flow-rate of 1 ml/min during the analysis using a stepwise isocratic elution with a buffer containing 10 mM acetate ammonium adjusted to pH 6.9. Detection was done with the diode array detector (PDA). The LC Solution workstation chromatography manager was used to pilot the HPLC instrument and to process the data. The products were monitored spectrophotometrically at 254 nm and quantified by integration of the peak absorbance area, employing a calibration curve established with various known concentrations of nucleosides. Finally, a correction coefficient was applied to correct raw data for minor differences in the total number of cells determined in each culture condition.

The effect of 5-cyclopropyl-2-(4-(2,6-difluorophenoxy)-3-isopropoxy-5-methyl-1H-pyrazol-1-yl)-3-fluoropyridine (example 5) on the growth of the Jurkat cell line which has been demonstrated to be sensitive to DHODH inhibitors was also assessed (J. Med. Chem. 2001, 44, 1986 and Transplant Immunology 2010, 23, 180). As shown in Figure 2, example 5 strongly inhibited the Jurkat cells proliferation (IC₅₀ = 0.02 µM), and its level of inhibition compares favorably with brequinar (IC₅₀ = 0.2 µM) or teriflunomide (IC₅₀ close to 60 µM, data not shown).

Material and Methods for the inhibition of the proliferation of Jurkat cells. Jurkat cells were cultured in at 5.10+4 cells per well in flat-bottom 96-well culture dishes. Cells were maintained at 37°C and 5% CO₂ in RPMI (Gibco-Invitrogen) containing 10% fetal calf serum (FCS), pyruvate sodium, non-essential amino acids, penicillin, and streptomycin. Number of living cells was determined by quantification of adenosine triphosphate (ATP) in culture wells using the CellTiter-Glo Assay (Promega) following manufacturer's recommendations. This luciferase-based assay evaluates by ATP quantification the number of metabolically active cells in culture wells.

To further demonstrate that DHODH is the cellular target of our series of antiviral compounds, the production of the recombinant human enzyme using the reported procedure was undertaken (Arch. Biochem. Biophys. 1971, 146, 256-270; Methods Enzymol. 1978, 51, 63-69; Methods Enzymol. 1978, 51, 58-63.) and the inhibition of this enzyme by representative example of this series of antiviral compounds was measured. As illustrated in the table II below, a correlation between the inhibition of measles virus replication and the inhibition of recombinant DHODH is apparent.

**Table II**

| **Comparison of measles virus and human DHODH inhibition** | | | | |
|---|---|---|---|---|
| | **Name** | **Structure** | **IC₅₀ on the measles virus (nM)** | **IC₅₀ on recombinant DHODH (nM)** |
| Example 122 | 5-cyclopropyl-2-(3-phenyl-1H-pyrazol-1-yl)pyrimidine | 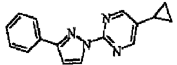 | 3800 | 22800 ± 2800 |
| Example 8 | 2-(4-(2,6-difluorophenoxy)-3-isopropoxy-5-methyl-1H-pyrazol-1-yl)-5-ethylpyrimidine | 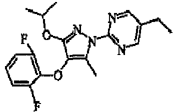 | 0.66 | 13 ± 1 |
| Example 67 | 5-cyclopropyl-2-(4-(2-fluorophenoxy)-3-isopropoxy-1H-pyrazol-1-yl)pyrimidine | 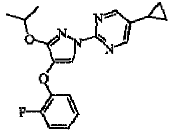 | 15 | 48 ± 7 |
| Example 20 | 2-(4-benzyl-3-isopropoxy-5-methyl-1H-pyrazol-1-yl)-5-cyclopropylpyridine | 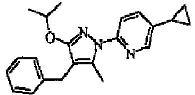 | 46 | 130 ± 5 |
| Example 10 | 5-cyclopropyl-2-(4-(2-fluorobenzyl)-3-isopropoxy-5-methyl-1H-pyrazol-1-yl)pyrimidine | 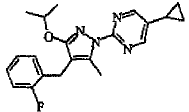 | 1.1 | 13 ± 2 |
| Example 176 | (1-(5-cyclopropylpyrimidin-2-yl)-3-isopropoxy-5-methyl-1H-pyrazol-4-yl)(phenyl)methanol | 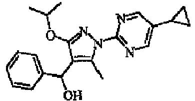 | 1.4 | 16 ± 3 |
| Example 81 | 2-(4-(2-bromophenoxy)-3-ethoxy-5-methyl-1H-pyrazol-1-yl)-5-ethylpyrimidine | 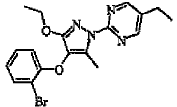 | 124 | 1150 ± 170 |
| Example 5 | 5-cyclopropyl-2-(4-(2,6-difluorophenoxy)-3-isopropoxy-5-methyl-1H-pyrazol-1-yl)-3-fluoropyridine | 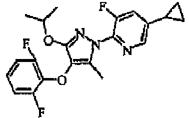 | 2.7 | 25 ± 5 |
| | brequinar | 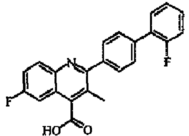 | 40 | 4 ± 2 |

Material and Methods for the Recombinant DHODH production and DHODH assay. I.M.A.G.E. full length cDNA clone IRATp970D0185D was purchased from Biovalle S.A. DNA polymerases, restriction enzymes, T₄ DNA ligase were from New England Biolabs. Oligonucleotides were purchased from Eurofins MWG Operon, BD TALON metal affinity resin was obtained from BD Biosciences Clontech and Hepes, Triton x-100, L-dihydroorotic acid (L-DHO), riboflavin 5'-monophosphate sodium salt (FMN), coenzyme Q1 (CoQ1) and 2,6-dichloroindophenol sodium salt (DCIP) from Sigma-Aldrich. *Plasmid construction and growth conditions* The *E. coli* DH5α strain (Life Technologies) was used for DNA purification. The cDNA encoding an N-terminally truncated (first 29 amino acids) human DHODH was amplified by PCR from full length cDNA clone IRATp970D0185D as template using primers 1 (GGGAATTCCATatggccacgggagatgagcgtttctat) and 2 (CCCCAAGCTTtcacctccgatgatctgctccaatgg), which introduce *Nde*I and *Hind*III restriction sites, respectively. The PCR product was inserted into the pCR_{™} II-TOPO vector (Life Technologies). This intermediate plasmid was digested by *Nde*I and *Hind*III restriction enzymes, and the purified 1104-bp insert was ligated into *Nde*I/*Hind*III linearized pET28a plasmid (Novagen, Inc.). The resulting plasmid pHL200-1 was sequenced to verify its integrity and introduced into the *E. coli* strain BL21(DE3)/pDIA17⁵⁷ to overproduce a N-terminal His-tagged human DHODH. *Purification of the recombinant human DHODH* Bacteria were grown at 25°C in 2YT medium supplemented with 0.1 mM flavin mononucleotide, 35 µg/ml kanamycin and 30 µg/ml chloramphenicol. Production of recombinant enzyme was induced with isopropyl-1-β-D-thiogalactopyranoside (5 µM final concentration) when cultures reached an absorbance of 0.5 at 600 nm. Cells were harvested after an additional 20 h of growth at 25°C. A cell pellet from 650 ml of culture was resuspended in 50 mL of lysis buffer A (50 mM HEPES pH 7.5, 300 mM NaCl, 20% glycerol) supplemented with Complete Protease Inhibitor Cocktail EDTA-free (Roche) and disrupted by sonication. Before centrifugation at 10,000 g for 30 minutes at 4°C, Triton X-100 was added to a final concentration of 1% into the lysate. The supernatant was added to 3 ml BD TALON resin (Clontech) previously equilibrated with buffer A and gently stirred at room temperature on a platform shaker. The additional steps are described by BD Biosciences in the Batch/gravity-Flow column purification section. The recombinant protein began to elute with 30 mM imidazole, as indicated by the appearance of a yellow color in the eluate. At this point, it was rapidly eluted with 160 mM imidazole. Fractions were analyzed by SDS-PAGE for purity, and appropriate fractions were pooled and concentrated using a stirred cell system (Pall Life Sciences). The His-tag was not removed for further studies. *Enzymatic assay* DHODH activity was determined at 25°C following the reduction of DCIP at 600 nm (ε = 18,800 M⁻¹ cm⁻¹) on a spectrophotometer. The inhibitory potency of the compounds at concentrations between 5 nM and 200 µM was evaluated by measuring the initial velocity of the DHODH-catalyzed reaction. The reaction medium used for this contained 50 mM Tris-HCl pH 8.0, 0.1% Triton X-100, 0.1 mM L-DHO, 0.025 mM CoQ₁ and 0.06 mM DCIP. The reaction was started by addition of the enzyme. Brequinar was assessed as a reference.

### Screening to identify broad-spectrum antivirals.

The screening pipeline used in the present experiment to select for broad-spectrum antivirals was recently described (Lucas-Hourani, M.; Munier-Lehmann, H.; Helynck, O.; Komarova, A.; Despres, P.; Tangy, F.; Vidalain, P. O. High-throughput screening for broad-spectrum chemical inhibitors of RNA viruses. J. Visualized Exp. 2014, in print ; PLOS Pathogens 2013, 9, e1003678). Briefly, stock solutions of compounds in DMSO were spiked in white, flat bottom, bar-coded tissue culture 96-wells plates. Then, culture wells were loaded with human HEK-293T cells infected with a recombinant strain of measles virus expressing firefly luciferase from an additional transcription unit (rMV2/Luc). Cells were grown for 24 hours at 37°C and 5% CO₂ in Dulbecco's modified Eagle's medium (DMEM) with stabilized L-glutamine and supplemented with 10% fetal calf serum (FCS), streptomycin (100 µg/ml) and penicillin (100 IU/ml). Luciferase activity was determined by adding 50 µl of luciferase substrate (Bright-GLO, Promega) directly to culture wells. After 6 min of incubation at room temperature, bioluminescence level was measured with a luminometer (Safire 2, TECAN). In parallel, a second set of culture plates containing test compounds were loaded with non-infected HEK-293T cells to determine cellular toxicity. After 24 hours of culture, cellular viability was determined by adding 50 µl of CellTiter-GLO (Promega), a luciferase-based reagent that evaluates, by ATP quantification, the number of metabolically active cells in culture. Compounds that inhibited rMV2/Luc replication by more than 75% without significant reduction of cellular viability were selected for further characterization. Finally, selected hit compounds were retested in dose-response experiments for their capacity to block the replication of both measles virus using rMV2/Luc, and tested as well for the inhibition of chikungunya virus. For this later experiment, chikungunya virus replication was assessed with the recombinant strain CHIKV/Ren that expresses renilla luciferase as a reporter.

The following table III provides the compounds concentration (in nmol/L) inhibiting 50% of the measles virus replication . The concentration inhibiting 50 % of the measles virus replication is ranked according to the following convention:
++++ : 0.5 nM < IC₅₀ < 50 nM
+++ : 50 nM < IC₅₀ < 500 nM
++ : 500 nM < IC₅₀ < 5000 nM
+ : 5000 nM < IC₅₀

**Table III**

| | structure | chemical name | MIC₅₀ |
|---|---|---|---|
| example 1 | 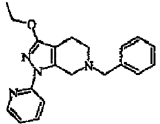 | 6-benzyl-3-ethoxy-1-(pyridin-2-yl)-4,5,6,7-tetrahydro-1H-pyrazolo[3,4-c]pyridine | + |
| example 2 | 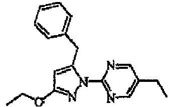 | 2-(5-benzyl-3-ethoxy-1H-pyrazol-1-yl)-5-ethylpyrimidine | + |
| example 3 | 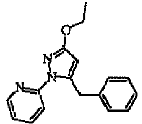 | 2-(5-benzyl-3-ethoxy-1H-pyrazol-1-yl)pyridine | + |
| example 4 | 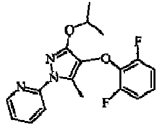 | 2-(4-(2,6-difluorophenoxy)-3-isopropoxy-5-methyl-1H-pyrazol-1-yl)pyridine | + |
| example 5 | 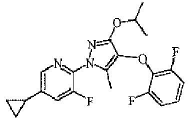 | 5-cyclopropyl-2-(4-(2,6-difluorophenoxy)-3-isopropoxy-5-methyl-1H-pyrazol-1-yl)-3-fluoropyridine | ++++ |
| example 5A | 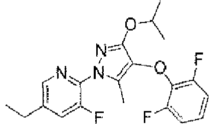 | 2-(4-(2,6-difluorophenoxy)-3-isopropoxy-5-methyl-1H-pyrazol-1-yl)-5-ethyl-3-fluoropyridine | +++ |
| example 6 | 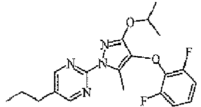 | 2-(4-(2,6-difluorophenoxy)-3-isopropoxy-5-methyl-1H-pyrazol-1-yl)-5-propylpyrimidine | ++++ |
| example 7 | 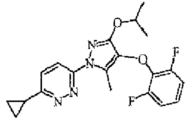 | 3-cyclopropyl-6-(4-(2,6-difluorophenoxy)-3-isopropoxy-5-methyl-1H-pyrazol-1-yl)pyridazine | ++++ |
| example 8 | 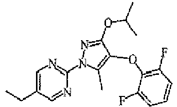 | 2-(4-(2,6-difluorophenoxy)-3-isopropoxy-5-methyl-1H-pyrazol-1-yl)-5-ethylpyrimidine | ++++ |
| example 9 | 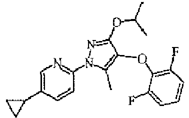 | 5-cyclopropyl-2-(4-(2,6-difluorophenoxy)-3-isopropoxy-5-methyl-1H-pyrazol-1-yl)pyridine | ++++ |
| example 9A | 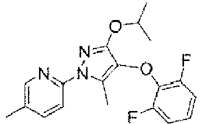 | 2-(4-(2,6-difluorophenoxy)-3-isopropoxy-5-methyl-1H-pyrazol-1-yl)-5-methylpyridine | ++++ |
| example 9B | 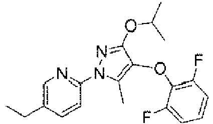 | 2-(4-(2,6-difluorophenoxy)-3-isopropoxy-5-methyl 1H-pyrazol-1-yl)-5-ethylpyridine | +++ |
| example 10 | 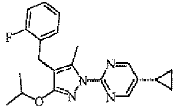 | 5-cyclopropyl-2-(4-(2-fluorobenzyl)-3-isopropoxy-5-methyl-1H-pyrazol-1-yl)pyrimidine | ++++ |
| example 11 | 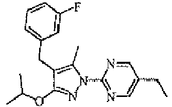 | 5-ethyl-2-(4-(3-fluorobenzyl)-3-isopropoxy-5-methyl-1H-pyrazol-1-yl)pyrimidine | ++++ |
| example 12 | 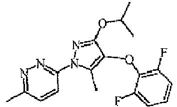 | 3-(4-(2,6-difluorophenoxy)-3-isopropoxy-5-methyl-1H-pyrazol-1-yl)-6-methylpyridazine | ++++ |
| example 13 | 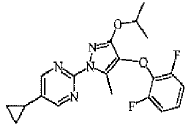 | 5-cyclopropyl-2-(4-(2,6-difluorophenoxy)-3-isopropoxy-5-methyl-1H-pyrazol-1-yl)pyrimidine | ++++ |
| example 14 | 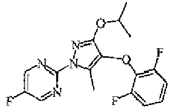 | 2-(4-(2,6-difluorophenoxy)-3-isopropoxy-5-methy)-1H-pyrazol-1-yl)-5-fluoropyrimidine | +++ |
| example 15 | 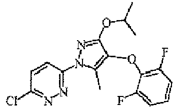 | 3-chloro-6-(4-(2,6-difluorophenoxy)-3-isopropoxy-5-methyl-1H-pyrazol-1-yl)pyridazine | +++ |
| example 16 | 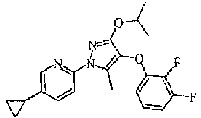 | 5-cyclopropyl-2-(4-(2,3-difluorophenoxy)-3-isopropoxy-5-methyl-1H-pyrazol-1-yl)pyridine | ++++ |
| example 17 | 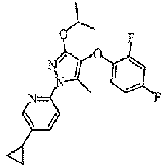 | 5-cyclopropyl-2-(4-(2,4-difluorophenoxy)-3-isopropoxy-5-methyl-1H-pyrazol-1-yl)pyridine | +++ |
| example 18 | 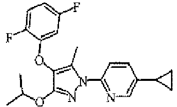 | 5-cyclopropyl-2-(4-(2,5-difluorophenoxy)-3-isopropoxy-5-methyl-1H-pyrazol-1-yl)pyridine | ++++ |
| example 19 | 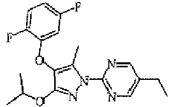 | 2-(4-(2,5-difluorophenoxy)-3-isopropoxy-5-methyl-1H-pyrazol-1-yl)-5-ethylpyrimidine | ++++ |
| example 20 | 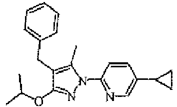 | 2-(4-benzyl-3-isopropoxy-5-methyl-1H-pyrazol-1-yl)-5 -cyclopropylpyridine | ++++ |
| example 21 | 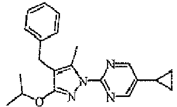 | 2-(4-benzyl-3-isopropoxy-5-methyl-1H-pyrazol-1-yl)-5-cyclopropylpyrimidine | ++++ |
| example 22 | 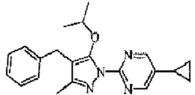 | 2-(4-benzyl-5-isopropoxy-3-methyl-1H-pyrazol-1-yl)-5-cyclopropylpyrimidine | +++ |
| example 23 | 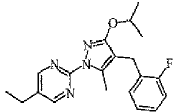 | 5-ethyl-2-(4-(2-fluorobenzyl)-3-isopropoxy-5-methyl-1H-pyrazol-1-yl)pyrimidine | ++++ |
| example 24 | 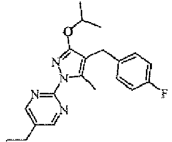 | 5-ethyl-2-(4-(4-fluorobenzyl)-3-isopropoxy-5-methyl-1H-pyrazol-1-yl)pyrimidine | ++++ |
| example 25 | 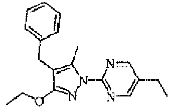 | 2-(4-benzyl-3-ethoxy-5-methyl-1H-pyrazol-1-yl)-5-ethylpyrimidine | ++++ |
| example 26 | 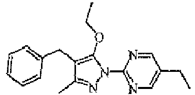 | 2-(4-benzyl-5-ethoxy-3-methyl-1H-pyrazol-1-yl)-5-ethylpyrimidine | ++ |
| example 27 | 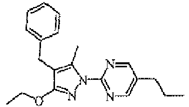 | 2-(4-benzyl-3-ethoxy-5-methyl-1H-pyrazol-1-yl)-5-propylpyrimidine | ++++ |
| example 28 | 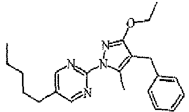 | 2-(4-benzyl-3-ethoxy-5-methyl-1H-pyrazol-1-yl)-5-pentylpyrimidine | + |
| example 29 | 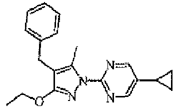 | 2-(4-benzyl-3-ethoxy-5-methyl-1H-pyrazol-1-yl)-5-cyclopropylpyrimidine | ++++ |
| example 30 | 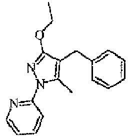 | 2-(4-benzyl-3-ethoxy-5-methyl-1H-pyrazol-1-yl)pyridine | + |
| example 31 | 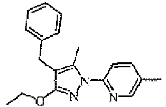 | 2-(4-benzyl-3-ethoxy-5-methyl-1H-pyrazol-1-yl)-5-methylpyridine | ++ |
| example 32 | 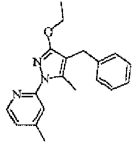 | 2-(4-benzyl-3-ethoxy-5-methyl-1H-pyrazol-1-yl)-4-methylpyridine | ++ |
| example 33 | 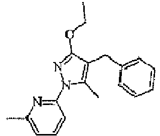 | 2-(4-benzyl-3-ethoxy-5-methyl-1H-pyrazol-1-yl)-6-methylpyridine | + |
| example 34 | 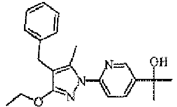 | 2-(6-(4-benzyl-3-ethoxy-5-methyl-1H-pyrazol-1-yl)pyridin-3-yl)propan-2-ol | + |
| example 35 | 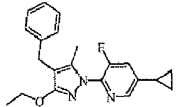 | 2-(4-benzyl-3-ethoxy-5-methyl-1H-pyrazol-1-yl)-5-cyclopropyl-3-fluoropyridine | +++ |
| example 36 | 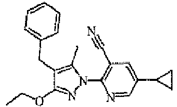 | 2-(4-benzyl-3-ethoxy-5-methyl-1H-pyrazol-1-yl)-5-cyclopropylnicotinonitrile | +++ |
| example 37 | 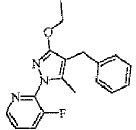 | 2-(4-benzyl-3-ethoxy-5-methyl-1H-pyrazol-yl)-3-fluoropyridine | + |
| example 38 | 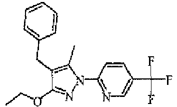 | 2-(4-benzyl-3-ethoxy-5-methyl-1H-pyrazol-1-yl)-5-(trifluoromethyl)pyridine | ++ |
| example 39 | 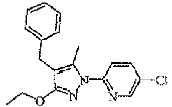 | 2-(4-benzyl-3-ethoxy-5-methyl-1H-pyrazol-1-yl)-5-chloropyridine | ++ |
| example 40 | 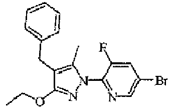 | 2-(4-benzyl-3-ethoxy-5-methyl-1H-pyrazol-1-yl)-5-bromo-3-fluoropyridine | ++ |
| example 41 | 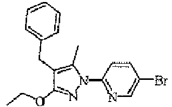 | 2-(4-benzyl-3-ethoxy-5-methyl-1H-pyrazol-1-yl)-5-bromopyridine | + |
| example 42 | 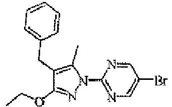 | 2-(4-benzyl-3-ethoxy-5-methyl-1H-pyrazol-1-yl)-5-bromopyrimidine | +++ |
| example 43 | 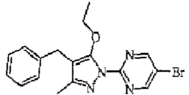 | 2-(4-benzyl-5-ethoxy-3-methyl-1H-pyrazol-1-yl)-5-bromopyrimidine | + |
| example 44 | 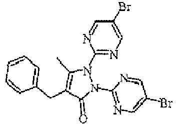 | 4-benzyl-1,2-bis(5-bromopyrimidin-2-yl)-5-methyl-1H-pyrazol-3(2H)-one | ++ |
| example 45 | 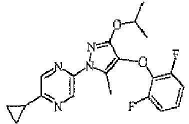 | 2-cyclopropyl-5-(4-(2,6-difluorophenoxy)-3-isopropoxy-5-methyl-1H-pyrazol-1-yl)pyrazine | ++++ |
| example 46 | 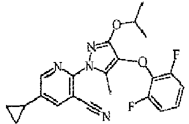 | 5-cyclopropyl-2-(4-(2,6-difluorophenoxy)-3-isopropoxy-5-methyl-1H-pyrazol-1-yl)nicotinonitrile | ++++ |
| example 47 | 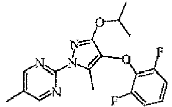 | 2-(4-(2,6-difluorophenoxy)-3-isopropoxy-5-methyl-1H-pyrazol-1-yl)-5-methylpyrimidine | ++++ |
| example 48 | 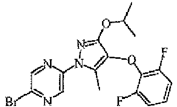 | 2-bromo-5-(4-(2,6-difluorophenoxy)-3-isopropoxy-5-methyl-1H-pyrazol-1-yl)pyrazine | ++ |
| example 49 | 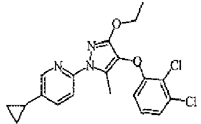 | 5-cyclopropyl-2-(4-(2,3-dichlorophenoxy)-3-ethoxy-5-methyl-1H-pyrazol-1-yl)pyridine | ++++ |
| example 50 | 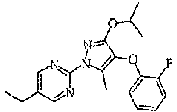 | 5-ethyl-2-(4-(2-fluorophenoxy)-3-isopropoxy-5-methyl-1H-pyrazol-1-yl)pyrimidine | ++++ |
| example 51 | 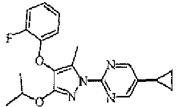 | 5-cyclopropyl-2-(4-(2-fluorophenoxy)-3-isopropoxy-5-methyl-1H-pyrazol-1-yl)pyrimidine | ++++ |
| example 52 | 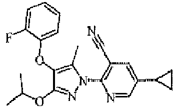 | 5-cyclopropyl-2-(4-(2-fluorophenoxy)-3-isopropoxy-5-methyl-1H-pyrazol-1-yl)nicotinonitrile | +++ |
| example 53 | 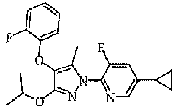 | 5-cyclopropyl-3-fluoro-2-(4-(2-fluorophenoxy)-3-isopropoxy-5-methyl-1H-pyrazol-1-yl)pyridine | +++ |
| example 54 | 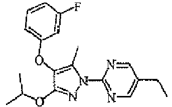 | 5-ethyl-2-(4-(3-fluorophenoxy)-3-isopropoxy-5-methyl-1H-pyrazol-1-yl)pyrimidine | +++ |
| example 55 | 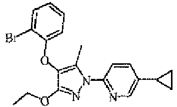 | 2-(4-(2-bromophenoxy)-3-ethoxy-5-methyl-1H-pyrazol-1-yl)-5-cyclopropylpyridine | ++++ |
| example 5 6 | 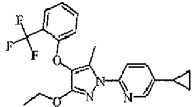 | 5-cyclopropyl-2-(3-ethoxy-5-methyl-4-(2-(trifluoromethyl)phenoxy)-1H-pyrazol-1-yl)pyridine | +++ |
| example 57 | 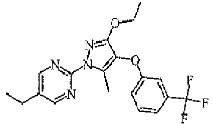 | 2-(3-ethoxy-5-methyl-4-(3-(trifluoromethyl)phenoxy)-1H-pyrazol-1-yl)-5-ethylpyrimidine | +++ |
| example 58 | 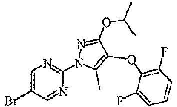 | 5-bromo-2-(4-(2,6-difluorophenoxy)-3-isopropoxy-5-methyl-1H-pyrazol-1-yl)pyrimidine | ++++ |
| example 59 | 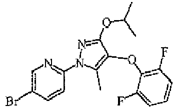 | 5-bromo-2-(4-(2,6-difluorophenoxy)-3-isopropoxy-5-methyl-1H-pyrazol-1-yl)pyridine | +++ |
| example 60 | 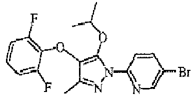 | 5-bromo-2-(4-(2,6-difluorophenoxy)-5-isopropoxy-3-methyl-1H-pyrazol-1-yl)pyridine | + |
| example 61 | 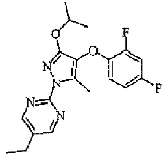 | 2-(4-(2,4-difluorophenoxy)-3-isopropoxy-5-methyl-1H-pyrazol-1-yl)-5-ethylpyrimidine | ++++ |
| example 62 | 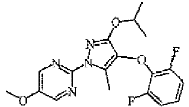 | 2-(4-(2,6-difluorophenoxy)-3-isopropoxy-5-methyl-1H-pyrazol-1-yl)-5-methoxypyrimidine | ++++ |
| example 63 | 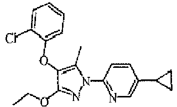 | 2-(4-(2-chlorophenoxy)-3-ethoxy-5-methyl-1H-pyrazol-1-yl)-5-cyclopropylpyridine | ++++ |
| example 64 | 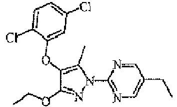 | 2-(4-(2,5-dichlorophenoxy)-3-ethoxy-5-methyl-1H-pyrazol-1-yl)-5-ethylpyrimidine | ++++ |
| example 65 | 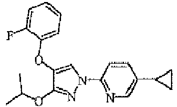 | 5-cyclopropyl-2-(4-(2-fluorophenoxy)-3-isopropoxy-1H-pyrazol-1-yl)pyridine | ++++ |
| example 66 | 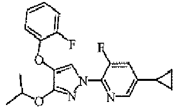 | 5-cyclopropyl-3-fluoro-2-(4-(2-fluorophenoxy)-3-isopropoxy-1H-pyrazol-1-yl)pyridine | ++++ |
| example 67 | 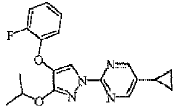 | 5-cyclopropyl-2-(4-(2-fluorophenoxy)-3-isopropoxy-1H-pyrazol-1-yl)pyrimidine | ++++ |
| example 68 | 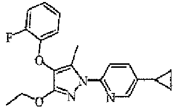 | 5-cyclopropyl-2-(3-ethoxy-4-(2-fluorophenoxy)-5-methyl-1H-pyrazol-1-yl)pyridine | ++++ |
| example 69 | 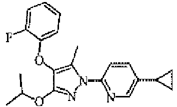 | 5-cyclopropyl-2-(4-(2-fluorophenoxy)-3-isopropoxy-5-methyl-1H-pyrazol-1-yl)pyridine | ++++ |
| example 70 | 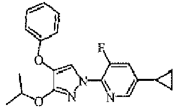 | 5-cyclopropyl-3-fluoro-2-(3-isopropoxy-4-phenoxy-1H-pyrazol-3-yl)pyridine | ++++ |
| example 71 | 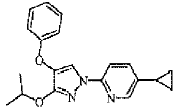 | 5-cyclopropyl-2-(3-isopropoxy-4-phenoxy-1H-pyrazol-1-yl)pyridine | +++ |
| example 72 | 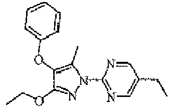 | 2-(3-ethoxy-5-methyl-4-phenoxy-1H-pyrazol-1-yl)-5-ethylpyrimidine | ++ |
| example 73 | 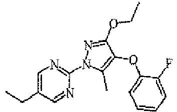 | 2-(3-ethoxy-4-(2-fluorophenoxy)-5-methyl-1H-pyrazol-1-yl)-5-ethylpyrimidine | +++ |
| example 74 | 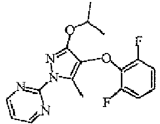 | 2-(4-(2,6-difluorophenoxy)-3-isopropoxy-5-methyl-1H-pyrazol-1-yl)pyrimidine | ++ |
| example 75 | 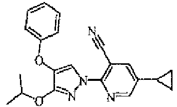 | 5-cyclopropyl-2-(3-isopropoxy-4-phenoxy-1H-pyrazol-1-yl)nicotinonitrile | + |
| example 76 | 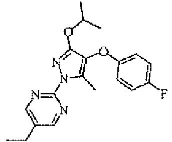 | 5-ethyl-2-(4-(4-fluorophenoxy)-3-isopropoxy-5-methyl-1H-pyrazol-1-yl)pyrimidine | +++ |
| example 77 | 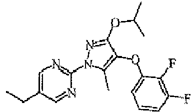 | 2-(4-(2,3-difluorophenoxy)-3-isopropoxy-5-methyl-1H-pyrazol-1-yl)-5-ethylpyrimidine | +++ |
| example 78 | 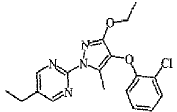 | 2-(4-(2-chlorophenoxy)-3-ethoxy-5-methyl-1H-pyrazol-1-yl)-5-ethylpyrimidine | +++ |
| example 79 | 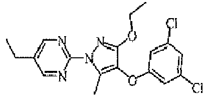 | 2-(4-(3,5-dichlorophenoxy)-3-ethoxy-5-methyl-1H-pyrazol-1-yl)-5-ethylpyrimitline | ++ |
| example 80 | 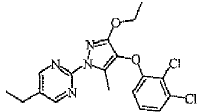 | 2-(4-(2,3-dichlorophenoxy)-3-ethoxy-5-methyl-1H-pyrazol-1-yl)-5-ethylpyrimidine | +++ |
| example 81 | 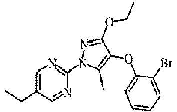 | 2-(4-(2-bromophenoxy)-3-ethoxy-5-methyl-1H-pyrazol-1-yl)-5-ethylpyrimidine | +++ |
| example 82 | 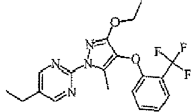 | 2-(3-ethoxy-3-methyl-4-(2-(triiluoromethyl)phenoxy)-1H-pyrazol-1-yl)-5-ethylpyrimidine | ++ |
| example 83 | 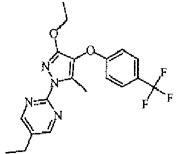 | 2-(3-ethoxy-5-methyl-4-(4-(trifluoromethyl)phenoxy)-1H-pyrazol-1-yl)-5-ethylpyrimidine | + |
| example 84 | 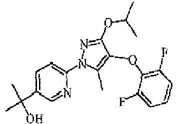 | 2-(6-(4-(2,6-difluorophonoxy)-3-isopropoxy-5-methyl-1H-pyrazol-1-yl)pyridin-3-yl)propan-2-ol | +++ |
| example 85 | 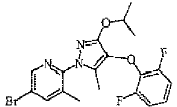 | 5-bromo-2-(4-(2,6-difluorophenoxy)-3-isopropoxy-5-methyl-1H-pyrazol-1-yl)-3-methylpyridine | ++ |
| example 86 | 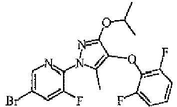 | 5-bromo-2-(4-(2,6-difluorophenoxy)-3-isopropoxy-5-methyl-1H-pyrazol-1-yl)-3-fluoropyridine | +++ |
| example 87 | 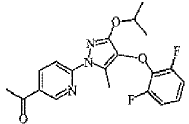 | 1-(6-(4-(2,6-difluorophenoxy)-3-isopropoxy-5-methyl-1H-pyrazol-1-yl)pyridin-3-yl)ehanone | +++ |
| example 88 | 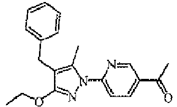 | 1-(6-(4-benzyl-3-ethoxy-5-methyl-1H-pyrazol-1-yl)pyridin-3-yl)ethanone | + |
| example 89 | 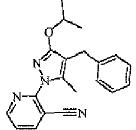 | 2-(4-benzyl-3-isopropoxy-5-methyl-1H-pyrazol-1-yl)nicotinonitrile | ++ |
| example 90 | 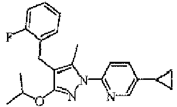 | 5-cyclopropyl-2-(4-(2-fluorobenzyl)-3-isopropoxy-5-methyl-1H-pyrazol-1-yl)pyridine | +++ |
| example 91 | 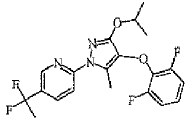 | 5-(1,1-difluoroethyl)-2-(4-(2,6-difluorophenoxy)-3-isopropoxy-5-methyl-1H-pyrazol-1-yl)pyridine | +++ |
| example 92 | 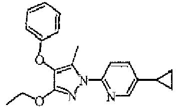 | 5-cyclopropyl-2-(3-ethoxy-5-methyl-4-phenoxy-1H-pyrazol-1-yl)pyridine | +++ |
| example 93 | 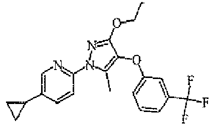 | 5-cyclopropyl-2-(3-ethoxy-5-methyl-4-(3-(trifluoromethyl)phenoxy)-1H-pyrazol-1-yl)pyridine | +++ |
| example 94 | 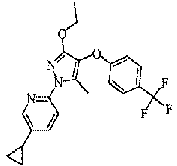 | 5-cyclopropyl-2-(3-ethoxy-5-methyl-4-(4-(trifluoromethyl)phenoxy)-1H-pyrazol-1-yl)pyridine | ++ |
| example 95 | 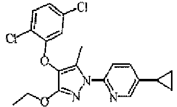 | 5-cyclopropyl-2-(4-(2,5-dichlorophenoxy)-3-ethoxy-5-methyl-1H-pyrazol-1-yl)pyridine | +++ |
| example 96 | 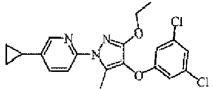 | 5-cyclopropyl-2-(4-(3,5-dichlorophenoxy)-3-methoxy-5-methyl-1H-pyrazol-1-yl)pyridine | +++ |
| example 97 | 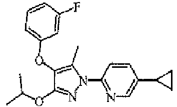 | 5-cyclopropyl-2-(4-(3-fluorophenoxy)-3-isopropoxy-5-methyl-1H-pyrazol-1-yl)pyridine | +++ |
| example 98 | 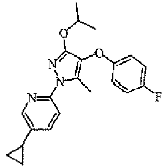 | 5-cyclopropyl-2-(4-(4-fluorophenoxy)-3-isopropoxy-5-methyl-1H-pyrazol-1-yl)pyridine | ++ |
| example 99 | 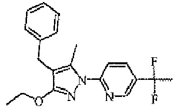 | 2-(4-benzyl-3-ethoxy-5-methyl-1H-pyrazol-1-yl)-5-(1,1-difluoroethyl)pyridine | ++ |
| example 100 | 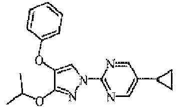 | 5-cyclopropyl-2-(3-isopropoxy-4-phenoxy-1H-pyrazol-1-yl)pyrimidine | +++ |
| example 101 | 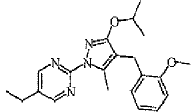 | 5-ethyl-2-(3-isopropoxy-4-(2-methoxybenzyl)-5-methyl-1H-pyrazol-1-yl)pyrimidine | ++++ |
| example 102 | 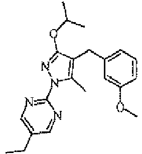 | 5-ethyl-2-(3-isopropoxy-4-(3-methoxybenzyl)-5-methyl-1H-pyrazol-1-yl)pyrimidine | ++++ |
| example 103 | 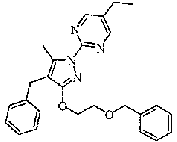 | 2-(4-benzyl-3-(2-(benzyloxy)ethoxy)-5-methyl-1H-pyrazol-1-yl)-5-ethylpyrimidine | ++++ |
| example 104 | 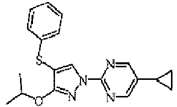 | 5-cyclopropyl-2-(3-isopropoxy-4-(phenylthio)-1H-pyrazol-1-yl)pyrimidine | ++++ |
| example 105 | 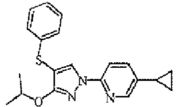 | 5-cyclopropyl-2-(3-isopropoxy-4-(phenylthio)-1H-pyrazol-1-yl)pyridine | +++ |
| example 106 | 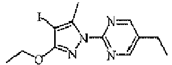 | 2-(3-ethoxy-4-iodo-5-methyl-1H-pyrazol-1-yl)-5-methylpyrimidine | + |
| example 107 | 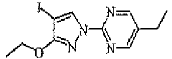 | 2-(3-ethoxy-4-iodo-1H-pyrazol-1-yl)-5-ethylpyrimidine | + |
| example 108 | 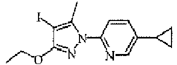 | 5-cyclopropyl-2-(3-ethoxy-4-iodo-5-methyl-1H-pyrazol-1-yl)pyridine | + |
| example 109 | 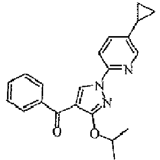 | (1-(5-cyclopropylpyridin-2-yl)-3-isopropoxy-1H-pyrazol-4-yl)(phenyl)methanone | ++ |
| example 110 | 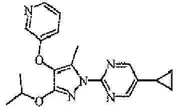 | 5-cyclopropyl-2-(3-isopropoxy-5-methyl-4-(pyridin-3-yloxy)-1H-pyrazol-1-yl)pyrimidine | ++ |
| example 111 | 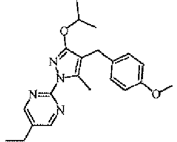 | 5-ethyl-2-(3-isopropoxy-4-(4-methoxybenzyl)-5-methyl-1H-pyrazol-1-yl)pyrimidine | +++ |
| example 112 | 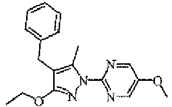 | 2-(4-benzyl-3-ethoxy-5-methyl-1H-pyrazol-1-yl)-5-methoxypyrimidine | ++ |
| example 113 | 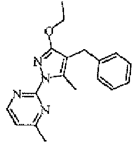 | 2-(4-benzyl-3-ethoxy-5-methyl-1H-pyrazol-1-yl)-4-methylpyrimidine | ++ |
| example 114 | 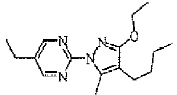 | 2-(4-butyl-3-ethoxy-5-methyl-1H-pyrazol-1-yl)-5-ethylpyrimidine | ++ |
| example 115 | 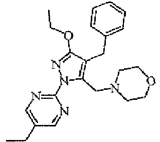 | 4-((4-benzyl-3-ethoxy-1-(5-ethylpyrimidin-2-yl)-1H-pyrazol-5-yl)methyl)morpholine | + |
| example 116 | 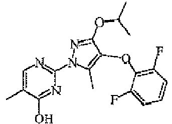 | 2-(4-(2,6-difluorophenoxy)-3-isopropoxy-5-methyl-1H-pyrazol-1-yl)-5-methylpyrimidin-4-ol | +++ |
| example 117 | 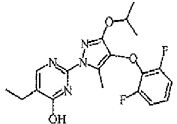 | 2-(4-(2,6-difluorophenoxy)-3-isopropoxy-5-methyl-1H-pyrazol-1-yl)-5-ethylpyrimidin-4-ol | ++++ |
| example 117A | 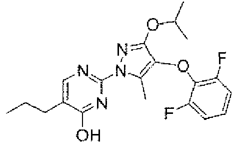 | 2-(4-(2,6-difluorophenoxy)-3-isopropoxy-5-methyl-1H-pyrazol-1-yl)-5-propylpyrimidin-4-ol | +++ |
| example 118 | 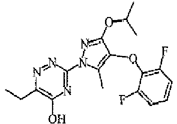 | 3-(4-(2,6-difluorophenoxy)-3-isopropoxy-5-methyl-1H-pyrazol-1-yl)-6-ethyl-1,2,4-triazin-5-ol | ++++ |
| example 119 | 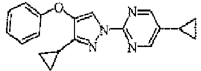 | 5-cyclopropyl-2-(3-cyclopropyl-4-phenoxy-1H-pyrazol-1-yl)pyrimidine | ++++ |
| example 120 | 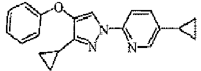 | 5-cyclopropyl-2-(3-cyclopropyl-4-phenoxy-1H-pyrazol-1-yl)pyridine | +++ |
| example 121 | 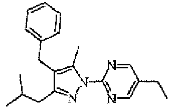 | 2-(4-benzyl-3-isobutyl-5-methyl-1H-pyrazol-1-yl)-5-ethylpyrimidine | ++++ |
| example 122 | 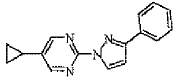 | 5-cyclopropyl-2-(3-phenyl-1H-pyrazol-1-yl)pyrimidine | ++ |
| example 123 | 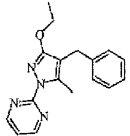 | 2-(4-benzyl-3-ethoxy-5-methyl-1H-pyrazol-1-yl)pyrimidine | ++ |
| example 124 | 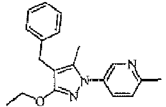 | 5-(4-benzyl-3-ethoxy-5-methyl-1H-pyrazol-1-yl)-2-methylpyridine | ++ |
| example 125 | 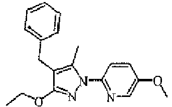 | 2-(4-benzyl-3-ethoxy-5-methyl-1H-pyrazol-1-yl)-5-methoxypyridine | ++ |
| example 126 | 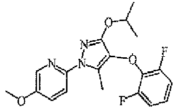 | 2-(4-(2,6-difluorophenoxy)-3-isopropoxy-5-methyl-1H-pyrazol-1-yl)-5-methoxypyridine | +++ |
| example 127 | 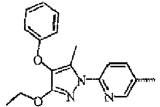 | 2-(3-ethoxy-5-methyl-4-phenoxy-1H-pyrazol-1-yl)-5-methylpyridine | ++ |
| example 128 | 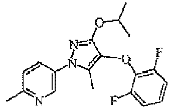 | 5-(4-(2,6-difluorophenoxy)-3-isopropoxy-5-methyl-1H-pyrazol-1-yl)-2-methylpyridine | ++++ |
| example 129 | 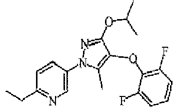 | 5-(4-(2,6-difluorophenoxy)-3-isopropoxy-5-methyl-1H-pyrazol-1-yl)-2-ethylpyridine | ++++ |
| example 130 | 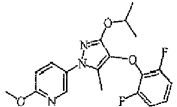 | 5-(4-(2,6-difluorophenoxy)-3-isopropoxy-5-methyl-1H-pyrazol-1-yl)-2-methoxypyridine | ++ |
| example 131 | 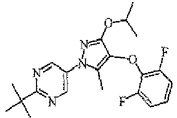 | 2-tert-butyl-5-(4-(2,6-difluorophenoxy)-3-isopropoxy-5-methyl-1H-pyrazol-1-yl)pyrimidine | +++ |
| example 132 | 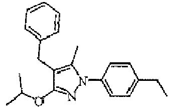 | 4-benzyl-1-(4-ethylphenyl)-3-isopropoxy-5-methyl-1H-pyrazole | ++ |
| example 133 | 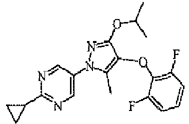 | 2-cyclopropyl-5-(4-(2,6-difluorophenoxy)-3-isopropoxy-5-methyl-1H-pyrazol-1-yl)pyrimidine | +++ |
| example 134 | 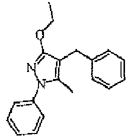 | 4-benzyl-3-ethoxy-5-methyl-1-phenyl-1H-pyrazole | ++ |
| example 135 | 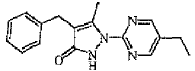 | 4-benzyl-1-(5-ethylpyrimidin-2-yl)-5-methyl-1H-pyrazol-3(2H)-one | ++ |
| example 136 | 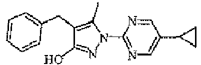 | 4-benzyl-1-(5-cyclopropylpyrimidin-2-yl)-5-methyl-1H-pyrazol-3-ol | ++ |
| example 137 | 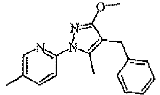 | 2-(4-benzyl-3-methoxy-5-methyl-1H-pyrazol-1-yl)-5-methylpyridine | ++ |
| example 138 | 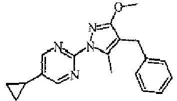 | 2-(4-benzyl-3-methoxy-5-methyl-1H-pyrazol-1-yl)-5-cyclopropylpyrimidine | +++ |
| example 139 | 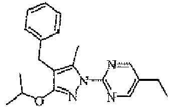 | 2-(4-benzyl-3-isopropoxy-5-methyl-1H-pyrazol-1-yl)-5-ethylpyrimidine | ++++ |
| example 140 | 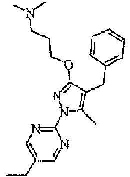 | 3-(4-benzyl-1-(5-ethylpyrimidin-2-yl)-5-methyl-1H-pyrazol-3-yloxy)-N,N-dimethylpropan-1-amine | ++ |
| example 141 | 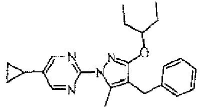 | 2-(4-benzyl-5-methyl-3-(pentan-3-yloxy)-1H-pyrazol-1-yl)-5-cyclopropylpyrimidine | ++++ |
| example 142 | 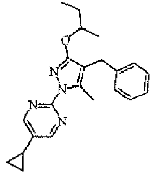 | 2-(4-benzyl-3-sec-butoxy-5-methyl-1H-pyrazol-1-yl)-5-cyclopropylpyrimidine | ++++ |
| example 143 | 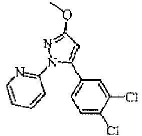 | 2-(5-(3,4-dichlorophenyl)-3-methoxy-1H-pyrazol-1-yl)pyridine | + |
| example 144 | 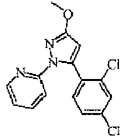 | 2-(5-(2,4-dichlorophenyl)-3-methoxy-1H-pyrazol-1-yl)pyridine | + |
| example 145 | 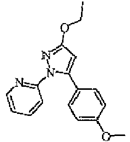 | 2-(3-ethoxy-5-(4-methoxyphenyl)-1H-pyrazol-1-yl)pyridine | + |
| example 146 | 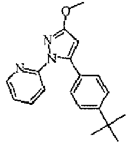 | 2-(5-(4-tert-butylphenyl)-3-methoxy-1H-pyrazol-1-yl)pyridine | + |
| example 147 | 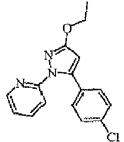 | 2-(5-(4-chlorophenyl)-3-ethoxy-1H-pyrazol-1-yl)pyridine | + |
| example 148 | 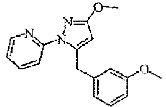 | 2-(3-methoxy-5-(3-methoxybenzyl)-1H-pyrazol-1-yl)pyridine | + |
| example 149 | 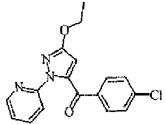 | (4-chlorophenyl)(3-ethoxy-1-(pyridin-2-yl)-1H-pyrazol-5-yl)methanone | + |
| example 150 | 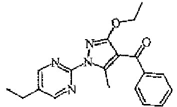 | (3-ethoxy-1-(5-ethylpyrimidin-2-yl)-5-methyl-1H-pyrazol-4-yl)(phenyl)methanone | ++ |
| example 151 | 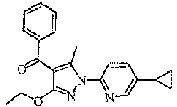 | (1-(5-cyclopropylpyridin-2-yl)-3-ethoxy-5-methyl-1H-pyrazol-4-yl)(phenyl)methanone | +++ |
| example 152 | 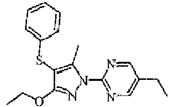 | 2-(3-ethoxy-5-methyl-4-(phenylthio)-1H-pyrazol-1-yl)-5-ethylpyrimidine | +++ |
| example 153 | 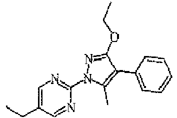 | 2-(3-ethoxy-5-methyl-4-phenyl-1H-pyrazol-1-yl)-5-ethylpyrimidine | ++ |
| example 154 | 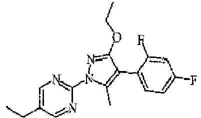 | 2-(4-(2,4-difluorophenyl)-3-ethoxy-5-methyl-1H-pyrazol-1-yl)-5-ethylpyrimidine | + |
| example 155 | 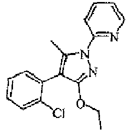 | 2-(4-(2-chlorophenyl)-3-ethoxy-5-methyl-1H-pyrazol-1-yl)pyridine | + |
| example 156 | 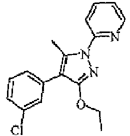 | 2-(4-(3-chlorophenyl)-3-ethoxy-5-methyl-1H-pyrazol-1-yl)pyridine | + |
| example 157 | 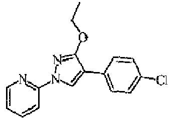 | 2-(4-(4-chlorophenyl)-3-ethoxy-1H-pyrazol-1-yl)pyridine | + |
| example 158 | 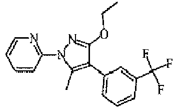 | 2-(3-ethoxy-5-methyl-4-(3-(trifluoromethyl)phenyl)-1H-pyrazol-1-yl)pyridine | + |
| example 159 | 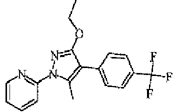 | 2-(3-ethoxy-5-methyl-4-(4-(trifluoromethyl)phenyl)-1H-pyrazol-1-yl)pyridine | + |
| example 160 | 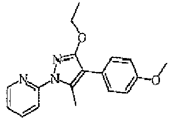 | 2-(3-ethoxy-4-(4-methoxyphenyl)-5-methyl-1H-pyrazol-1-yl)pyridine | + |
| example 161 | 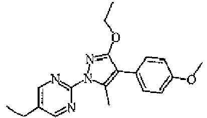 | 2-(3-ethoxy-4-(4-methoxyphenyl)-5-methyl-1H-pyrazol-1-yl)-5-ethylpyrimidine | ++ |
| example 161A | 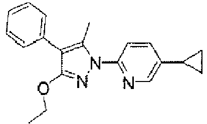 | 5-cyclopropyl-2-(3-ethoxy-5-methyl-4-phenyl-1H-pyrazol-1-yl)pyridine | ++ |
| example 162 | 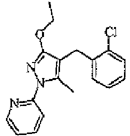 | 2-(4-(2-chlorobenzyl)-3-ethoxy-3-methyl-1H-pyrazol-1-yl)pyridine | + |
| example 163 | 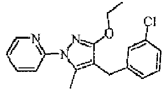 | 2-(4-(3-chlorobenzyl)-3-ethoxy-5-methyl-1H-pyrazol-1-yl)pyridine | + |
| example 164 | 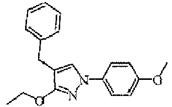 | 4-benzyl-3-ethoxy-1-(4-methoxyphenyl)-1H-pyrazole | + |
| example 165 | 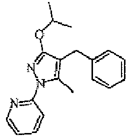 | 2-(4-benzyl-3-isopropoxy-5-methyl-1H-pyrazol-1-yl)pyridine | + |
| example 166 | 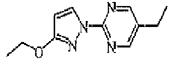 | 2-(3-ethoxy-1H-pyrazol-1-yl)-5-ethylpyrimidine | ++ |
| example 167 | 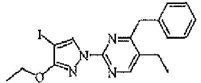 | 4-benzyl-2-(3-ethoxy-4-iodo-1H-pyrazol-1-yl)-5-ethylpyrimidine | ++ |
| example 168 | 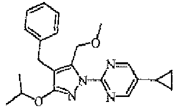 | 2-(4-benzyl-3-isopropoxy-5-(methoxymethyl)-1H-pyrazol-1-yl)-5-cyclopropylpyrimidine | +++ |
| example 169 | 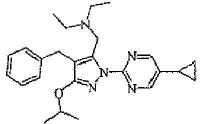 | N-((4-benzyl-1-(5-cyclopropylpyrimidin-2-yl)-3-isopropoxy-1H-pyrazol-5-yl)methyl)-N-ethylethanamine | + |
| example 170 | 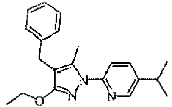 | 2-(4-benzyl-3-ethoxy-5-methyl-1H-pyrazol-1-yl)-5-isopropylpyridine | ++ |
| example 171 | 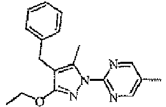 | 2-(4-benzyl-3-ethoxy-5-methyl-1H-pyrazol-1-yl)-5-methylpyrimidine | ++++ |
| example 172 | 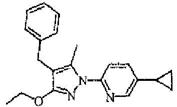 | 2-(4-benzyl-3-ethoxy-5-methyl-1H-pyrazol-1-yl)-5-cyclopropylpyridine | +++ |
| example 173 | 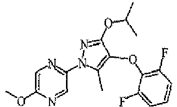 | 2-(4-(2,6-difluorophenoxy) -3 -isopropoxy- 5 -methyl-1H-pyrazol-1-yl)-5-methoxypyrazine | ++ |
| example 174 | 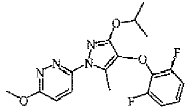 | 3-(4-(2,6-di-fluorophenoxy)-3-isopropoxy-5-methyl-1H-pyrazol-1-yl)-6-methoxypyridazine | +++ |
| example 174A | 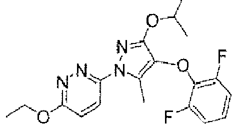 | 3-(4-(2,6-difluorophenoxy)-3-isopropoxy-5-methyl-1H-pyrazol-1-yl)-6-ethoxypyridazine | +++ |
| example 175 | 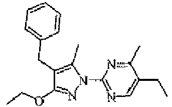 | 2-(4-benzyl-3-ethoxy-5-methyl-1H-pyrazol-1-yl)-5-ethyl-4-methylpyrimidine | ++++ |
| example 176 | 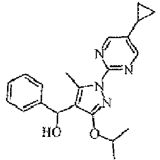 | (1-(5-cyclopropylpyrimidin-2-yl)-3-isopropoxy-5-methyl-1H-pyrazol-4-yl)(phenyl)methanol | ++++ |
| example 177 | 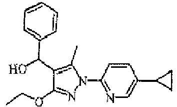 | (1-(5-cyclopropylpyridin-2-yl)-3-ethoxy-5-methyl-1H-pyrazol-4-yl)(phenyl)methanol | ++++ |
| example 178 | 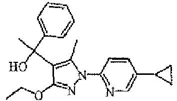 | 1-(1-(5-cyclopropylpyridin-2-yl)-3-ethoxy-5-methyl-1H-pyrazol-4-yl)-1-phenylethanol | ++ |
| example 179 | 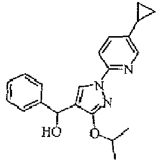 | (1-(5-cyclopropylpyridin-2-yl)-3-isopropoxy-1H-pyrazol-4-yl)(phenyl)methanol | +++ |
| example 180 | 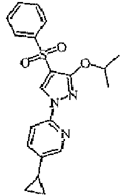 | 5-cyclopropyl-2-(3-isopropoxy-4-(phenylsulfonyl)-1H-pyrazol-1-yl)pyridine | +++ |
| example 181 | 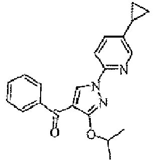 | 5-cyclopropyl-2-(3-isopropoxy-4-(phenylsulfinyl)-1H-pyrazol-1-yl)pyridine | ++ |
| example 182 | 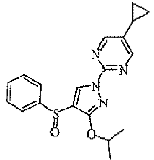 | 5-cyclopropyl-2-(3-isopropoxy-4-(phenylsulfinyl)-1H-pyrazol-1-yl)pyrimidine | ++ |
| example 183 | 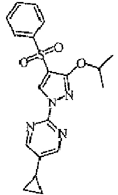 | 5-cyclopropyl-2-(3-isopropoxy-4-(phenylsulfonyl)-1H-pyrazol-1-yl)pyrimidine | ++++ |
| example 184 | 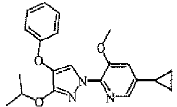 | 5-cyclopropyl-2-(3-isopropoxy-4-phenoxy-1H-pyrazol-1-yl)-3-methoxypyridine | ++++ |
| example 185 | 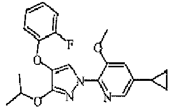 | 5-cyclopropyl-2-(4-(2-fluorophenoxy)-3-isopropoxy-1H-pyrazol-1-yl)-3-methoxypyridine | +++ |
| example 186 | 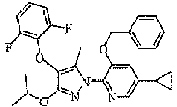 | 3-(benzyloxy)-5-cyclopropyl-2-(4-(2,6-difluorophenoxy)-3-isopropoxy-5-methyl-1H-pyrazol-1-yl)pyridine | +++ |
| example 187 | 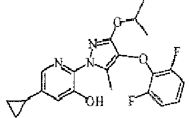 | 5-cyclopropyl-2-(4-(2,6-difluorophenoxy)-3-isopropoxy-5-methyl-1H-pyrazol-1-yl)pyridin-3-ol | ++++ |
| example 188 | 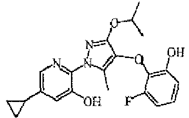 | 5-cyclopropyl-2-(4-(2-fluoro-6-hydroxyphenoxy)-3-isopropoxy-5-methyl-1H-pyrazol-1-yl)pyridin-3-ol | ++++ |
| example 189 | 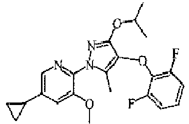 | 5-cyclopropyl-2-(4-(2,6-difluorophenoxy)-3-isopropoxy-5-methyl-1H-pyrazol-1-yl)-3-methoxypyridine | ++++ |
| example 190 | 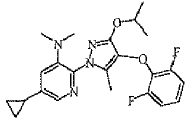 | 5-cyclopropyl-2-(4-(2,6-difluorophenoxy)-3-isopropoxy-5-methyl-1H-pyrazol-1-yl)-N,N-dimethylpyridin-3 -amine | ++++ |
| example 190A | 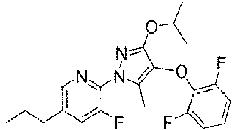 | 2-(4-(2,6-difluorophenoxy)-3-isopropoxy-5-methyl-1H-pyrazol-1-yl)-3-fluoro-5-propylpyridine | +++ |
| example 191 | 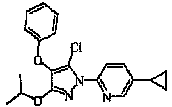 | 2-(5-chloro-3-isopropoxy-4-phenoxy-1H-pyrazol-1-yl)-5-cyclopropylpyridine | +++ |
| example 192 | 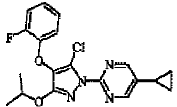 | 2-(5-chloro-4-(2-fluorophenoxy)-3-isopropoxy-1H-pyrazol-1-yl)-5-cyclopropylpyrimidine | ++++ |
| example 193 | 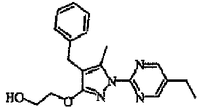 | 2-(4-benzyl-1-(5-ethylpyrimidin-2-yl)-5-methyl-1H-pyrazol-3-yloxy)ethanol | +++ |

## Claims

1. A compound of formula (I) : 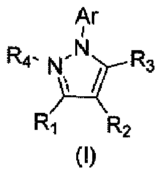 wherein:
Ar is independently selected from C₆-C₁₀ aryl or 5 to 7 membered heteroaryl, wherein said aryl or heteroaryl groups are optionally substituted by one to three R₅;
R₁ is independently selected from C₁-C₆ alkyl, C₃-C₆ cycloalkyl, C₆-C₁₀ aryl, OH, C₁-C₆ alkoxy, =0, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from H, F, Cl, Br, I, C₁-C₆ alkyl, C₆-C₁₀ aryl, C₆-C₁₀ aryloxy, (C₆-C₁₀)aryl(C₁-C₆)alkyl, 5 to 7 membered heteroaryloxy, CN, CO₂R₉, C₆-C₁₀ aroyl, C₆-C₁₀ arylthio, C₆-C₁₀ arylsulfinyl, C₆-C₁₀ arylsulfonyl, wherein said aryl groups are optionally substituted by one to three R₁₀ and wherein said alkyl groups are optionally substituted by OH;
R₃ is independently selected from H, Cl, C₁-C₆ alkyl, C₁-C₆ alkoxy, (C₁-C₆)alkoxy(C₁-C₆)alkyl, C₆-C₁₀ aryl, C₆-C₁₀ aroyl, (C₆-C₁₀)aryl(C₁-C₆)alkyl, 5 to 7 membered heterocyclyl, (5 to 7 membered heterocyclyl)-CH₂-, (CH₂)ₙCO₂R₁₁, CH₂NR₇R₈, wherein said aryl or heterocyclyl groups are optionally substituted by one to three R₁₂;
or
R₂ and R₃ together form a 5 to 7 membered heterocyclyl, optionally substituted by (C₆-C₁₀)aryl(C₁-C₆)alkyl;
R₄ is, at each occurrence, independently selected from H, or 5 to 7 membered heteroaryl optionally substituted by R₁₃;
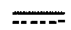 is, at each occurrence, independently selected from a double bond C=N or a single bond C-N,
provided that when 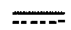 is a double bond, R₄ is absent and R₁ cannot be =O;
R₅ is at each occurrence, independently selected from C₁-C₆ alkyl, C₁-C₆ alkenyl, C₃-C₆ cycloalkyl, C₁-C₆ alkoxy, (C₆-C₁₀)aryl(C₁-C₆)alkyl, (C₆-C₁₀)aryl(C₁-C₆)alkoxy, OH, F, Cl, Br, I, NR₇R₈, CF₃, CF₂CH₃, CN, C(=O)R₆, CO₂R₆, wherein said alkyl groups are optionally substituted by one or more OH, F, Cl, or Br;
R₆, R₇, R₈, are, at each occurrence, independently selected from each independently selected from C₁-C₆ alkyl;
R₉ is, at each occurrence, independently selected from H or C₁-C₆ alkyl;
R₁₀ is at each occurrence, independently selected from C₁-C₆ alkoxy, OH, F, Cl, Br, CF₃;
R₁₁ is, at each occurrence, independently selected from H, C₁-C₆ alkyl;
R₁₂ is, at each occurrence, independently selected from F, Cl, Br, CN, C₁-C₆ alkyl or C₁-C₆ alkoxy;
R₁₃ is, at each occurrence, independently selected from F, Cl, Br, I;
n is, at each occurrence, independently selected from 0 or 1;
and pharmaceutically acceptable salts thereof,
for use in the treatment and/or prevention of auto-immune or auto-immune related diseases, cancer, viral infections, and central nervous system diseases and disorders, by inhibiting human dehydroorate dehydrogenase (DHODH).

2. The compound for use according to claim 1, in the treatment of rheumatoid arthritis, multiple sclerosis, melanoma, spinal cord injury, psoriasis, inflammatory bowel disease or prevention of graft rejection.

3. The compound for use of any of claims 1 or 2, wherein 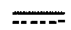 is a double bond C=N.

4. The compound for use of any of claims 1 to 3, wherein at least one of R₂ or R₃ is different from H.

5. The compound for use of any of claims 1 to 4, wherein Ar is phenyl, pyridyl, pyrazine, pyrimidinyl, pyridazine, triazinyl, preferably 2-pyridyl, 3-pyridyl, 2-pyrazinyl, 2-pyrimidinyl, 5-pyrimidinyl, 3-pyridazinyl or 3-(1,2,4-triazinyl).

6. The compound for use of any of claims 1 to 5, wherein Ar is substituted, preferably monosubstituted.

7. The compound for use of any of claims 1 to 6, wherein R₅ is C₁-C₆ alkyl, C₃-C₆ cycloalkyl, C₁-C₆ alkoxy F, Cl, Br, CF₂CH₃, or CF₃, preferably methyl, ethyl, cyclopropyl, methoxy, F, Cl, Br, CF₂CH₃, or CF₃, more preferably ethyl, cyclopropyl or methoxy.

8. The compound of any of claims 1 to 7, wherein at least one of R₅ is located at *para* position with regard to the attached pyrazole ring

9. The compound for use of any of claims 1 to 8, wherein R₁ is C₁-C₆ alkyl, C₃-C₆ cycloalkyl, or C₁-C₆ alkoxy, preferably C₁-C₆ alkoxy, preferably methoxy, ethoxy or isopropyloxy, more preferably ethoxy or isopropyloxy.

10. The compound for use of any of claims 1 to 9, wherein R₂ is C₆-C₁₀ aryloxy, or (C₆-C₁₀)aryl(C₁-C₆)alkyl, preferably benzyl, PhCH(OH)-, or phenoxy.

11. The compound for use of any of claims 1 to 10, wherein R₁₀ is F, Cl, OH, methoxy, or CF₃, preferably F.

12. The compound for use of any of claims 1 to 11, wherein R₃ is H, C₁-C₆ alkyl, C₆-C₁₀ aryl, (C₆-C₁₀)aryl(C₁-C₆)alkyl, or Cl, preferably methyl, benzyl or phenyl, more preferably H or methyl.

13. The compound for use of any of claims 1 to 12, wherein R₁₂ is Cl or C₁-C₆ alkoxy, preferably Cl or methoxy.

14. The compound for use according to any of claims 1 to 13, which is selected from:
2-((4-(2,6-difluorophenoxy)-3-isopropoxy-5-methyl-1H-pyrazol-1-yl)-5-ethylpyrimidine (example 8)
5-((4-(2,6-difluorophenoxy)-3-isopropoxy-5-methyl-1H-pyrazol-1-yl)-2-ethylpyridine (example 129)
5-cyclopropyl-2-(4-(2,6-difluorophenoxy)-3-isopropoxy-5-methyl-1H-pyrazol-1-yl)pyrimidine (example 13)
5-((4-(2,6-difluorophenoxy)-3-isopropoxy-5-methyl-1H-pyrazol-1-yl)-2-ethylpyridine (example 129)
(1-((5-cyclopropylpyrimidin-2-yl)-3-isopropoxy-5-methyl-1H-pyrazol-4-yl)(phenyl)methanol (example 176)
2-((4-benzyl-5-methyl-3-(pentan-3-yloxy)-1H-pyrazol-1-yl)-5-cyclopropylpyrimidine (example 141)
2-((4-benzyl-3-sec-butoxy-5-methyl-1H-pyrazol-1-yl)-5-cyclopropylpyrimidine (example 142) 3-cyclopropyl-6-(4-(2,6-difluorophenoxy)-3-isopropoxy-5-methyl-1H-pyrazol-1-yl)pyridazine (example 7)
2-cyclopropyl-5-(4-(2,6-difluorophenoxy)-3-isopropoxy-5-methyl-1H-pyrazol-1-yl)pyrazine (example 45)
5-cyclopropyl-2-(4-(2,6-difluorophenoxy)-3-isopropoxy-5-methyl-1H-pyrazol-1-yl)-3-fluoropyridine (example 5)
2-((4-(2,6-difluorophenoxy)-3-isopropoxy-5-methyl-1H-pyrazol-1-yl)-5-propylpyrimidine (example 6)
2-((4-benzyl-3-isopropoxy-5-methyl-1H-pyrazol-1-yl)-5-ethylpyrimidine (example 139) 5-cyclopropyl-2-(4-(2-fluoro-6-hydroxyphenoxy)-3-isopropoxy-5-methyl-1H-pyrazol-1-yl)pyridin-3-ol (example 188)

15. A compound of formula (Ia) : 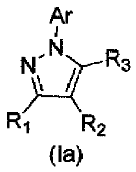 Wherein:
R₁ is independently selected from OH, C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl ;
R₂ is independently selected from C₆-C₁₀ aryloxy, 5 to 7 membered heteroaryloxy,
wherein said aryl groups are optionally substituted by one to three R₁₀ and wherein said alkyl groups are optionally substituted by OH;
R₃ is independently selected from C₁-C₆ alkyl, (C₁-C₆)alkoxy(C₁-C₆)alkyl, (C₆-C₁₀)aryl(C₁-C₆)alkyl, (5 to 7 membered heterocyclyl)-CH₂-, CH₂CO₂R₁₁, CH₂NR₇R₈, wherein said aryl or heterocyclyl groups are optionally substituted by one to three R₁₂,
Ar, R₇, R₈, R₁₀, R₁₁, R₁₂ being as defined in any of claims 1 to 14,
and pharmaceutically acceptable salts thereof.

16. A compound of formula (Ib): 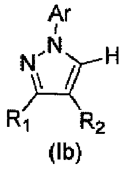 Wherein:
R₁ is independently selected from C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl ;
R₂ is independently selected from (C₆-C₁₀)aryl(C₁-C₆)alkyl, C₆-C₁₀ aryloxy, 5 to 7 membered heteroaryloxy, wherein said aryl groups are optionally substituted by one to three R₁₀ and wherein said alkyl groups are optionally substituted by OH ;
Ar, R₇, R₈, R₁₀, being as defined in any of claims 1 to 14,
and pharmaceutically acceptable salts thereof.

17. A compound of formula (Ic): 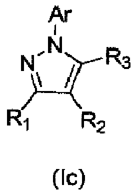 Wherein :
Ar is 2- or 3-pyridyl,
R₁ is independently selected from C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from (C₆-C₁₀)aryl(C₁-C₆)alkyl, wherein said aryl groups are optionally substituted by one to three R₁₀ and wherein said alkyl groups are optionally substituted by OH ;
R₃, R₇, R₈, being as defined in any of claims 1 to 14,
With the exclusion of the following compounds:
2-(4-benzyl-3-ethoxy-5-methyl-1H-pyrazol-1-yl)pyridine, 4-benzyl-5-methyl-1-(pyridin-2-yl)-1H-pyrazol-3(2H)-one, and/or 4-benzyl-5-anethyl-1-(pyridin-2-yl)-1H-pyrazol-3-ol,
and pharmaceutically acceptable salts thereof.

18. A compound of formula (Id): 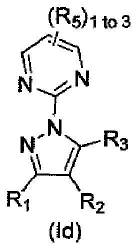 Wherein:
R₁ is independently selected from C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from C₁-C₆ alkyl, (C₆-C₁₀)aryl(C₁-C₆)alkyl, ,wherein said aryl groups are optionally substituted by one to three R₁₀ and wherein said alkyl groups are optionally substituted by OH;
R₃, R₅, R₇, R₈, R₁₀, being as defined in any of claims 1 to 14,
and pharmaceutically acceptable salts thereof.

19. A compound of formula (Ie): 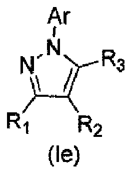 Wherein:
Ar is 2-pyrazinyl, 3-pyridazinyl, 5-pyrimidinyl, or 3-(1,2,4-triazinyl),
R₁ is independently selected from C₁-C₆ alkoxy, wherein said alkoxy is optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₂ is independently selected from C₁-C₆ alkyl, C₆-C₁₀ aryl, (C₆-C₁₀)aryl(C₁-C₆)alkyl, CN, CO₂R₉, C₆-C₁₀ aroyl, wherein said aryl groups are optionally substituted by one to three R₁₀ and wherein said alkyl groups are optionally substituted by OH;
R₅ is at each occurrence, independently selected from C₁-C₆ alkyl, C₁-C₆ alkenyl, C₃-C₆ cycloalkyl, C₁-C₆ alkoxy, (C₆-C₁₀)aryl(C₁-C₆)alkyl, (C₆-C₁₀)aryl(C₁-C₆)alkoxy, OH, F, Cl, Br, I, CF₃, CF₂CH₃, CN, C(=O)R₆, CO₂R₆, wherein said alkyl groups are optionally substituted by one or more OH, F, Cl, or Br;
R₃, R₆, R₇, R₈, R₁₀, being as defined in any of claims 1 to 14,
and pharmaceutically acceptable salts thereof.

20. A pharmaceutical composition comprising a compound of formula (I), as defined in any of claims 1 to 14 in admixture with one or more pharmaceutically acceptable excipients or carriers.

21. The pharmaceutical composition of claim 20, wherein the compound of formula (I) is a compound of formula (Ia), (Ib), (Ic), (Id) or (Ie) as defined in any of claims 15 to 19.

22. A method of preparation of compounds of formula (I) according to any of claims 1 to 14 comprising the steps consisting of:
i) Reacting a compound of formula (II) with R₁₄NHNH₂ thereby obtaining a compound of formula (III) : 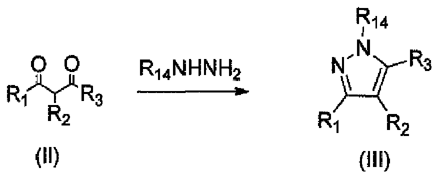 Wherein
R₁, R₂ are as defined in formula (I)
R₃ is C₁-C₆ alkoxy,
R₁₄ is H or Ar,
ii) If R₁₄ is H, then reacting the compound of formula (III) with Ar-X, thereby
obtaining a compound of formula (I) 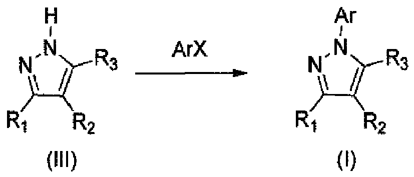 Wherein
Ar is as defined in any of claims 1 to 14 and X is a halogen atom, notably F, Cl, Br or I, iii) Recovering the obtained compound of formula (I).

23. A compound of formula (IV) : 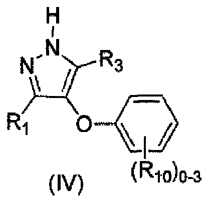 Wherein:
R₁ is C₁-C₆ alkoxy, optionally substituted by OH, C₆-C₁₀ aryl, (C₆-C₁₀)aryl(C₁-C₆)alkyl, (C₆-C₁₀)aryl(C₁-C₆)alkoxy, NR₇R₈, or a 5 to 7 membered heterocyclyl;
R₃, R₁₀ are as defined in any of claims 1 to 14.


SEQUENCE LISTING

<110>  Institut Pasteur
       CNRS
       Inserm

<120>  Pyrazole derivatives as dihydroorate dehydrogenase inhibitors

<130>  F226 CAS 198 EP

<160>  2

<170>  PatentIn version 3.5

<210>  1
<211>  38
<212>  DNA
<213>  Artificial Sequence

<220>
<223>  Synthetic Primer

<400>  1
gggaattcca tatggccacg ggagatgagc gtttctat                              38


<210>  2
<211>  36
<212>  DNA
<213>  Artificial sequence

<220>
<223>  Synthetic Primer

<400>  2
ccccaagctt tcacctccga tgatctgctc caatgg                                36